# EUROPEAN PATENT APPLICATION

(11) **EP 1 659 123 A1**
(43) Date of publication of application: **24.05.2006**
(21) Application number: 04772573.4
(22) Date of filing: 25.08.2004
(51) Int. Cl.: C07D 487/04, A61K 31/4985, A61P 3/10, A61P 43/00

(54) **BICYCLIC PYRAZOLE DERIVATIVE**

(30) Priority: 29.08.2003 JP 2003306948
(71) Applicant: Dainippon Sumitomo Pharma Co., Ltd., Osaka 541-8524 (JP)
(72) Inventor: NAKAHIRA, Hiroyuki c/o Dainippon Sumitomo Pharma, Osaka-shi, Osaka 5540022 (JP); HOCHIGAI, Hitoshi c/o Dainippon Sumitomo Pharma, Osaka-shi, Osaka 5540022 (JP); TAKEDA, Tatsuya c/o Dainippon Sumitomo Pharma Co,, Osaka-shi, Osaka 5540022 (JP); KOBAYASHI, Tomonori c/o Dainippon Sumitomo Pharma, Osaka-shi, Osaka 5540022 (JP); HUME, William Ewan c/o Dainippon Sumitomo Pharma, Osaka-shi, Osaka 5540022 (JP)
(74) Representative: Duckett, Anthony Joseph
(86) International application number: PCT/JP2004/012617
(87) International publication number: WO 2005/021550

(57) **Abstract**

A compound represented by the following formula (I), a prodrug thereof, or a pharmaceutically acceptable salt of either. These are compounds having high DPP-IV inhibitory activity and improved in safety, nontoxicity, etc. (I) [In the formula, R¹ represents hydrogen, optionally substituted alkyl, etc.; the solid line and dotted line between A¹ and A² indicate a double bond (A¹=A²), etc.; A¹ represents a group represented by the formula C(R²), etc.; A² represents a group represented by the formula C(R⁴), etc.; R² represents hydrogen, optionally substituted alkyl, etc.; R⁴ represents hydrogen, optionally substituted alkyl, etc.; R⁶ represents hydrogen, optionally substituted aryl, etc.; and -Y represents, e.g.; a group represented by the formula (A): (A) (wherein ml is 0, 1, 2, or 3; and R⁷ is absent, or one or two R⁷'s are present and each independently represents optionally substituted alkyl, etc.).]

## Description

### TECHNICAL FIELD

The present invention relates to a novel condensed imidazole useful as medicine. More particularly, it relates to a novel condensed imidazole effective as a dipeptidyl peptidase IV (DPP-IV) inhibitor. Furthermore, it relates to a pharmaceutical composition for the treatment of diabetes containing a novel condensed imidazole effective as a dipeptidyl peptidase IV (DPP-IV) inhibitor, as an active ingredient.

### BACKGROUND ART

DPP-IV is a serine protease widely present in the body, is one of dipeptidyl aminopeptidases capable of hydrolyzing and releasing a N-terminal peptide and markedly acts on, in particular, peptides containing proline as the second amino acid from the N-terminal. Therefore, DPP-IV is referred to also prolyl end peptidase. DPP-IV is known to accept, as substrates, various biological peptides concerned in the endocrine system, the neuroendocrine system, immunological functions and the like. It is known that many physiologically active peptides such as the pancreatic polypeptide family represented by pancreatic polypeptides (PP), neuropeptide Y (NPY) and the like; the glucagon/VIP family represented by vasoactive intestinal polypeptides (VIP), glucagon-like peptide-1 (GLP-1), glucose-dependent insulinotropic polypeptides (GIP), growth hormone release accelerating factor (GRF) and the like; and the chemocaine family are substrates for DPP-IV and feel the influences of DPP-IV, such as activation/inactivation, metabolism acceleration and the like (J. Langer and S. Ansorge, "Cellular Peptidases in Immune Functions and Disease 2", Advances in Experimental Medicine and Biology Vol. 477).

DPP-IV severs two amino acids (His-Ala) from the N-terminal of GLP-1. It is known that although the severed peptide binds weekly to a GLP-1 receptor, it has no activating effect on the receptor and acts as an antagonist (L.B. Knudsen et al., European Journal of Pharmacology, Vol. 318, p429-435, 1996). The metabolism of GLP-1 by DPP-1V in blood is known to be very rapid, and the concentration of active GLP-1 in blood is increased by the inhibition of DPP-IV (T.J. Kieffer et al., Endocrinology, Vol. 136, p3585-3596, 1995). GLP-1 is a peptide secreted from intestinal tract by the ingestion of sugars and is a main accelerating factor for the glucose-responsive secretion of insulin by pancreas. In addition, GLP-1 is known to have accelerating effect on insulin synthesis in pancreatic β cells and accelerating effect on β cell proliferation. Moreover, it is known that GLP-1 receptors appear also in digestive tracts, liver, muscle, adipose tissue and the like, and it is also known that in these tissues, GLP-1 affects working of digestive tracts, the secretion of acid in stomach, the synthesis and degradation of glycogen, insulin-dependent glucose uptake, and the like. Accordingly, there is expected the development of a DPP-IV inhibitor effective against type 2 diabetes (non-insulin-dependent diabetes) which brings about effects such as the acceleration of insulin secretion dependent on blood sugar level, the improvement of pancreas function, the improvement of a high postprandial blood sugar level, the improvement of glucose tolerance abnormality, the improvement of insulin resistance, and the like, by increasing the concentration of GLP-1 in blood (R.A. Pederson et al., Diabetes Vol. 47, p1253-1258, 1998).

Various DPP-IV inhibitors have been reported. For example, International Publication No. WO02/02560 pamphlet reports that xanthine derivatives having a piperazine ring or the like are effective as DPP-IV inhibitors. International Publication No. WO02/068420 pamphlet and International Publication No. WO03/004496 pamphlet report that xanthine derivatives having a piperidine ring or the like are effective as DPP-IV inhibitors. International Publication No. WO03/024965 pamphlet reports that xanthine derivatives containing a 2-aminocyclohexylamino group are effective as DPP-IV inhibitors. International Publication No. WO02/024698 pamphlet reports that xanthine derivatives are effective as phosphodiesterase V inhibitors.

### DISCLOSURE OF THE INVENTION

An object of the present invention is to provide a novel compound having an excellent DPP-IV inhibitory activity.

The present inventors earnestly investigated in order to achieve the above object, and consequently found that the following compound, a prodrug thereof or a pharmaceutically acceptable salt of the compound or prodrug (hereinafter abbreviated as the present inventive compound in some cases if necessary) has an excellent DPP-IV inhibitory effect, whereby the present invention has been accomplished.

That is, the present invention relates to the following:
[1] A compound represented by the formula (I): 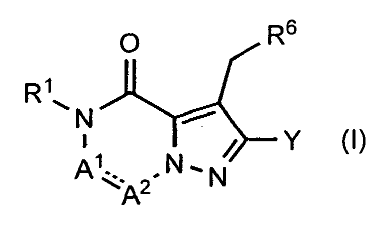 wherein R¹ is a hydrogen atom, an optionally substituted alkyl group, an optionally substituted cycloalkyl group, an optionally substituted aryl group, or an optionally substituted heteroaryl group;
   the solid line and dotted line between A¹ and A² indicate a double bond (A¹=A²) or a single bond (A¹⁻A²);
   A¹ is a nitrogen atom or a group represented by the formula C(R²) and A² is a group represented by the formula C(R⁴), in the case of the solid line and dotted line between A¹ and A² being a double bond (A¹=A²);
   A¹ is a group represented by the formula C(R²)(R³) and A² is a group represented by the formula C(R⁴)(R⁵), in the case of the solid line and dotted line between A¹ and A² being a single bond (A¹-A²);
   R¹ and R² may be taken together with the adjacent nitrogen atom and carbon atom to form an optionally substituted 4- to 7-membered ring in the case of the solid line and dotted line between A¹ and A² being a double bond (A¹=A²) and A¹ being a group represented by the formula C(R²);
   R² is a hydrogen atom, a halogen atom, a cyano group, a formyl group, an optionally substituted alkyl group, an optionally substituted cycloalkyl group, an optionally substituted cycloalkyloxy group, an optionally substituted alkenyl group, an optionally substituted alkynyl group, an optionally substituted amino group, an optionally substituted carbamoyl group, a carboxyl group, an optionally substituted alkoxy group, an optionally substituted alkoxycarbonyl group, an optionally substituted cycloalkyloxycarbonyl group, a tetrahydrofuranyloxycarbonyl group, an optionally substituted aryl group, an optionally substituted aryloxy group, an optionally substituted aryloxycarbonyl group, an optionally substituted aralkyl group, an optionally substituted aralkyloxy group, an optionally substituted aroyl group, an optionally substituted arylthio group, an optionally substituted arylsulfinyl group, an optionally substituted arylsulfonyl group, an optionally substituted alkylthio group, an optionally substituted alkylsulfinyl group, an optionally substituted alkylsulfonyl group, an optionally substituted heteroaryl group, an optionally substituted heteroarylalkyl group, an optionally substituted heteroarylcarbonyl group, an optionally substituted heteroaryloxy group, an optionally substituted alkylcarbonyl group, an optionally substituted cycloalkylcarbonyl group, or an optionally substituted nitrogen-containing saturated heterocyclic group;
   R³ is a hydrogen atom, a halogen atom, a cyano group, an optionally substituted alkyl group, an optionally substituted cycloalkyl group, an optionally substituted carbamoyl group, a carboxyl group, an optionally substituted alkoxy group, an optionally substituted alkoxycarbonyl group, an optionally substituted aryl group, an optionally substituted aryloxycarbonyl group, an optionally substituted aralkyl group, an optionally substituted aroyl group, an optionally substituted aryloxy group, an optionally substituted arylthio group, an optionally substituted arylsulfonyl group, an optionally substituted heteroaryl group, an optionally substituted heteroarylalkyl group, an optionally substituted heteroarylcarbonyl group, an optionally substituted heteroaryloxy group, or an optionally substituted alkylcarbonyl group;
   R⁴ and R⁵ are independently a hydrogen atom, a halogen atom, a cyano group, an optionally substituted alkyl group, an optionally substituted cycloalkyl group, an optionally substituted carbamoyl group, a carboxyl group, an optionally substituted alkoxy group, an optionally substituted cycloalkyloxy group, an optionally substituted alkoxycarbonyl group, an optionally substituted amino group, an optionally substituted aryl group, an optionally substituted aryloxycarbonyl group, an optionally substituted aralkyl group, an optionally substituted aroyl group, an optionally substituted aryloxy group, an optionally substituted arylthio group, an optionally substituted arylsulfonyl group, an optionally substituted heteroaryl group, an optionally substituted heteroarylalkyl group, an optionally substituted heteroarylcarbonyl group, or an optionally substituted alkylcarbonyl group;
   R⁶ is a hydrogen atom, an optionally substituted alkyl group, an optionally substituted cycloalkyl group, an optionally substituted aryl group, an optionally substituted vinyl group, an optionally substituted ethynyl group, an optionally substituted nitrogen-containing saturated heterocyclic group, or an optionally substituted heteroaryl group; and
   -Y is any of groups represented by the formula (A), formula (B), formula (C) and formula (D) shown below: 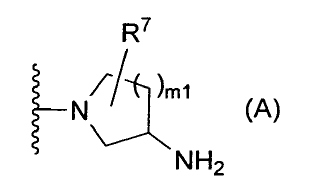 wherein m1 is 0, 1, 2 or 3, and R⁷ is absent or one or two R⁷s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁷s, when taken together, represent methylene or ethylene and may bind to one or more carbon atoms constituting the ring, to form a new ring; 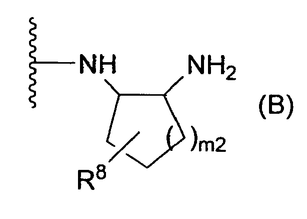 wherein m2 is 0, 1, 2 or 3, and R⁸ is absent or one or two R⁸s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁸s, when taken together, represent methylene or ethylene and may bind to one or more carbon atoms constituting the ring, to form a new ring; 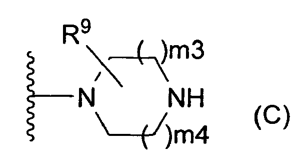 wherein m3 and m4 are independently 0 or 1, and R⁹ is absent or one or two R⁹s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁹s, when taken together, represent methylene or ethylene and may bind to one or more carbon atoms constituting the ring, to form a new ring; and 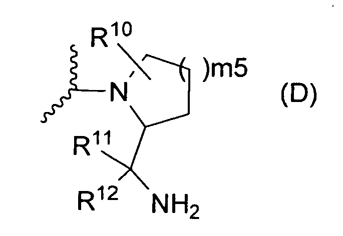 wherein m5 is 1, 2 or 3, R¹⁰ is absent or one or two R¹⁰s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R¹⁰s, when taken together, represent methylene or ethylene and may bind to one or more carbon atoms constituting the ring, to form a new ring, and R¹¹ and R¹² are independently a hydrogen atom, methyl, ethyl, propyl or isopropyl, or R¹¹ and R¹², when taken together with the adjacent carbon atom, represent cyclopropyl, cyclobutyl or cyclopentyl, a prodrug of said compound, or a pharmaceutically acceptable salt of said compound or prodrug.
[2] A compound according to [1], which is represented by the formula (II): 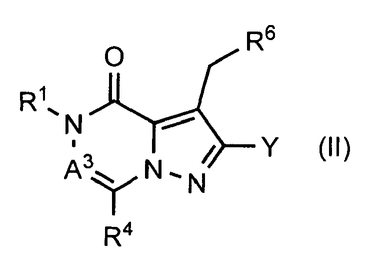 wherein A³ is a nitrogen atom or a group represented by the formula C(R²), and R¹, R², R⁴, R⁶ and Y are as defined in the item [1], a prodrug thereof or a pharmaceutically acceptable salt of the compound or prodrug.
[3] A compound, a prodrug thereof or a pharmaceutically acceptable salt of the compound or prodrug according to [1] or [2], wherein R⁶ is the following formula (E), formula (F), formula (G) or formula (H): 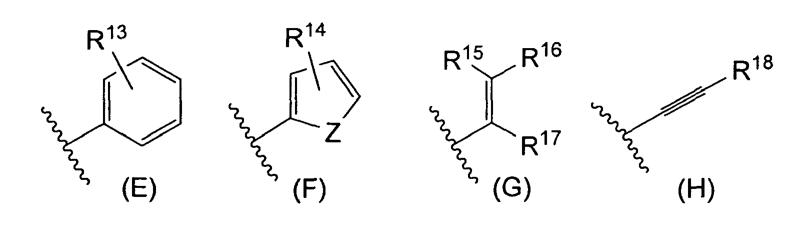 wherein Z is an oxygen atom, the formula S(O)p or N(R¹⁹);
   R¹³ is absent or one or two R¹³s are present and are independently a halogen atom, a hydroxyl group, a formyl group, a carboxyl group, a cyano group, an alkylthio group, an alkylsulfinyl group, an alkylsulfonyl group, an alkyl group, a haloalkyl group, a cycloalkyl group, an alkoxy group, a haloalkoxy group, an optionally substituted amino group, an optionally substituted carbamoyl group, an alkoxycarbonyl group, an optionally substituted alkylcarbonyl group, a cycloalkylcarbonyl group, an optionally substituted phenyl group, an optionally substituted heteroaryl group or a nitrogen-containing saturated heterocyclic group, or two R¹³s, when taken together, represent a C₁₋₃ alkylenedioxy group;
   R¹⁴ is absent or one or two R¹⁴s are present and are independently a halogen atom, a cyano group, an alkyl group, a haloalkyl group, a cycloalkyl group, an alkoxy group or a haloalkoxy group;
   R¹⁵ is methyl, ethyl, a chlorine atom or a bromine atom;
   R¹⁶ is a hydrogen atom, methyl, ethyl, a chlorine atom or a bromine atom;
   R¹⁷ is a hydrogen atom, methyl or ethyl;
   R¹⁸ is a hydrogen atom, methyl, ethyl, cyclopropyl or cyclobutyl;
   p is 0, 1 or 2; and
   R¹⁹ is a hydrogen atom or an alkyl group.
[4] A compound, a prodrug thereof or a pharmaceutically acceptable salt of the compound or prodrug according to [3], wherein R⁶ is the formula (E) or the formula (H).
[5] A compound, a prodrug thereof or a pharmaceutically acceptable salt of the compound or prodrug according to [4], wherein R⁶ is the formula (E) and one or two R¹³ are present and are independently a halogen atom, a cyano group, an alkylthio group, an alkylsulfonyl group, a C₁₋₃ alkylenedioxy group, an alkyl group, a haloalkyl group, a cycloalkyl group, an alkoxy group or a haloalkoxy group.
[6] A compound, a prodrug thereof or a pharmaceutically acceptable salt of the compound or prodrug according to either of [1] and [2], wherein R⁶ is the following formula (J): 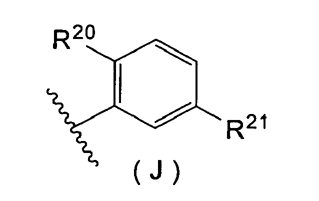 wherein R²⁰ is a halogen atom, a cyano group, an alkylthio group, an alkylsulfonyl group, an alkyl group, a haloalkyl group, a cycloalkyl group, an alkoxy group or a haloalkoxy group, and R²¹ is a hydrogen atom or a fluorine atom.
[7] A compound, a prodrug thereof or a pharmaceutically acceptable salt of the compound or prodrug according to any one of [1] to [6], wherein Y is the formula (A) or (B).
[8] A compound, a prodrug thereof or a pharmaceutically acceptable salt of the compound or prodrug according to any one of [1] to [6], wherein Y is the formula (A).
[9] A compound, a prodrug thereof or a pharmaceutically acceptable salt of the compound or prodrug according to any one of [1] to [8], wherein R² is a hydrogen atom, a cyano group, an optionally substituted alkyl group, an optionally substituted cycloalkyl group, an optionally substituted alkenyl group, an optionally substituted carbamoyl group, a carboxyl group, an optionally substituted alkoxycarbonyl group, an optionally substituted cycloalkyloxycarbonyl group, a tetrahydrofuranyloxycarbonyl group, an optionally substituted aryl group, an optionally substituted aryloxycarbonyl group, an optionally substituted aralkyl group, an optionally substituted aroyl group, an optionally substituted heteroaryl group, an optionally substituted heteroarylalkyl group, an optionally substituted heteroarylcarbonyl group or an optionally substituted alkylcarbonyl group.
[10] A compound, a prodrug thereof or a pharmaceutically acceptable salt of the compound or prodrug according to any one of [1] to [8], wherein R² is a hydrogen atom, a cyano group, an optionally substituted alkyl group, an optionally substituted carbamoyl group, a carboxyl group, an optionally substituted alkoxycarbonyl group, an optionally substituted cycloalkyloxycarbonyl group, a tetrahydrofuranyloxycarbonyl group, an optionally substituted aryl group, an optionally substituted aryloxycarbonyl group, an optionally substituted aralkyl group, an optionally substituted aroyl group, an optionally substituted heteroaryl group or an optionally substituted alkylcarbonyl group.
[11] A compound, a prodrug thereof or a pharmaceutically acceptable salt of the compound or prodrug according to any one of [1] to [10], wherein R⁴ is a hydrogen atom, an optionally substituted alkyl group, a cyano group, an optionally substituted carbamoyl group, a carboxyl group, an optionally substituted alkoxy group, an optionally substituted cycloalkyloxy group, an optionally substituted alkoxycarbonyl group, an optionally substituted aryl group, an optionally substituted aryloxycarbonyl group, an optionally substituted aralkyl group, an optionally substituted aroyl group, an optionally substituted heteroaryl group, an optionally substituted heteroarylalkyl group, an optionally substituted heteroarylcarbonyl group or an optionally substituted alkylcarbonyl group.
[12] A compound, a prodrug thereof or a pharmaceutically acceptable salt of the compound or prodrug according to any one of [1] to [10], wherein R⁴ is a hydrogen atom or an optionally substituted alkyl group.
[13] A compound, a prodrug thereof or a pharmaceutically acceptable salt of the compound or prodrug according to any one of [1] to [10], wherein R⁴ is the formula: -C(R²²)(R²³)-A⁴-R²⁴ in which R²² and R²³ are independently a hydrogen atom, methyl, ethyl, propyl, isopropyl, methoxy or ethoxy, or R²² and R²³, when taken together with the adjacent carbon atom, represent cyclopropyl, cyclobutyl or cyclopentyl; A⁴ is a single bond, methylene or ethylene; and R²⁴ is a hydrogen atom, a cyano group, an alkyl group, a haloalkyl group, a cycloalkyl group, a tetrahydrofuranyl group, an optionally substituted carbamoyl group, a carboxyl group, an optionally substituted alkoxy group, an optionally substituted aryloxy group, an optionally substituted alkoxycarbonyl group, an optionally substituted aryl group, an optionally substituted aryloxycarbonyl group, an optionally substituted aroyl group, an optionally substituted heteroarylcarbonyl group or an optionally substituted alkylcarbonyl group.
[14] A compound, a prodrug thereof or a pharmaceutically acceptable salt of the compound or prodrug according to any one of [1] to [13], wherein R¹ is a hydrogen atom, an optionally substituted alkyl group of 1 to 3 carbon atoms, or an optionally substituted aryl group, and the substituent(s) of the optionally substituted alkyl group is selected from fluorine atom, optionally substituted aroyl groups, carboxyl group, optionally substituted alkoxycarbonyl groups, optionally substituted aryl groups and optionally substituted aryloxy groups.
[15] A compound, a prodrug thereof or a pharmaceutically acceptable salt of the compound or prodrug according to any one of [1] to [13], wherein R¹ is a hydrogen atom, methyl or a group represented by the formula: -Ra-Rb-Rc in which
   Ra is an alkylene chain;
   Rb is a single bond or a carbonyl group; and
   Rc is an optionally substituted alkyl group, an optionally substituted alkoxy group, an optionally substituted aryl group, an optionally substituted heteroaryl group, an optionally substituted heteroaryloxy group or an optionally substituted aryloxy group.
[16] A pharmaceutical composition comprising a compound, a prodrug thereof or a pharmaceutically acceptable salt of the compound or prodrug according to any one of [1] to [15] as an active ingredient.
[17] A dipeptidyl peptidase IV inhibitor comprising a compound, a prodrug thereof or a pharmaceutically acceptable salt of the compound or prodrug according to any one of [1] to [15] as an active ingredient.
[18] A pharmaceutical composition for the treatment of diabetes comprising a compound, a prodrug thereof or a pharmaceutically acceptable salt of the compound or prodrug according to any one of [1] to [15] as an active ingredient.
[19] Use of a compound, a prodrug thereof or a pharmaceutically acceptable salt of the compound or prodrug according to any one of [1] to [15] in the manufacture of a dipeptidyl peptidase IV inhibitor.
[20] Use of a compound, a prodrug thereof or a pharmaceutically acceptable salt of the compound or prodrug according to any one of [1] to [15] in the manufacture of a pharmaceutical composition for the treatment of diabetes.
[21] A method for treating diabetes comprising administering an effective amount of a compound, a prodrug thereof or a pharmaceutically acceptable salt of the compound or prodrug according to any one of [1] to [15] to a patient who needs treatment.

The present inventive compound has an excellent DPP-IV inhibitory activity and is useful as a therapeutic agent for diabetes.

The present invention is explained below in further detail.

In the present specification, the number of substituents of each group defined by the term "optionally substituted" or "substituted" is not particularly limited so long as the substitution is possible, and it is 1 or more. Unless otherwise specified, the explanation of each group applies also to the case where the group is a portion or the substituent of another group.

The "halogen atom" includes, for example, fluorine atom, chlorine atom, bromine atom and iodine atom.

The "alkyl group" includes, for example, linear or branched alkyl groups of 1 to 6 carbon atoms. Specific examples thereof are methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl. Preferable examples thereof are linear or branched alkyl groups of 1 to 4 carbon atoms. Specific examples of such groups are methyl, ethyl, propyl, isopropyl, butyl and tert-butyl.

The "alkenyl group" includes, for example, alkenyl groups of 2 to 6 carbon atoms. Specific examples thereof are vinyl, propenyl, methylpropenyl, butenyl and methylbutenyl.

The "alkynyl group" includes, for example, alkynyl groups of 2 to 6 carbon atoms. Specific examples thereof are ethynyl, 1-propynyl, 2-propynyl, butynyl, pentynyl and hexynyl.

The "cycloalkyl group" includes, for example, cycloalkyl groups of 3 to 10 carbon atoms. Specific examples thereof are cyclopropyl, cyclobutyl, cyclopentyl, cyclohexyl, cycloheptyl, adamantyl and norbornyl. Preferable examples thereof are cycloalkyl groups of 3 to 6 carbon atoms. Specific examples of such groups are cyclopropyl, cyclobutyl, cyclopentyl and cyclohexyl.

The "aryl group" includes, for example, aryl groups of 6 to 10 carbon atoms. Specific examples thereof are phenyl, 1-naphthyl and 2-naphthyl.

The "aralkyl group" includes, for example, groups formed by bonding of an aryl group to an alkylene chain. Specific examples thereof are benzyl, 2-phenylethyl and 1-naphthylmethyl.

The "alkylene chain" includes, for example, alkylene chains of 1 to 3 carbon atoms. Specific examples thereof are methylene, ethylene and trimethylene.

The "heteroaryl group" includes, for example, 5- to 10-membered monocyclic or polycyclic groups containing one or more (for example, 1 to 4) heteroatoms selected from nitrogen atom, sulfur atom and oxygen atom. Specific examples thereof are pyrrolyl, thienyl, benzothienyl, benzofuranyl, benzoxazolyl, benzothiazolyl, furyl, oxazolyl, thiazolyl, isoxazolyl, imidazolyl, pyrazolyl, pyridyl, pyrazyl, pyrimidyl, pyridazyl, quinolyl, isoquinolyl, triazolyl, triazinyl, tetrazolyl, indolyl, imidazo[1,2-a]pyridyl, dibenzofuranyl, benzimidazolyl, quinoxalyl, cinnolyl, quinazolyl, indazolyl, naphthyridyl, quinolinolyl and isoquinolinolyl. Preferable examples thereof are 5- or 6-membered groups containing a heteroatom selected from nitrogen atom, sulfur atom and oxygen atom. Specific examples of such groups are pyridyl, thienyl and furyl.

The heteroaryl portion of the "heteroarylalkyl group" includes the groups exemplified above as the heteroaryl group.

The "alkylcarbonyl group" includes, for example, alkylcarbonyl groups of 2 to 4 carbon atoms. Specific examples thereof are acetyl, propionyl and butyryl.

The "cycloalkylcarbonyl group" includes, for example, cycloalkylcarbonyl groups of 4 to 11 carbon atoms. Specific examples thereof are cyclopropylcarbonyl, cyclobutylcarbonyl, cyclopentylcarbonyl, cyclohexylcarbonyl, adamantylcarbonyl and norbornylcarbonyl. Preferable examples thereof are cycloalkylcarbonyl groups of 4 to 7 carbon atoms. Specific examples of such groups are cyclopropylcarbonyl, cyclobutylcarbonyl, cyclopentylcarbonyl and cyclohexylcarbonyl.

The "aroyl group" includes, for example, aroyl groups of 7 to 11 carbon atoms. Specific examples thereof are benzoyl, 1-naphthoyl and 2-naphthoyl.

The heteroaryl portion of the "heteroarylcarbonyl group" includes the groups exemplified above as the heteroaryl group.

The "alkoxycarbonyl group" includes, for example, alkoxycarbonyl groups of 2 to 5 carbon atoms. Specific examples thereof are methoxycarbonyl, ethoxycarbonyl, propoxycarbonyl, 2-propoxycarbonyl and tert-butoxycarbonyl.

The "aryloxycarbonyl group" includes, for example, aryloxycarbonyl groups of 7 to 11 carbon atoms. Specific examples thereof are phenyloxycarbonyl, 2-naphthyloxy- carbonyl and 1-naphthyloxycarbonyl.

The "alkoxy group" includes, for example, alkoxy groups of 1 to 4 carbon atoms. Specific examples thereof are methoxy, ethoxy, propoxy, isopropoxy, butoxy, isobutoxy, sec-butoxy and tert-butoxy.

The "cycloalkyloxy group" includes, for example, cycloalkyloxy groups of 3 to 10 carbon atoms. Specific examples thereof are cyclopropyloxy, cyclobutoxy, cyclopentyloxy, cyclohexyloxy, cycloheptyloxy, adamantyloxy and norbornyloxy. Preferable examples thereof are cycloalkyloxy groups of 3 to 6 carbon atoms. Specific examples of such groups are cyclopropyloxy, cyclobutoxy, cyclopentyloxy and cyclohexyloxy.

The "aryloxy group" includes, for example, aryloxy groups of 6 to 10 carbon atoms. Specific examples thereof are phenoxy, 1-naphthyloxy and 2-naphthyloxy.

The aralkyl portion of the "aralkyloxy group" includes the groups exemplified above as the aralkyl group. Specific examples thereof are benzyloxy and 2-phenylethyloxy.

The heteroaryl portion of the "heteroaryloxy group" includes the groups exemplified above as the heteroaryl group.

The "alkylthio group" includes, for example, alkylthio groups of 1 to 6 carbon atoms. Specific examples thereof are methylthio, ethylthio, propylthio, isopropylthio, butylthio, sec-butylthio, tert-butylthio, pentylthio and hexylthio. Preferable examples thereof are alkylthio groups of 1 to 4 carbon atoms. Specific examples of such groups are methylthio, ethylthio, propylthio, isopropylthio, butylthio, sec-butylthio and tert-butylthio.

The "alkylsulfinyl group" includes, for example, alkylsulfinyl groups of 1 to 6 carbon atoms. Specific examples thereof are methylsulfinyl, ethylsulfinyl, propylsulfinyl, isopropylsulfinyl, butylsulfinyl, pentylsulfinyl and hexylsulfinyl. Preferable examples thereof are alkylsulfinyl groups of 1 to 4 carbon atoms. Specific examples of such groups are methylsulfinyl, ethylsulfinyl, propylsulfinyl, isopropylsulfinyl and butylsulfinyl.

The "alkylsulfonyl group" includes, for example, alkylsulfonyl groups of 1 to 6 carbon atoms. Specific examples thereof are methylsulfonyl, ethylsulfonyl, propylsulfonyl, isopropylsulfonyl, butylsulfonyl, pentylsulfonyl and hexylsulfonyl. Preferable examples thereof are alkylsulfonyl groups of 1 to 4 carbon atoms. Specific examples of such groups are methylsulfonyl, ethylsulfonyl, propylsulfonyl, isopropylsulfonyl and butylsulfonyl.

The "arylthio group" includes, for example, arylthio groups of 6 to 10 carbon atoms. Specific examples thereof are phenylthio, 1-naphthylthio and 2-naphthylthio.

The "arylsulfinyl group" includes, for example, arylsulfinyl groups of 6 to 10 carbon atoms. Specific examples thereof are phenylsulfinyl, 1-naphthylsulfinyl and 2-naphthylsulfinyl.

The "arylsulfonyl group" includes, for example, arylsulfonyl groups of 6 to 10 carbon atoms. Specific examples thereof are phenylsulfonyl, tosyl, 1-naphthylsulfonyl and 2-naphthylsulfonyl.

The "nitrogen-containing saturated heterocyclic group" includes, for example, 5- or 6-membered saturated heterocyclic rings which have one or two nitrogen atoms and may further have an oxygen atom or a sulfur atom. Specific examples thereof are pyrrolidinyl, imidazolidinyl, piperidinyl, morpholinyl, thiomorpholinyl, dioxothiomorpholinyl, hexamethyleniminyl, oxazolidinyl, thiazolidinyl, imidazolidinyl, oxoimidazolidinyl, dioxoimidazolidinyl, oxooxazolidinyl, dioxooxazolidinyl, dioxothiazolidinyl, tetrahydrofuranyl and tetrahydropyridinyl.

The substituent(s) of the "optionally substituted alkyl group" includes, for example, (1) halogen atoms, (2) hydroxyl group, (3) cyano group, (4) carboxyl group, (5) optionally substituted cycloalkyl groups, (6) optionally substituted aryl groups, (7) optionally substituted heteroaryl groups, (8) optionally substituted aroyl groups, (9) optionally substituted heteroarylcarbonyl groups, (10) optionally substituted arylaminocarbonyl groups, (11) optionally substituted heteroarylaminocarbonyl groups, (12) optionally substituted aryloxy groups, (13) optionally substituted arylsulfonyl groups, (14) optionally substituted aralkylsulfonyl groups, (15) optionally substituted alkoxy groups, (16) optionally substituted cycloalkyloxy groups, (17) optionally substituted alkoxycarbonyl groups, (18) optionally substituted aryloxycarbonyl groups, (19) optionally substituted amino groups, (20) optionally substituted carbamoyl groups, (21) alkylsulfonyl groups, (22) optionally substituted vinyl groups, (23) optionally substituted ethynyl groups, (24) optionally substituted alkylcarbonyl groups, (25) cycloalkyloxycarbonyl groups, (26) optionally substituted quinolonyl groups, (27) optionally substituted isoquinolonyl groups, (28) tetrahydrofuranyloxycarbonyl group, and (29) tetrahydrofuranyl group.

Here, the above items (1) to (29) are explained below.

The substituents of the "optionally substituted cycloalkyl groups" of the above item (5) include, for example, alkyl groups, aralkyl groups, alkoxy groups and fluorine atom.

The substituents of the "optionally substituted aryl groups" of the above item (6) include those hereinafter exemplified as the substituents of the "optionally substituted aryl groups".

The substituents of the "optionally substituted heteroaryl groups" of the above item (7) include, for example,
(a) hydroxyl group,
(b) halogen atoms,
(c) alkyl groups,
(d) alkyl groups substituted by a halogen atom(s) or an alkoxy group (for example, fluoromethyl, difluoromethyl, trifluoromethyl, 2,2-difluoroethyl, 2,2,2-trifluoroethyl, perfluoroethyl, 2-fluoro-1-(fluoromethyl)ethyl, 1-(difluoromethyl)-2,2-difluoroethyl, methoxymethoxy, ethoxymethoxy, methoxyethoxy, ethoxyethoxy, methoxypropoxy and ethoxypropoxy),
(e) alkoxy groups,
(f) alkoxy groups substituted by a halogen atom(s) or an alkoxy group (for example, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
(g) cyano group,
(h) carboxyl group,
(i) alkoxycarbonyl groups,
(j) carbamoyl groups which may be substituted by an alkyl group(s) (for example, carbamoyl, methylcarbamoyl, dimethylcarbamoyl, ethylcarbamoyl and diethylcarbamoyl),
(k) aryl groups,
and (l) amino group.

The substituents of the "optionally substituted aroyl groups" of the above item (8) include those exemplified as the substituents of the "optionally substituted aryl groups" of the above item (6).

The substituents of the "optionally substituted heteroarylcarbonyl groups" of the above item (9) include those exemplified as the substituents of the "optionally substituted heteroaryl groups" of the above item (7).

The substituents of the "optionally substituted arylaminocarbonyl groups" of the above item (10) include those exemplified as the substituents of the "optionally substituted aryl groups" of the above item (6).

The substituents of the "optionally substituted heteroarylaminocarbonyl groups" of the above item (11) include those exemplified as the substituents of the "optionally substituted heteroaryl groups" of the above item (7).

The substituents of the "optionally substituted aryloxy groups" of the above item (12) and the "optionally substituted arylsulfonyl groups" of the above item (13) include those exemplified as the substituents of the "optionally substituted aryl groups" of the above item (6).

The aralkyl portion of each of the "optionally substituted aralkylsulfonyl groups" of the above item (14) includes the groups exemplified above as the aralkyl group.

The substituents of the "optionally substituted aralkylsulfonyl groups" include those exemplified as the substituents of the "optionally substituted aryl groups" of the above item (6).

The substituents of the "optionally substituted alkoxy groups" of the above item (15) include, for example,
(a) hydroxyl group,
(b) carboxyl group,
(c) alkyl groups,
(d) alkoxy groups,
(e) alkylcarbonyloxy groups (for example, methylcarbonyloxy, ethylcarbonyloxy, propylcarbonyloxy, isopropylcarbonyloxy, butylcarbonyloxy and tert-butylcarbonyloxy),
(f) alkoxycarbonyl groups,
(g) amino groups substituted by an alkyl group(s),
(h) carbamoyl groups substituted by an alkyl group(s),
(i) sulfamoyl groups substituted by an alkyl group(s),
(j) ureido groups substituted by an alkyl group(s),
(k) alkoxycarbonyloxy groups (for example, methoxycarbonyloxy, ethoxycarbonyloxy, 2-propoxycarbonyloxy, and tert-butoxycarbonyloxy),
(l) cycloalkyloxycarbonyloxy groups (for example, cyclopentyloxycarbonyloxy, cyclohexyloxycarbonyloxy and cycloheptyloxycarbonyloxy),
(m) phenyl groups which may be substituted by a halogen atom or an alkoxy group (for example, phenyl, 2-fluorophenyl, 3-fluorophenyl, 4-fluorophenyl, 2-chlorophenyl, 3-chlorophenyl, 4-chlorophenyl, 2-methoxyphenyl, 3-methoxyphenyl, 4-methoxyphenyl, 2-ethoxyphenyl, 3-ethoxyphenyl, 4-ethoxyphenyl, 2-isopropoxyphenyl and 3-isopropoxyphenyl),
(n) 5-methyl-2-oxo-1,3-dioxolen-4-yl,
(o) 5-oxo-2-tetrahydrofuranyl,
(p) 1,3-dihydro-3-oxo-1-isobenzofuranyl,
(q) tetrahydrofuranyl,
(r) nitrogen-containing saturated heterocyclic groups,
(s) alkoxy groups substituted by a halogen atom(s) or an alkoxy group (for example, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
(t) cycloalkyl groups,
(u) cycloalkyl groups substituted by a halogen atom(s) or an alkoxy group (for example, 2-fluorocyclopropyl, 2-methoxycyclopropyl, 2-fluorocyclobutyl, 3-fluorocyclobutyl and 3-methoxycyclobutyl),
and (v) halogen atoms.

The substituents of the "optionally substituted cycloalkyloxy groups" of the above item (16) and the "optionally substituted alkoxycarbonyl groups" of the above item (17) include those exemplified as the substituents of the "optionally substituted alkoxy groups" of the above item (15).

The substituents of the "optionally substituted aryloxycarbonyl groups" of the above item (18) include those exemplified as the substituents of the "optionally substituted aryl groups" of the above item (6).

The substituents of the "optionally substituted amino groups" of the above item (19) include, for example,
(a) alkyl groups,
(b) alkylcarbonyl groups,
(c) aroyl groups,
(d) alkylsulfonyl groups,
(e) arylsulfonyl groups,
(f) optionally substituted aryl groups (whose substituent(s) includes, for example, halogen atoms, alkyl groups and alkoxy groups),
(g) alkoxycarbonylmethyl (the carbon atom of the methyl portion may be substituted by one or two alkyl groups, and the two alkyl groups on the carbon atom of the methyl portion may bind to each other to form cyclopropyl, cyclobutyl or cyclopentyl together with the carbon atom of the methyl portion),
and (h) aralkyl groups.

As the optionally substituted amino groups, (i) imides are also exemplified.

The substituents of the "optionally substituted carbamoyl groups" of the above item (20) include, for example, alkyl groups and cycloalkyl groups. The two substituents of the carbamoyl group may bind to each other to form an aliphatic heterocyclic ring which may contain carbon, nitrogen or oxygen, such as pyrrolidine (which may be substituted by a hydroxyl group), piperidine, morpholine, thiomorpholine, thiomorpholine oxide, thiomorpholine dioxide, piperazine (whose nitrogen atom may be substituted by methyl or ethyl), or the like.

Specific examples of the "optionally substituted carbamoyl groups" are carbamoyl, methylcarbamoyl, dimethylcarbamoyl, ethylcarbamoyl, diethylcarbamoyl, ethylmethylcarbamoyl, methylpropylcarbamoyl, cyclopropylcarbamoyl, cyclopropylmethylcarbamoyl, pyrrolidinocarbonyl, piperidinocarbonyl and morpholinocarbonyl.

The substituents of the "optionally substituted vinyl groups" of the above item (22) include, for example, halogen atoms and alkyl groups.

Specific examples of the substituted vinyl groups are 1-propylene, 2-methyl-1-propylene and 2-chloro-1-propylene.

The substituents of the "optionally substituted ethynyl groups" of the above item (23) include, for example, alkyl groups and cycloalkyl groups.

Specific examples of the substituted ethynyl groups are ethylidyne, propylidyne and 2-cyclopropyl-1-ethylidyne.

The substituents of the "optionally substituted alkylcarbonyl groups" of the above item (24) include, for example,
(a) halogen atoms,
(b) alkoxy groups,
(c) cycloalkyl groups,
(d) alkoxycarbonyl groups,
and (e) optionally substituted aryl groups (whose substituent(s) includes, for example, halogen atoms, alkyl groups, alkoxy groups and alkoxycarbonyl groups).

The substituents of the "optionally substituted quinolonyl groups" of the above item (26) and the "optionally substituted isoquinolonyl groups" of the above item (27) include, for example, alkyl groups.

The substituent(s) of each of the "optionally substituted alkylthio group", "optionally substituted alkylsulfinyl group" and "optionally substituted alkylsulfonyl group" includes those exemplified as the substituents of the above-mentioned "optionally substituted alkyl group".

The substituent(s) of each of the "optionally substituted alkenyl group" and the "optionally substituted alkynyl group" includes
(1) hydroxyl group,
(2) halogen atoms,
(3) alkyl groups,
(4) alkyl groups substituted by a halogen atom(s) or an alkoxy group (for example, fluoromethyl, difluoromethyl, trifluoromethyl, 2,2-difluoroethyl, 2,2,2-trifluoroethyl, perfluoroethyl, 2-fluoro-1-(fluoromethyl)ethyl, 1-(difluoromethyl)-2,2-difluoroethyl, methoxymethyl, ethoxymethyl, methoxyethyl, ethoxyethyl, methoxypropyl and ethoxypropyl),
(5) alkoxy groups,
(6) alkoxy groups substituted by a halogen atom(s) or an alkoxy group (for example, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
(7) phenyl groups or aroyl groups, which may be substituted by the following (aa), (bb) or (cc):
   (aa) an alkoxy group which may be substituted by a halogen atom(s) or an alkoxy group (for example, methoxy, ethoxy, propoxy, isopropoxy, butoxy, isobutoxy, sec-butoxy, tert-butoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy or ethoxypropoxy),
   (bb) an alkyl group which may be substituted by a halogen atom(s) (for example, methyl, ethyl, propyl, isopropyl, butyl, fluoromethyl, difluoromethyl, trifluoromethyl, 2,2-difluoroethyl, 2,2,2-trifluoroethyl, perfluoroethyl, 2-fluoro-1-(fluoromethyl)ethyl or 1-(difluoromethyl)-2,2-difluoroethyl),
   (cc) a halogen atom(s),
(8) cyano group,
(9) carboxyl group,
(10) alkoxycarbonyl groups,
(11) carbamoyl groups which may be substituted by an alkyl group(s) (for example, carbamoyl, methylcarbamoyl, dimethylcarbamoyl, ethylcarbamoyl and diethylcarbamoyl),
(12) alkylsulfonyl groups,
   and (13) phenyloxy.

The substituent(s) of the "optionally substituted vinyl group" includes those exemplified as the substituents of (22) the "optionally substituted vinyl groups" as the substituent(s) of the above-mentioned "optionally substituted alkyl group".

The substituent(s) of the "optionally substituted ethynyl group" includes those exemplified as the substituents of (23) the "optionally substituted ethynyl groups" as the substituent(s) of the above-mentioned "optionally substituted alkyl group".

The substituent(s) of the "optionally substituted cycloalkyl group" includes those exemplified as the substituents of (5) the "optionally substituted cycloalkyl groups" as the substituent(s) of the above-mentioned "optionally substituted alkyl group".

The substituent(s) of the "optionally substituted aryl group" includes, for example,
(1) hydroxyl group,
(2) halogen atoms,
(3) alkyl groups,
(4) alkyl groups substituted by a halogen atom(s), an alkoxy group or a cycloalkyl group (for example, fluoromethyl, difluoromethyl, trifluoromethyl, 2,2-difluoroethyl, 2,2,2-trifluoroethyl, perfluoroethyl, 2-fluoro-1-(fluoromethyl)ethyl, 1-(difluoromethyl)-2,2-difluoroethyl, methoxymethyl, ethoxymethyl, methoxyethyl, ethoxyethyl, methoxypropyl and ethoxypropyl),
(5) phenyl groups which may be substituted by the following (aa), (bb) or (cc):
   (aa) an alkoxy group which may be substituted by a halogen atom(s) or an alkoxy group (for example, methoxy, ethoxy, propoxy, isopropoxy, butoxy, isobutoxy, sec.-butoxy, tert.-butoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy or ethoxypropoxy),
   (bb) an alkyl group which may be substituted by a halogen atom(s) (for example, methyl, ethyl, propyl, isopropyl, butyl, fluoromethyl, difluoromethyl, trifluoromethyl, 2,2-difluoroethyl, 2,2,2-trifluoroethyl, perfluoroethyl, 2-fluoro-1-(fluoromethyl)ethyl or 1-(difluoromethyl)-2,2-difluoroethyl),
   (cc) a halogen atom(s),
(6) cyano group,
(7) carboxyl group,
(8) alkoxycarbonyl groups which may be substituted by a halogen atom(s) (for example, methoxy, ethoxy, propoxy, isopropoxy, butoxy, isobutoxy, sec-butoxy, tert-butoxy, fluoromethoxycarbonyl, difluoromethoxycarbonyl, 2,2-difluoroethoxycarbonyl, 2,2,2-trifluoroethoxycarbonyl, methoxycarbonyl and ethoxycarbonyl),
(9) carbamoyl groups which may be substituted by an alkyl group(s) (for example, carbamoyl, methylcarbamoyl, dimethylcarbamoyl, ethylcarbamoyl and diethylcarbamoyl),
(10) alkylsulfonyl groups,
(11) C₁₋₃ alkylenedioxy groups,
(12) formyl group,
(13) optionally substituted phenyloxy groups (whose substituent(s) includes, for example, halogen atoms, alkyl groups and alkoxy groups),
(14) nitrogen-containing saturated heterocyclic groups (for example, pyrrolidinyl, piperidinyl, morpholinyl and piperazinyl (whose nitrogen atoms may be substituted, for example, by methyl, ethyl or propyl)),
(15) cycloalkyloxy groups which may be substituted by a hydroxyl group, an oxo group, a carboxyl group, a carboxymethyl group, an alkoxycarbonyl group, an alkoxycarbonylalkyl group (e.g. methoxycarbonylmethyl, ethoxycarbonylmethyl or isopropoxycarbonylmethyl), an alkyl group, a fluoroalkyl group (e.g. fluoromethyl, difluoromethyl, trifluoromethyl, 2,2-difluoroethyl, 2,2,2-trifluoroethyl or perfluoroethyl), an alkoxyalkyl group (e.g. methoxymethyl, ethoxymethyl or isopropoxymethyl), a cycloalkyloxyalkyl group (e.g. cyclopropyloxymethyl, cyclopropyloxyethyl or cyclobutoxy), an alkoxy group, a cycloalkyloxy group or a halogen atom(s) (for example, 3-carboxycyclobutoxy, 3-methoxycarbonylcyclobutoxy, 3-ethoxycarbonylbutoxy, 2-methylcyclopropyloxy, 2-fluorocyclopropyloxy, 3-methoxycyclobutoxy, 3-fluorocyclobutoxy, 3,3-difluorocyclobutoxy and 3-(2-fluoroethyl)cyclobutoxy),
(16) alkoxy groups which may be substituted by a hydroxyl group, an oxo group, a carboxyl group, an alkoxycarbonyl group, a cycloalkyl group, an alkoxy group, a cycloalkyloxy group, an optionally substituted oxygen-containing heterocyclic group (e.g. a 5- or 6-membered saturated heterocyclic group having an oxygen atom(s), specific examples of which are tetrahydrofuranyl and tetrahydropyranyl; its substituent(s) includes, for example, halogen atoms, oxo group and alkoxy groups), or a halogen atom(s) (for example, methoxy, ethoxy, propoxy, isopropoxy, butoxy, isobutoxy, sec-butoxy, tert-butoxy, 2-hydroxyethoxy, carboxymethoxy, methoxycarbonylmethoxy, ethoxycarbonylmethoxy, tert-butoxycarbonylmethoxy, cyclopropylmethoxy, cyclobutylmethoxy, methoxymethoxy, ethoxymethoxy, methoxyethoxy, ethoxyethoxy, isopropoxy-methoxy, cyclopropyloxymethoxy, cyclobutoxymethoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy and 1-(difluoromethyl)-2,2-difluoroethoxy),
(17) difluoromethylenedioxy,
(18) alkenyl groups which may be substituted by a halogen atom(s) (for example, vinyl, propenyl, methylpropenyl, butenyl and methylbutenyl),
(19) amino groups which may be substituted by an alkyl group(s) (for example, amino, methylamino, ethylamino, propylamino, dimethylamino, methylethylamino and diethylamino),
(20) optionally substituted alkylcarbonyl groups (whose substituent(s) includes, for example, halogen atoms, alkoxy groups and cycloalkyl groups),
(21) alkylcarbonyloxy groups (for example, methylcarbonyloxy, ethylcarbonyloxy and isopropylcarbonyloxy),
(22) cycloalkyl groups which may be substituted by a fluorine atom (for example, cyclopropyl, cyclobutyl, cyclopentyl, 2-fluorocyclopropyl, 2-fluorocyclobutyl, 3-fluorocyclobutylcyclobutyl, adamantyl and norbornyl),
(22) cycloalkylcarbonyl groups which may be substituted by a fluorine atom (for example, cyclopropylcarbonyl, 2-fluorocyclopropylcarbonyl, cyclobutylcarbonyl and cyclopentylcarbonyl),
(23) alkylthio groups,
(24) alkylsulfinyl groups,
(25) optionally substituted heteroaryl groups (whose substituent(s) includes, for example, halogen atoms, alkyl groups, alkoxy groups, haloalkyl groups and haloalkoxy groups),
(26) groups represented by the following formulas (T1) to (T16): 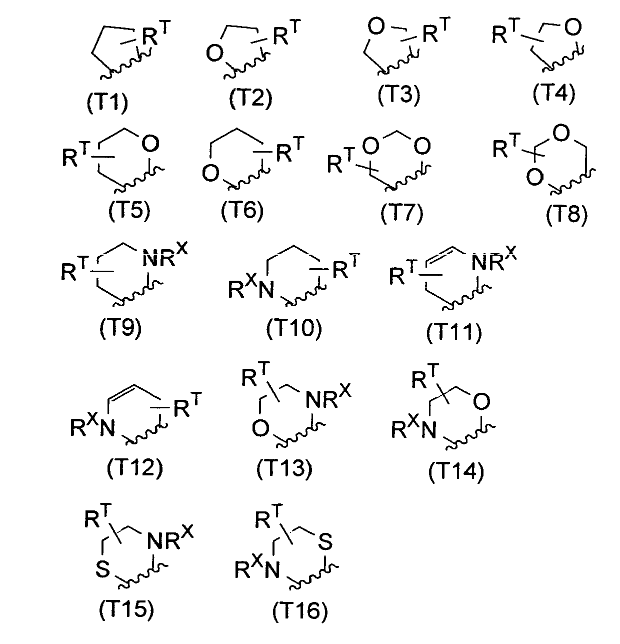
wherein R^{T} is absent or one or more R^{T}s are present and are independently a halogen atom, a hydroxyl group, an oxo group, a carboxyl group, an optionally substituted alkyl group (whose substituent(s) includes, for example, halogen atoms and alkoxy groups), an optionally substituted alkoxycarbonyl group (whose substituent(s) includes, for example, halogen atoms and alkoxy groups), an optionally substituted alkoxy group (whose substituent(s) includes, for example, halogen atoms and alkoxy groups), an optionally substituted carbamoyl group (whose substituent(s) includes, for example, alkyl groups), or a saturated heterocyclic group oxycarbonyl group (the saturated heterocyclic group includes, for example, 5- or 6-membered saturated heterocyclic groups having one or two oxygen atoms, nitrogen atoms and/or sulfur atoms, specific examples of which are tetrahydrofuranyl, tetrahydropyranyl, dihydrofuranyl, tetrahydrothiopyranyl, tetrahydrodioxothiopyranyl, pyrrolidinyl, piperidinyl, piperazinyl, imidazolidinyl, oxazolidinyl and thiazolidinyl), or two R^{T}s, when taken together, may represent methylene, ethylene, trimethylene, tetramethylenel or butenylene and may bind to one or more carbon atoms constituting the ring, to form a new ring; and R^{x} is a hydrogen atom or an alkyl group, and (27) aroyl groups.

The substituent(s) of each of the "optionally substituted heteroaryl group", "optionally substituted aralkyl group", "optionally substituted heteroarylalkyl group", "optionally substituted aroyl group", "optionally substituted heteroarylcarbonyl group", "optionally substituted aryloxycarbonyl group", "optionally substituted aryloxy group", "optionally substituted aralkyloxy group", "optionally substituted heteroaryloxy group", "optionally substituted arylthio group", "optionally substituted arylsulfinyl group" and "optionally substituted arylsulfonyl group" includes those exemplified as the substituent(s) of the above-mentioned "optionally substituted aryl group".

The substituent(s) of the "optionally substituted alkylcarbonyl group" includes those exemplified as the substituents of (24) the "optionally substituted alkylcarbonyl groups" as the substituent(s) of the above-mentioned "optionally substituted alkyl group".

The substituent(s) of the "optionally substituted cycloalkylcarbonyl group" includes, for example, halogen atoms and alkoxy groups.

The substituent(s) of each of the "optionally substituted alkoxy group" and the "optionally substituted alkoxycarbonyl group" includes those exemplified as the substituents of (15) the "optionally substituted alkoxy groups" as the substituent(s) of the above-mentioned "optionally substituted alkyl group".

The substituent(s) of each of the "optionally substituted cycloalkyloxy group" and the "optionally substituted cycloalkyloxycarbonyl group" includes those exemplified as the substituents of (16) the "optionally substituted cycloalkyloxy groups" as the substituent(s) of the above-mentioned "optionally substituted alkyl group".

The substituent(s) of the "optionally substituted amino group" includes those exemplified as the substituents of (19) the "optionally substituted amino groups" as the substituent(s) of the above-mentioned "optionally substituted alkyl group".

The substituent(s) of the "optionally substituted carbamoyl group" includes, for example,
(1) alkyl groups,
(2) cycloalkyl groups,
(3) aryl groups which may be substituted by the following (aa), (bb), (cc) or (dd):
   (aa) a halogen atom(s),
   (bb) an alkoxy group which may be substituted by a halogen atom(s) (for example, methoxy, ethoxy, propoxy, isopropoxy, butoxy, isobutoxy, sec-butoxy, tert-butoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy or 1-(difluoromethyl)-2,2-difluoroethoxy),
   (cc) an alkyl group which may be substituted by a halogen atom(s) (for example, methyl, ethyl, propyl, isopropyl, butyl, methyl, ethyl, propyl, isopropyl, butyl, fluoromethyl, difluoromethyl, trifluoromethyl, 2,2-difluoroethyl, 2,2,2-trifluoroethyl, perfluoroethyl, 2-fluoro-1-(fluoromethyl)ethyl or 1-(difluoromethyl)-2,2-difluoroethyl),
   (dd) a C₁₋₃ alkylenedioxy group,
(4) alkylsulfonyl groups,
(5) cycloalkylsulfonyl groups,
(6) optionally substituted arylsulfonyl groups (whose substituent(s) includes, for example, halogen atoms, alkyl groups, haloalkyl groups, alkoxy groups and haloalkoxy groups),
(7) alkylcarbonyl groups,
(8) alkoxycarbonyl groups,
   and (9) optionally substituted aroyl groups (whose substituent(s) includes, for example, halogen atoms, alkyl groups, haloalkyl groups, alkoxy groups, haloalkoxy groups, alkoxycarbonyl groups and C₁₋₃ alkylenedioxy groups).

Specific examples of the "optionally substituted carbamoyl group" are carbamoyl, methylcarbamoyl, dimethylcarbamoyl, ethylcarbamoyl, diethylcarbamoyl, ethylmethylcarbamoyl, phenylcarbamoyl and phenylmethylcarbamoyl.

The two substituents of the carbamoyl group may bind to each other to form an aliphatic heterocyclic ring which may contain carbon, nitrogen, oxygen or sulfur, such as pyrrolidine, piperidine, morpholine, thiomorpholine, thiomorpholine oxide, thiomorpholine dioxide, piperazine (whose nitrogen atom may be substituted by methyl, ethyl or propyl), or the like. Specific examples the carbamoyl group are pyrrolidinocarbamoyl, piperidinocarbamoyl and morpholinocarbamoyl.

The substituent(s) of the "optionally substituted nitrogen-containing saturated heterocyclic group" includes, for example,
(1) halogen atoms,
(2) alkyl groups,
(3) alkyl groups substituted by a halogen atom(s) or an alkoxy group (for example, fluoromethyl, difluoromethyl, trifluoromethyl, 2-fluoroethyl, 2,2-difluoroethyl, perfluoroethyl and methoxyethyl),
(4) alkoxy groups,
(5) alkoxy groups substituted by a halogen atom(s) or an alkoxy group (for example, fluoromethoxy, difluoromethoxy, trifluoromethoxy, methoxymethoxy, ethoxymethoxy, methoxyethoxy, ethoxyethoxy, methoxypropoxy and ethoxypropoxy),
(6) cyano group,
   and (7) oxo group.

When two R⁷s, R⁸s, R⁹s or R¹⁰s are present, they may be present on different carbon atoms, respectively.

The phrase "two R⁷s, R⁸s, R⁹s or R¹⁰s, when taken together, represent methylene or ethylene and bind to one or more carbon atoms constituting the ring, to form a new ring" means that they form a spiro ring or bicyclo ring through one and the same carbon atom or different carbon atoms.

The phrase "two R^{T}s, when taken together, represent methylene, ethylene, trimethylene, tetramethylene or butenylene and bind to one or two carbon atoms constituting the ring, to form a new ring" means that they form a spiro ring or bicyclo ring through one and the same carbon atom or different carbon atoms.

The "haloalkoxy group" includes, for example, alkoxy groups of 1 to 4 carbon atoms substituted by a halogen atom(s). Specific examples thereof are fluoromethoxy, difluoromethoxy and trifluoromethoxy.

The "haloalkyl group" includes, for example, alkyl groups of 1 to 4 carbon atoms substituted by a halogen atom(s). Specific examples thereof are fluoromethyl, difluoromethyl, trifluoromethyl, 2-fluoroethyl and perfluoroethyl.

The "C₁₋₃ alkylenedioxy group" includes, for example, methylenedioxy, ethylenedioxy and trimethylenedioxy.

The phrase "R¹ and R² may be taken together with the adjacent nitrogen atom and carbon atom to form an optionally substituted 4- to 7-membered ring in the case of the solid line and dotted line between A¹ and A² being a double bond (A¹=A²) and A¹ being the formula C(R²)" means that the compound represented by the general formula (I) of the item [1] is represented by the formula (III): 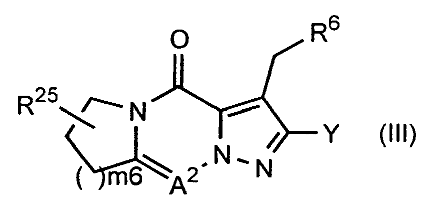 wherein m6 is 0, 1, 2 or 3, and R²⁵ is absent or one or two R²⁵s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group.

As each of the "optionally substituted amino group", "optionally substituted carbamoyl group", "optionally substituted alkylcarbonyl group", "optionally substituted phenyl group" and "optionally substituted heteroaryl group" for R¹³, the corresponding groups explained as the substituent(s) of the above-mentioned "optionally substituted aryl group" are exemplified.

As each of the "optionally substituted carbamoyl group", "optionally substituted alkoxy group", "optionally substituted alkoxycarbonyl group", "optionally substituted aryl group", "optionally substituted aryloxycarbonyl group", "optionally substituted aroyl group", "optionally substituted heteroarylcarbonyl group" and "optionally substituted alkylcarbonyl group" for R²⁴, the corresponding groups explained as the substituent(s) of the above-mentioned "optionally substituted alkyl group" are exemplified.

The substituent(s) of the "optionally substituted alkyl group" for Rc includes, for example, halogen atoms, alkoxy groups and cycloalkyl groups.

The substituent(s) of each of the "optionally substituted heteroaryl group" and "optionally substituted heteroaryloxy group" for Rc includes those exemplified as the substituents of (7) the "optionally substituted heteroaryl group" as the substituent(s) of the above-mentioned "optionally substituted alkyl group".

As the "prodrug", there are exemplified those which are easily hydrolyzed in a living body to regenerate the compound (I) of the present invention. Specific examples thereof are compounds obtained by converting the amino group of the compound represented by the formula (I) to -NHQ. Here, the following are exemplified as Q:
(1) 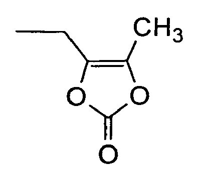
(2) -COR²⁰
(3) -COO-CR²¹(R²²)-OCOR²³
(4) -COOR²⁴
   wherein R²⁰ is a hydrogen atom, an alkyl group or an optionally substituted aryl group; R²¹ and R²² are independently a hydrogen atom or an alkyl group; R²³ is a hydrogen atom, an alkyl group, an aryl group or a benzyl group; and R²⁴ is an alkyl group or a benzyl group.

Preferable examples of Q are the group of (1) and the groups of (3). Preferable examples of the groups of (3) are groups in which R²¹ is a hydrogen atom, R²² is a hydrogen atom, methyl or ethyl and R²³ is a hydrogen atom, methyl or ethyl. These compounds may be produced according to conventional processes (for example, J. Med. Chem. 35, 4727 (1992) and WO 01/40180). In addition, the prodrug may be one which changes to the original compound under physiological conditions such as those described in "Development of Medicines Vol.7, Molecular Design", pp. 163-198, Hirokawa Shoten, 1990.

As the "pharmaceutically acceptable salt", there are exemplified inorganic acid salts such as hydrochloride, hydrobromide, sulfate, phosphate, nitrate, etc., and organic acid salts such as acetate, propionate, oxalate, succinate, lactate, malate, tartrate, citrate, maleate, fumarate, methanesulfonate, benzenesulfonate, p-toluenesulfonate, ascorbate, etc.

In addition, the present invention includes compounds represented by the formula (I), prodrugs thereof and pharmaceutically acceptable salts of the compounds or prodrugs. The present invention also includes their hydrates or solvates (e.g. ethanol solvate). Furthermore, the present invention includes all tautomers, all existing stereoisomers and all crystal forms of the compound (I) of the present invention.

Examples of the compound of the present invention are given below but the compound of the present invention is not limited thereto.

Preferable examples of the bicyclic pyrazole derivative of the present invention are the following bicyclic pyrazole derivatives. In the compounds listed in the following tables, the following abbreviations are used in some cases for the simplification of description.

Ph: phenyl group, Et: ethyl group, Me: methyl group, n-Pr: n-propyl group, i-Pr: isopropyl group, n-Bu: n-butyl group, t-Bu: tert-butyl group, cycpro:
cyclopropyl group, cycbu: cyclobutyl group, etoet:
ethoxyethyl group.

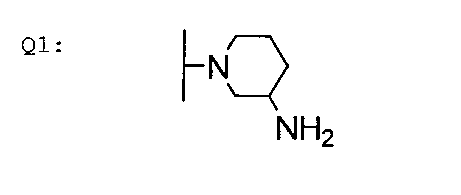

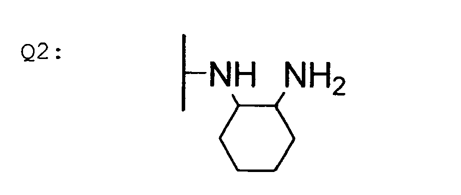

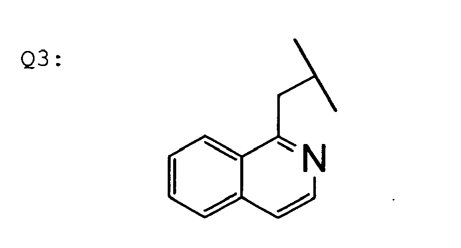

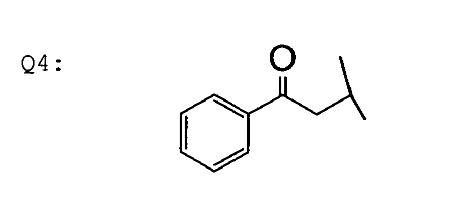

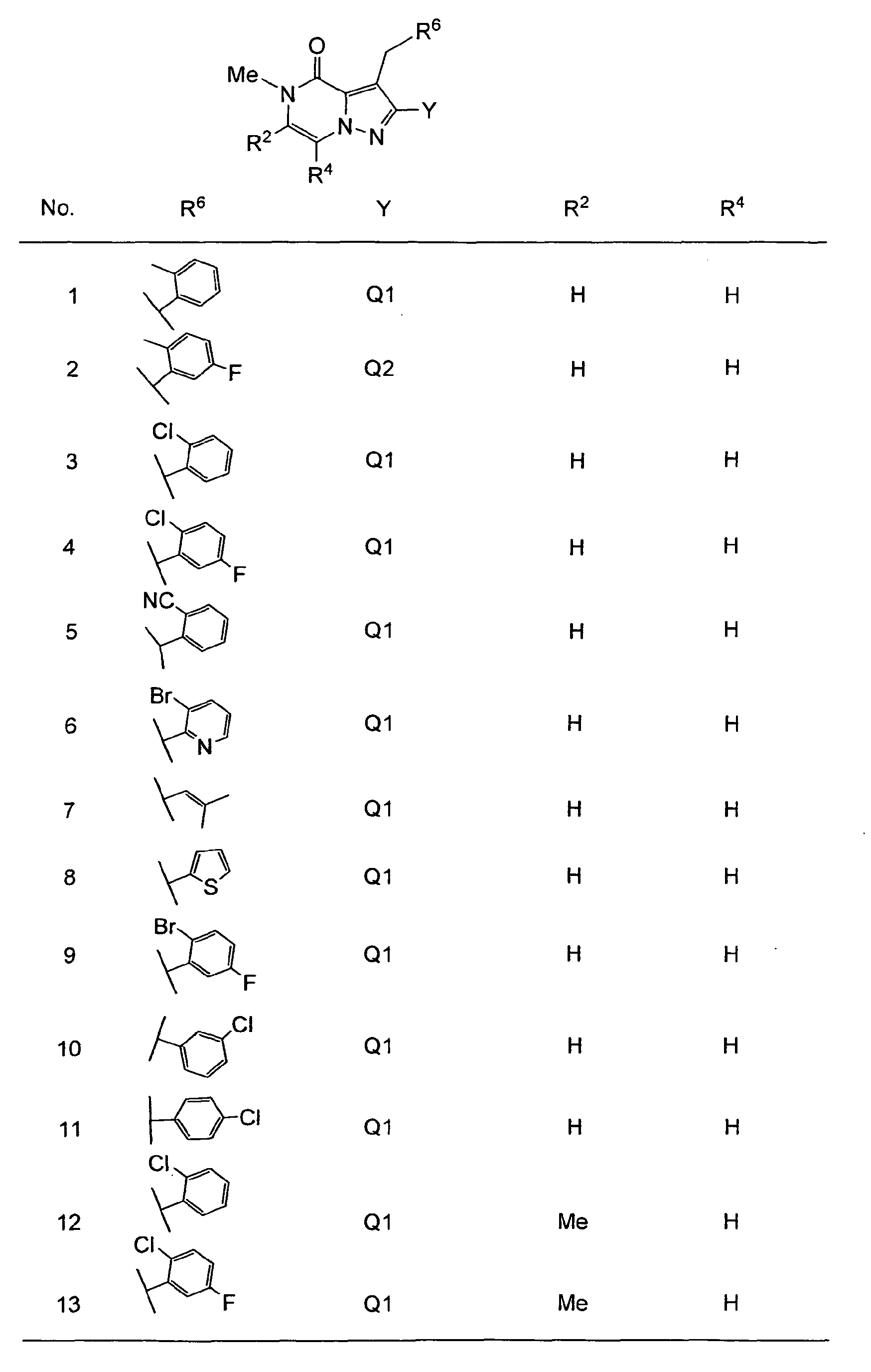

| 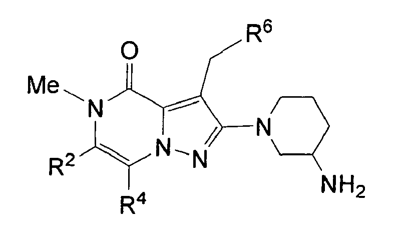 | | | | | | | |
|---|---|---|---|---|---|---|---|
| No. | R⁶ | R² | R⁴ | No. | R⁶ | R² | R⁴ |
| 14 | 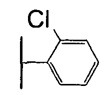 | H | Me | 21 | 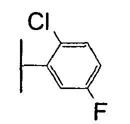 | H | CF₃ |
| 15 | 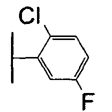 | H | Me | 22 | 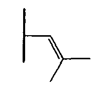 | CF₃ | H |
| 16 | 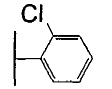 | Me | Me | 23 | 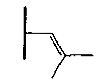 | CN | H |
| 17 | 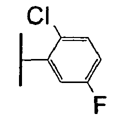 | Me | Et | 24 | 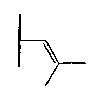 | C(O)Me | H |
| 18 | 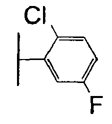 | CF₃ | H | 25 | 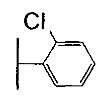 | Et | H |
| 19 | 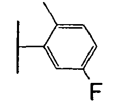 | CF₃ | H | 26 | 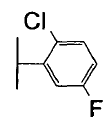 | Et | H |
| 20 | 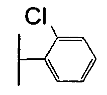 | Me | CF₃ | | | | |

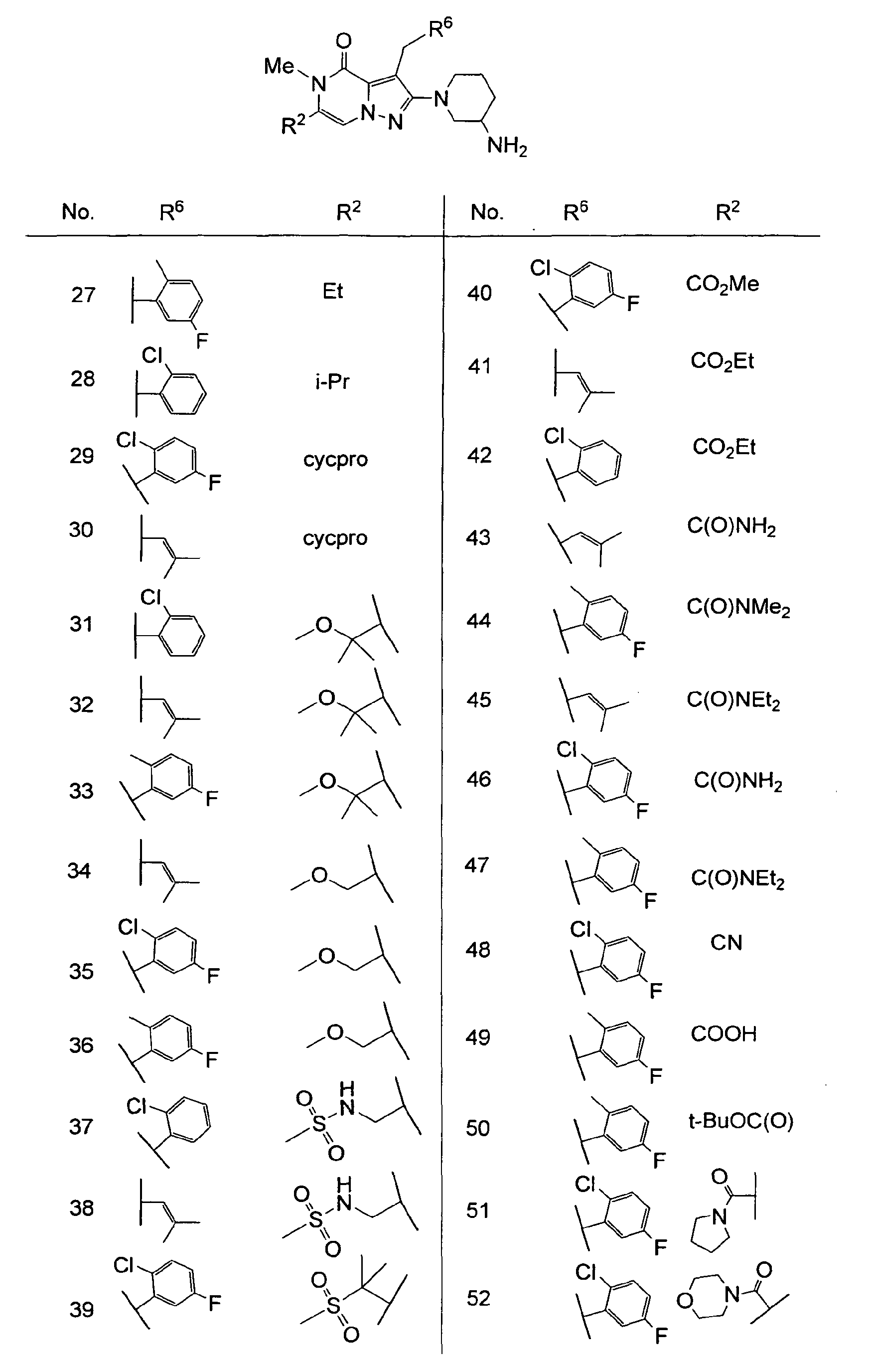

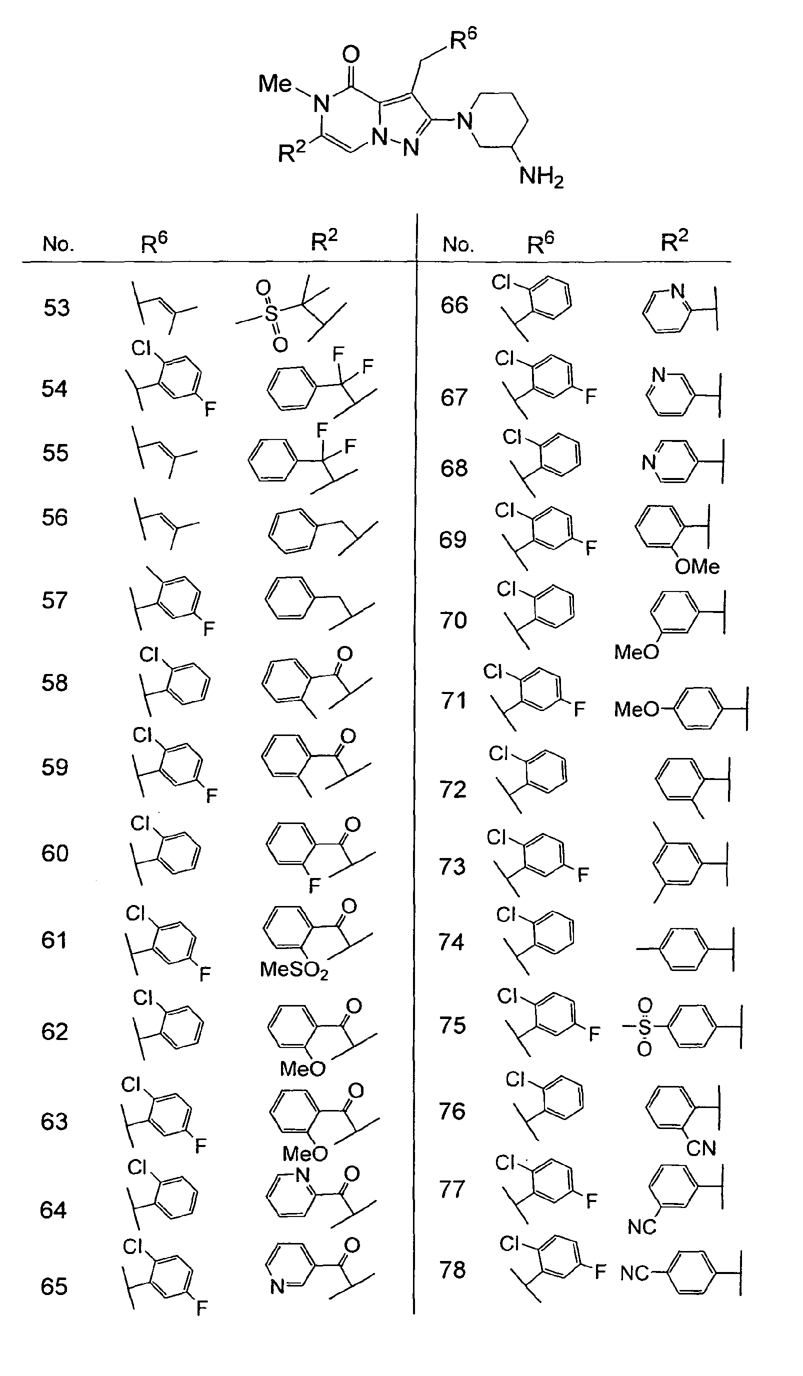

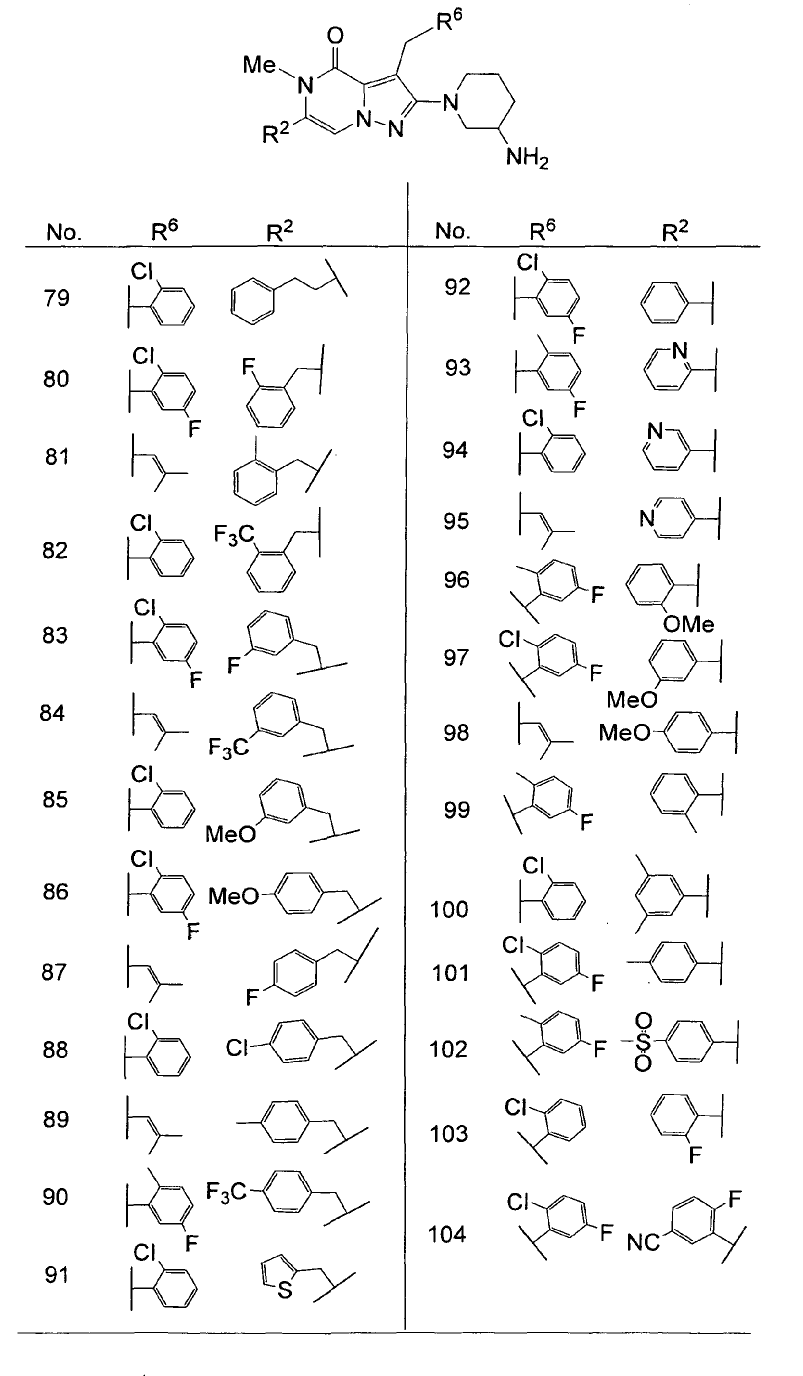

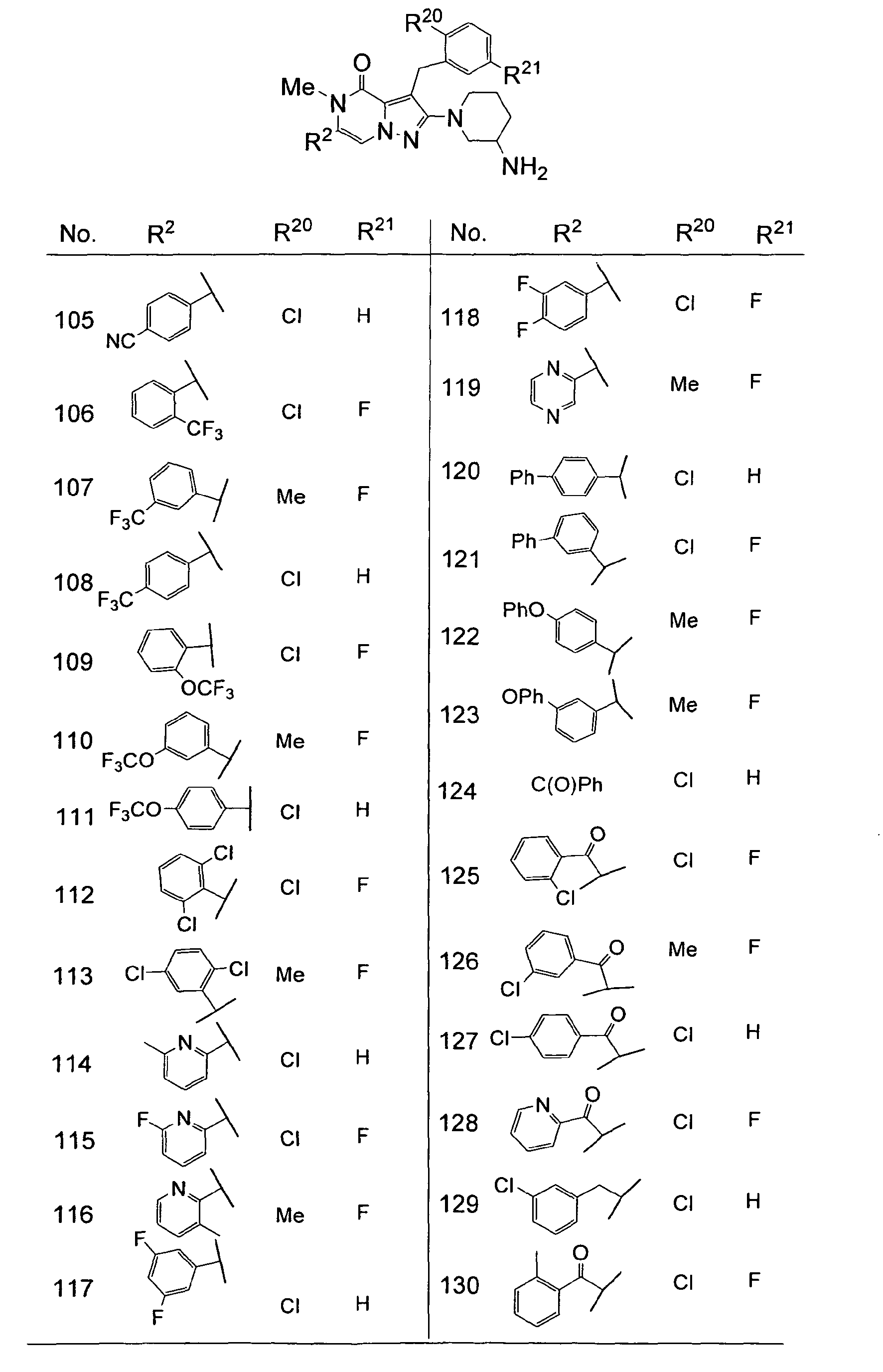

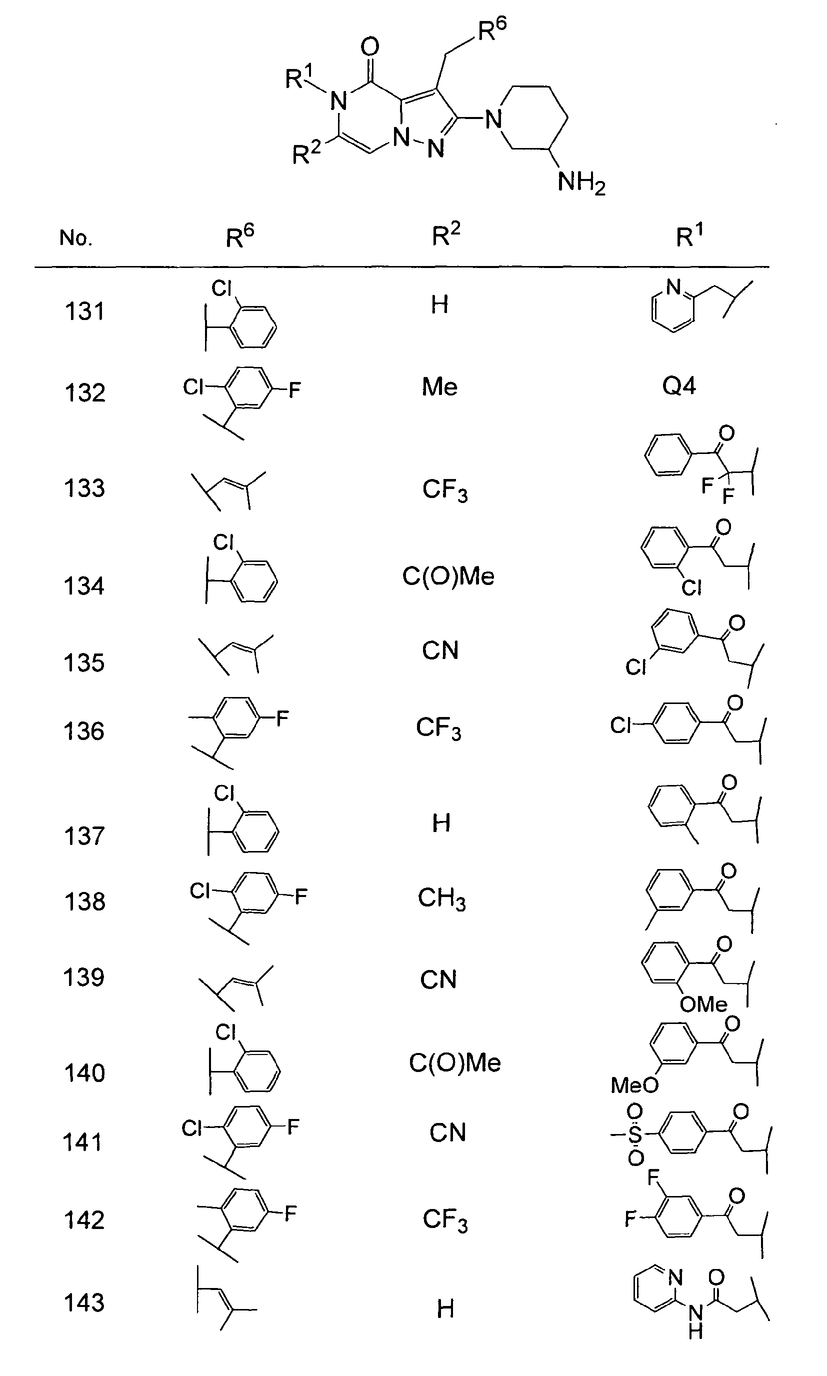

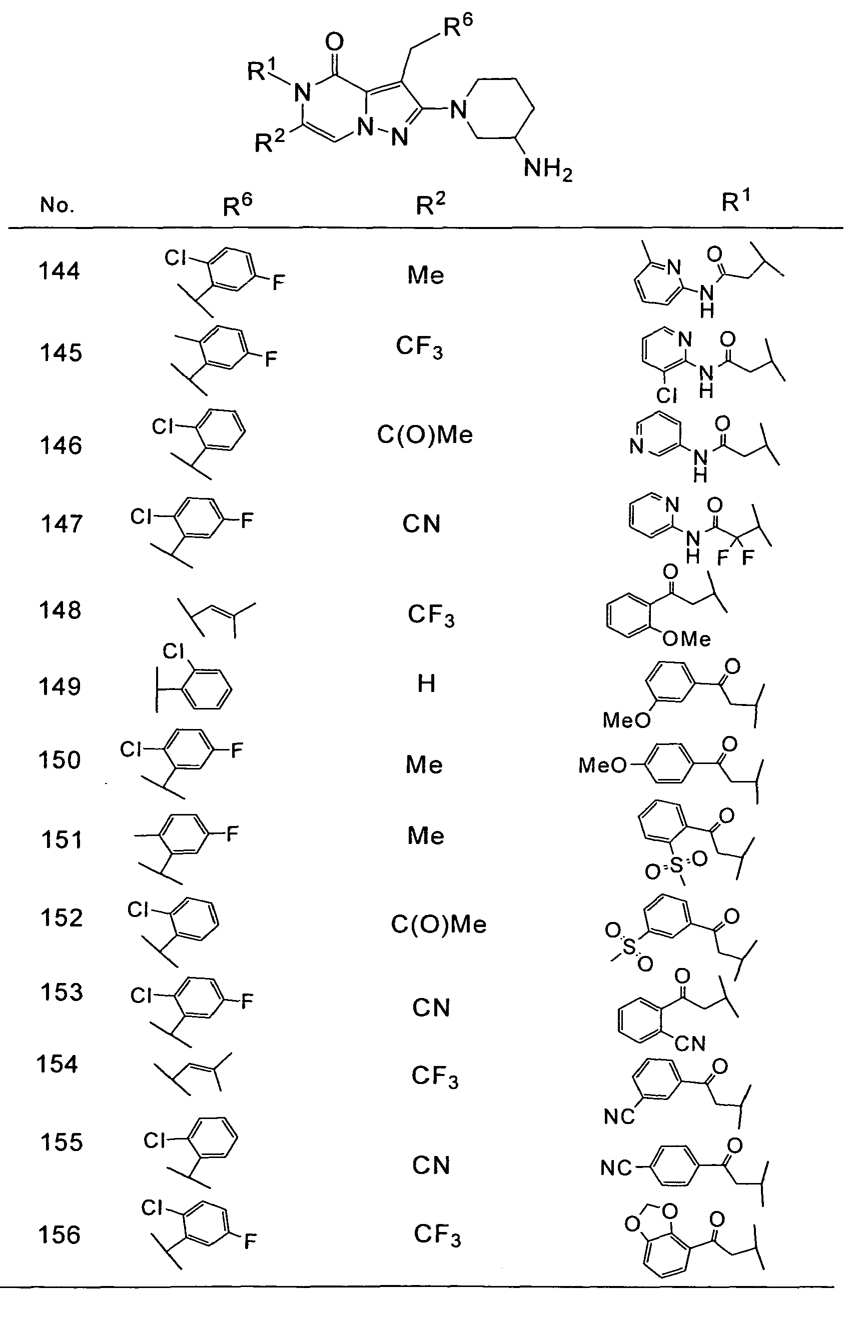

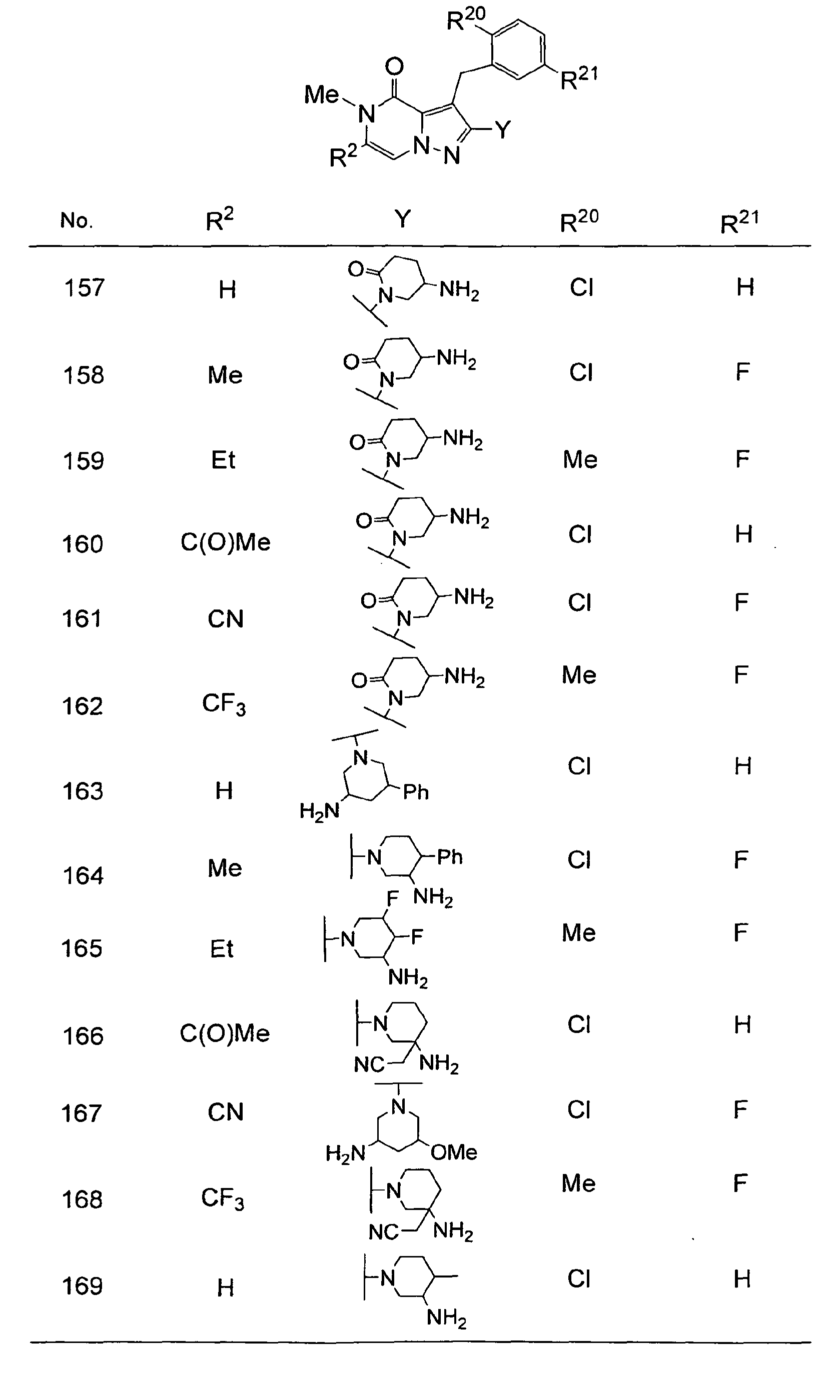

| 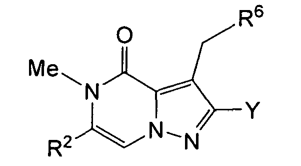 | | | | | | | |
|---|---|---|---|---|---|---|---|
| No. | R⁶ | R² | Y | No. | R⁶ | R² | Y |
| 170 | 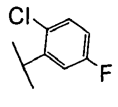 | Me | 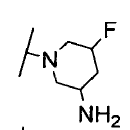 | 176 | 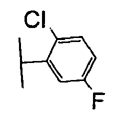 | Me | 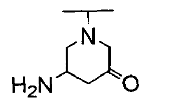 |
| 171 | 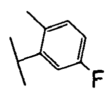 | Et | 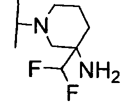 | 177 | 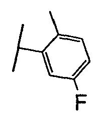 | Me | 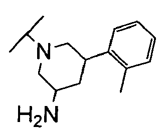 |
| 172 | 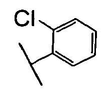 | C(O)Me | 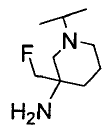 | 178 | 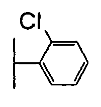 | C(O)Me | 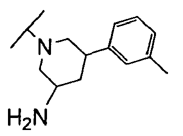 |
| 173 | 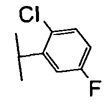 | CN | 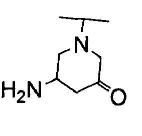 | 179 | 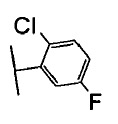 | CN | 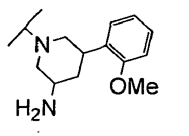 |
| 174 | 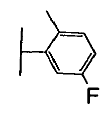 | CF₃ | 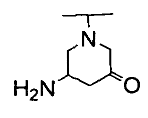 | 180 | 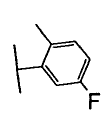 | CF₃ | 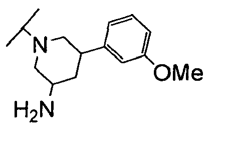 |
| 175 | 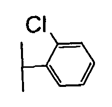 | H | 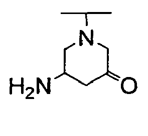 | 181 | 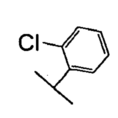 | CN | 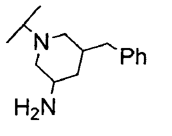 |
| | | | | 182 | 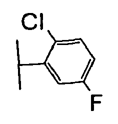 | CF₃ | 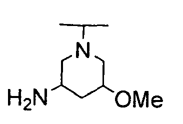 |

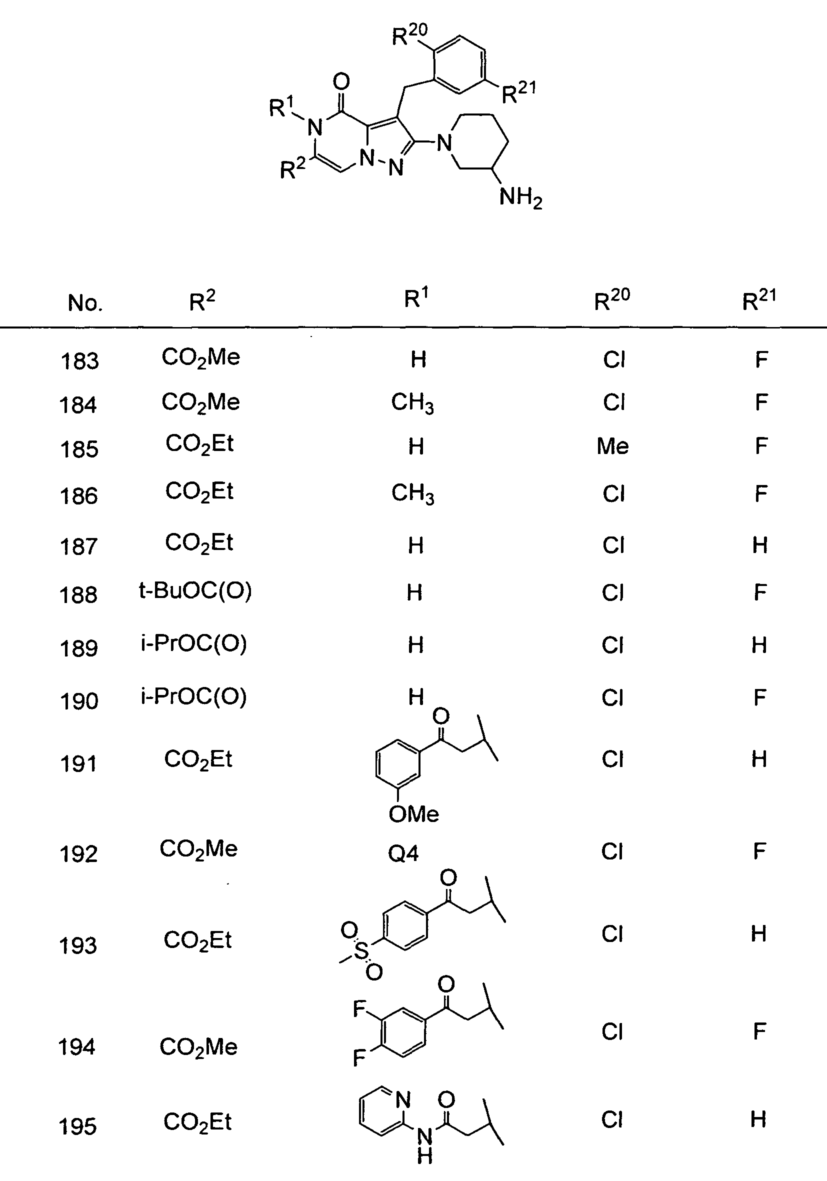

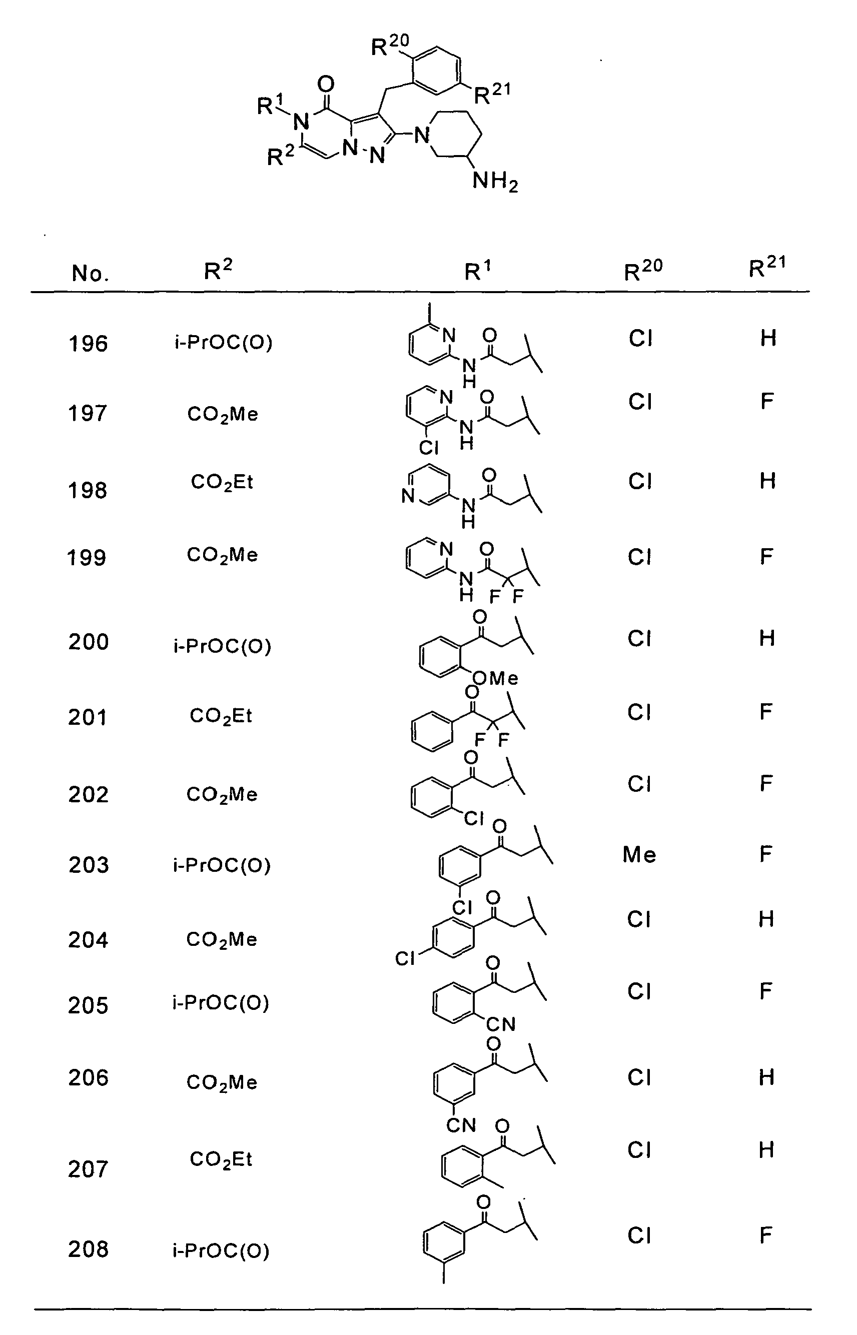

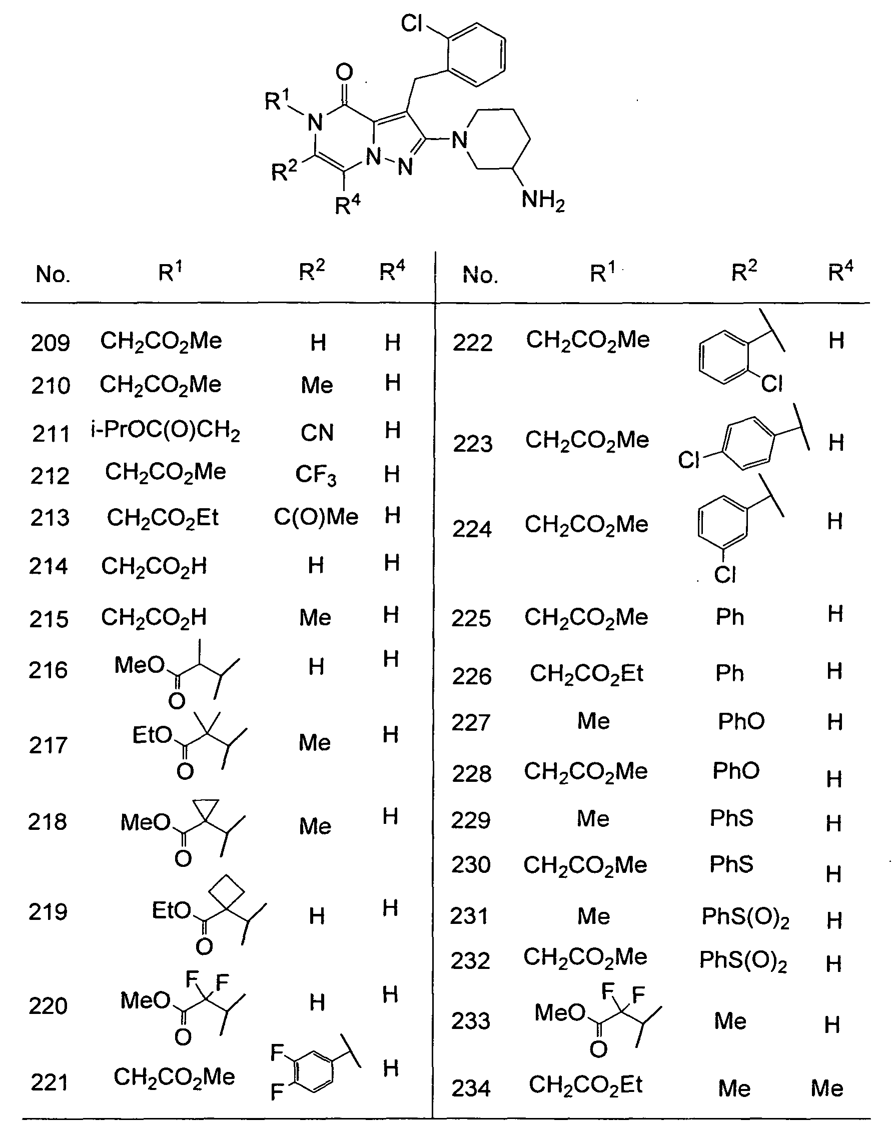

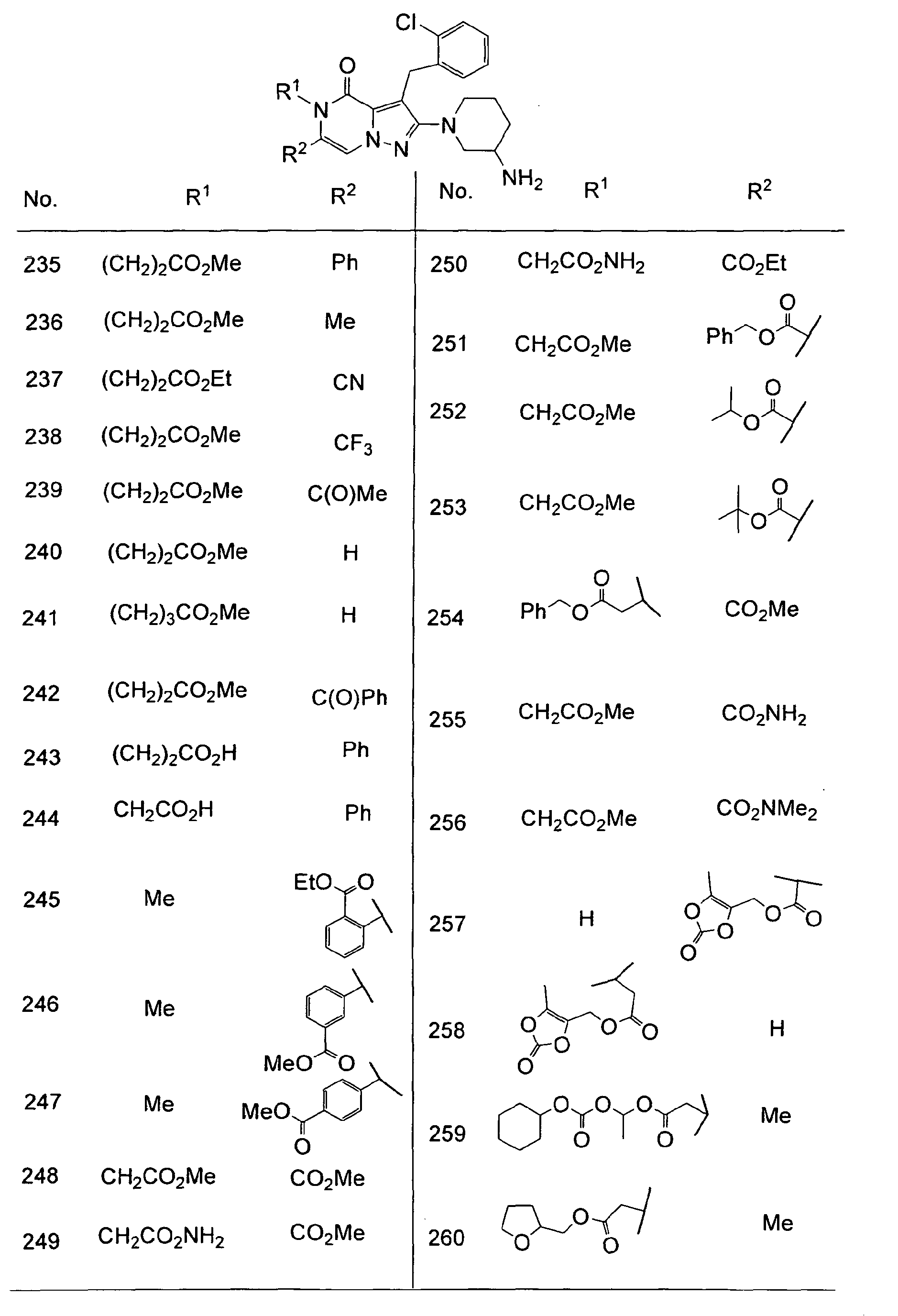

| 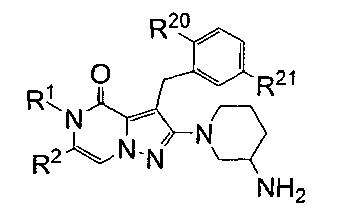 | | | | |
|---|---|---|---|---|
| No. | R¹ | R² | R²⁰ | R²¹ |
| 261 | 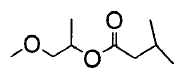 | Ph | Me | H |
| 262 | 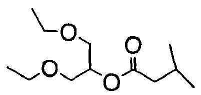 | Me | Cl | H |
| 263 | 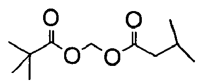 | CN | Me | F |
| 264 | 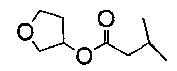 | CF₃ | Cl | F |
| 265 | 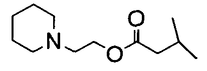 | C(O)Me | Me | H |
| 266 | 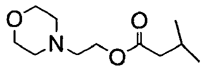 | H | Cl | H |
| 267 | 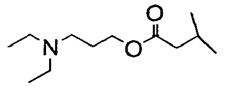 | Ph | Me | F |
| 268 | 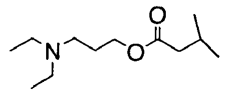 | Me | Cl | F |
| 269 | 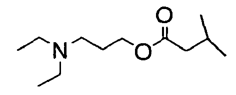 | H | Me | H |
| 270 | 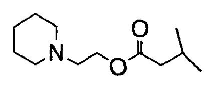 | CN | Cl | H |
| 271 | 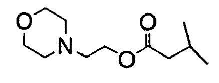 | H | Me | F |
| 272 | H | 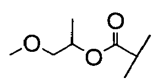 | Cl | F |
| 273 | H | 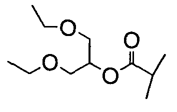 | Me | H |

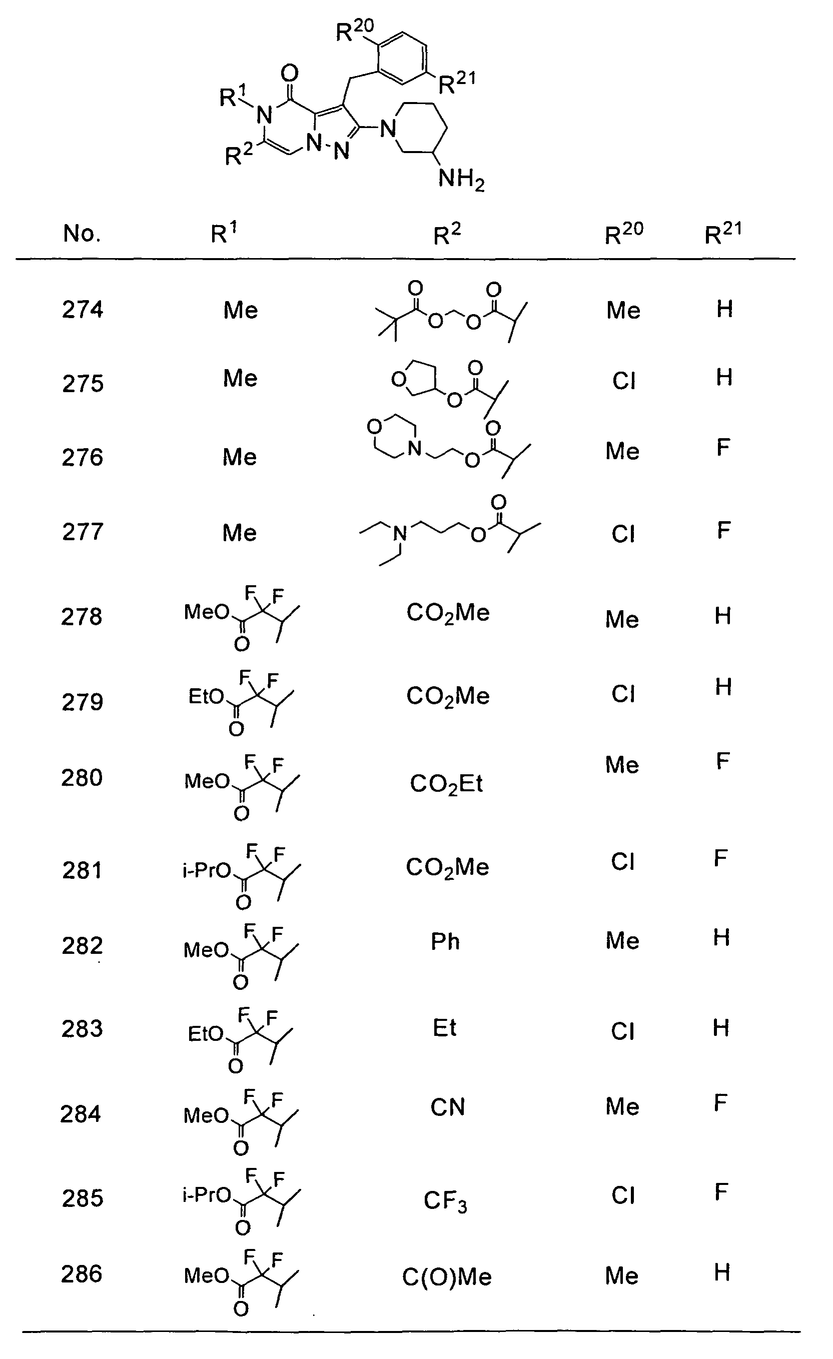

| 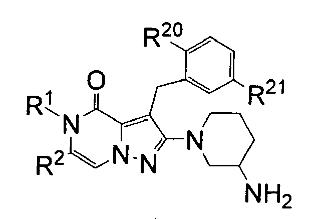 | | | | | | | | | |
|---|---|---|---|---|---|---|---|---|---|
| No. | R² | R¹ | R²⁰ | R²¹ | No. | R² | R¹ | R²⁰ | R²¹ |
| 287 | CO₂Me | H | Me | H | 314 | CO₂Me | H | Br | F |
| 288 | COOH | H | Me | H | 315 | COOH | Me | Br | F |
| 289 | CO₂Et | Me | Me | H | 316 | CO₂Et | Me | Br | F |
| 290 | COOH | H | Cl | F | | | | | |
| 291 | COOH | H | Cl | H | 317 | COOH | H | Cl | Cl |
| 292 | COOH | H | CN | H | 318 | CO₂Me | H | Cl | Cl |
| 293 | CO₂Me | H | CN | H | 319 | COOH | H | Me | Cl |
| 294 | COOH | H | CN | F | 320 | CO₂Me | H | Me | CI |
| 295 | CO₂Et | H | CN | F | 321 | COOH | H | CN | Cl |
| 296 | COOH | H | OMe | H | 322 | CO₂Me | H | CN | CI |
| 297 | CO₂Me | H | OMe | H | 323 | COOH | H | OMe | CI |
| 298 | COOH | H | OMe | F | 324 | CO₂Me | H | OMe | Cl |
| 299 | CO₂Me | H | OMe | F | 325 | CO₂Et | H | OMe | Cl |
| 300 | COOH | Me | CF₃ | H | 326 | CO₂cycpro | H | Cl | F |
| 301 | i-PrOC(O) | H | CF₃ | H | 327 | CO₂cycbu | H | Cl | F |
| 302 | COOH | H | CF₃ | F | 328 | CO₂CH(Et)(Me) | H | Cl | F |
| 303 | CO₂Me | H | CF₃ | F | 329 | CO₂CH₂cycpro | H | Cl | F |
| 304 | COOH | H | CF₃ | Cl | 330 | CO₂CH₂CH(Me)₂ | H | Cl | F |
| 305 | CO₂Me | H | CF₃ | Cl | 331 | CO₂Et | H | OMe | F |
| 306 | COOH | H | CHF₂ | H | 332 | i-PrOC(O) | H | OMe | F |
| 307 | C02Et | H | CHF₂ | H | | | | | |
| 308 | COOH | H | OCHF₂ | H | 333 | CO₂cycpro | H | OMe | F |
| 309 | CO₂Me | H | OCHF₂ | H | 334 | CO₂CH₂CH(Me)₂ | H | OMe | F |
| 310 | COOH | H | Br | H | 335 | CO₂Et | H | OMe | H |
| 311 | CO₂Me | H | Br | H | 336 | i-PrOC(O) | H | OMe | H |
| 312 | CO₂Et | H | Br | H | 337 | CO₂cycpro | H | OMe | H |
| 313 | COOH | H | Br | F | 338 | CO₂CH₂CH(Me)₂ | H | OMe | H |

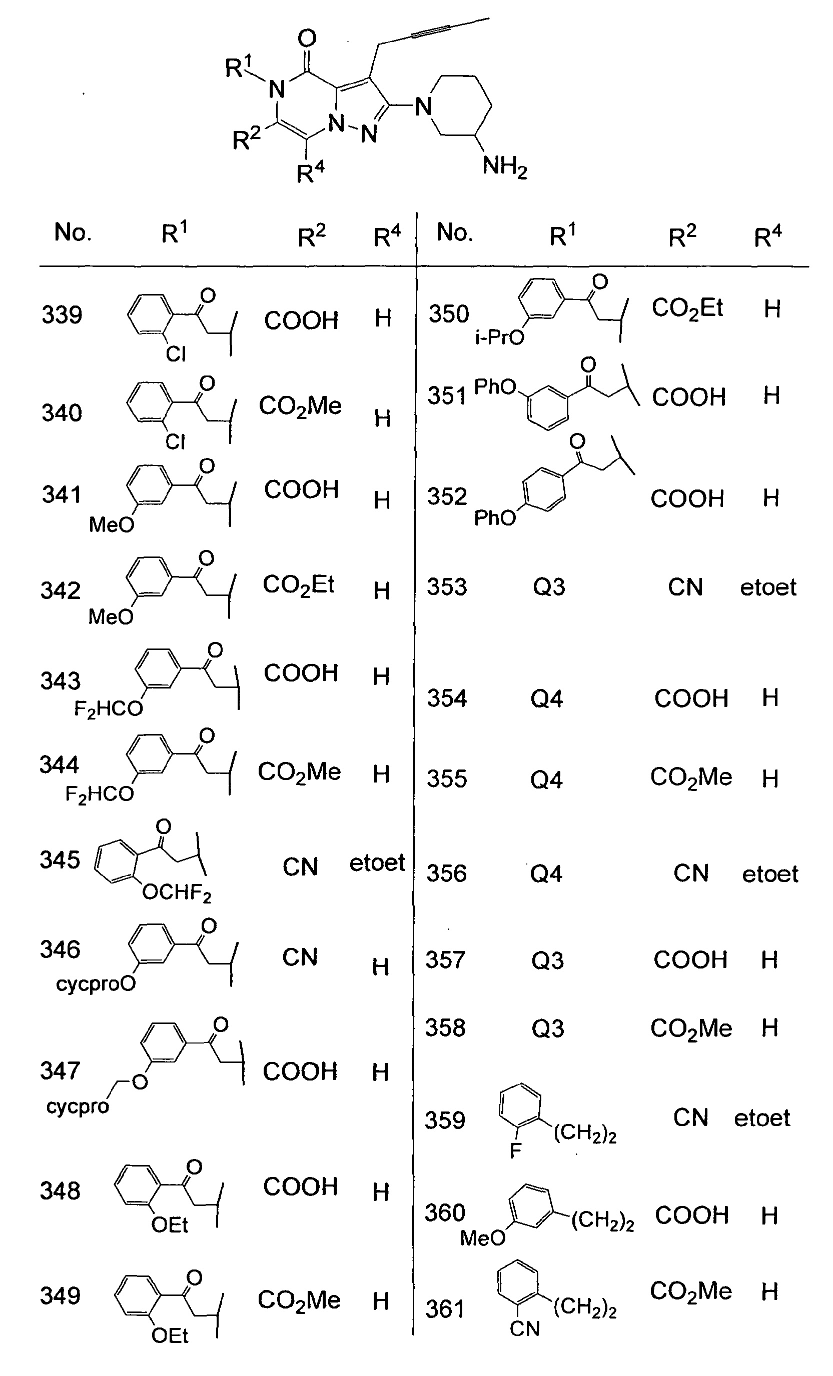

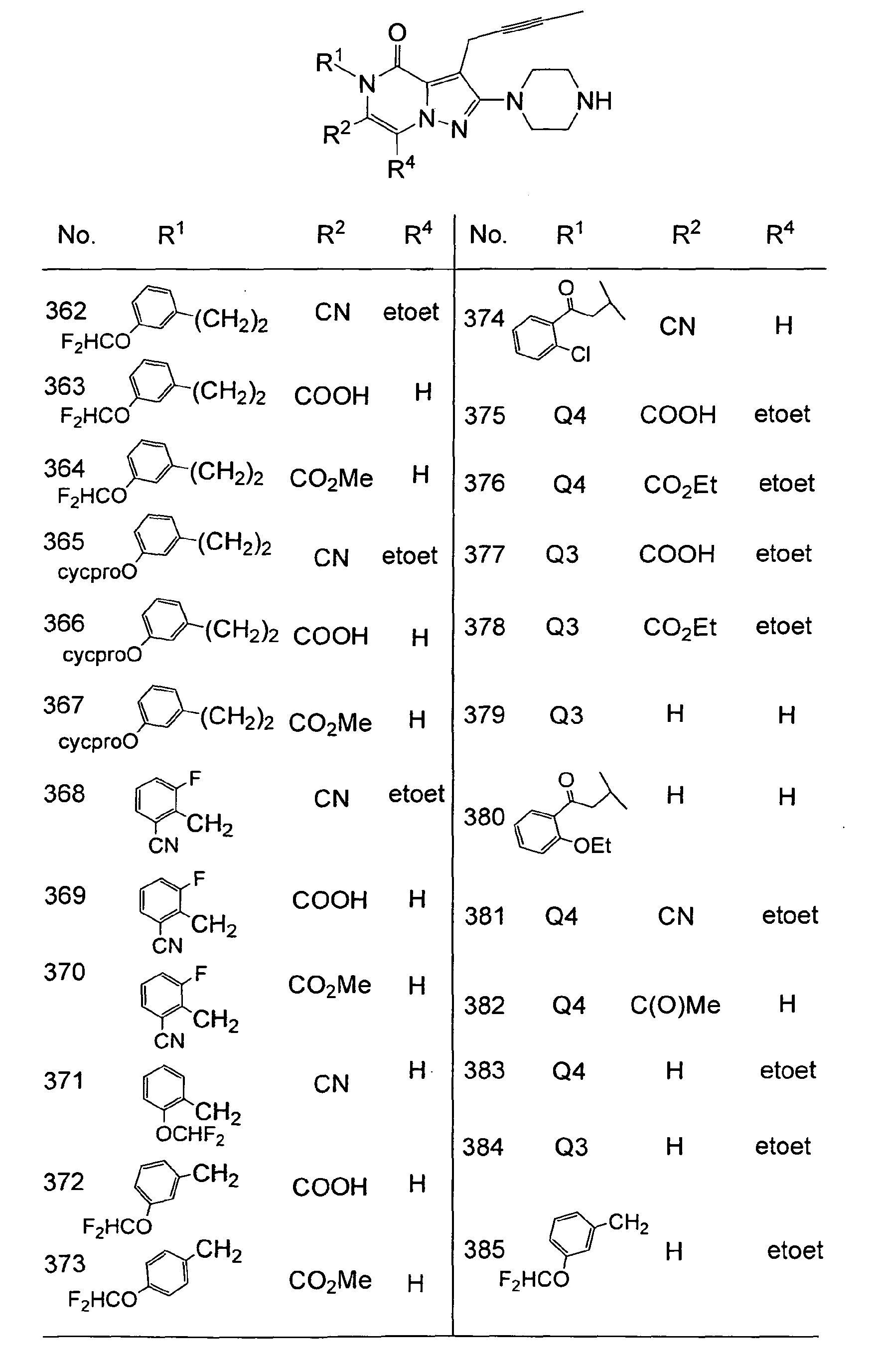

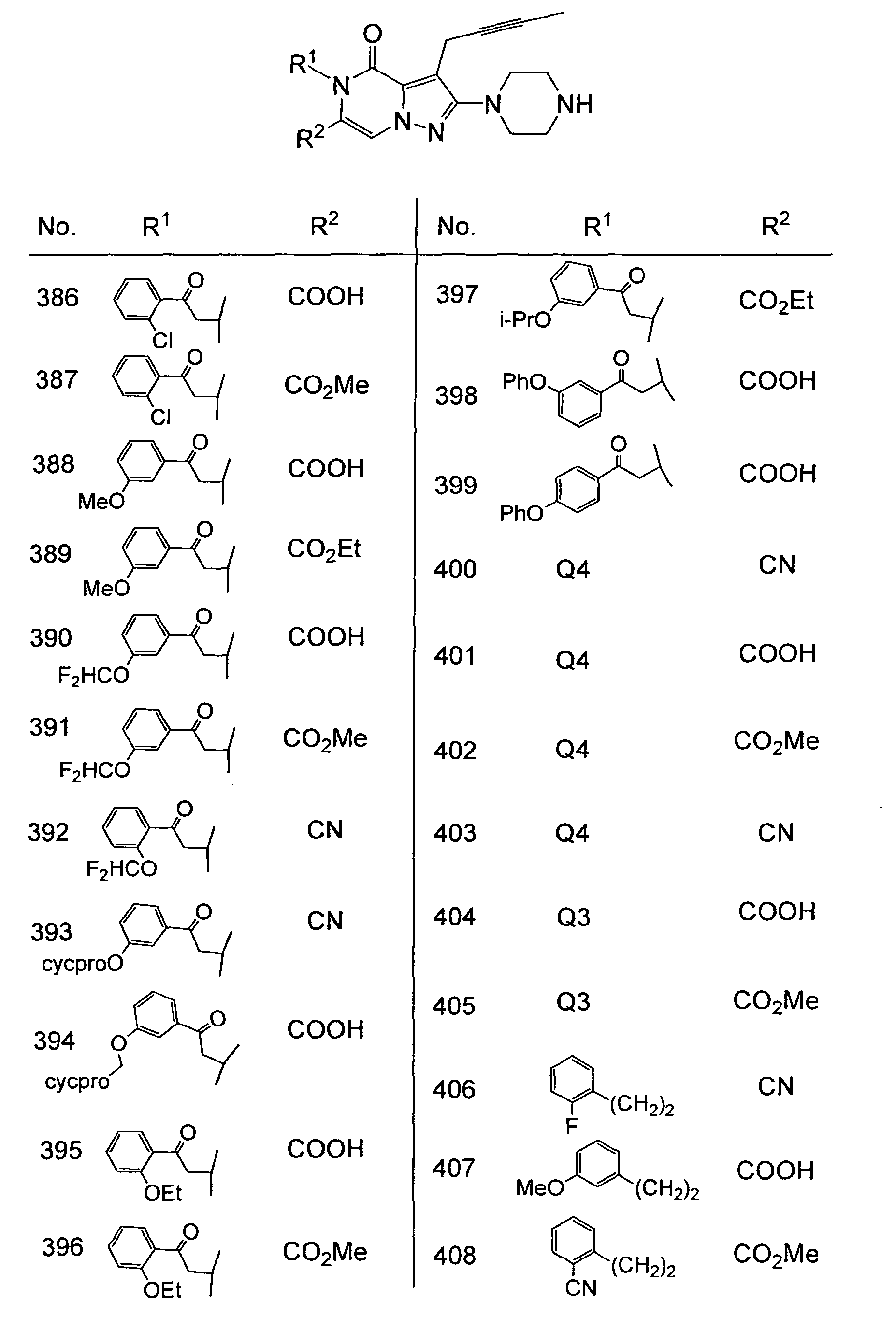

| 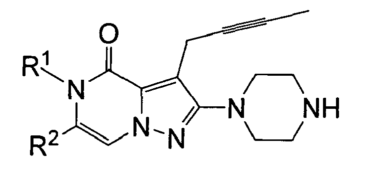 | | | | | |
|---|---|---|---|---|---|
| No. | R¹ | R² | No. | R¹ | R² |
| 409 | Ph(CH₂)₂ | CN | 421 | Q4 | Me |
| 410 | Ph(CH₂)₂ | COOH | 422 | Q3 | etoet |
| 411 | Ph(CH₂)₂ | CO₂Me | 423 | Q4 | Me |
| 412 | 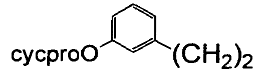 | CN | 424 | Q3 | etoet |
| 413 | 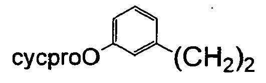 | i-PrOC(O) | 425 | Q3 | C(O)Me |
| 414 | 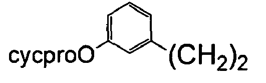 | CO₂Et | 426 | Q4 | CF₃ |
| 415 | 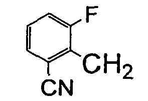 | CN | 427 | Q3 | CF₃ |
| 416 | 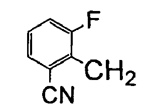 | COOH | 428 | Q4 | CHF₂ |
| 417 | 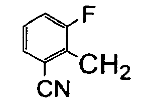 | CO₂Et | 429 | Q4 | 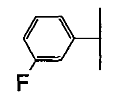 |
| 418 | 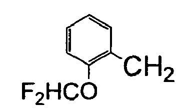 | CN | 430 | 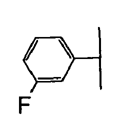 | CN |
| 419 | 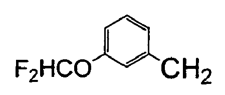 | i-PrOC(O) | 431 | Q4 | C(O)Ph |
| 420 | 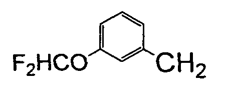 | COOH | 432 | Q3 | cycpro |

| 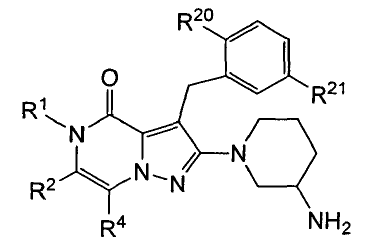 | | | | | |
|---|---|---|---|---|---|
| No. | R¹ | R² | R⁴ | R²⁰ | R²¹ |
| 433 | H | CO₂Me | CI | Cl | F |
| 434 | 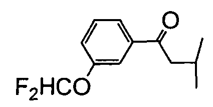 | CO₂Me | Cl | Cl | F |
| 435 | H | COOH | Cl | Cl | F |
| 436 | 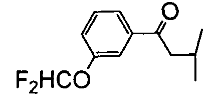 | COOH | Cl | Cl | F |
| 437 | Q3 | CO₂Me | Me | CN | H |
| 438 | Q3 | CO₂Me | Me | Cl | F |
| 439 | H | CO₂Me | Me | Cl | F |
| 440 | 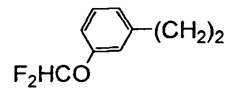 | CN | Cl | Cl | F |
| 441 | H | COOH | etoet | Cl | F |
| 442 | H | CO₂Et | etoet | Cl | F |
| 443 | H | COOH | etoet | OMe | H |
| 444 | H | CO₂Et | etoet | OMe | H |
| 445 | H | CN | etoet | Cl | F |

| 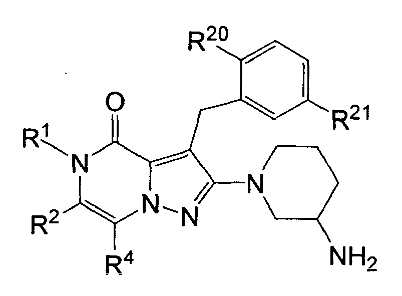 | | | | | |
|---|---|---|---|---|---|
| No. | R¹ | R² | R⁴ | R²⁰ | R²¹ |
| 446 | H | CO₂Me | CH₂CO₂Me | Cl | F |
| 447 | 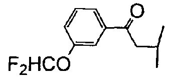 | CO₂Me | CH₂CO₂Et | Cl | F |
| 448 | H | COOH | CH₂CO₂H | Cl | F |
| 449 | 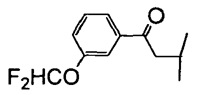 | COOH | MeNHC(O)CH₂ | Cl | F |
| 450 | Me | CO₂Me | 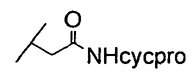 | CN | H |
| 451 | Q3 | CO₂Me | 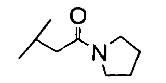 | Cl | F |
| 452 | H | CO₂Me | 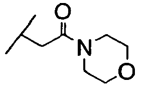 | Cl | F |
| 453 | 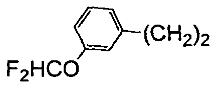 | CN | CH₂C(O)NHPh | Cl | F |
| 454 | 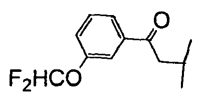 | COOH | 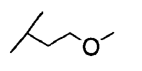 | Cl | F |
| 455 | Me | CO₂Me | etoet | CN | H |
| 456 | Q3 | CO₂Me | 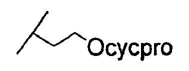 | Cl | F |
| 457 | H | CO₂Me | 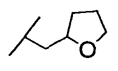 | Cl | F |
| 458 | 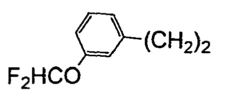 | CN | 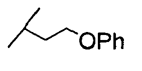 | Cl | F |

| 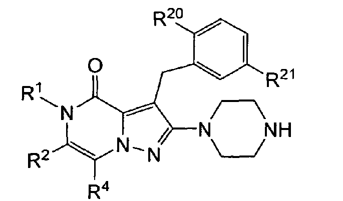 | | | | | |
|---|---|---|---|---|---|
| No. | R¹ | R² | R⁴ | R²⁰ | R²¹ |
| 459 | H | CO₂Me | CH₂CO₂Me | Cl | F |
| 460 | 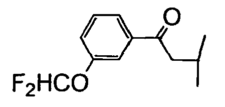 | CO₂Me | CH₂CO₂Et | Cl | F |
| 461 | H | COOH | CH₂CO₂H | Cl | F |
| 462 | 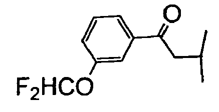 | COOH | CH₂C(O)NHMe | Cl | F |
| 463 | Me | CO₂Me | 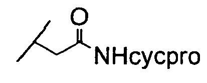 | CN | H |
| 464 | Q3 | CO₂Me | 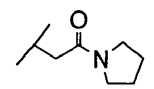 | Cl | F |
| 465 | H | CO₂Me | 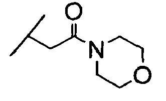 | Cl | F |
| 466 | 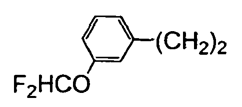 | CN | CH₂C(O)NHPh | Cl | F |
| 467 | 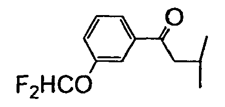 | COOH | 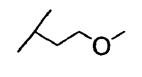 | Cl | F |
| 468 | Me | CO₂Me | etoet | CN | H |
| 469 | Q3 | CO₂Me | 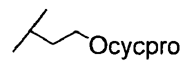 | Cl | F |
| 470 | H | CO₂Me | 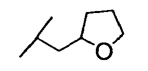 | Cl | F |
| 471 | 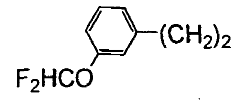 | CN | 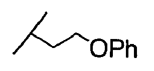 | Cl | F |

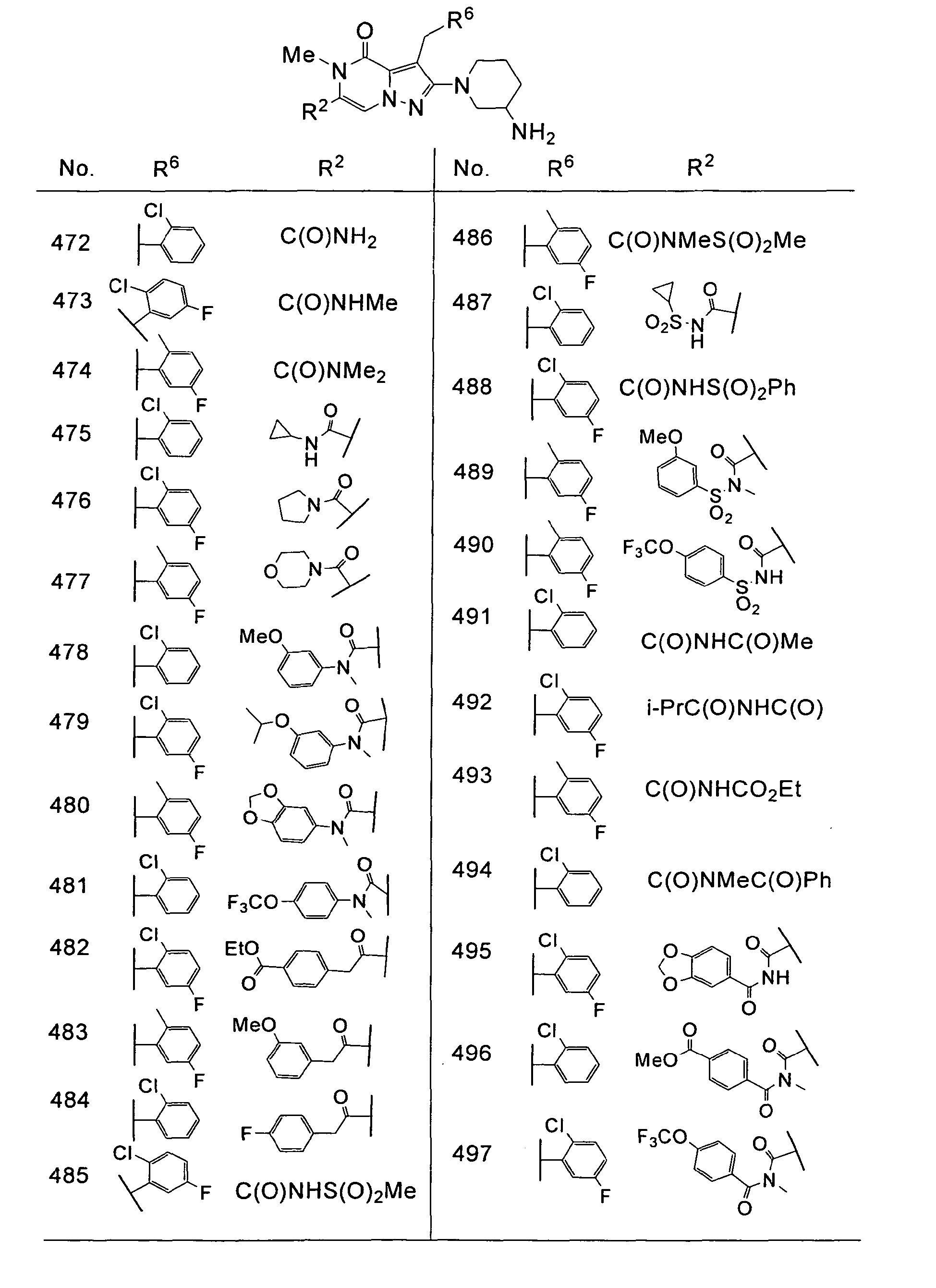

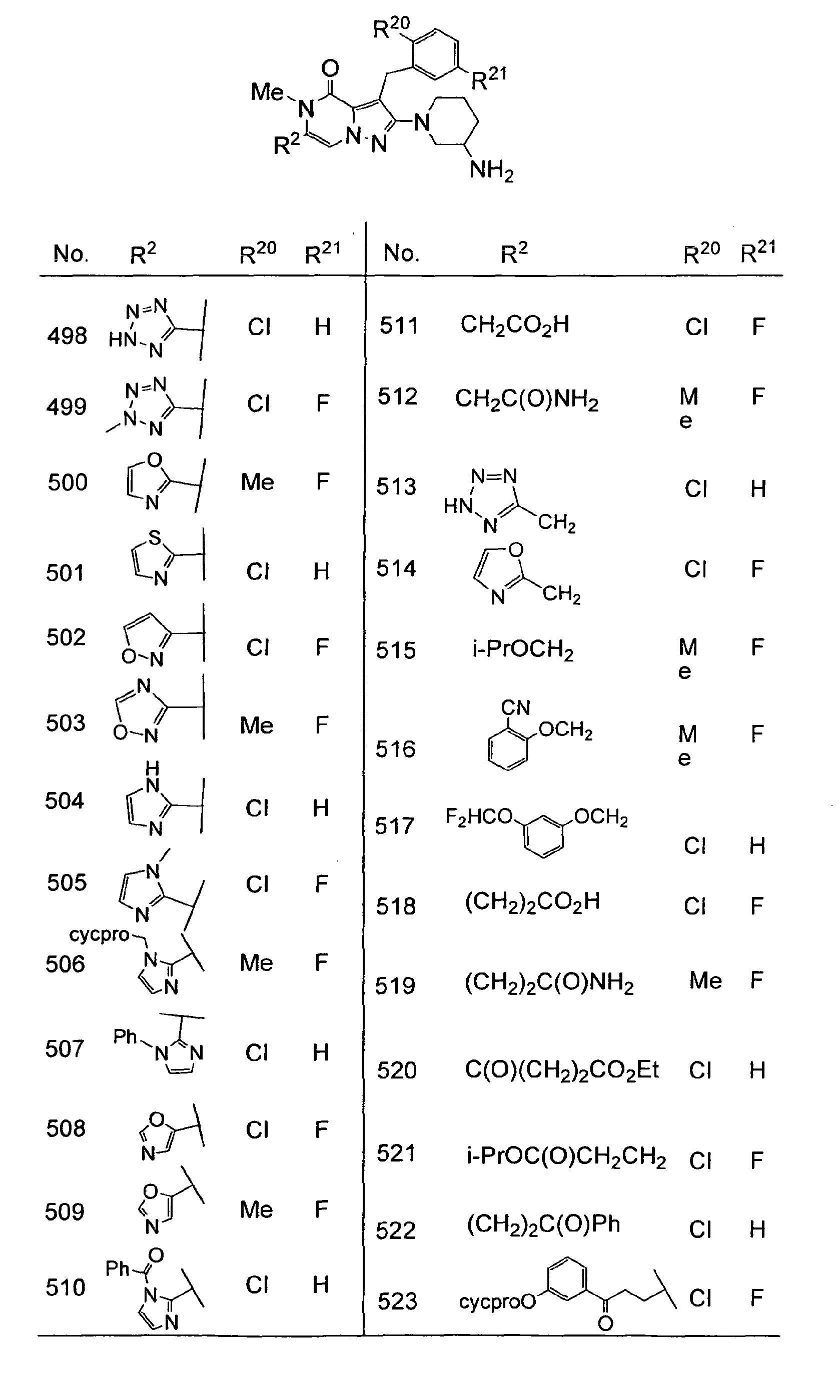

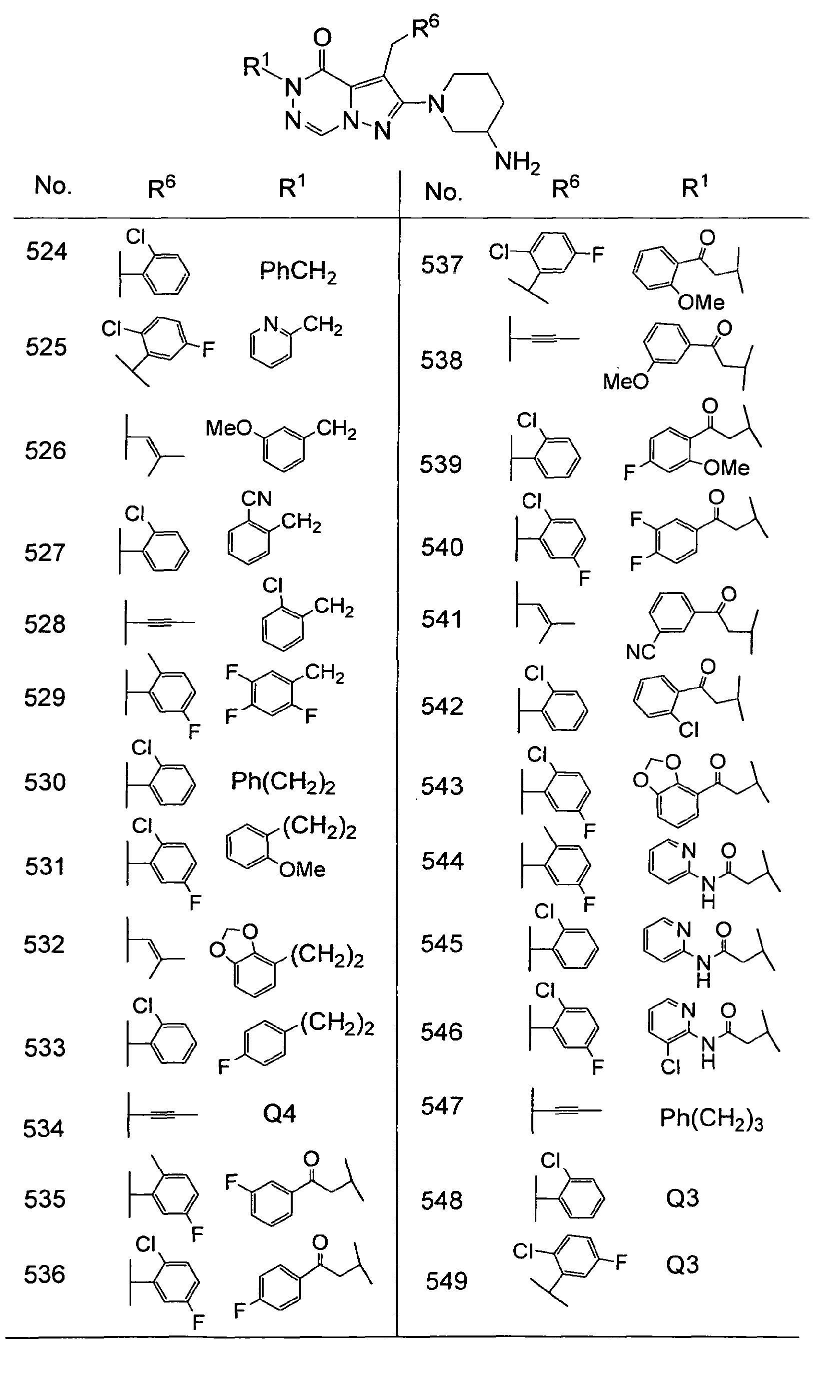

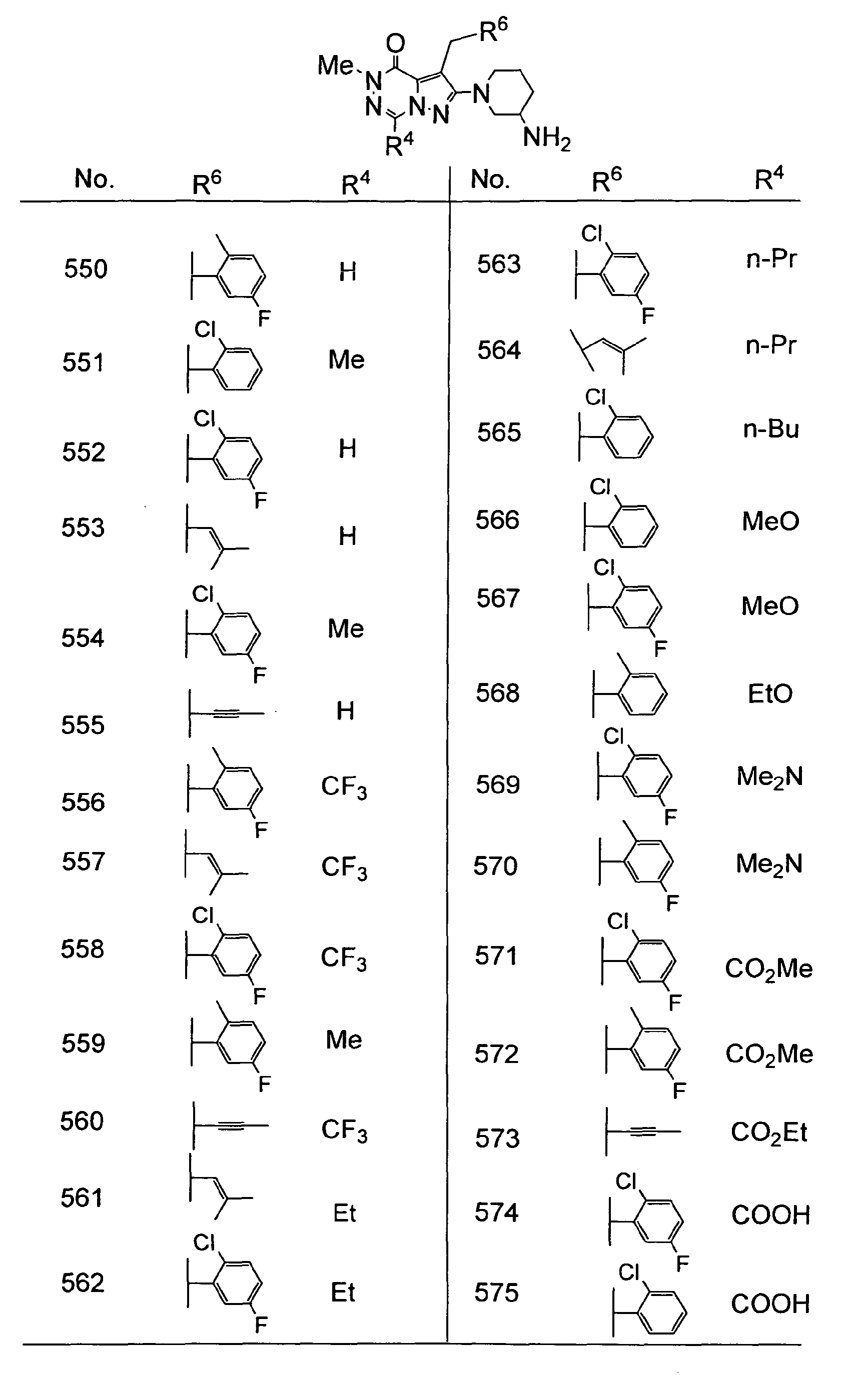

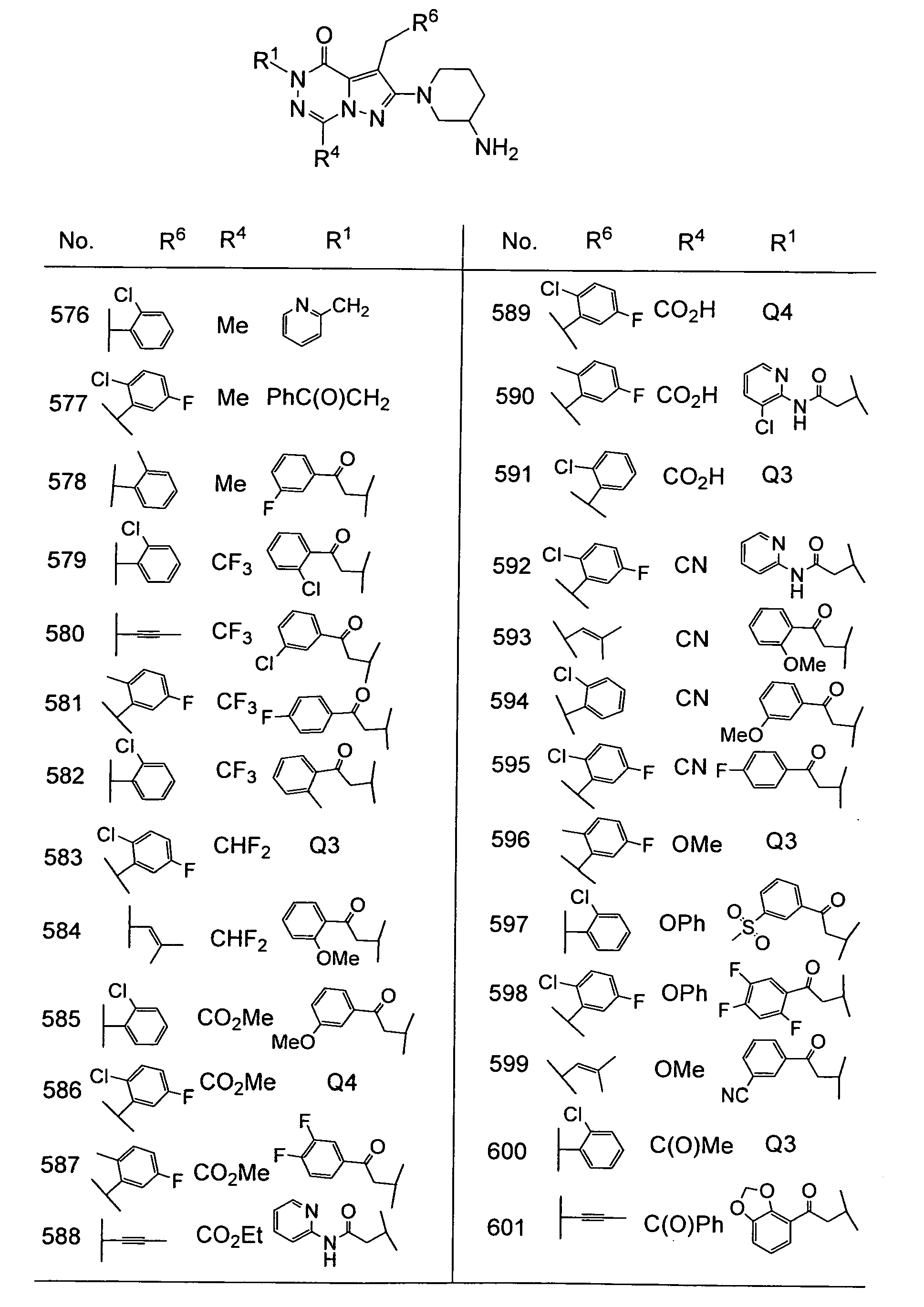

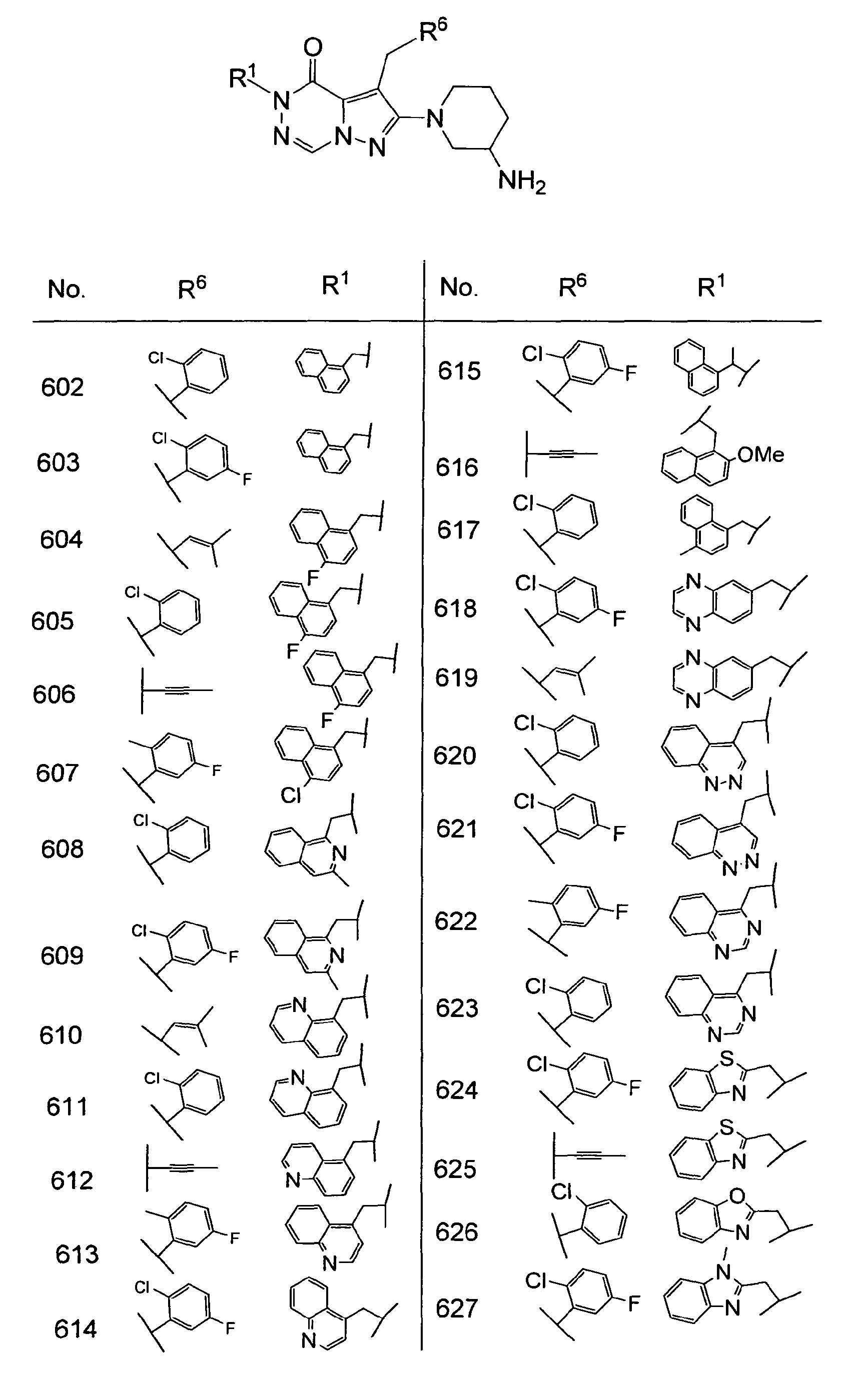

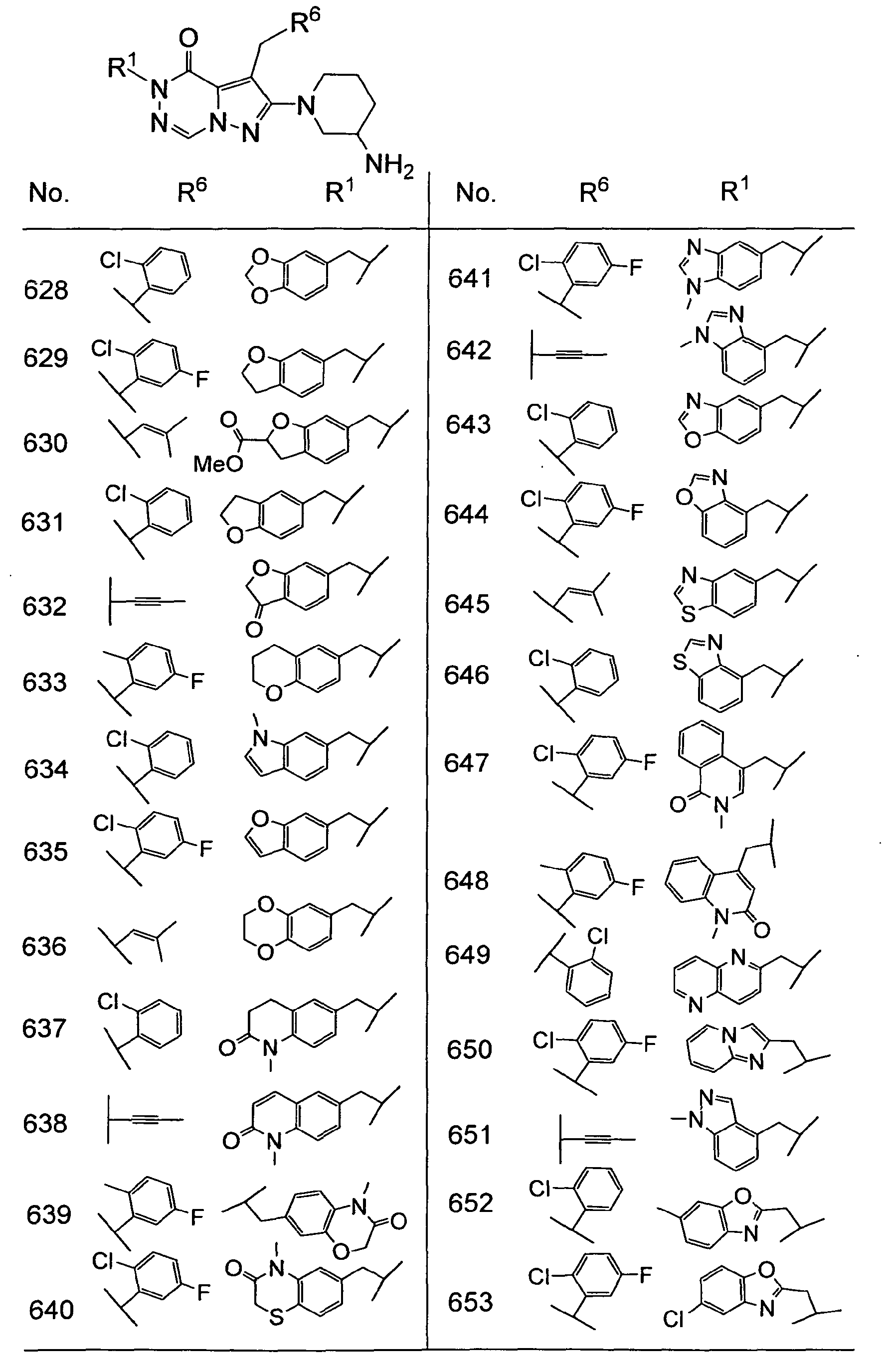

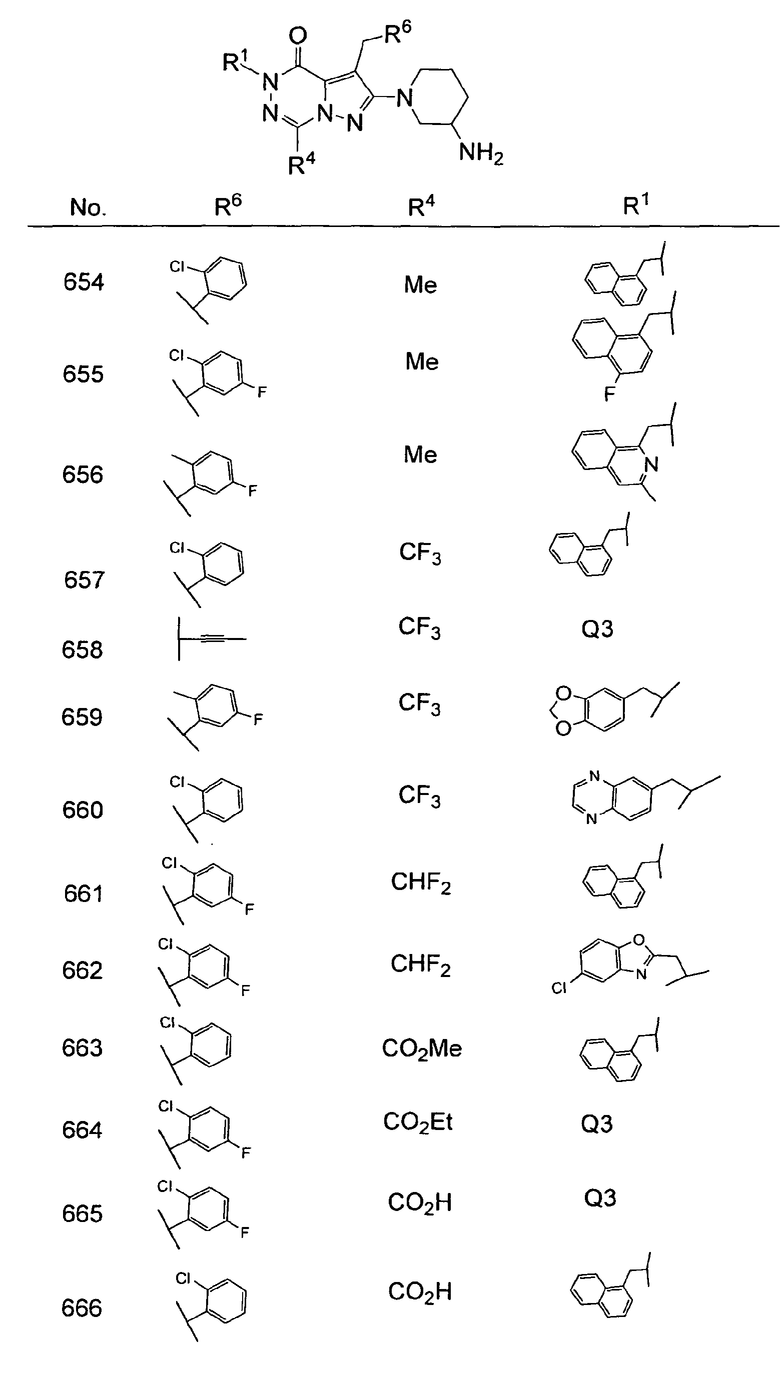

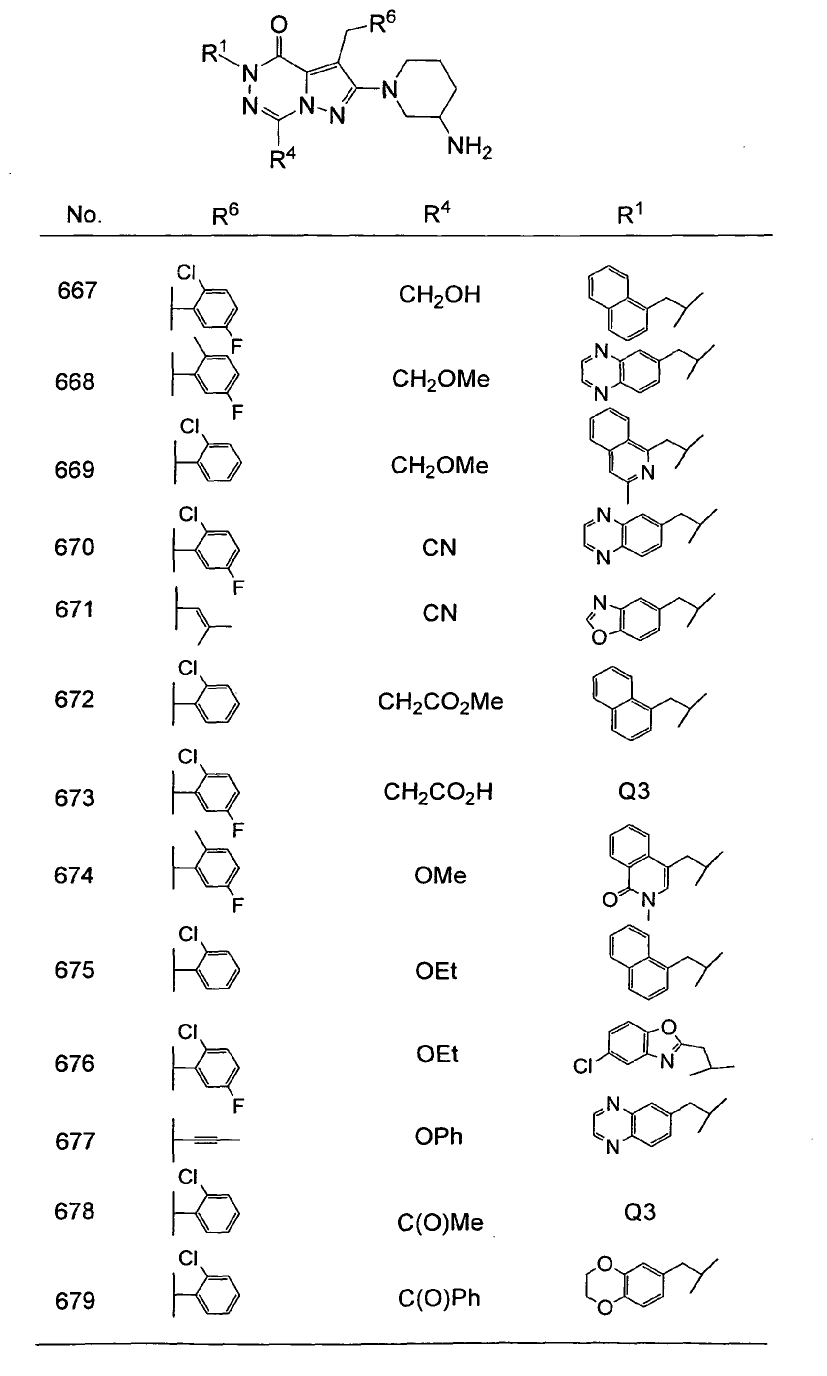

| 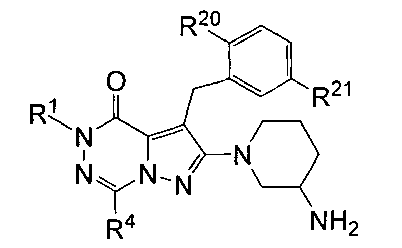 | | | | |
|---|---|---|---|---|
| No. | R¹ | R⁴ | R²⁰ | R²¹ |
| 680 | H | CH₂CO₂Me | CI | F |
| 681 | Q4 | CH₂CO₂Et | CI | F |
| 682 | Me | CH₂CO₂H | Cl | F |
| 683 | 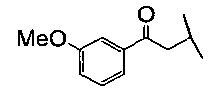 | CH₂C(O)NHMe | Cl | F |
| 684 | 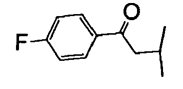 | 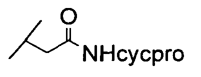 | CN | H |
| 685 | Q3 | 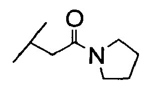 | Cl | F |
| 686 | 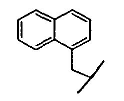 | 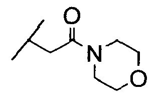 | Cl | F |
| 687 | (CH₂)₂Ph | CH₂C(O)NHPh | Cl | F |
| 688 | 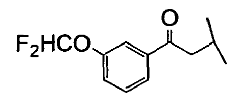 | 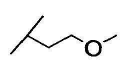 | Cl | F |
| 689 | 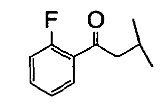 | etoet | CN | F |
| 690 | Q3 | 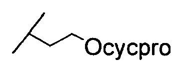 | CN | H |
| 691 | 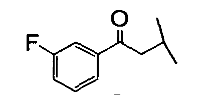 | 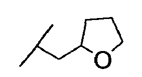 | Cl | F |
| 692 | 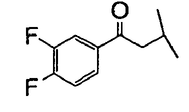 | 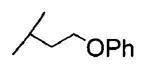 | Cl | F |

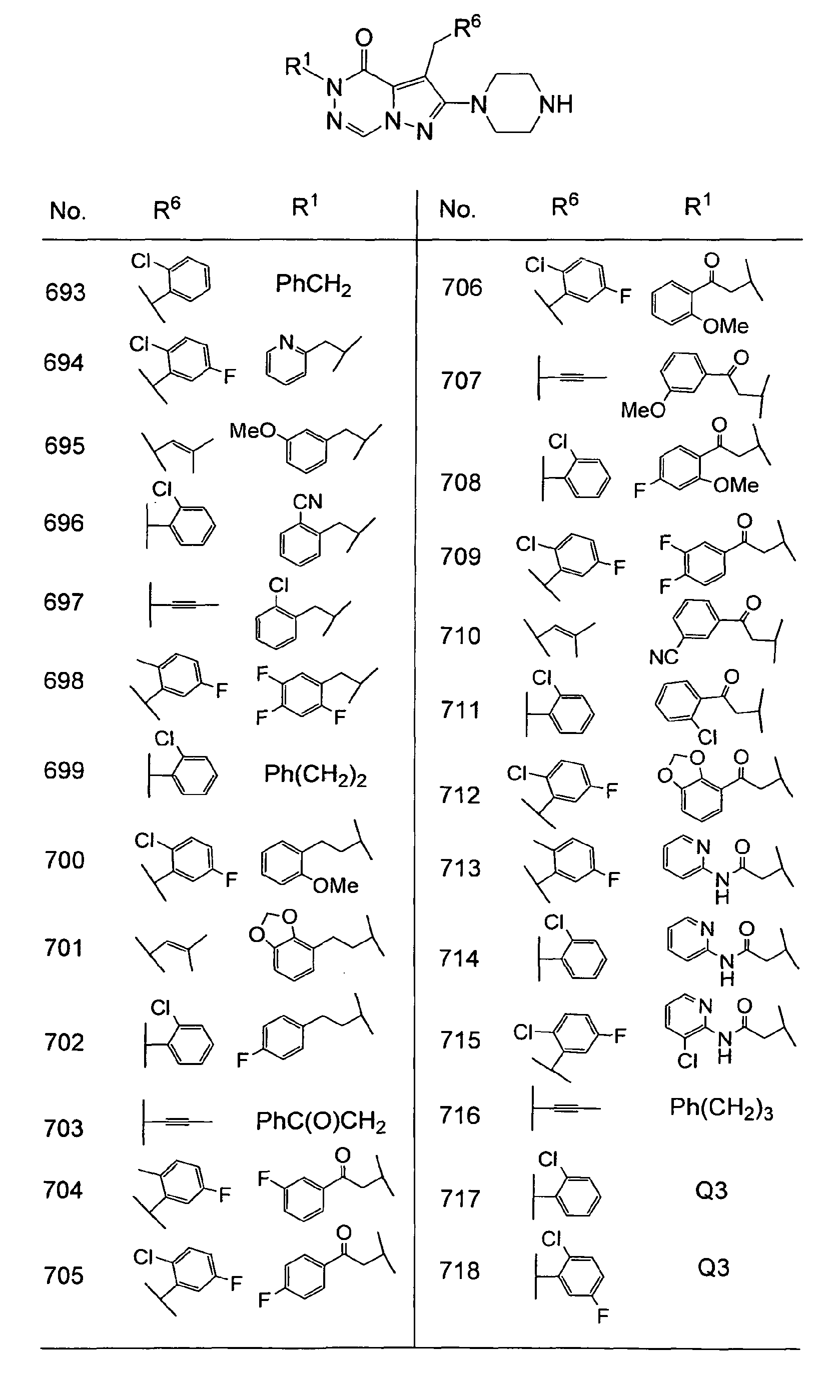

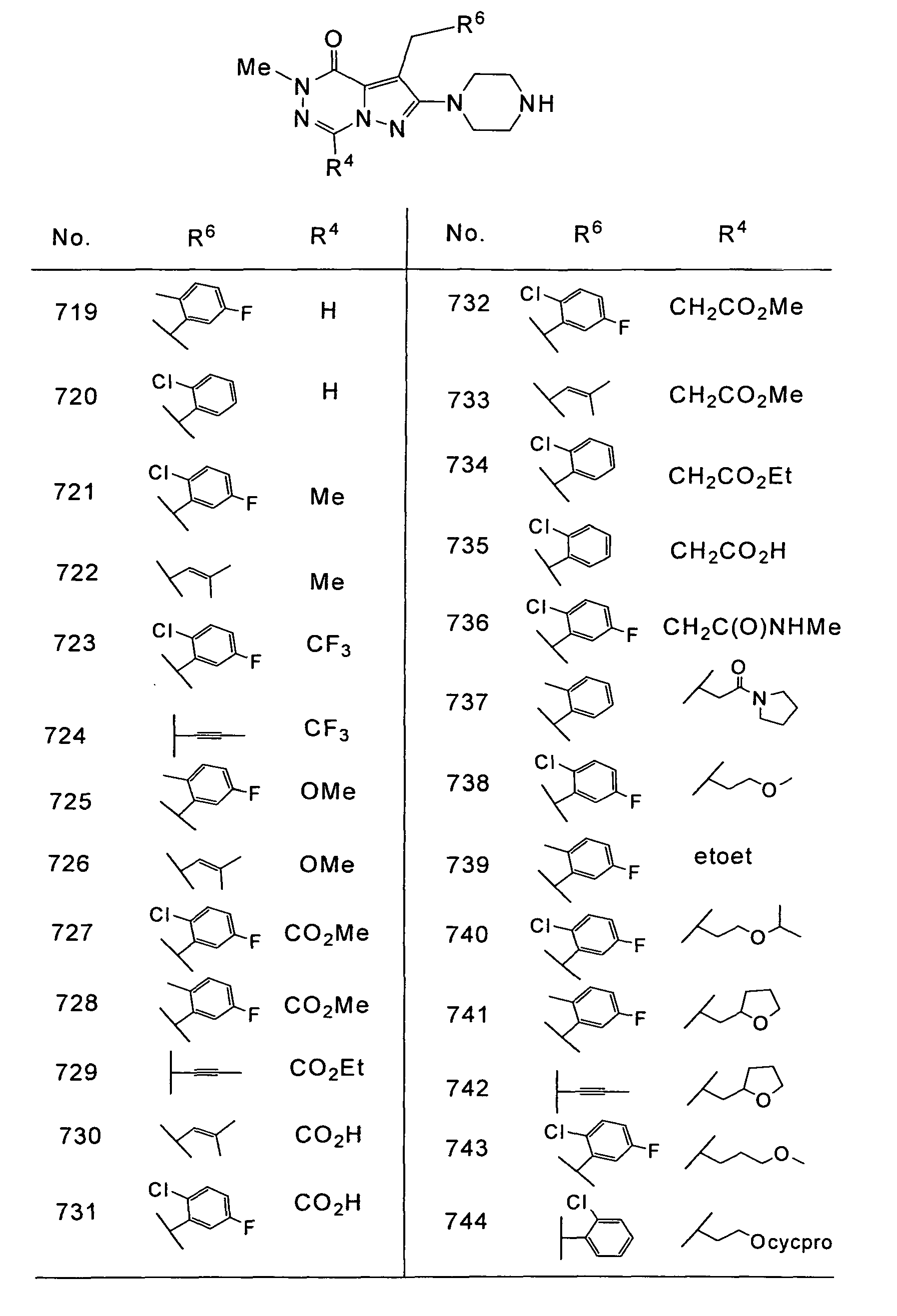

| 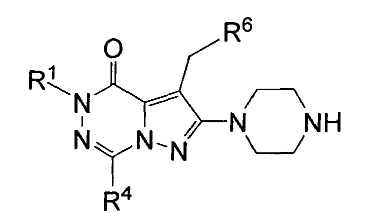 | | | |
|---|---|---|---|
| No. | R⁶ | R⁴ | R¹ |
| 745 | 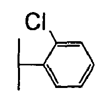 | Me | 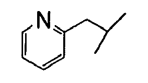 |
| 746 | 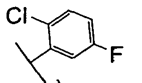 | Me | Q4 |
| 747 | 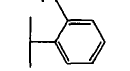 | CF₃ | 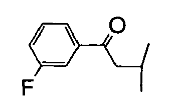 |
| 748 | 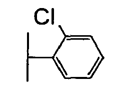 | CF₃ | 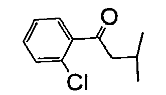 |
| 749 | 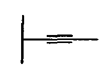 | CO₂Me | 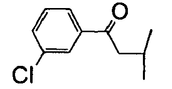 |
| 750 | 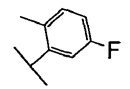 | CO₂Me | 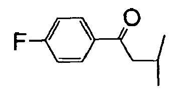 |
| 751 | 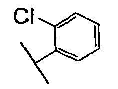 | CO₂Et | 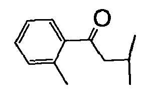 |
| 752 | 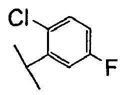 | CO₂H | Q3 |
| 753 | 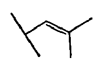 | CN | 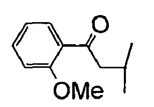 |
| 754 | 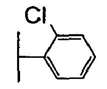 | OMe | 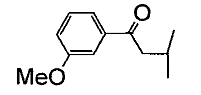 |
| 755 | 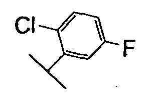 | OPh | Q4 |
| 756 | 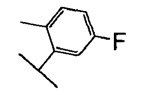 | C(O)Me | 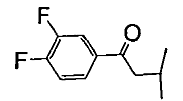 |
| 757 | 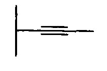 | C(O)Ph | 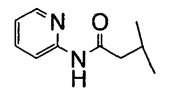 |

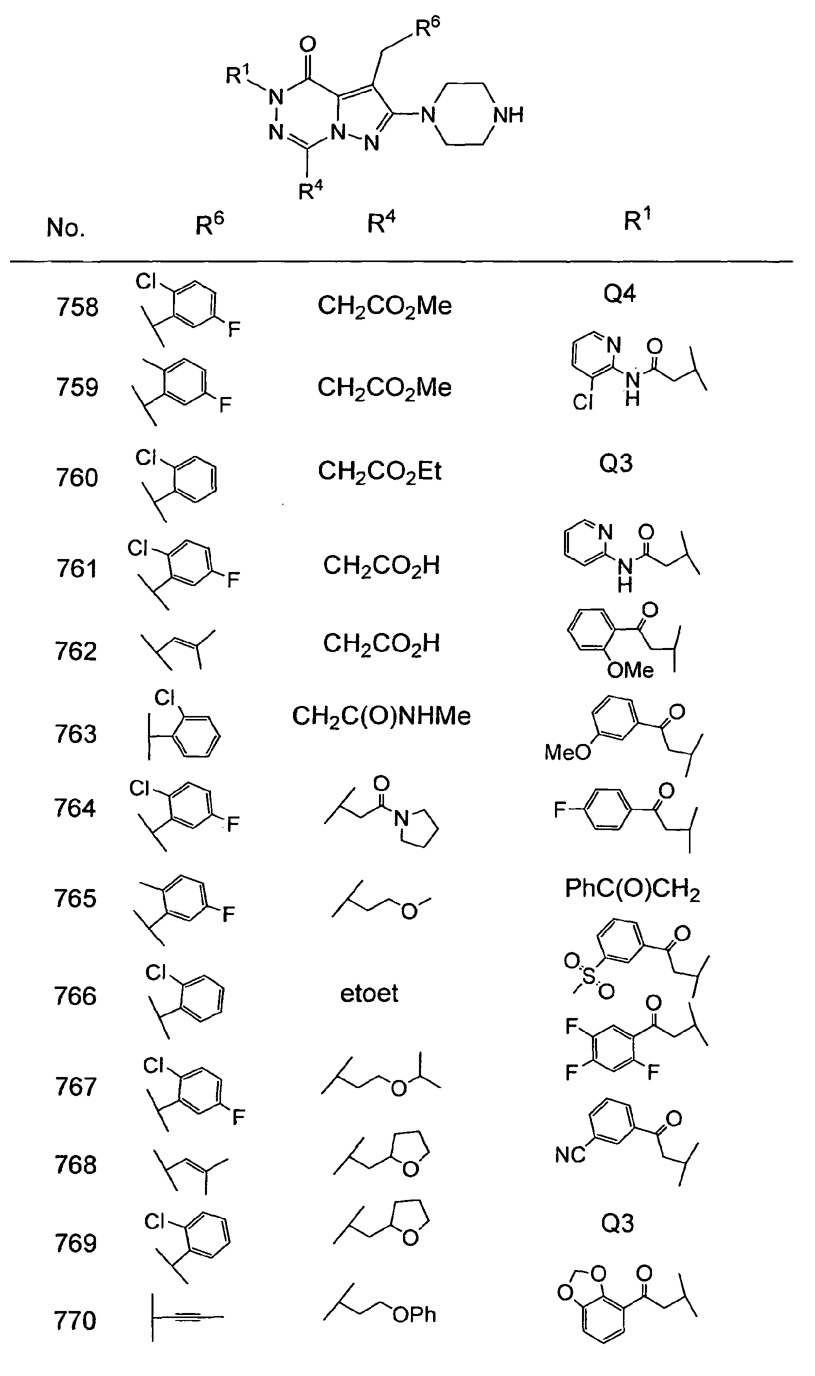

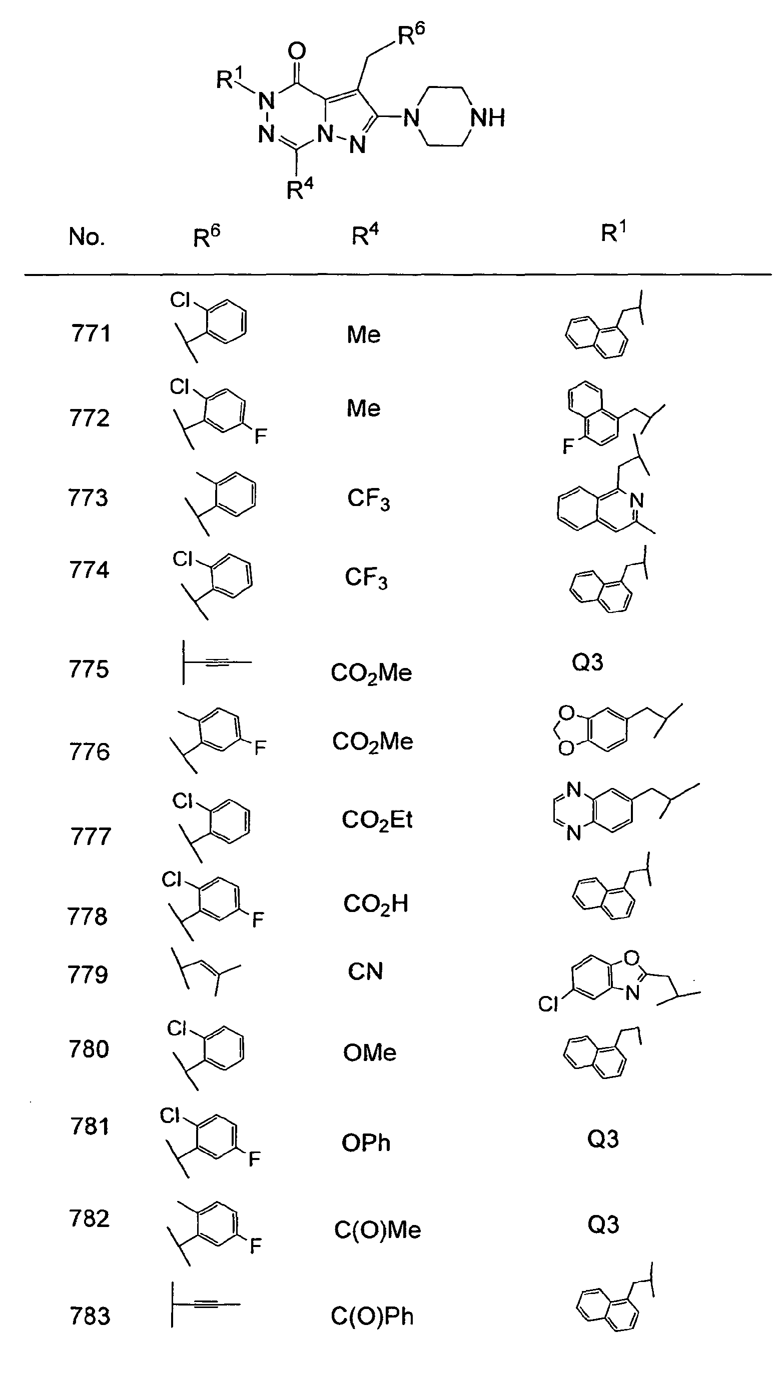

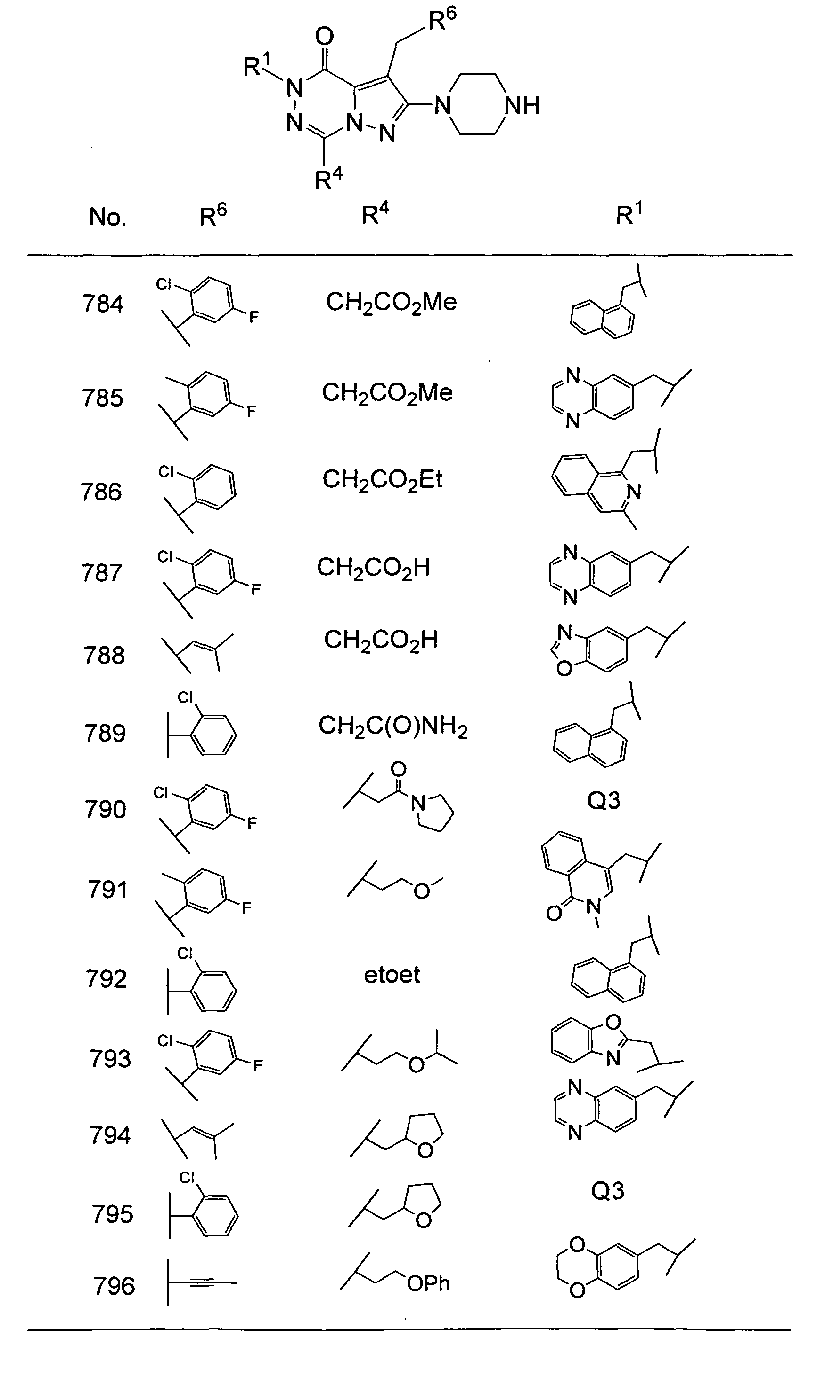

| 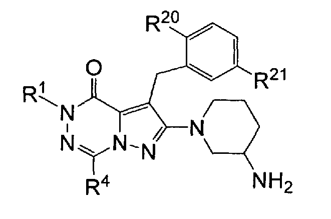 | | | | | | | | | |
|---|---|---|---|---|---|---|---|---|---|
| No. | R¹ | R⁴ | R²⁰ | R²¹ | No. | R¹ | R⁴ | R²⁰ | R²¹ |
| 797 | H | H | CN | H | 813 | Q4 | etoet | Cl | F |
| 798 | Me | H | CN | H | 814 | Q3 | etoet | Cl | F |
| 799 | Me | Me | CN | H | 815 | H | H | Me | H |
| 800 | Me | etoet | CN | H | 816 | Me | H | Me | H |
| 801 | Q4 | H | CN | H | 817 | Me | Me | Me | H |
| 802 | Q3 | H | CN | H | 818 | Me | etoet | Me | H |
| 803 | Q4 | etoet | CN | H | 819 | Q4 | H | Me | H |
| 804 | Q3 | etoet | CN | H | 820 | Q3 | H | Me | H |
| 805 | H | H | OMe | H | 821 | Q4 | etoet | Me | H |
| 806 | Me | H | OMe | H | 822 | Q3 | etoet | Me | H |
| 807 | Me | Me | OMe | H | 823 | Me | Me | Me | F |
| 808 | Me | etoet | OMe | H | 824 | Me | etoet | Me | F |
| 809 | Q4 | H | OMe | H | 825 | Q4 | H | Me | F |
| 810 | Q3 | H | OMe | H | 826 | Q3 | H | Me | F |
| 811 | Q4 | etoet | OMe | H | 827 | Q4 | etoet | Me | F |
| 812 | Q3 | etoet | OMe | H | 828 | Q3 | etoet | Me | F |

| | 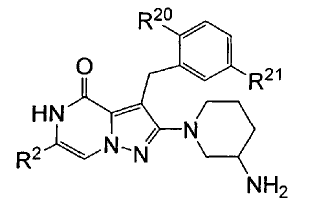 | | | | | | |
|---|---|---|---|---|---|---|---|
| No. | R² | R²⁰ | R²¹ | No. | R² | R²⁰ | R 2 |
| 829 | CO₂Ph | Cl | F | 850 | CO₂CH₂Ph | OMe | ¹H |
| 830 | CO₂CH₂Ph | Me | F | 851 | t-BuOC(O) | OMe | H |
| 831 | t-BuOC(O) | Me | H | 852 | CO₂Ph | OMe | H |
| 832 | i-PrOC(O) | Me | H | 853 | n-PrOC(O) | OMe | H |
| 833 | CO₂Ph | Me | H | 854 | C(O)OCH(Me)(Et) | OMe | H |
| 834 | CO₂CH₂Ph | Me | H | 855 | C(O)OCH₂cycpro | OMe | H |
| 835 | CO₂Et | Me | H | 856 | n-PrOC(O) | CN | H |
| 836 | C(O)Ocycpro | Me | H | 857 | t-BuOC(O) | CN | H |
| 837 | C(O)OCH(Me)(Et) | Me | H | 858 | i-PrOC(O) | CN | H |
| 838 | C(O)OCH₂CH(Me)₂ | Me | H | 859 | CO₂Ph | CN | H |
| 839 | C(O)OCH₂cycpro | Me | H | 860 | CO₂Me | CN | F |
| 840 | CO₂H | Me | F | 861 | CO₂Et | CN | H |
| 841 | t-BuOC(O) | Me | F | 862 | C(O)Ocycpro | CN | H |
| 842 | i-PrOC(O) | Me | F | 863 | C(O)OCH(Me)(Et) | CN | H |
| 843 | CO₂Ph | Me | F | 864 | C(O)OCH₂CH(Me)₂ | CN | H |
| 844 | CO₂Me | Me | F | | | | |
| 845 | CO₂Et | Me | F | 865 | C(O)OCH₂cycpro | CN | H |
| 846 | C(O)Ocycpro | Me | F | 866 | C(O)OCH₂cycbu | Me | H |
| 847 | C(O)OCH(Me)(Et) | Me | F | 867 | C(O)OCH₂cycbu | Me | F |
| 848 | C(O)OCH₂CH(Me)₂ | Me | F | 868 | C(O)OCH₂cycbu | CN | H |
| 849 | C(O)OCH₂cycpro | Me | F | 869 | C(O)OCH₂cycbu | OMe | H |

| 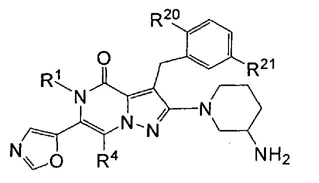 | | | | | | | | | |
|---|---|---|---|---|---|---|---|---|---|
| No. | R¹ | R⁴ | R²⁰ | R²¹ | No. | R¹ | R⁴ | R²⁰ | R²¹ |
| 870 | H | H | Cl | F | 884 | H | Me | Cl | F |
| 871 | Me | H | CN | F | 885 | Me | Me | Cl | F |
| 872 | H | H | MeO | H | 886 | H | (CH₂)₂OCH₂CHF₂ | Cl | F |
| 873 | Me | H | MeO | H | 887 | Me | (CH₂)₂OCH₂CHF₂ | Cl | F |
| 874 | Q3 | H | Cl | F | 888 | H | 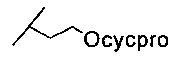 | Cl | F |
| 875 | Q4 | H | Cl | F | 889 | Me | 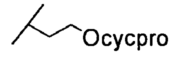 | Cl | F |
| 876 | H | H | CN | H | 890 | H | (CH₂)₂OMe | Cl | F |
| 877 | Me | H | CN | H | 891 | Me | (CH₂)₂OMe | Cl | F |
| 878 | H | H | MeO | F | 892 | H | 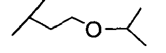 | Cl | F |
| 879 | Me | H | MeO | F | 893 | Me | 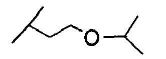 | Cl | F |
| 880 | Me | etoet | Cl | F | 894 | H | etoet | Me | H |
| 881 | Me | 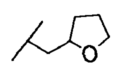 | Cl | F | 895 | Me | etoet | Me | H |
| 882 | H | etoet | Cl | F | 896 | H | etoet | Me | F |
| 883 | H | 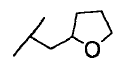 | Cl | F | 897 | Me | etoet | Me | F |
| | | | | | 898 | H | H | Me | H |
| | | | | | 899 | Me | H | Me | H |

| | | | | | | | |
|---|---|---|---|---|---|---|---|
| 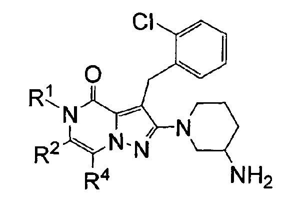 | | | | 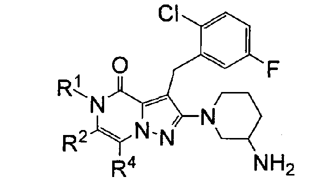 | | | |

| No. | R¹ | R² | R⁴ | No. | R¹ | R² | R⁴ |
|---|---|---|---|---|---|---|---|
| 900 | H | CO₂Et | CH₂CO₂Me | 925 | H | CO₂Et | CH₂CO₂Me |
| 901 | Me | CO₂Et | CH₂CO₂Me | 926 | Me | CO₂Et | CH₂CO₂Me |
| 902 | Me | CO₂Et | CH₂CO₂Et | 927 | Me | CO₂Et | CH₂CO₂Et |
| 903 | H | i-PrOC(O) | CH₂CO₂Me | 928 | H | i-PrOC(O) | CH₂CO₂Me |
| 904 | H | i-PrOC(O) | CH₂CO₂Et | 929 | H | i-PrOC(O) | CH₂CO₂Et |
| 905 | H | i-PrOC(O) | i-PrOC(O)CH₂ | 930 | H | i-PrOC(O) | i-PrOC(O)CH₂ |
| 906 | Me | i-PrOC(O) | CH₂CO₂Me | 931 | Me | i-PrOC(O) | CH₂CO₂Me |
| 907 | Me | i-PrOC(O) | CH₂CO₂Et | 932 | Me | i-PrOC(O) | CH₂CO₂Et |
| 908 | Me | i-PrOC(O) | i-PrOC(O)CH₂ | 933 | Me | i-PrOC(O) | i-PrOC(O)CH₂ |
| 909 | H | 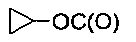 | CH₂CO₂Me | 934 | H | 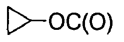 | CH₂CO₂Me |
| 910 | H | 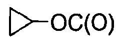 | CH₂CO₂Et | 935 | H | 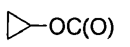 | CH₂CO₂Et |
| 911 | H | 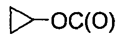 | i-PrOC(O)CH₂ | 936 | H | 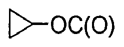 | i-PrOC(O)CH₂ |
| 912 | Me | 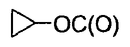 | CH₂CO₂Me | 937 | Me | 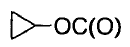 | CH₂CO₂Me |
| 913 | Me | 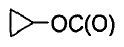 | CH₂CO₂Et | 938 | Me | 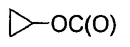 | CH₂CO₂Et |
| 914 | Me | 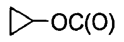 | i-PrOC(O)CH₂ | 939 | Me | 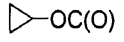 | i-PrOC(O)CH₂ |
| 915 | H | CO₂Me | 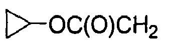 | 940 | H | CO₂Me | 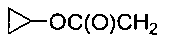 |
| 916 | H | CO₂Et | 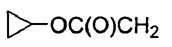 | 941 | H | CO₂Et | 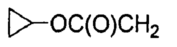 |
| 917 | H | i-PrOC(O) | 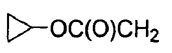 | 942 | H | i-PrOC(O) | 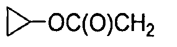 |
| 918 | Me | CO₂Me | 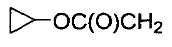 | 943 | Me | CO₂Me | 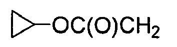 |
| 919 | Me | CO₂Et | 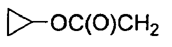 | 944 | Me | CO₂Et | 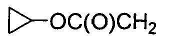 |
| 920 | Me | i-PrOC(O) | 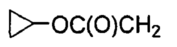 | 945 | Me | i-PrOC(O) | 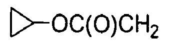 |
| 921 | H | CO₂Et | CH₂CO₂Et | 946 | H | CO₂Et | CH₂CO₂Et |
| 922 | Me | CO₂Me | CH₂CO₂Me | 947 | Me | CO₂Me | CH₂CO₂Me |
| 923 | Me | CO₂H | CH₂CO₂H | 948 | Me | CO₂H | CH₂CO₂H |
| 924 | H | CO₂H | CH₂CO₂H | | | | |

| 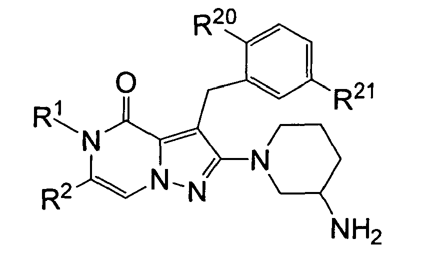 | | | | | | | | | |
|---|---|---|---|---|---|---|---|---|---|
| No. | R¹ | R² | R²⁰ | R²¹ | No. | R¹ | R² | R²⁰ | R²¹ |
| 949 | Q4 | CN | Cl | H | 965 | Q4 | C(O)Me | Cl | H |
| 950 | Q4 | CN | Cl | F | 966 | Q4 | C(O)Me | Cl | F |
| 951 | Q4 | CN | OMe | H | 967 | Q4 | C(O)Me | OMe | H |
| 952 | Q4 | CN | CN | H | 968 | Q4 | C(O)Me | CN | H |
| 953 | Q4 | CN | Me | H | 969 | Q4 | C(O)Me | Me | H |
| 954 | Q4 | CN | OMe | F | 970 | Q4 | C(O)Me | OMe | F |
| 955 | Q4 | CN | CN | F | 971 | Q4 | C(O)Me | CN | F |
| 956 | Q4 | CN | Me | F | 972 | Q4 | C(O)Me | Me | F |
| 957 | Q3 | CN | Cl | H | 973 | Q3 | C(O)Me | Cl | H |
| 958 | Q3 | CN | Cl | F | 974 | Q3 | C(O)Me | Cl | F |
| 959 | Q3 | CN | OMe | H | 975 | Q3 | C(O)Me | OMe | H |
| 960 | Q3 | CN | CN | H | 976 | Q3 | C(O)Me | CN | H |
| 961 | Q3 | CN | Me | H | 977 | Q3 | C(O)Me | Me | H |
| 962 | Q3 | CN | OMe | F | 978 | Q3 | C(O)Me | OMe | F |
| 963 | Q3 | CN | CN | F | 979 | Q3 | C(O)Me | CN | F |
| 964 | Q3 | CN | Me | F | 980 | Q3 | C(O)Me | Me | F |

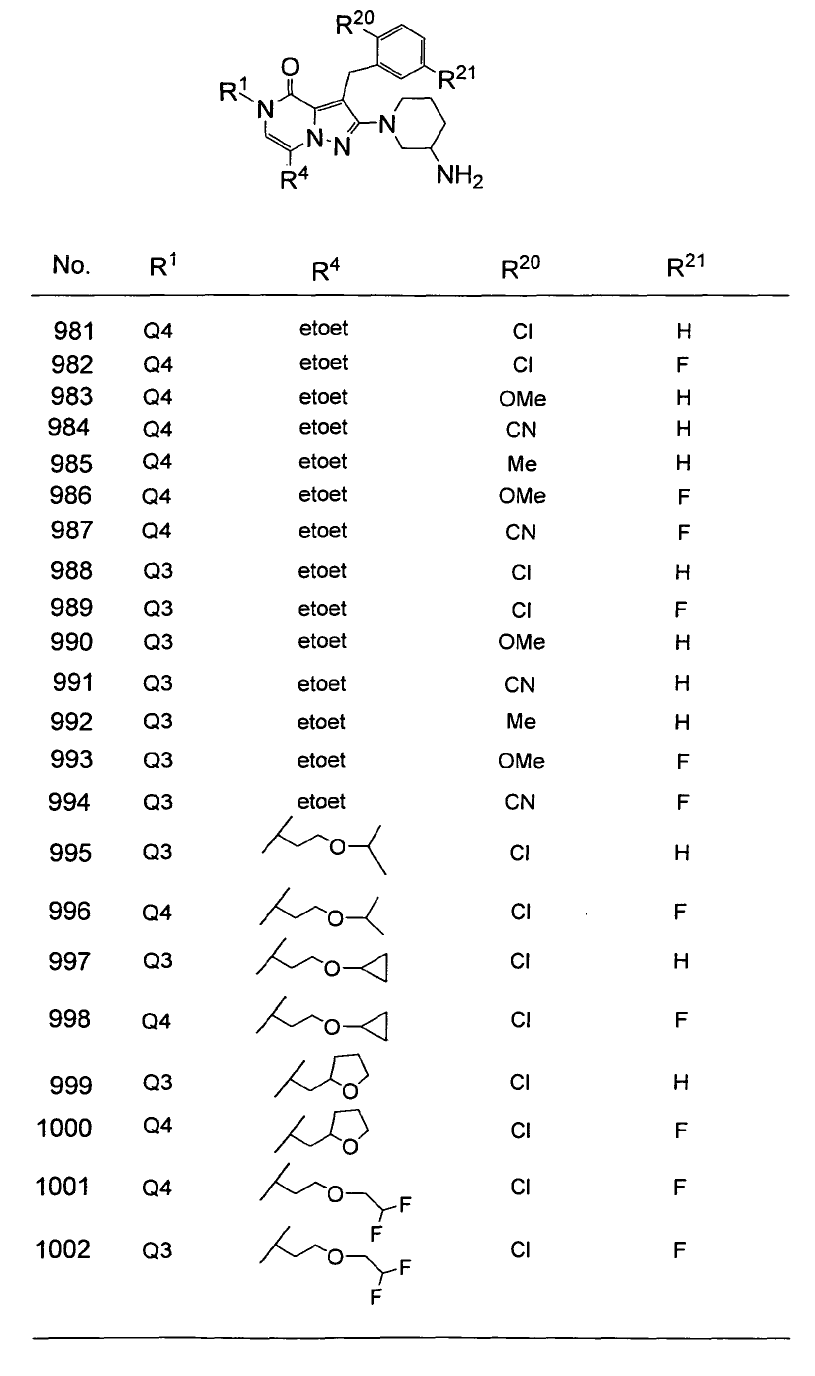

| 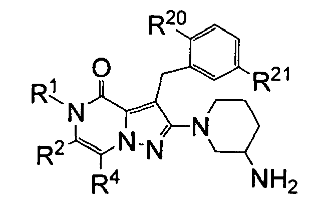 | | | | | |
|---|---|---|---|---|---|
| No. | R¹ | R² | R⁴ | R²⁰ | R²¹ |
| 1003 | Q⁴ | CN | H | Cl | H |
| 1004 | Q4 | C(O)Me | H | Cl | F |
| 1005 | Q4 | CN | H | OMe | H |
| 1006 | Q4 | C(O)Me | H | CN | H |
| 1007 | Q4 | CN | H | Me | H |
| 1008 | Q4 | C(O)Me | H | OMe | F |
| 1009 | Q4 | CN | H | CN | F |
| 1010 | Q3 | CN | H | Cl | H |
| 1011 | Q3 | C(O)Me | H | Cl | F |
| 1012 | Q3 | CN | H | OMe | H |
| 1013 | Q3 | C(O)Me | H | CN | H |
| 1014 | Q3 | CN | H | Me | H |
| 1015 | Q3 | C(O)Me | H | OMe | F |
| 1016 | Q3 | CN | H | CN | F |
| 1017 | Q4 | CN | etoet | Cl | H |
| 1018 | Q4 | C(O)Me | etoet | Cl | F |
| 1019 | Q4 | CO₂Et | etoet | Cl | F |
| 1020 | Q4 | C(O)Me | i-PrO(CH₂)₂ | Cl | H |
| 1021 | Q4 | CN | cycproO(CH₂)₂ | Cl | F |
| 1022 | Q4 | C(O)Me | 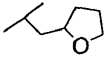 | Cl | F |
| 1023 | Q4 | CN | etoet | CN | H |
| 1024 | Q4 | C(O)Me | etoet | Me | H |
| 1025 | Q4 | CO₂Et | etoet | OMe | H |
| 1026 | Q3 | CN | etoet | Cl | H |
| 1027 | Q3 | C(O)Me | etoet | Cl | F |
| 1028 | Q3 | CO₂Et | etoet | Cl | F |
| 1029 | Q3 | CN | etoet | CN | H |
| 1030 | Q3 | C(O)Me | etoet | Me | H |
| 1031 | Q3 | CO₂Et | etoet | OMe | H |
| 1032 | Q4 | CO₂H | etoet | Cl | F |
| 1033 | Q4 | CO₂H | etoet | OMe | H |
| 1034 | Q3 | CO₂H | etoet | Cl | F |
| 1035 | Q3 | CO₂H | etoet | OMe | H |

When in the above compounds having compound numbers 1 to 1035, the portion corresponding to Y described in the item [1] is an unsubstituted or substituted 3-aminopyrrolidin-1-yl group, an unsubstituted or substituted 3-aminopiperidin-1-yl group or an unsubstituted or substituted (3-amino)hexahydroazepin-1-yl group, bicyclic pyrazole derivatives whose amino group at the 3-position has an absolute configuration represented by the following formula (F₁) are more preferable. 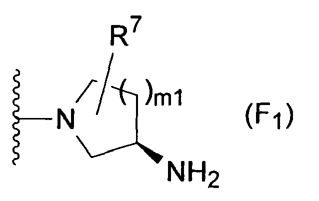 In the above formula, m and R⁷ are as defined in the item [1].

When in the above compounds having compound numbers 1 to 1035, the portion corresponding to Y described in the item [1] is an unsubstituted or substituted (2-aminocycloalkyl)amino group, bicyclic pyrazole derivatives whose amino groups at the 1-position and 2-position have an absolute configuration represented by the following formula (F₂) or (F₃) are more preferable. 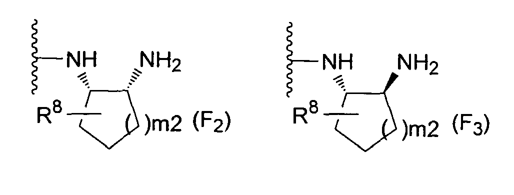 In the above formulas, m2 and R⁸ are as defined in the item [1].

Bicyclic pyrazole derivatives whose amino groups at the 1-position and 2-position have an absolute configuration represented by the following formula (F₄) are still more preferable. 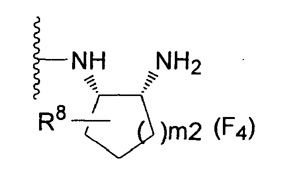 In the above formula, m2 and R⁸ are as defined in the item [1].

In the following description, a bond shown by a wedge-shaped solid line or dotted line as in the formula (J₁) and formula (J₂) indicates the absolute configuration of an amino group, and a bond shown by a thick line as in the formula (J₃) indicates the relative configuration of an amino group (for example, the formula (J₃) represents a (±)-cis form). 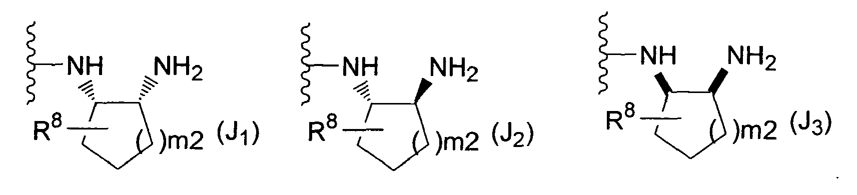 In the above formulas, m2 and R⁸ are as defined in the item [1].

In the compounds among the above compounds having compound numbers 1 to 1035, in which each of the portions corresponding to R¹, R² and R⁴, respectively, described in the item [1] is "an optionally substituted alkoxycarbonyl group", "an optionally substituted cycloalkoxycarbonyl group", "an optionally substituted aryloxycarbonyl group" or "an optionally substituted aralkyloxycarbonyl group", each of these substituents is changed to "a carboxyl group" in some cases under physiological conditions in a living body by oxidation, reduction, hydrolysis or the like by an enzyme, or hydrolysis by acid in the stomach, or the like.

A process for producing the compound represented by the formula (I) of the present invention is explained below with reference to examples, which should not be construed as limiting the scope of the invention. In the present specification, the following abbreviations are used in some cases for the simplification of description.
Boc: tert-butoxycarbonyl group
Cbz: benzyloxycarbonyl group
TBS: tert-butyldimethylsilyl group
MOM: methoxymethyl group
Ph: phenyl group
Bn: benzyl group
Et: ethyl group
Me: methyl group

The bicyclic pyrazole derivative represented by the formula (I) may be synthesized from a well-known compound by a combination of well-known synthesis processes. It may be synthesized, for example, by any of the following processes.

### Production Process 1

A compound of the formula (1-7) as the compound of the formula (I), or a salt thereof is produced, for example, by the following process: 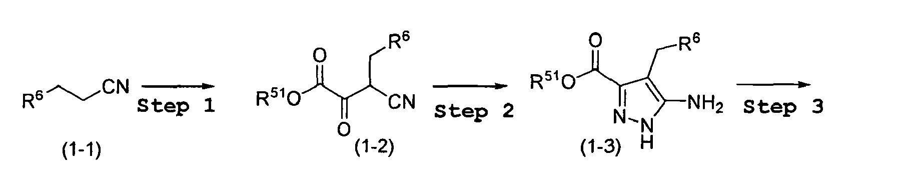 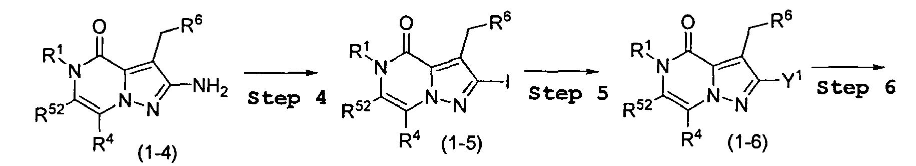 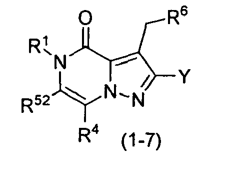 wherein R¹, R⁴, R⁶ and Y are as defined in the item [1]; R⁵¹ is an alkyl group; R⁵² is "a hydrogen atom", "an optionally substituted alkyl group", "an optionally substituted alkoxycarbonyl group", "an optionally substituted aryloxycarbonyl group", "an optionally substituted cycloalkyl group", "an optionally substituted aryl group", "an optionally substituted aralkyl group", "an optionally substituted heteroarylalkyl group" or "an optionally substituted heteroaryl group", which is represented by R² described in the item [1]; and Y¹ is a group formed by protecting the NH or NH₂ of Y with a protective group.

### 1) Step 1

A compound (1-2) may be produced by reacting a compound (1-1) with a compound (1-8) represented by the formula: 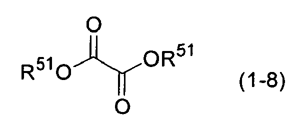 wherein R⁵¹ is as defined above, in an inert solvent in the presence of a base. The base includes sodium ethoxide, sodium methoxide, potassium tert-butoxide, sodium hydride and the like. A preferable example thereof is sodium ethoxide. The amount of the base used is usually chosen in the range of 1 to 5 equivalents per equivalent of the compound (1-1). The amount of the compound (1-8) used is usually chosen in the range of 0.5 to 3 equivalents per equivalent of the compound (1-1). The inert solvent includes, for example, alcohol solvents (e.g. methanol, ethanol and 2-propanol), ether solvents (tetrahydrofuran and 1,4-dioxane), and mixed solvents thereof. When sodium ethoxide is used as the base, ethanol is preferable as the inert solvent. The reaction temperature may be chosen in the range of about 50°C to about 100°C. Sodium ethoxide may be produced from sodium and ethanol chosen as the inert solvent.

### 2) Step 2

A compound (1-3) is produced by reacting the compound (1-2) with hydrazine monohydrate in an inert solvent. The amount of hydrazine monohydrate used is usually chosen in the range of 1 to 3 equivalents per equivalent of the compound (1-2). The inert solvent includes, for example, alcohol solvents (e.g. methanol, ethanol and 2-propanol), acetic acid, and mixed solvents thereof. The reaction temperature is chosen in the range of about 50°C to about 120°C, and the reaction is usually carried out with refluxing.

### 3) Step 3

A compound (1-4) may be produced from the compound (1-3) by carrying out the following reactions (1) to (4).
(1) The compound (1-3) is reacted with di-tert-butyl dicarbonate in an inert solvent in the presence of a base. The amount of di-tert-butyl dicarbonate used is usually chosen in the range of 3 to 6 equivalents per equivalent of the compound (1-3). The inert solvent includes ether solvents (tetrahydrofuran and 1,4-dioxane). The reaction temperature is chosen in the range of about -10°C to about 40°C.
(2) The product obtained in the above item (1) is reacted in an inert solvent in the presence of a base. The base includes sodium hydroxide, potassium hydroxide, lithium hydroxide and the like. The amount of the base used is usually chosen in the range of 3 to 10 equivalents. The inert solvent includes mixed solvents of water and alcohol solvents (e.g. methanol, ethanol and 2-propanol). The volume ratio of water to the alcohol is chosen in the range of 0.5 to 1.0. The reaction temperature is chosen in the range of about 40°C to about 80°C.
(3) The product obtained in the above item (2) is reacted with a compound (1-9a) represented by the formula: 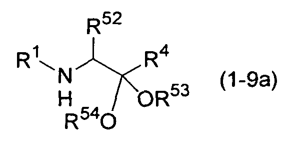 wherein R¹, R⁴ and R⁵² are as defined above, and each of R⁵³ and R⁵⁴, which are the same, is methyl, ethyl or isopropyl, or R⁵³ and R⁵⁴, when taken together, may form ethylene or trimethylene, or
   a compound (1-9b) represented by the formula: 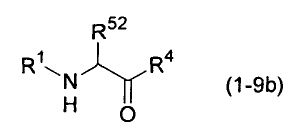 wherein R¹, R⁴ and R⁵² are as defined above, in an inert solvent by the use of a condensing agent optionally in the presence of a base. As the compound (1-9b), a commercial reagent may be used, or the compound (1-9b) may be produced by converting the ester portion of the compound (12-8) described hereinafter in the production process 12 to a carboxylic acid by the method described in the step 3, (2) in the production process 1, and converting the carboxylic acid to a ketone by the method described in the step 1 to step 2 in the production process 9 described hereinafter. As the compound (1-9a), a commercial reagent may be used, or the compound (1-9a) may be produced from the compound (1-9b) by the same production process as described in literature (for example, Tetrahedron 50, 6299 (1994) and R.C. Ralock, "Comprehensive Organic transformation", VCH publisher Inc., 1989).
   The base is not particularly limited so long as it is used as a base in a usual reaction. The base includes, for example, organic bases such as 1-hydroxybenzotriazole, N-methylmorpholine, triethylamine, diisopropylethylamine, tributylamine, 1,8-diazabicyclo[5,4,0]undec-7-ene, 1,5-diazabicyclo[4,3,0]nona-5-ene, 1,4-diazabicyclo[5,4,0]undec-7-ene, pyridine, dimethylaminopyridine, picoline, etc., and inorganic bases such as sodium hydrogencarbonate, potassium hydrogencarbonate, sodium carbonate, potassium carbonate, sodium hydroxide, sodium hydride, etc. As the condensing agent, those described in Japanese Chemical Association, Jikken Kagaku Koza (Experimental Chemistry) Vol. 22, Maruzen Co., Ltd. are exemplified. The condensing agent includes, for example, phosphoric esters such as diethyl cyanophosphate, diphenylphosphorylazide, etc.; carbodiimides such as 1-ethyl-3-(3-dimethylaminopropyl)carbodiimide hydrochloride, dicyclohexylcarbodiimide, etc.; combinations of a disulfide (e.g. 2,2'-dipyridyl disulfide) and a phosphine (e.g. triphenylphosphine); phosphorus halides such as N,N'-bis(2-oxo-3-oxazolidinyl)phosphinic chloride, etc.; combinations of an azodicarboxylic acid diester (e.g. diethyl azodicarboxylate) and a phosphine (e.g. triphenylphosphine); and 2-halo-1-lower-alkylpyridinium halides such as 2-chloro-1-methylpyridinium iodide, etc. The inert solvent includes, for example, ether solvents such as tetrahydrofuran, diethyl ether, 1,4-dioxane, 1,2-dimethoxyethane, etc.; hydrocarbon solvents such as hexane, heptane, toluene, benzene, xylene, etc.; halogenated hydrocarbon solvents such as dichloromethane, chloroform, 1,2-dichloroethane, etc.; ketone solvents such as acetone, etc.; and aprotic solvents such as acetonitrile, N,N-dimethylformamide, dimethyl sulfoxide, hexamethylenephosphoamide, etc. Mixed solvents of these solvents may also be used as the inert solvent. The reaction temperature is chosen in the range of about -70°C to about 80°C.
   A production example in this step is described below. The product obtained in the above item (2) is reacted with the compound of the formula (1-9a) or the formula (1-9b) in an inert solvent in the presence of 1-hydroxybenzotriazole by using 1-ethyl-3-(3-dimethylaminopropyl)carbodiimide hydrochloride as a condensing agent. The inert solvent includes dimethylformamide, ether solvents (e.g. tetrahydrofuran and 1,4-dioxane) and halogenated hydrocarbon solvents (e.g. dichloromethane and 1,2-dichloroethane). A preferable example thereof is dimethylformamide. The reaction temperature is chosen in the range of about -10°C to about 40°C.
(4) The product obtained in the above item (3) is reacted in an inert solvent in the presence of an acid. The acid includes hydrochloric acid, phosphoric acid, sulfuric acid and the like. A preferable example thereof is hydrochloric acid. The amount of the acid used is usually chosen in the range of 10 to 20 equivalents. The inert solvent includes ethers (e.g. tetrahydrofuran and 1,4-dioxane). A preferable example thereof is 1,4-dioxane. In addition, 1,4-dioxane containing hydrogen chloride may be used as the solvent. The reaction temperature is chosen in the range of about -10°C to about 100°C.

### 4) Step 4

A compound (1-5) is produced by reacting the compound (1-4) with diiodomethane and isoamyl nitrite in an inert solvent. The amount of diiodomethane used is usually chosen in the range of 10 to 50 equivalents per equivalent of the compound (1-4). Diiodomethane may be used also as a solvent. The amount of isoamyl nitrite used is usually chosen in the range of 1 to 10 equivalents per equivalent of the compound (1-4). The inert solvent includes hydrocarbon solvents such as toluene, benzene, xylene, etc.; and halogenated hydrocarbon solvents such as dichloromethane, chloroform, 1,2-dichloroethane, etc. The reaction temperature is chosen in the range of about 20°C to about 40°C.

### 5) Step 5

A compound (1-6) is produced by reacting the compound (1-5) with a compound selected from a compound (1-10), a compound (1-11), a compound (1-12), a compound (1-13), a compound (1-14) and a compound (1-15) represented by the formulas: 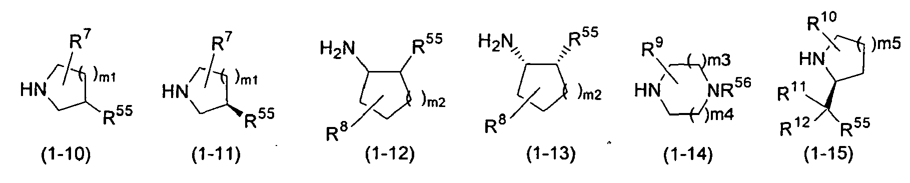 wherein m1, m2, m3, m4, m5, R⁷, R⁸, R⁹, R¹⁰, R¹¹ and R¹² are as defined in the item [1], R⁵⁵ is N=C(Ph)₂, NHBoc, NHCbz or the following formula (1-16): 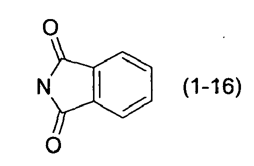 and R⁵⁶ is Boc or Cbz, in an inert solvent in the presence of potassium phosphate, ethylene glycol and copper iodide. The amount of the compound (1-10), compound (1-11), compound (1-12), compound (1-13), compound (1-14) or compound (1-15) used is usually chosen in the range of 1 to 5 equivalents per equivalent of the compound (1-5). The amount of potassium phosphate used is usually chosen in the range of 1 to 5 equivalents per equivalent of the compound (1-5). The amount of ethylene glycol used is usually chosen in the range of 1 to 5 equivalents per equivalent of the compound (1-5). The amount of copper iodide used is usually chosen in the range of 1 to 5 equivalents per equivalent of the compound (1-5). The inert solvent includes, for example, alcohol solvents (e.g. methanol, ethanol and 2-propanol). The reaction temperature is chosen in the range of about 50°C to about 150°C. It is also possible to carry out the reaction in a closed reaction vessel such as an autoclave.

The compound (1-11) and the compound (1-13) may be produced by the process described hereinafter as production process 23 and the process described hereinafter as production process 24, respectively.
The compound (1-14) may be produced by the process described in literature (for example, Synthesis 391 (1994), Org. Lett. 5, 1591 (2003), Synthesis 1065 (1992), Synlett 755 (2002), J. Org. Chem. 56, 3063 (1991), J. Org. Chem. 60, 4177 (1995) and J. Org. Chem. 57, 6653 (1992)) or the process described hereinafter as production process 25. The compound (1-10) may be produced by the process described hereinafter as production process 22 or the process described in literature (for example, US5232929). The compound (1-15) may be produced by the same process as that described in literature (for example, J. Org. Chem. 61, 6700 (1996)) or the like. The compound (1-12) may be produced by the same process as that described in literature (for example, US6075167) or the like.

### 6) Step 6

The compound (1-7) may be produced from the compound (1-6) by the same process as that described in literature (for example, Protective Groups in Organic Synthesis 2nd Edition (John Wiley & Sons, Inc.)) or the like.

### Production Process 2

A compound of the formula (2-4) as the compound of the formula (I), or a salt thereof is produced, for example, by the following process: 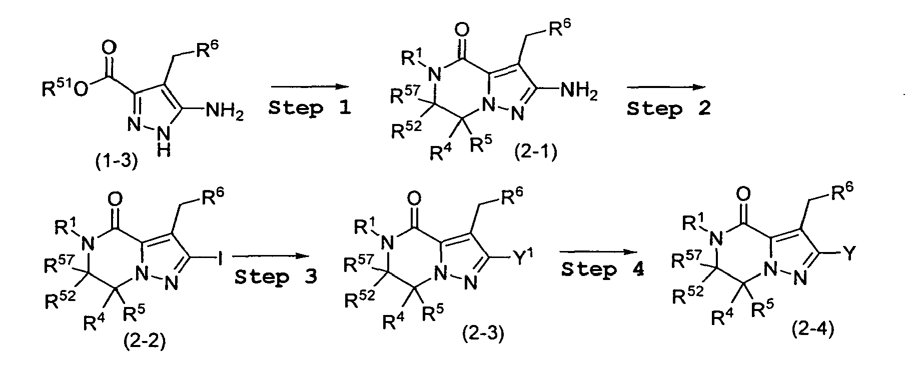 wherein R¹, R⁴, R⁵, R⁶ and Y are as defined in the item [1]; R⁵¹, R⁵² and Y¹ are as defined in the production process 1; and R⁵⁷ has the same meaning as that of R⁵² defined in the production process 1.

### 1) Step 1

A compound (2-1) may be produced from a compound (1-3) by carrying out the following reactions (1) to (6).
(1) Reaction is carried out by the same method as in the production process described in the step 3, (1) in the production process 1.
(2) Reaction is carried out by the same method as in the production process described in the step 3, (2) in the production process 1.
(3) The product obtained in the above item (2) is reacted with a compound (2-5) represented by the formula: 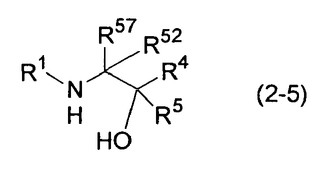 wherein R¹, R⁴, R⁵, R⁵² and R⁵⁷ are as defined above, by the same method as in the production process described in the step 3, (3) in the production process 1.
(4) The product obtained in the above item (3) is reacted with carbon tetrabromide in an inert solvent in the presence of triphenylphosphine. The amount of triphenylphosphine used is usually chosen in the range of 1 to 3 equivalents. The amount of carbon tetrabromide used is usually chosen in the range of 1 to 3 equivalents. The inert solvent includes aprotic solvents such as N,N-dimethylformamide, dimethyl sulfoxide, and acetonitrile. The reaction temperature is chosen in the range of about -10°C to about 40°C.
(5) A base is added to the reaction solution obtained in the above item (4) and the reaction is carried out. The base includes potassium tert-butoxide, sodium tert-butoxide, cesium carbonate, potassium carbonate, sodium carbonate, sodium hydride and the like. A preferable example thereof is potassium carbonate. The amount of the base used is chosen usually in the range of 1 to 5 equivalents. The reaction temperature may be chosen in the range of about 30°C to about 100°C.
(6) From the product obtained in the above item (5), Boc is removed by the same method as that described in literature (for example, Protective Groups in Organic Synthesis 2nd Edition (John Wiley & Sons, Inc.)) or the like.

### 2) Step 2

A compound (2-2) may be produced from the compound (2-1) by the same production process as described in the step 4 in the production process 1.

### 3) Step 3

A compound (2-3) may be produced from the compound (2-2) by the same production process as described in the step 5 in the production process 1.

### 4) Step 4

The compound (2-4) may be produced from the compound (2-3) by the same production process as described in the step 6 in the production process 1.

### Production Process 3

A compound (3-1) corresponding to the compound (1-4) described in the production process 1 in the case where R⁴ is a hydrogen atom, may be produced also by the following process: 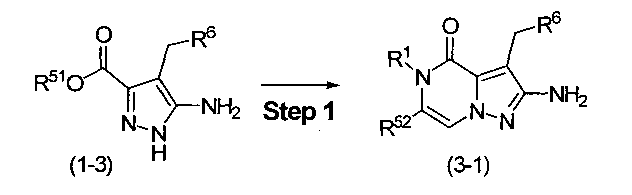 wherein R¹ and R⁶ are as defined in the item [1], and R⁵¹ and R⁵² are as defined in the production process 1.

The compound (3-1) may be produced from a compound (1-3) by carrying out the following reactions (1) to (5) in the step 1.

### 1) Step 1

(1) Reaction is carried out by the same method as in the production process described in the step 3, (1) in the production process 1.
(2) Reaction is carried out by the same method as in the production process described in the step 3, (2) in the production process 1.
(3) The product obtained in the above item (2) is reacted with a compound (3-2) represented by the formula: 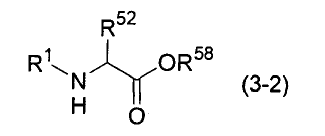 wherein R¹ and R⁵² are as defined above, and R⁵⁸ is methyl, ethyl or isopropyl, by the same method as in the production process described in the step 3, (3) in the production process 1. As the compound (3-2), a commercial reagent may be used, or the compound (3-2) may be produced by the same process as the production process of a compound (12-8) described hereinafter in the production process 12.
(4) The product obtained in the above item (3) is reacted with diisobutylaluminum hydride in an inert solvent. The amount of diisobutylaluminum hydride used is usually chosen in the range of 3 to 10 equivalents. The inert solvent includes toluene, xylene and ether solvents (e.g. tetrahydrofuran). A preferable example thereof is toluene. The reaction temperature is chosen in the range of about -100°C to about 0°C, preferably about -80°C to about -60°C.
(5) Reaction is carried out by the same method as in the production process described in the step 3, (4) in the production process 1.

### Production Process 4

The compound (3-1) described in the production process 3 may be produced also by the following process: 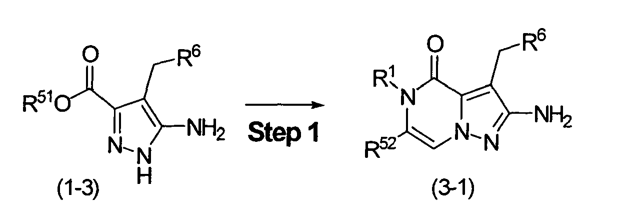 wherein R¹ and R⁶ are as defined in the item [1], and R⁵¹ and R⁵² are as defined in the production process 1.

The compound (3-1) may be produced from a compound (1-3) by carrying out the following reactions (1) to (5) in the step 1.

### 1) Step 1

(1) Reaction is carried out by the same method as in the production process described in the step 3, (1) in the production process 1.
(2) Reaction is carried out by the same method as in the production process described in the step 3, (2) in the production process 1.
(3) The product obtained in the above item (2) is reacted with a compound (4-1) represented by the formula: 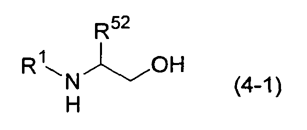 wherein R¹ and R⁵² are as defined above, by the same method as in the production process described in the step 3, (3) in the production process 1. As the compound (4-1), a commercial reagent may be used, or the compound (4-1) may be produced from the compound (12-8) described hereinafter in the production process 12, by the same production process as described in literature (for example, Synth. Commun. 33, 2907 (2003), Synlett 37 (2002), and R.C. Ralock, "Comprehensive Organic transformation", VCH publisher Inc., 1989).
(4) The hydroxylmethyl group of the product obtained in the above item (3) is converted to a formyl group by the same method as in the production process described in literature (for example, J. Am. Chem. Soc. 118, 12246 (1996), J. Comb. Chem. 3, 223 (1999), J. Comb. Chem. 5, 516 (2002), Org. Lett. 3, 3041 (2001) and J. Org. Chem.23, 7907 (2001)).
(5) Reaction is carried out by the same method as in the production process described in the step 3, (4) in the production process 1.

### Production Process 5

Each of compounds of the formula (5-13) and the formula (5-16) as the compound of the formula (I), or a salt thereof is produced, for example, by the following process: 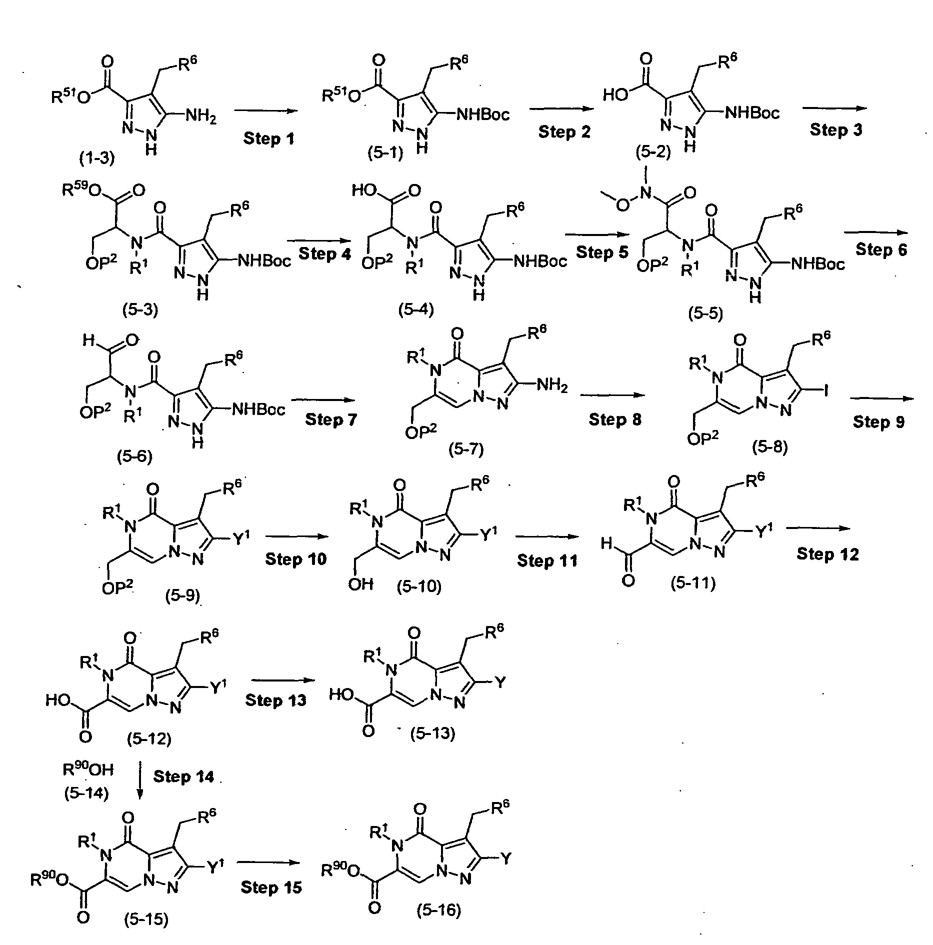 wherein R¹, R⁶ and Y are as defined in the item [1]; R⁵¹ and Y¹ are as defined in the production process 1; R⁵⁹ is methyl or ethyl; P² is a protective group for hydroxyl group; and R⁹⁰ is an optionally substituted alkyl group, an optionally substituted aryl group or an optionally substituted heteroaryl group.

A compound (5-1) may be produced from a compound (1-3) by carrying out the following reactions (1) to (5).

### 1) Step 1

A compound (5-1) may be produced from a compound (1-3) by the same production process as described in the step 3, (1) in the production process 1.

### 2) Step 2

A compound (5-2) may be produced from the compound (5-1) by the same production process as described in the step 3, (2) in the production process 1.

### 3) Step 3

A compound (5-3) may be produced from the compound (5-2) and a compound (5-17) represented by the formula: 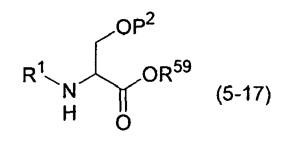 wherein R¹, R⁵⁹ and P² are as defined above, by the same production process as described in the step 3, (3) in the production process 1. Preferable examples of 2 are methoxymethyl, benzyl, p-methoxybenzyl, tert-butyldimethylsilyl and triisopropylsilyl. As the compound (5-17), a commercial reagent may be used, or the compound (5-17) may be produced by the same process as the production process of a compound (12-8) described hereinafter in the production process 12.

### 4) Step 4

A compound (5-4) may be produced from the compound (5-3) by the same production process as described in the step 3, (2) in the production process 1.

### 5) Step 5

A compound (5-5) may be produced from the compound (5-4) and N,O-dimethylhydroxylamine hydrochloride by the same production process as described in literature (for example, Bioorg. Med. Chem. Lett. 11, 2951 (2001), Synthesis 1852 (2000), Organic Letters 2, 4091 (2000) and Bioorg. Med. Chem. Lett. 11, 287 (2001)).

### 6) Step 6

A compound (5-6) may be produced from the compound (5-5) by the same production process as described in literature (for example, Bioorg. Med. Chem. Lett. 13, 265 (2003), Tetrahedron Letters 40, 5179 (1999), Tetrahedron Letters 34, 7371 (1993), Tetrahedron 55, 12907 (1999), Synlett 700 (1995) and J. Org. Chem. 58, 2446 (1993)).

### 7) Step 7

A compound (5-7) may be produced from the compound (5-6) by the same production process as described in the step 3, (4) in the production process 1.

### 8) Step 8

A compound (5-8) may be produced from the compound (5-7) by the same production process as described in the step 4 in the production process 1.

### 9) Step 9

A compound (5-9) may be produced from the compound (5-8) by the same production process as described in the step 5 in the production process 1.

### 10) Step 10

A compound (5-10) may be produced from the compound (5-9) by the same process as that described in literature (for example, Protective Groups in Organic Synthesis 2nd Edition (John Wiley & Sons, Inc.)) or the like.

### 11) Step 11

A compound (5-11) may be produced from the compound (5-10) by the same production process as described in literature (for example, J. Org. Chem. 65, 7757 (2000), Pharmazie 55, 273 (2000), Pharmazie 55, 645 (2000), J. Am. Chem. Soc. 122, 7144 (2000), Tetrahedron Lett. 36, 8513 (1995), Tetrahedron Lett. 36, 9117 (1995) and Tetrahedron Lett. 36, 8513 (1995)).

### 12) Step 12

A compound (5-12) may be produced from the compound (5-11) by the same production process as described in literature (for example, J. Chem. Soc. Perkin Trans. I 529 (2002), Heterocycles 32, 1933 (1991), Synthesis 295 (1993), Tetrahedron Lett. 35, 2959 (1994), Tetrahedron Lett. 40, 9085 (1999), Synthesis 1878 (1999) and Synth. Commun. 26, 2775 (1996)).

### 13) Step 13

The compound (5-13) may be produced from the compound (5-12) by the same production process as described in the step 6 in the production process 1.

### 14) Step 14

A compound (5-15) may be produced from the compound (5-12) and a compound (5-14) by the same process as that described in literature (for example, R.C. Ralock, "Comprehensive Organic transformation", VCH publisher Inc., (1989)) or the like.

### 15) Step 15

The compound (5-16) may be produced from the compound (5-15) by the same production process as described in the step 6 in the production process 1.

### Production Process 6

In the production process 1, the compound of the formula (1-6) or a salt thereof may be produced also by, for example, the following process: 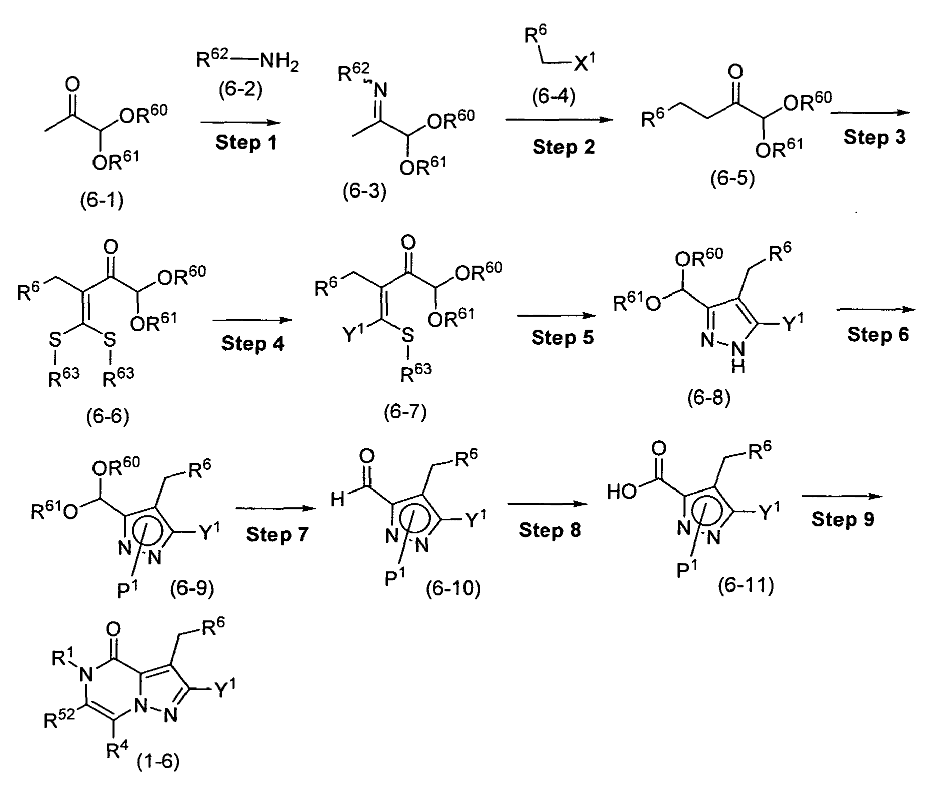 wherein R¹, R⁴ and R⁶ are as defined in the item [1]; R⁵² and Y¹ are as defined in the production process 1; each of R⁶⁰ and R⁶¹, which are the same, is methyl, ethyl or isopropyl, or R⁶⁰ and R⁶¹, when taken together, may form ethylene or trimethylene; X¹ is a leaving group (for example, a bromine atom, a chlorine atom, methanesulfonyloxy, trifluoromethanesulfonyloxy or p-toluenesulfonyloxy); P¹ is a protective group for nitrogen atom; R⁶² is a cycloalkyl group; R⁶³ is methyl or ethyl; and the following formula (G1): 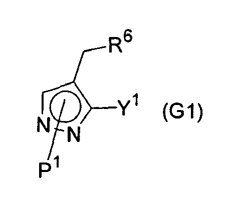 as the partial structural formula of each of the compound (6-9), compound (6-10) and compound (6-11) represents the following formula (G2) or the following formula (G3):

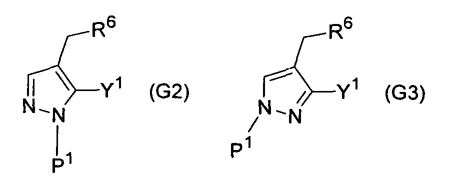

### 1) Step 1

A compound (6-3) may be produced from a compound (6-1) by the same process as that described in literature (for example, J. Am. Chem. Soc. 125, 9900 (2003)) or the like.

### 2) Step 2

A compound (6-5) may be produced from the compound (6-3) by carrying out the following reactions (1) and (2).
(1) The compound (6-3) is treated with a base in an inert solvent and reacted with a compound (6-4). The base includes lithium diisopropylamide, lithium bis(trimethylsilyl)amide, potassium bis(trimethylsilyl)amide and the like. The amount of the base used is usually chosen in the range of 1 to 5 equivalents per equivalent of the compound (6-3). The amount of the compound (6-4) used is usually chosen in the range of 1 to 5 equivalents per equivalent of the compound (6-3). The inert solvent includes, for example, ether solvents such as diethyl ether, tetrahydrofuran, 1,4-dioxane, etc. Mixed solvents of these solvents may also be used. The reaction temperature is chosen in the range of about -100°C to about 20°C.
(2) The product obtained in the above item (1) is reacted with an acid in an inert solvent. The acid includes inorganic acids such as hydrochloric acid, sulfuric acid, etc.; and organic acids such as trifluoroacetic acid, acetic acid, etc. The amount of the acid used is usually chosen in the range of 1 equivalent to large excess equivalents per equivalent of the product obtained in the above item (1). The inert solvent includes water, tetrahydrofuran and the like. The reaction temperature is chosen in the range of about 0°C to about 30°C.

### 3) Step 3

A compound (6-6) may be produced from the compound (6-5) by continuously carrying out the following procedures ((1) to (4)).
(1) The compound (6-5) is treated with potassium bis(trimethylsilyl)amide in an inert solvent such as tetrahydrofuran. The amount of potassium bis(trimethylsilyl)amide used is chosen in the range of 1 to 2 equivalents per equivalent of the compound (6-5). The reaction temperature is chosen in the range of about -100°C to about -50°C.
(2) Carbon disulfide is added to the reaction solution obtained in the above item (1). The amount of carbon disulfide used is chosen in the range of 1 to 2 equivalents per equivalent of the compound (6-5). The reaction temperature is chosen in the range of about -100°C to about -50°C.
(3) Potassium bis(trimethylsilyl)amide is added to the reaction solution obtained in the above item (2). The amount of potassium bis(trimethylsilyl)amide used is chosen in the range of 1 to 2 equivalents per equivalent of the compound (6-5). The reaction temperature is chosen in the range of about -100°C to about -50°C.
(4) Methyl iodide or ethyl iodide is added to the reaction solution obtained in the above item (3). The amount of methyl iodide or ethyl iodide used is chosen in the range of 2 to 5 equivalents per equivalent of the compound (6-5). The reaction temperature is chosen in the range of about -100°C to about 40°C.

### 4) Step 4

A compound (6-7) may be produced by reacting the compound (6-6) with a compound selected from the compound (1-10), compound (1-11), compound (1-12), compound (1-13), compound (1-14) and compound (1-15) described in the step 5 in the production process 1, in an inert solvent in the presence or absence of a base. The base includes sodium ethoxide, sodium methoxide, potassium tert-butoxide, sodium hydride and the like. The amount of the base used is usually chosen in the range of 1 to 5 equivalents per equivalent of the compound (6-6). The amount of the compound (1-10), compound (1-11), compound (1-12), compound (1-13), compound (1-14) or compound (1-15) used is usually chosen in the range of 1 to 3 equivalents per equivalent of the compound (6-6). The inert solvent includes, for example, alcohol solvents such as methanol, ethanol, 2-propanol, etc.; ether solvents such as tetrahydrofuran, 1,4-dioxane, etc.; hydrocarbon solvents such as toluene, o-xylene, m-xylene, p-xylene, etc.; and aprotic solvents such as dimethylformamide, etc. The reaction temperature may be chosen in the range of about 50°C to about 180°C.

### 5) Step 5

A compound (6-8) may be produced by reacting the compound (6-7) with hydrazine monohydrate in an inert solvent. The amount of hydrazine monohydrate used is usually chosen in the range of 1 to 3 equivalents per equivalent of the compound (6-7). The inert solvent includes, for example, alcohol solvents such as methanol, ethanol, 2-propanol, etc., and aprotic solvents such as dimethylformamide, etc. The reaction temperature is chosen in the range of about 50°C to about 150°C.

### 6) Step 6

A compound (6-9) may be produced from the compound (6-8) by the production process described in literature (for example, Protective Groups in Organic Synthesis 2nd Edition (John Wiley & Sons, Inc.)). Preferable examples of P¹ are dimethylsulfamoyl group, methoxymethyl group and p-methoxybenzyl group.

### 7) Step 7

A compound (6-10) may be produced by reacting the compound (6-9) with an organic acid in an inert solvent. The organic acid includes acetic acid, propionic acid and the like. The amount of the organic acid used is usually chosen in the range of 1 equivalent to large excess equivalents per equivalent of the compound (6-9). The inert solvent includes, for example, water, dioxane and alcohol solvents (e.g. methanol, ethanol and 2-propanol). The reaction temperature is chosen in the range of about 30°C to about 100°C.

### 8) Step 8

A compound (6-11) may be produced from the compound (6-10) by the same process as that described in literature (for example, Tetrahedron 59, 6045 (2003)) or the like.

### 9) Step 9

The compound (1-6) may be produced from the compound (6-11) by carrying out the following reactions (1) and (2).
(1) Reaction is carried out by the same method as in the production process described in the step 3, (3) in the production process 1.
(2) Reaction is carried out by the same method as in the production process described in the step 3, (4) in the production process 1. In this reaction, a compound in which the protective group for primary amino group or secondary amino group in Y¹ has been removed is produced in some cases. The primary amino group or secondary amino group in Y may be protected again with a protective group (e.g. Boc or Cbz) by the same method as in the production process described in literature (for example, Protective Groups in Organic Synthesis 2nd Edition (John Wiley & Sons, Inc.)).

### Production Process 7

The compound of the formula (6-9) in the production process 6 or a salt thereof may be produced also by, for example, the following process: 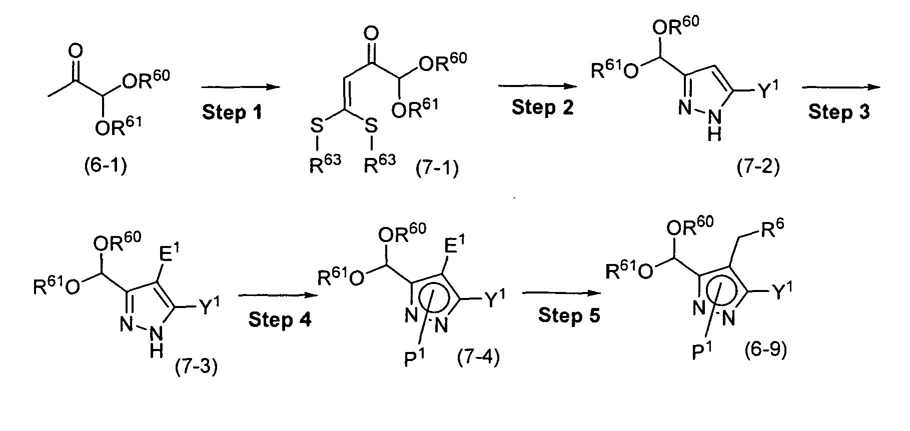 wherein R⁶ is as defined in the item [1]; Y¹ is as defined in the production process 1; P¹, R⁶⁰, R⁶¹ and R⁶³ are as defined in the production process 6; E¹ is an iodine atom, a bromine atom or a chlorine atom; and the following formula (G4): 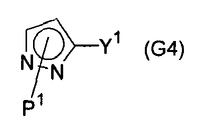 as the partial structural formula of each of a compound (7-4) and the compound (6-9) represents the following formula (G5) or the following formula (G6):

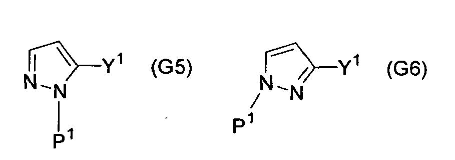

### 1) Step 1

A compound (7-1) may be produced from a compound (6-1) by continuously carrying out the following procedures ((1) to (3)).
(1) The compound (6-1) is treated with, for example, an alkali metal hydride such as sodium hydride or potassium hydride in an inert solvent such as dimethylformamide or tetrahydrofuran. The amount of the alkali metal hydride used is chosen in the range of 1 to 10 equivalents per equivalent of the compound (6-1). The reaction temperature is chosen in the range of about -10°C to about 40°C.
(2) Carbon disulfide is added to the reaction solution obtained in the above item (1). The amount of carbon disulfide used is chosen in the range of 1 to 5 equivalents per equivalent of the compound (6-1). The reaction temperature is chosen in the range of about -10°C to about 50°C.
(3) Methyl iodide or ethyl iodide is added to the reaction solution obtained in the above item (2). The amount of methyl iodide or ethyl iodide used is chosen in the range of 2 to 10 equivalents per equivalent of the compound (6-1). The reaction temperature is chosen in the range of about -10°C to about 40°C.

### 2) Step 2

A compound (7-2) may be produced from the compound (7-1) by continuously carrying out the following procedures ((1) and (2)).
(1) Reaction is carried out by the same method as in the production process described in the step 4 in the production process 6.
(2) Reaction is carried out by the same method as in the production process described in the step 5 in the production process 6.

### 3) Step 3

A compound (7-3) may be produced by reacting the compound (7-2) with a halogen (iodine, bromine or chlorine) in an inert solvent in the presence of an inorganic base. The amount of the halogen used is usually chosen in the range of 1 to 5 equivalents per equivalent of the compound (7-2). The inorganic base includes sodium hydrogencarbonate, potassium hydrogencarbonate, sodium carbonate, potassium carbonate and the like. The amount of the inorganic base used is usually chosen in the range of 1 to 5 equivalents per equivalent of the compound (7-2). The inert solvent includes, for example, halogenated hydrocarbon solvents such as chloroform, dichloromethane, dichloroethane, etc. The reaction temperature is chosen in the range of about -10°C to about 40°C.

### 4) Step 4

A compound (7-4) may be produced from the compound (7-3) by the same production process as described in the step 6 in the production process 6.

### 5) Step 5

The compound (6-9) may be produced from the compound (7-4) by the same production process as described in literature (for example, Chem. Rev. 95, 2457 (1995), Organic Process Research & Development 5, 254 (2001), J. Med. Chem. 45, 999 (2002), Synthesis 563 (1997), J. Org. Chem. 65, 9001 (2000), J. Org. Chem. 64, 4196 (1999), J. Org. Chem. 67, 3904 (2002), Adv. Synth. Catal. 345, 620 (2003) and J. Med. Chem. 43, 675 (2000)).

### Production Process 8

Each of compounds of the formula (8-2), the formula (8-5) and the formula (8-7) as the compound of the formula (I), or a salt thereof is produced, for example, by the following process: 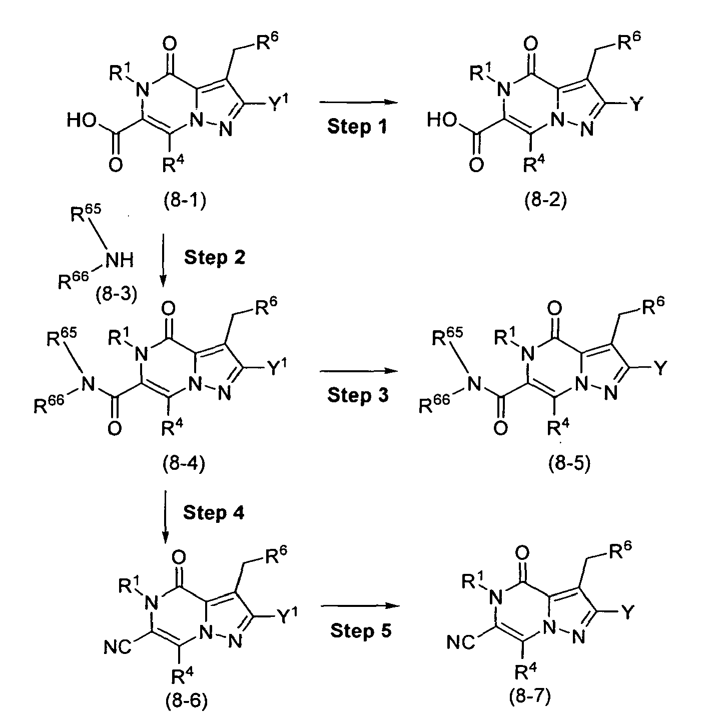 wherein R¹, R⁴, R⁶ and Y are as defined in the item [1]; the compound represented by the formula (8-1) corresponds to the compound (12-6) described in the production process 12 when R⁶⁹ is a carboxyl group; Y¹ is as defined in the production process 1; and R⁶⁵R⁶⁶NC(O) represents "an optionally substituted carbamoyl group" for R² described in the item [1].

### 1) Step 1

The compound (8-2) may be produced from the compound (8-1) by the same production process as described in the step 6 in the production process 1.

### 2) Step 2

A compound (8-4) may be produced by condensing the compound (8-1) with a compound (8-3) in an inert solvent by the use of a dehydrating-condensation agent such as dicyciohexyicarbodiimide or carbonyldiimidazole optionally in the presence of an additive such as 4-(dimethylamino)pyridine. The inert solvent includes, for example, ether solvents such as diethyl ether, tetrahydrofuran, 1,4-dioxane, etc.; aprotic solvents such as N,N-dimethylformamide, etc.; and halogenated hydrocarbon solvents such as dichloromethane, dichloroethane, etc. Mixed solvents of these solvents may also be used. A preferable example thereof is N,N-dimethylformamide. The reaction temperature is usually chosen in the range of about 0°C to about 50°C.

### 3) Step 3

The compound (8-5) may be produced from the compound (8-4) by the same production process as described in the step 6 in the production process 1.

### 4) Step 4

A compound (8-6) may be produced from the compound (8-4) by the same production process as described in literature (for example, R.C. Ralock, "Comprehensive Organic transformation", VCH publisher Inc., 1989).

### 5) Step 5

The compound (8-7) may be produced from the compound (8-6) by the same production process as described in the step 6 in the production process 1.

### Production Process 9

A compound of the formula (9-4) as the compound of the formula (I), or a salt thereof is produced, for example, by the following process: 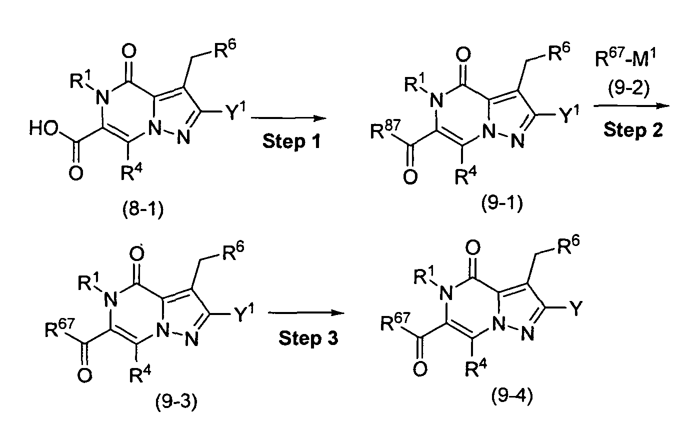 wherein R¹, R⁴, R⁶ and Y are as defined in the item [1]; the formula (8-1) is as defined above; Y¹ is as defined in the production process 1; R⁸⁷ is 4-morpholinyl or N(CH₃)OCH₃; R⁶⁷C(O) is "an optionally substituted aroyl group", "an optionally substituted heteroarylcarbonyl group" or "an optionally substituted alkylcarbonyl group", which is represented by R² described in the item [1]; and M¹ is lithium, magnesium chloride, magnesium bromide or magnesium iodide.

### 1) Step 1 to Step 2

A compound (9-3) may be produced from a compound (8-1) by the same production process as described in literature (for example, Bioorg. Med. Chem. Lett. 11, 2951 (2001), Tetrahedron Letters 42, 8955 (2001), Synthesis 1852 (2000), Organic Letters 2, 4091 (2000), Tetrahedron Letters 42, 5609 (2001), Synthesis 2239 (2001), Synlett 5, 715 (2002), J. Org. Chem. 67, 5032 (2002), Bioorg. Med. Chem. Lett. 11, 287 (2001), Tetrahedron Letters 42, 3763 (2001), J. Org. Chem. 67, 8938 (2002), Bioorg. Med. Chem. Lett. 12, 2887 (2002) and Tetrahedron Letters 43, 6313 (2002)).

As a compound (9-2), a commercial one may be used, or the compound (9-2) may be produced by the process described, for example, in Japanese Chemical Association, Jikken Kagaku Koza (Experimental Chemistry) Vol. 25, Maruzen Co., Ltd.

In the step 2, anhydrous cerium(III) chloride may be added. The amount of anhydrous cerium(III) chloride used is usually chosen in the range of 1 to 5 equivalents per equivalent of a compound (9-1).

### 2) Step 3

The compound (9-4) may be produced from the compound (9-3) by the same production process as described in the step 6 in the production process 1.

### Production Process 10

Each of compounds of the formula (10-5) and the formula (10-7) as the compound of the formula (I), or a salt thereof is produced, for example, by the following process: 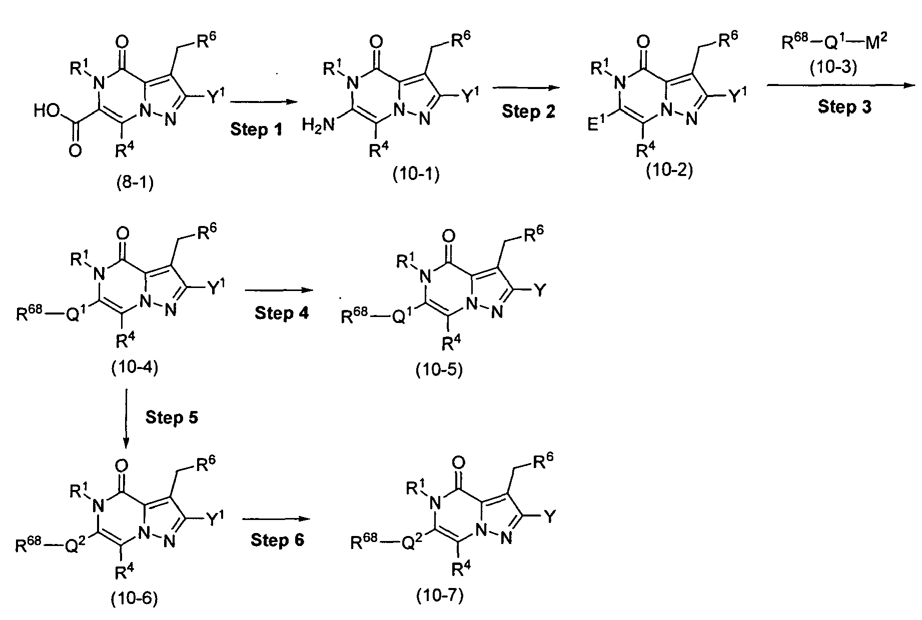 wherein R¹, R⁴, R⁶ and Y are as defined in the item [1]; Y¹ is as defined in the production process 1; R⁶⁸-Q¹ is "an optionally substituted alkoxy group", "an optionally substituted aryloxy group", "an optionally substituted arylthio group" or "an optionally substituted heteroaryloxy group", which is represented by R² described in the item [1]; R⁶⁸-Q² is "an optionally substituted arylsulfonyl group" represented by R² described in the item [1]; E¹ is a chlorine atom, a bromine atom or an iodine atom; and M² is lithium, potassium or cesium.

### 1) Step 1

A compound (10-1) may be produced from a compound (8-1) by the same production process as described in literature (for example, R.C. Ralock, "Comprehensive Organic transformation", VCH publisher Inc., 972-976 (1989) and Eur. J. Org. Chem. 1353 (2000)) .

### 2) Step 2

A compound (10-2) may be produced from the compound (10-1) by the same production process as described in literature (for example, R.C. Ralock, "Comprehensive Organic transformation", VCH publisher Inc., 1989).

### 3) Step 3

A compound (10-4) may be produced from the compound (10-2) by the same production process as described in literature (for example, Heterocycles 52, 253 (2000), WO95/18109 and WO00/58309).

### 4) Step 4

The compound (10-5) may be produced from the compound (10-4) by the same production process as described in the step 6 in the production process 1.

### 5) Step 5

When Q¹ of the compound (10-4) is a sulfur atom, a compound (10-6) obtained by converting Q¹ of the compound (10-4) to sulfonyl may be produced by the same production process as described in literature (for example, R.C. Ralock, "Comprehensive Organic transformation", VCH publisher Inc., 1989).

### 6) Step 6

The compound (10-7) may be produced from the compound (10-6) by the same production process as described in the step 6 in the production process 1.

### Production Process 11

The compound of the formula (6-9) described in the production process 7 may be produced also by, for example, the following process: 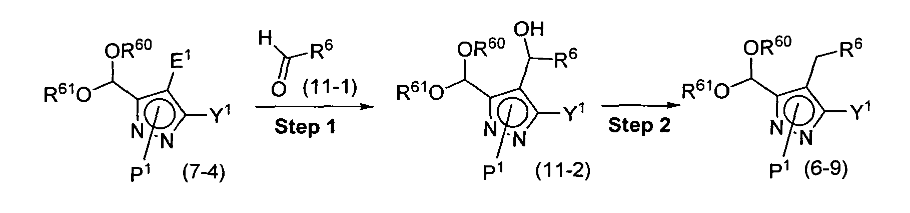 wherein R⁶ is as defined in the item [1]; Y¹ is as defined in the production process 1; P¹, R⁶⁰ and R⁶¹ are as defined in the production process 6; and E¹ is as defined in the production process 7.

### 1) Step 1

A compound (11-2) may be produced from a compound (7-4) by the same production process as described in literature (for example, Bioorg. Med. Chem. Lett. 8, 183 (1998) and Tetrahedron, 51, 11043 (1995)).

### 2) Step 2

The compound (6-9) may be produced from the compound (11-2) by the same production process as described in literature (for example, Bioorg. Med. Chem. Lett. 8, 183 (1998), Tetrahedron, 51, 11043 (1995), Tetrahedron, 57, 4817 (2001), Tetrahedron Lett. 42, 1073 (2001) and Bioorg. Med. Chem. Lett. 12, 2643 (2002)).

### Production Process 12

A compound of the formula (12-7) as the compound of the formula (I), or a salt thereof is produced, for example, by the following process: 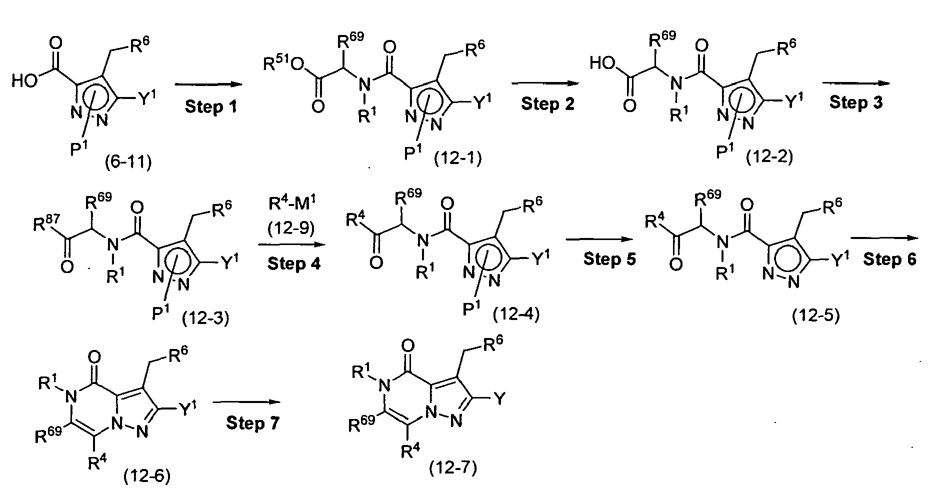 wherein R¹, R⁴, R⁶ and Y are as defined in the item [1]; Y¹ and R⁵¹ are as defined in the production process 1; R⁸⁷ and M¹ are as defined in the production process 9; R⁶⁹ is "a hydrogen atom", "an optionally substituted alkyl group", "an optionally substituted cycloalkyl group", "a carboxyl group", "an optionally substituted alkoxycarbonyl group", "an optionally substituted aryl group", "an optionally substituted aryloxycarbonyl group", "an optionally substituted aralkyl group", "an optionally substituted aroyl group", "an optionally substituted heteroaryl group", "an optionally substituted heteroarylalkyl group", "an optionally substituted heteroarylcarbonyl group" or "an optionally substituted alkylcarbonyl group", which is represented by R² described in the item [1]; and P¹ is as defined in the production process 6.

### 1) Step 1

A compound (12-1) may be produced by reacting a compound (6-11) with a compound represented by the formula: 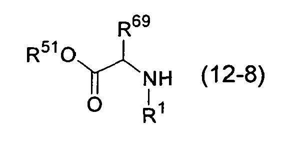 wherein R¹, R⁵¹ and R⁶⁹ are as defined above, by the same method as in the production process described in the step 3, (3) in the production process 1. As the compound (12-8), a commercial reagent may be used, or the compound (12-8) may be produced by the process described in literature (for example, Chem. Rev. 103, 3013 (2003), Chem. Rev. 103, 2795 (2003), Acc. Chem. Res. 36, 342 (2003), Acc. Chem. Res. 36, 10 (2003), J. Heterocyclic Chemistry 39, 437 (2002) and R.C. Ralock, "Comprehensive Organic transformation", VCH publisher Inc., 1989).

### 2) Step 2

A compound (12-2) may be produced from the compound (12-1) by the same production process as described in the step 3, (2) in the production process 1.

### 3) Step 3 to Step 4

A compound (12-4) may be produced from the compound (12-2) by the same production process as described in the step 1 to step 2 in the production process 9.

### 4) Step 5

A compound (12-5) may be produced from the compound (12-4) by the same production process as that described in literature (for example, Protective Groups in Organic Synthesis 2nd Edition (John Wiley & Sons, Inc.)) or the like.

### 5) Step 6

A compound (12-6) may be produced from the compound (12-5) by the same production process as described in the step 3, (4) in the production process 1.

In this reaction, a compound in which the protective group for primary amino group or secondary amino group in Y¹ has been removed is produced in some cases. The primary amino group or secondary amino group in Y may be protected again with a protective group (e.g. Boc or Cbz) by the same method as in the production process described in literature (for example, Protective Groups in Organic Synthesis 2nd Edition (John Wiley & Sons, Inc.)).

### 6) Step 7

The compound (12-7) may be produced from the compound (12-6) by the same production process as described in the step 6 in the production process 1.

### Production Process 13

Each of compounds of the formula (13-7) and the formula (13-10) as the compound of the formula (I), or a salt thereof is produced, for example, by the following process: 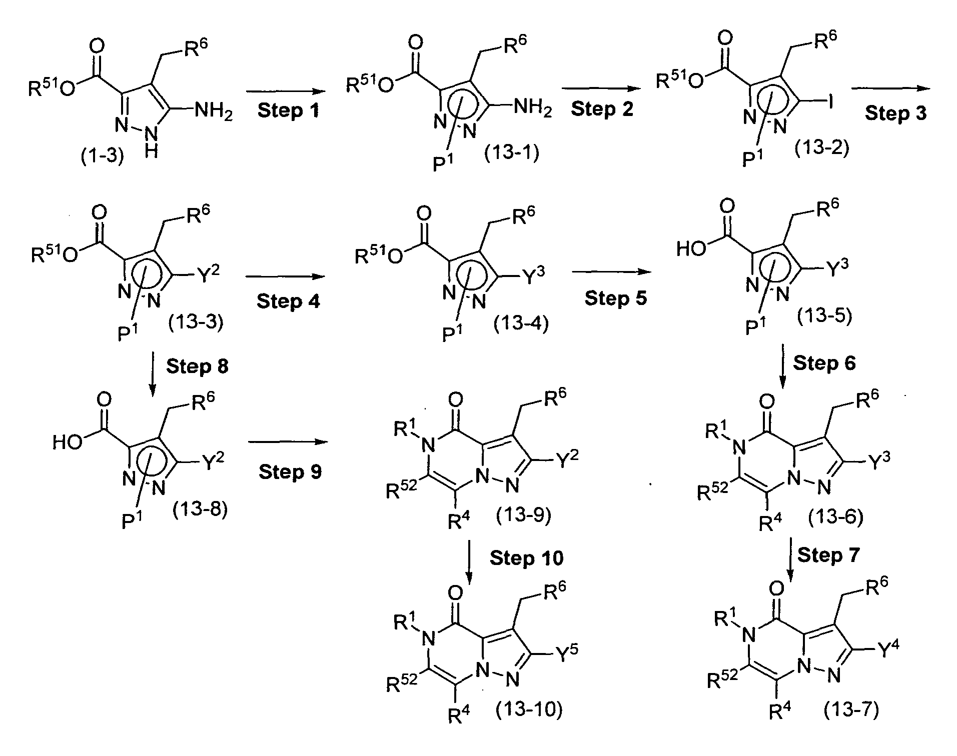 wherein R¹, R⁴ and R⁶ are as defined in the item [1]; R⁵¹ and R⁵² are as defined in the production process 1; P¹ is as defined in the production process 6; Y² represents the following formula (13-11), the following formula (13-12), the following formula (13-13) or the following formula (13-14): 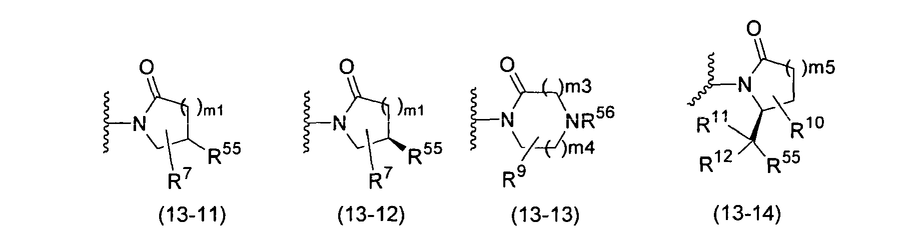 wherein m1, m3, m4, m5, R⁷, R⁹, R¹⁰, R¹¹, R¹², R⁵⁵ and R⁵⁶ are as defined above;
Y³ represents the following formula (13-16), the following formula (13-17), the following formula (13-18) or the following formula (13-19): 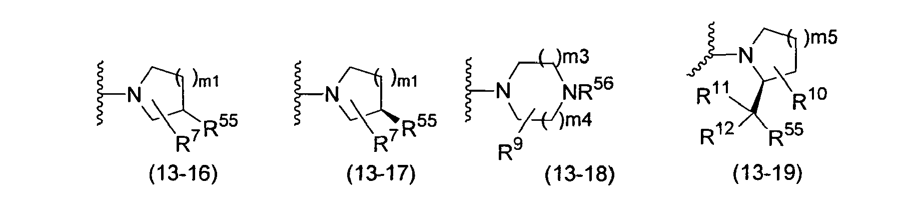 wherein m1, m3, m4, m5, R⁷, R⁹, R¹⁰, R¹¹, R¹², R⁵⁵ and R⁵⁶ are as defined above;
Y⁴ represents the following formula (13-20), the following formula (13-21), the following formula (13-22) or the following formula (13-23): 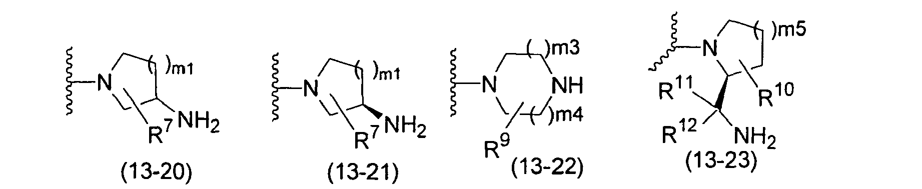 wherein m1, m3, m4, m5, R⁷, R⁹, R¹⁰, R¹¹ and R¹² are as defined above; and
Y⁵ represents the following formula (13-24), the following formula (13-25), the following formula (13-26) or the following formula (13-27): 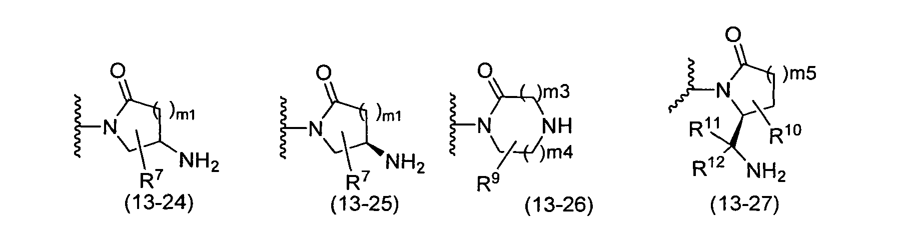 wherein m1, m3, m4, m5, R⁷, R⁹, R¹⁰, R¹¹ and R¹² are as defined above.

### 1) Step 1

A compound (13-1) may be produced from a compound (1-3) by the same production process as described in the step 6 in the production process 6.

### 2) Step 2

A compound (13-2) may be produced from the compound (13-1) by the same production process as described in the step 4 in the production process 1.

### 3) Step 3

A compound (13-3) may be produced by reacting the compound (13-2) with a compound selected from a compound (13-28), a compound (13-29), a compound (13-30) and a compound (13-31) which are represented by the formulas: 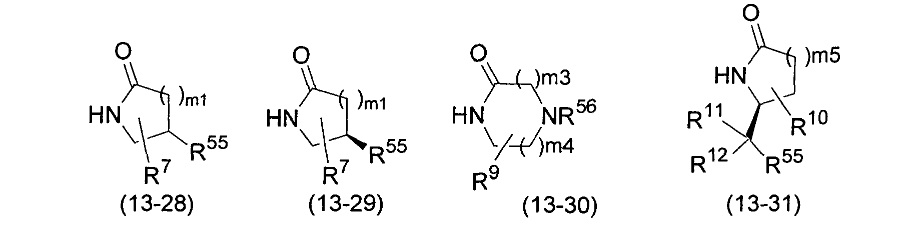 wherein m1, m3, m4, m5, R⁷, R⁹, R¹⁰, R¹¹, R¹², R⁵⁵ and R⁵⁶ are as defined above, and the compound of the formula (13-29) is the same as the compound of the formula (23-4) described in the production process 23, in an inert solvent in the presence of potassium phosphate, ethylene glycol and copper iodide. The amount of the compound (13-28), compound (13-29), compound (13-30) or compound (13-31) used is usually chosen in the range of 1 to 5 equivalents per equivalent of the compound (13-2). The amount of potassium phosphate used is usually chosen in the range of 1 to 5 equivalents per equivalent of the compound (13-2). The amount of ethylene glycol used is usually chosen in the range of 1 to 5 equivalents per equivalent of the compound (13-2). The amount of copper iodide used is usually chosen in the range of 1 to 5 equivalents per equivalent of the compound (13-2). The inert solvent includes, for example, alcohol solvents (e.g. methanol, ethanol and 2-propanol). The reaction temperature may be chosen in the range of about 50°C to about 150°C. It is also possible to carry out the reaction in a closed reaction vessel such as an autoclave.

The compound (13-29) and the compound (13-30) may be produced by the process described hereinafter as production process 23 and the process described hereinafter as production process 25, respectively. As the compound (13-28), a commercial reagent may be used, or the compound (13-28) may be produced by the process described in literature (for example, US5232929). As the compound (13-31), a commercial reagent may be used, or the compound (13-31) may be produced by the process described in literature (for example, Tetrahedron 51, 13309-20 (1995), Tetrahedron: Asymmetry 5, 887-94 (1994) and Tetrahedron: Asymmetry 4, 91-100 (1993)).

### 4) Step 4

A compound (13-4) may be produced by reacting the compound (13-3) with a reducing agent in an inert solvent. The reducing agent includes lithium aluminum hydride, borane complexes (e.g. borane-dimethyl sulfide complexes and borane-tetrahydrofuran complexes) and the like. The inert solvent includes tetrahydrofuran, 1,4-dioxane, mixed solvents thereof, and the like. The reaction temperature is chosen in the range of about - 20°C to about 60°C. A preferable reaction temperature is chosen in the range of about -10°C to about 30°C.

### 5) Step 5

A compound (13-5) may be produced from the compound (13-4) by the same production process as described in the step 3, (2) in the production process 1.

### 6) Step 6

A compound (13-6) may be produced from the compound (13-5) by the same production process as described in the step 3, (3) and (4) in the production process 1.

### 7) Step 7

The compound (13-7) may be produced from the compound (13-6) by the same production process as described in the step 6 in the production process 1.

### 8) Step 8

A compound (13-8) may be produced from the compound (13-3) by the same production process as described in the step 3, (2) in the production process 1.

### 9) Step 9

A compound (13-9) may be produced from the compound (13-8) by the same production process as described in the step 3, (3) and (4) in the production process 1.

### 10) Step 10

The compound (13-10) may be produced from the compound (13-9) by the same production process as described in the step 6 in the production process 1.

### Production Process 14

A compound of the formula (14-4) as the compound of the formula (I), or a salt thereof is produced, for example, by the following process: 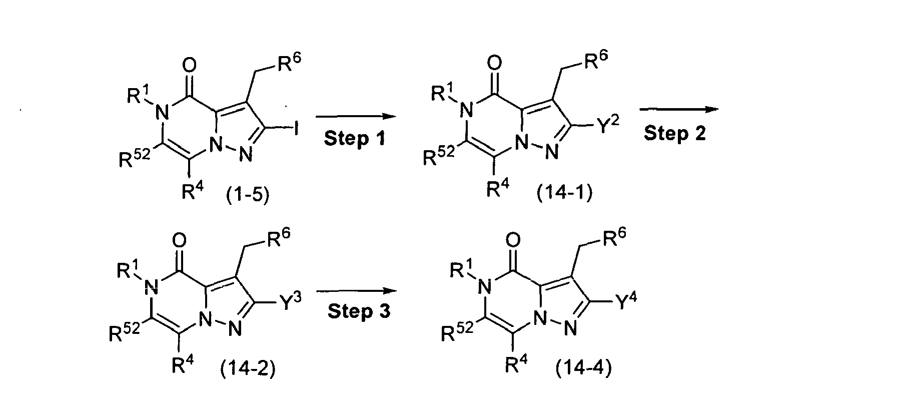 wherein R¹, R⁴ and R⁶ are as defined in the item [1]; R⁵² is as defined in the production process 1; and Y², Y³ and Y⁴ are as defined in the production process 13.

### 1) Step 1

A compound (14-1) may be produced from a compound (1-5) by the same production process as described in the step 3 in the production process 13.

### 2) Step 2

A compound (14-2) may be produced from the compound (14-1) by the same production process as described in the step 4 in the production process 13.

### 3) Step 3

The compound (14-4) may be produced from the compound (14-2) by the same production process as described in the step 6 in the production process 1.

### Production Process 15

A compound of the formula (15-10) as the compound of the formula (I), or a salt thereof is produced, for example, by the following process: 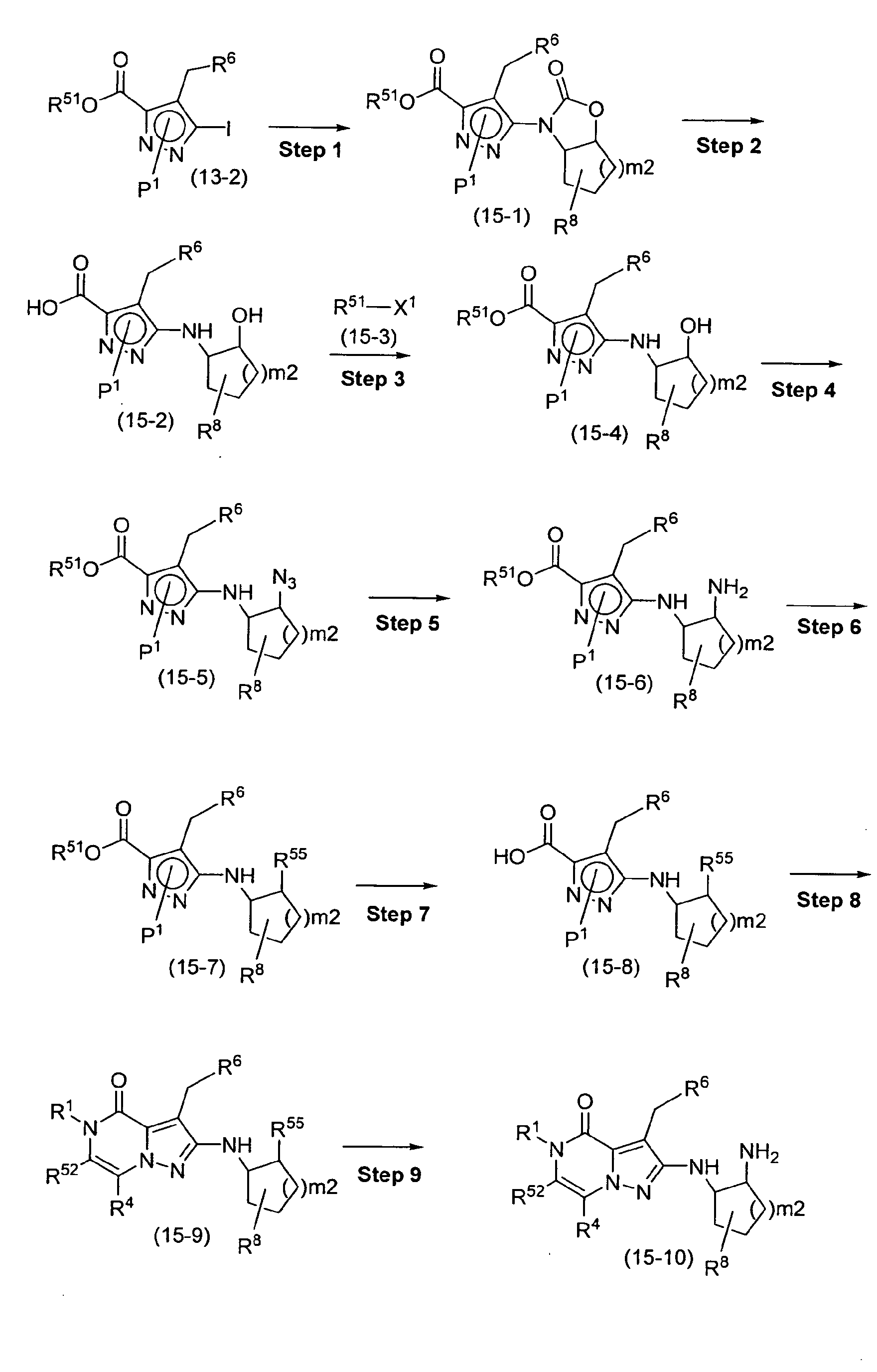 wherein m2, R¹, R⁴, R⁶ and R⁸ are as defined in the item [1]; R⁵¹, R⁵² and R⁵⁵ are as defined in the production process 1; and X¹ and P¹ are as defined in the production process 6.

### 1) Step 1

A compound (15-1) may be produced by reacting a compound (13-2) with a compound represented by the formula: 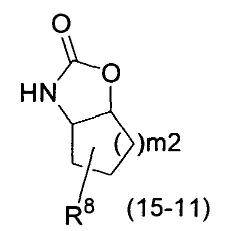 wherein R⁸ and m2 are as defined above, by the same method as in the production process described in literature (for example, J. Am. Chem. Soc. 124, 7421 (2002), Org. Lett. 5, 963 (2003) and Synlett 427 (2002)).

### 2) Step 2

A compound (15-2) may be produced from the compound (15-1) by the same production process as described in the step 3, (2) in the production process 1.

### 3) Step 3

A compound (15-4) may be produced by reacting the compound (15-2) with a compound (15-3) in an inert solvent in the presence of a base. The base includes sodium hydrogencarbonate, potassium hydrogencarbonate, sodium carbonate, potassium carbonate, lithium carbonate and the like. The amount of the base used is usually chosen in the range of 1 to 5 equivalents per equivalent of the compound (15-2). The amount of the compound (15-3) used is usually chosen in the range of 1 to 5 equivalents per equivalent of the compound (15-2). The inert solvent includes, for example, dimethylformamide, tetrahydrofuran and 1,4-dioxane. Mixed solvents thereof may also be used. The reaction temperature is chosen in the range of about -10°C to about 40°C.

### 4) Step 4

A compound (15-5) may be produced from the compound (15-4) by the same production process as described in literature (for example, Synthesis 130 (1990)).

### 5) Step 5

A compound of the formula (15-6) may be produced by reacting the compound of the formula (15-5) in an inert solvent under a hydrogen atmosphere in the presence of a catalyst such as palladium-carbon. The inert solvent includes, for example, alcohol solvents such as ethanol, methanol, 2-propanol, etc.; ether solvents such as tetrahydrofuran, 1,4-dioxane, etc.; aprotic solvents such as dimethylformamide, etc.; organic acids such as acetic acid, propionic acid, etc.; and hydrocarbon solvents such as benzene, toluene, xylene, etc. Mixed solvents of these solvents may also be used. The reaction temperature is chosen in the range of about 0°C to about 40°C.

### 6) Step 6

A compound (15-7) may be produced from the compound (15-6) by the same process as that described in literature (for example, Protective Groups in Organic Synthesis 2nd Edition (John Wiley & Sons, Inc.)) or the like.

### 7) Step 7

A compound (15-8) may be produced from the compound (15-7) by the same production process as described in the step 3, (2) in the production process 1.

### 8) Step 8

A compound (15-9) may be produced from the compound (15-8) by the same production process as described in the step 3, (3) to (4) in the production process 1.

### 9) Step 9

The compound (15-10) may be produced from the compound (15-9) by the same production process as described in the step 6 in the production process 1.

### Production Process 16

In the production process 15, the compound of the formula (15-8) or a salt thereof may be produced also by, for example, the following process: 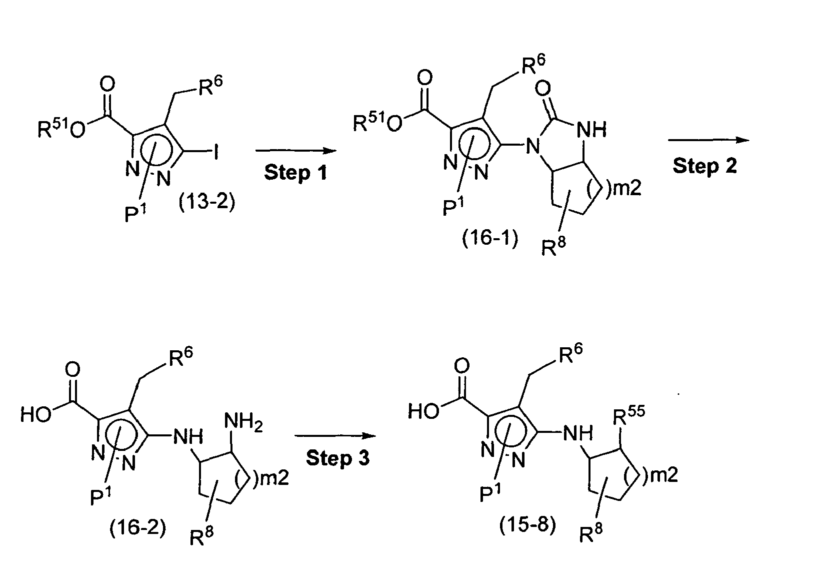 wherein m2, R⁶ and R⁸ are as defined in the item [1]; R⁵¹ and R⁵⁵ are as defined in the production process 1; and P¹ is as defined in the production process 6.

### 1) Step 1

A compound (16-1) may be produced by reacting a compound (13-2) with a compound (16-3) represented by the formula: 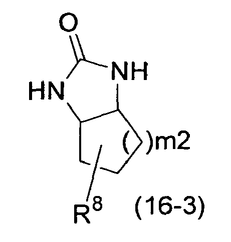 wherein R⁸ and m2 are as defined above, by the same method as in the production process described in literature (for example, J. Am. Chem. Soc. 124, 7421 (2002)).

### 2) Step 2

A compound (16-2) may be produced from the compound (16-1) by the same production process as described in the step 3, (2) in the production process 1.

### 3) Step 3

The compound (15-8) may be produced from the compound (16-2) by the same process as that described in literature (for example, Protective Groups in Organic Synthesis 2nd Edition (John Wiley & Sons, Inc.)) or the like.

### Production Process 17

A compound of the formula (17-4) as the compound of the formula (I), or a salt thereof is produced, for example, by the following process: 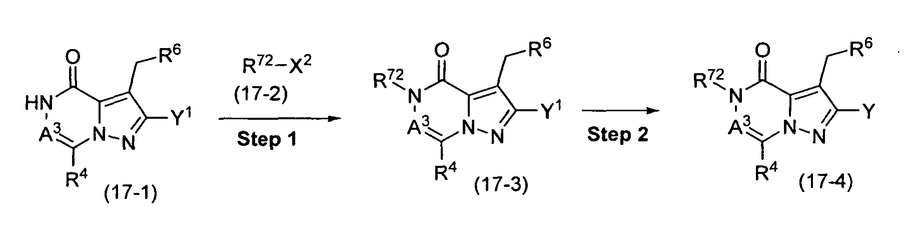 wherein R⁴, R⁶ and Y are as defined in the item [1]; A³ is as defined in the item [2]; Y¹ is as defined in the production process 1; the compound of the formula (17-1) is the compound (26-6) described in the production process 26 or the compound (27-3) described in the production process 27, or corresponds to the following compound when R¹ is a hydrogen atom: the compound (1-6) described in the production process 1 or the production process 6, the compound (8-1), compound (8-4) or compound (8-6) described in the production process 8, the compound (9-3) described in the production process 9, the compound (10-4) or compound (10-6) described in the production process 10, the compound (12-6) described in the production process 12, the compound (13-6) or compound (13-9) described in the production process 13, the compound (14-2) described in the production process 14, the compound (15-9) described in the production process 15, the compound (28-10) described in the production process 28, the compound (29-7) described in the production process 29, or the compound (30-2) described in the production process 30; R⁷² is "an optionally substituted alkyl group" or "an optionally substituted cycloalkyl group", which is represented by R¹ described in the item [1]; and X² is a leaving group (for example, a bromine atom, a chlorine atom, methanesulfonyloxy, trifluoromethanesulfonyloxy or p-toluenesulfonyloxy).

### 1) Step 1

A compound (17-3) may be produced by reacting a compound (17-1) with a compound (17-2) in an inert solvent in the presence of a base. The base includes potassium tert-butoxide, sodium tert-butoxide, cesium carbonate, potassium carbonate, sodium carbonate, sodium phenoxide, potassium phenoxide, sodium hydride and the like. The amount of the base used is usually chosen in the range of 1 to 5 equivalents per equivalent of the compound (17-3). The inert solvent includes tetrahydrofuran, 1,4-dioxane, dimethylformamide, mixed solvents thereof, and the like. The reaction temperature may be chosen in the range of about 10°C to about 50°C.

### 2) Step 2

The compound (17-4) may be produced from the compound (17-3) by the same production process as described in the step 6 in the production process 1.

### Production Process 18

A compound of the formula (18-3) as the compound of the formula (I), or a salt thereof is produced, for example, by the following process: 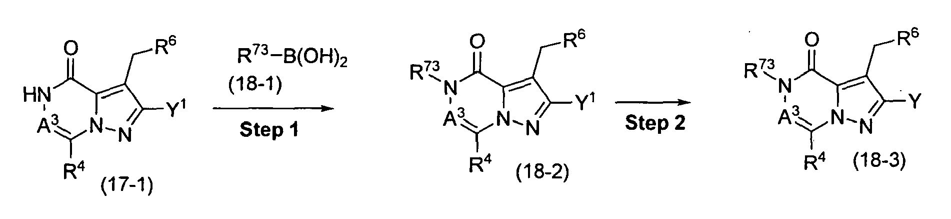 wherein R⁴, R⁶ and Y are as defined in the item [1]; A³ is as defined in the item [2]; Y¹ is as defined in the production process 1; the formula (17-1) is as defined above; and R⁷³ is "an optionally substituted aryl group" or "an optionally substituted heteroaryl group", which is represented by R¹ described in the item [1].

### 1) Step 1

A compound (18-2) may be produced from a compound (17-1) by the same process as that described in literature (for example, Tetrahedron 55, 12757 (1999)) or the like.

### 2) Step 2

The compound (18-3) may be produced from the compound (18-2) by the same production process as described in the step 6 in the production process 1.

### Production Process 19

Each of compounds of the formula (19-3), the formula (19-6) and the formula (19-9) as the compound of the formula (I), or a salt thereof is produced, for example, by the following process: 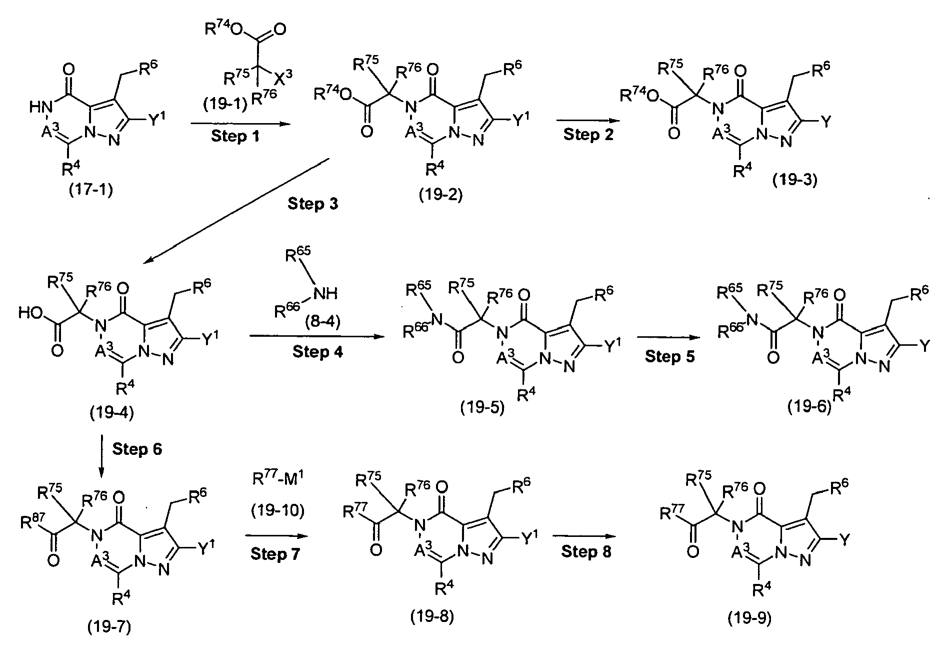 wherein R⁴, R⁶ and Y are as defined in the item [1]; A³ is as defined in the item [2]; Y¹ is as defined in the production process 1; the formula (17-1) is as defined above; R⁶⁵ and R⁶⁶ are as defined in the production process 8; R⁸⁷ and M¹ are as defined in the production process 9; X³ is a leaving group (for example, a bromine atom, a chlorine atom, methanesulfonyloxy, trifluoromethanesulfonyloxy or p-toluenesulfonyloxy); R⁷⁴ is an alkyl group; R⁷⁵ and R⁷⁶ are independently a hydrogen atom, methyl, ethyl, propyl, isopropyl, methoxy, ethoxy or isopropoxy, or R⁷⁵ and R⁷⁶, when taken together with the adjacent carbon atom, may form cyclopropyl, cyclobutyl or cyclopentyl; and R⁷⁷ is an optionally substituted alkyl group, an optionally substituted aryl group or an optionally substituted heteroaryl group.

### 1) Step 1

A compound (19-2) may be produced from a compound (17-1) by the same production process as described in the step 1 in the production process 17.

### 2) Step 2

The compound (19-3) may be produced from the compound (19-2) by the same production process as described in the step 6 in the production process 1.

### 3) Step 3

A compound (19-4) may be produced from the compound (19-2) by the same production process as described in the step 3, (2) in the production process 1.

### 4) Step 4

A compound (19-5) may be produced from the compound (19-4) by the same production process as described in the step 2 in the production process 8.

### 5) Step 5

The compound (19-6) may be produced from the compound (19-5) by the same production process as described in the step 6 in the production process 1.

### 6) Step 6 to Step 7

A compound (19-8) may be produced from the compound (19-4) by the same production process as described in the step 1 to step 2 in the production process 9.

### 7) Step 8

The compound (19-9) may be produced from the compound (19-8) by the same production process as described in the step 6 in the production process 1.

### Production Process 20

A compound of the formula (20-3) as the compound of the formula (I), or a salt thereof is produced, for example, by the following process: 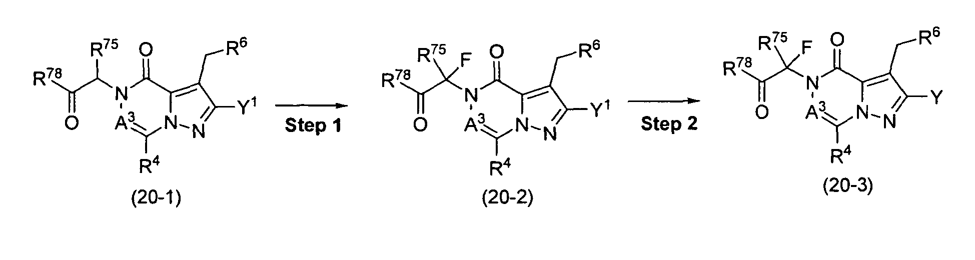 wherein R⁴, R⁶ and Y are as defined in the item [1]; A³ is as defined in the item [2]; Y¹ is as defined in the production process 1; the compound of the formula (20-1) corresponds to the compound (19-2), compound (19-5) or compound (19-8) described in the production process 19 when R⁷⁶ is a hydrogen atom; R⁷⁸ corresponds to R⁷⁴O, R⁶⁵R⁶⁶N or R⁷⁷ in the production process 19; and R⁶⁵, R⁶⁶, R⁷⁴ and R⁷⁷ are as defined above.

### 1) Step 1

A compound (20-2) may be produced from a compound (20-1) by the same production process as described in literature (for example, Angew. Chem. 108, 1082 (1996), Bioorg. Med. Chem. Lett. 8, 3275 (1998) and Tetrahedron Lett. 32, 1779 (1991)).

### 2) Step 2

The compound (20-3) may be produced from the compound (20-2) by the same production process as described in the step 6 in the production process 1.

### Production Process 21

A compound of the formula (21-7) as the compound of the formula (I), or a salt thereof is produced, for example, by the following process: 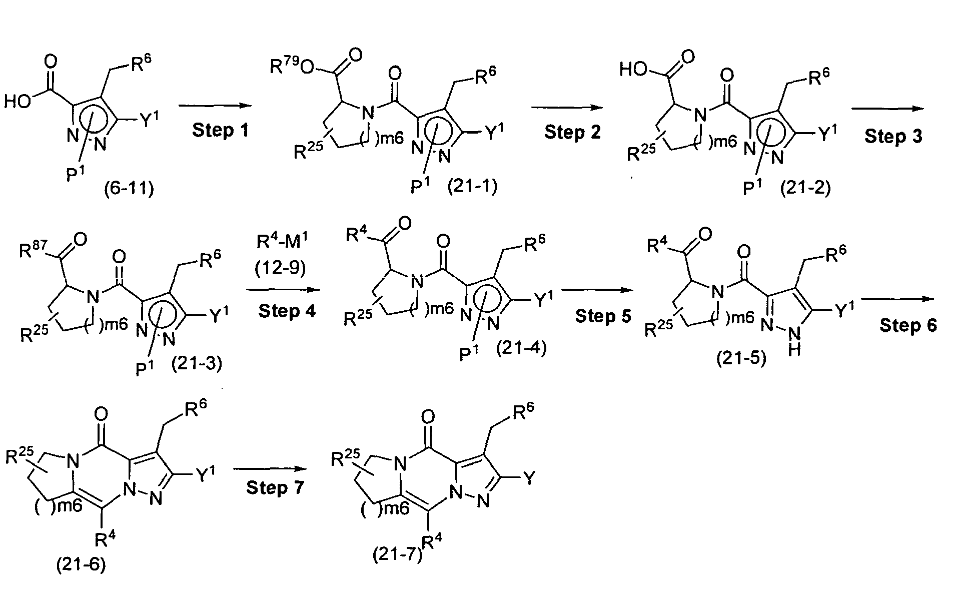 wherein R⁴, R⁶ and Y are as defined in the item [1]; R⁷⁹ is an alkyl group; Y¹ is as defined in the production process 1; P¹ is as defined in the production process 6; R⁸⁷ and M¹ are as defined in the production process 9; and m6 and R²⁵ are as defined above.

### 1) Step 1

A compound (21-1) may be produced by reacting a compound (6-11) with a compound (21-8) represented by the formula: 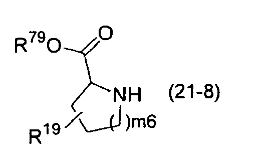 wherein m6, R²⁵ and R⁷⁹ are as defined above, by the same method as in the production process described in the step 3, (3) in the production process 1.

### 2) Step 2

A compound (21-2) may be produced from the compound (21-1) by the same production process as described in the step 3, (2) in the production process 1.

### 3) Step 3 to Step 4

A compound (21-4) may be produced from the compound (21-2) by the same production process as described in the step 1 to step 2 in the production process 9.

### 4) Step 5

A compound (21-5) may be produced from the compound (21-4) by the same process as that described in literature (for example, Protective Groups in Organic Synthesis 2nd Edition (John Wiley & Sons, Inc.)) or the like.

### 5) Step 6

A compound (21-6) may be produced from the compound (21-5) by the same production process as described in the step 3, (4) in the production process 1.

### 6) Step 7

The compound (21-7) may be produced from the compound (21-6) by the same production process as described in the step 6 in the production process 1.

### Production Process 22

The compound (1-10) described in the production process 1 may be produced, for example, by the following process: 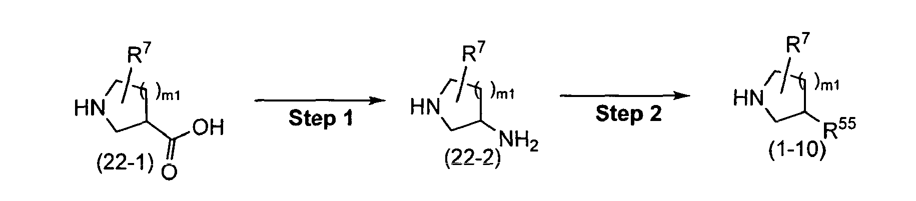 wherein R⁷ and ml are as defined in the item [1], and R⁵⁵ is as defined in the production process 1.

### 1) Step 1

A compound (22-2) may be produced from a compound (22-1) by the same production process as described in literature (for example, J. Org. Chem. 58, 879 (1993)).

### 2) Step 2

The compound (1-10) may be produced from the compound (22-2) by the same process as that described in literature (for example, Protective Groups in Organic Synthesis 2nd Edition (John Wiley & Sons, Inc.)) or the like.

### Production Process 23

Each of the compound (1-11) described in the production process 1 and the compound described in the production process 13 may be produced, for example, by the following process: 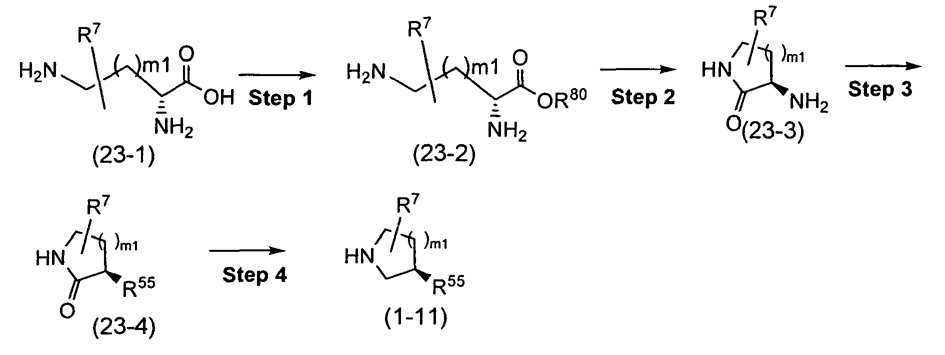 wherein R⁷ and ml are as defined in the item [1]; R⁵⁵ is as defined in the production process 1; and R⁸⁰ is an alkyl group.

### 1) Step 1

A compound (23-2) may be produced by reacting a compound (23-1) with thionyl chloride in an alcohol solvent. The alcohol solvent includes methanol, ethanol and the like. The amount of thionyl chloride used is usually chosen in the range of 2 to 10 equivalents per equivalent of the compound (23-1). The reaction temperature may be chosen in the range of about -90°C to about 30°C.

### 2) Step 2

A compound (23-3) may be produced by reacting the compound (23-2) with a base in water solvent. The base includes sodium hydrogencarbonate, potassium hydrogencarbonate, sodium carbonate, potassium carbonate and the like. The reaction temperature may be chosen in the range of about 30°C to about 100°C.

### 3) Step 3

A compound (23-4) may be produced from the compound (23-3) by the same process as that described in literature (for example, Protective Groups in Organic Synthesis 2nd Edition (John Wiley & Sons, Inc.)) or the like.

### 4) Step 4

The compound (1-11) may be produced by reacting the compound (23-4) with a reducing agent in an inert solvent. The reducing agent includes lithium aluminum hydride, borane complexes (e.g. borane-dimethyl sulfide complexes and borane-tetrahydrofuran complexes) and the like. The inert solvent includes tetrahydrofuran, 1,4-dioxane, mixed solvents thereof, and the like. The reaction temperature is chosen in the range of about -20°C to about 60°C.

Examples of synthesis of compounds (1-10a) to (1-10j) as specific examples of the compound (1-10) are given below. The compounds (1-10a) to (1-10j) include pharmaceutically acceptable salts thereof.

| Compound | Production process |
|---|---|
| 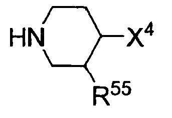 | WO 02/48138 |
| | J. Chem. Soc., Perkin Trans. 1, 2233 (1999) Protective Groups in Organic Synthesis 2nd Edition (John Wiley & Sons, Inc.) |
| (1-10a): X⁴ = CH₃ | |
| (1-10b): X⁴ = CH₂CH₃ | |
| (1-10c): X⁴ = CH₂CH₂OH | |
| (1-10d): X⁴ = CH₂CH₂F | |
| (1-10e): X⁴ = H | |
| 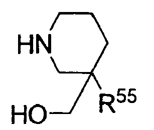 | J. Org. Chem. 44, 2732 (1979) |
| (1-10f) | J. Chem. Soc., Perkin Trans. 1, 2233 (1999) Protective Groups in Organic Synthesis 2nd Edition (John Wiley & Sons, Inc.) |
| 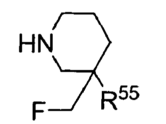 | Compound (1-10f) is used as a starting material and the process described in, for example, J. Org. Chem. 44, 3872 (1979), J. Chem. Soc., Perkin Trans. 1, 2233 (1999) Protective Groups in Organic Synthesis 2nd Edition (John Wiley & Sons, Inc.) is adopted. |
| (1-10g) | |
| 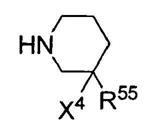 | Arch. Pharm. 322, 499 (1989) J. Chem. Soc., Perkin Trans. 1, 2233 (1999) Protective Groups in Organic Synthesis 2nd Edition (John Wiley & Sons, Inc.) |
| (1-10h): X⁴ = CH₃ | |
| (1-10i): X⁴ = CH₂CH₃ | |
| (1-10j): X⁴= CH₂CH₂CH₃ | |
| In the above formulas, R⁵⁵ is as defined in the production process 1. | |

As hydrochloride of the compound (1-10e), a commercial one may also be used. It is also possible to synthesize the compound (1-10) from a substituted DL-ornithine by a well-known process. A specific example of the process is the process described in literature (for example, R.C. Ralock, "Comprehensive Organic transformation", VCH publisher Inc., 1989).

Examples of synthesis of compounds (1-11a) to (1-11j) as specific examples of the compound (1-11) are given below. The compounds (1-11a) to (1-11j) include pharmaceutically acceptable salts thereof.

| Compound | Production process |
|---|---|
| 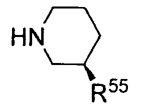 | WO 01/27082 J. Chem. Soc., Perkin Trans. 1, 2233 (1999) Protective Groups in Organic Synthesis 2nd Edition (John Wiley & Sons, Inc.) |
| (1-11a) | |
| 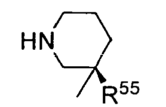 | lnt. J. Peptide Protein Res. 40, 119 (1992) WO 01/27082 J. Chem. Soc., Perkin Trans. 1, 2233 (1999) Protective Groups in Organic Synthesis 2nd Edition (John Wiley & Sons, Inc.) |
| (1-11b) | |
| 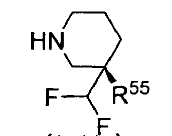 | US 4413141 WO 01/27082 J. Chem. Soc., Perkin Trans. 1, 2233 (1999) Protective Groups in Organic Synthesis 2nd Edition (John Wiley & Sons, Inc.) |
| (1-11c) | |
| 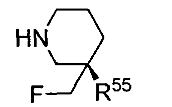 | Tetrahedron: Asymmetry 8, 327 (1997) WO 01/27082 J. Chem. Soc., Perkin Trans. 1, 2233 (1999) Protective Groups in Organic Synthesis 2nd Edition (John Wiley & Sons, Inc.) |
| (1-11d) | |
| 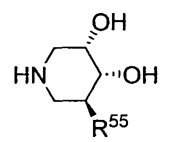 | Tetrahedron: Asymmetry 11, 567 (2000) J. Chem. Soc., Perkin Trans. 1, 2233 (1999) Protective Groups in Organic Synthesis 2nd Edition (John Wiley & Sons, Inc.) |
| (1-11e) | |
| 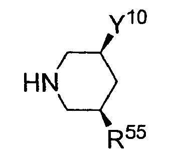 | Chem. Eur. J. 6, 2830 (2000) WO 00/26332 J. Chem. Soc., Perkin Trans. 1, 2233 (1999) Protective Groups in Organic Synthesis 2nd Edition (John Wiley & Sons, Inc.) |
| (1-11f) | |
| 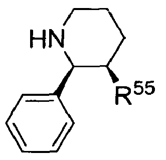 | Japanese Patent Application Kohyo No. 2002-525325 J. Chem. Soc., Perkin Trans. 1, 2233 (1999) Protective Groups in Organic Synthesis 2nd Edition (John Wiley & Sons, Inc.) |
| (1-11g) | |
| 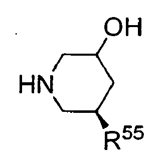 | Bull. Chem. Soc. Jpn. 53, 2605 (1980) J. Chem. Soc., Perkin Trans. 1, 2233 (1999) Protective Groups in Organic Synthesis 2nd Edition (John Wiley & Sons, Inc.) |
| (1-11h) | |
| 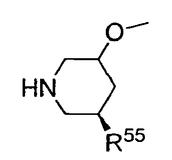 | Compound (1-11h) is used as a starting material and the process described in, for example, J. Am. Chem. Soc. 80, 2584 (1958). J. Chem. Soc. PT1 499 (1972). J. Chem. Soc., Perkin Trans. 1, 2233 (1999) Protective Groups in Organic Synthesis 2nd Edition (John Wiley & Sons, Inc.) is adopted. |
| (1-11i) | |
| In the above formulas, R⁵⁵ is as defined in the production process 1, and Y¹⁰ is NH₂, NHBoc or NHCbz. | |

Examples of synthesis of compounds (1-11j) to (1-11v) as specific examples of the compound (1-11) are given below. The compounds (1-11j) to (1-11v) include pharmaceutically acceptable salts thereof.

| Compound | Production process |
|---|---|
| 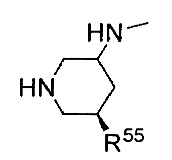 | Compound (1-11f in which R⁵⁵ is NH₂) is used as a starting material and the process described in, for example, J. Chem. Soc. Chem. Commun. 611 (1981), J. Chem. Soc., Perkin Trans. 1, 2233 (1999) Protective Groups in Organic Synthesis 2nd Edition (John Wiley & Sons, Inc.) is adopted. |
| (1-11j) | |
| 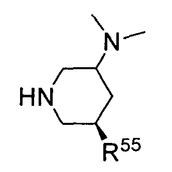 | Compound (1-11f in which R⁵⁵ is NH₂) is used as a starting material and the process described in, for example, J. Chem. Soc. Chem. Commun. 611 (1981), J. Chem. Soc., Perkin Trans. 1, 2233 (1999) Protective Groups in Organic Synthesis 2nd Edition (John Wiley & Sons, Inc.) is adopted. |
| (1-11k) | |
| 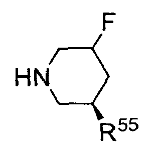 | Compound (1-11h) is used as a starting material and the process described in, for example, J. Org. Chem. 44, 3872 (1979), J. Chem. Soc., Perkin Trans. 1, 2233 (1999) Protective Groups in Organic Synthesis 2nd Edition (John Wiley & Sons, Inc.) is adopted. |
| (1-11l) | |
| 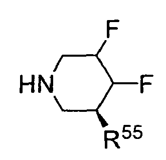 | Compound (1-11e) is used as a starting material and the process described in, for example, J. Org. Chem. 44, 3872 (1979), J. Chem. Soc., Perkin Trans. 1, 2233 (1999) Protective Groups in Organic Synthesis 2nd Edition (John Wiley & Sons, Inc.) is adopted. |
| (1-11m) | |
| 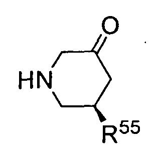 | Compound (1-11h) is used as a starting material and the process described in, for example, Bull. Chem. Soc. Jpn. 64, 2857 (1991), J. Chem. Soc., Perkin Trans. 1, 2233 (1999) Protective Groups in Organic Synthesis 2nd Edition (John Wiley & Sons, Inc.) is adopted. |
| (1-11n) | |
| 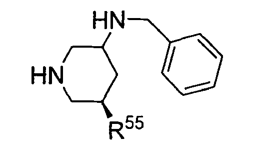 | Compound (1-11f i which R⁵⁵ is NH₂) is used as a starting material and the process described in, for example, Tetrahedron Lett. 40, 5609(1999), J. Chem. Soc., Perkin Trans. 1, 2233 (1999) Protective Groups in Organic Synthesis 2nd Edition (John Wiley & Sons, Inc.) is adopted. |
| (1-11o) | |
| 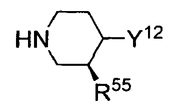 | J. Med. Chem. 35, 833 (1992), R.C. Ralock, "Comprehensive Organic transformation", VCH publisher Inc., 1989, J. Chem. Soc., Perkin Trans. 1, 2233 (1999) Protective Groups in Organic Synthesis 2nd Edition (John Wiley & Sons, Inc.) |
| (1-11p): Y¹² = (R)-C₆H₅ | |
| (1-11q): Y¹² = (S)-C₆H₅ | |
| 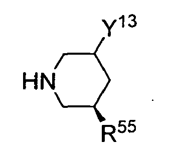 | Compound (1-11f in which R⁵⁵ is NH₂) is used as a starting material and the process described in, for example, R.C. Ralock, "Comprehensive Organic transformation", VCH publisher Inc., 1989. J. Chem. Soc., Perkin Trans. 1, 2233 (1999) Protective Groups in Organic Synthesis 2nd Edition (John Wiley & Sons, Inc.) is adopted. |
| (1-11r): Y¹³ = NHS(O)₂CH₃ | |
| (1-11s): Y¹³ = NHC(O)CH₃ | |
| (1-11t): Y¹³ = NHC(O)C₆H₅ | |
| (1-11u): Y¹³ = N(CH₃)C(O)CH₃ | |
| 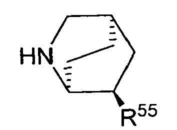 | WO 02/068420 J. Chem. Soc., Perkin Trans. 1, 2233 (1999) Protective Groups in Organic Synthesis 2nd Edition (John Wiley & Sons, Inc.) |
| (1-11v) | |
| In the above formula, R⁵⁵ is as defined in the production process 1. | |

Examples of synthesis of compounds (1-11w) to (1-11dd) as specific examples of the compound (1-11) are given below. The compounds (1-11w) to (1-11dd) include pharmaceutically acceptable salts thereof.

| | |
|---|---|
| Compound | Production Process |
| 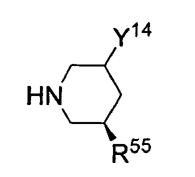 | |
| (1-11w): Y¹⁴=2-CH₃-C₆H₅ | Compound (1-11n) is used as a starting material and the process described in, for example, R.C. Ralock, "Comprehensive Organic transformation", VCH publisher Inc., 1989 J. Org.. Chem. 66, 3593 (2001), J. Prakt. Chem. 342, 421 (2000), Tetrahedron Lett. 36, 5611 (1994), J. Org.. Chem. 53, 5143 (1988), Bioorg. Med. Chem. Lett. 11, 1281 (2001), J. Chem. Soc., Perkin Trans. 1, 2233 (1999) Protective Groups in Organic Synthesis 2nd Edition (John Wiley & Sons, Inc.) is adopted. |
| (1-11x): Y¹⁴ = 3-CH₃-C₆H₅ | |
| (1-11y): Y¹⁴ = 4-CH₃-C₆H₅ | |
| (1-11z): Y¹⁴ = 2-CH₃O-C₆H₅ | |
| (1-11aa): Y¹⁴ = 3-CH₃O-C₆H₅ | |
| (1-11bb): Y¹⁴ = 4-CH₃O-C₆H₅ | |
| (1-11cc): Y¹⁴ = C₆H₅ | |
| (1-11dd): Y¹⁴ = CH₂C₆H₅ | |
| In the above formula, R⁵⁵ is as defined in the production process 1. | |

The compound (1-11) may be synthesized from a substituted D-ornithine by a well-known process. A specific example of the process is the process described in literature (for example, R.C. Ralock, "Comprehensive Organic transformation", VCH publisher Inc., 1989).

### Production Process 24

The compound (1-13) described in the production process 1 may be produced, for example, by the following process: 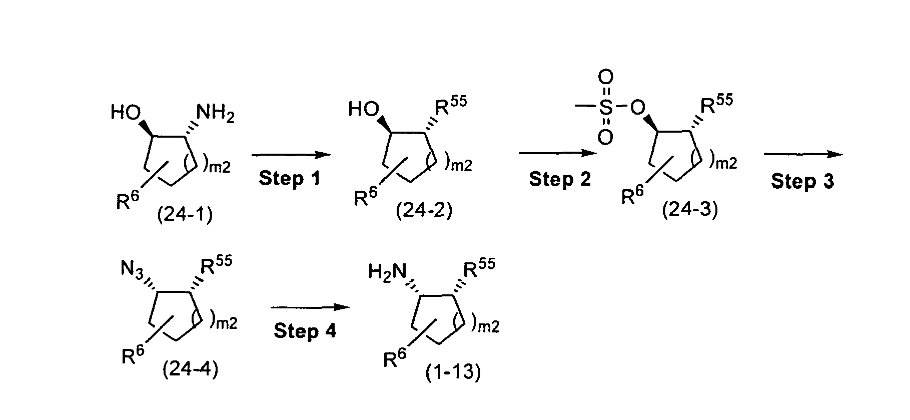 wherein R⁶ and m2 are as defined in the item [1], and R⁵⁵ is as defined in the production process 1.

### 1) Step 1

A compound (24-2) may be produced from a compound (24-1) by the same process as described in literature (for example, Protective Groups in Organic Synthesis 2nd Edition (John Wiley & Sons, Inc.)) or the like. The compound (24-1) may be produced by the same production process as described in literature (for example, J. Org. Chem. 50, 4154 (1985)).

### 2) Step 2 to Step 4

The compound (1-13) may be produced from the compound (24-2) by the same process as described in literature (for example, R.C. Ralock, "Comprehensive Organic transformation", VCH publisher Inc., 1989).

Examples of synthesis of compounds (1-13a) to (1-13aa) as specific examples of the compound (1-13) are given below. The compounds (1-13a) to (1-13aa) include pharmaceutically acceptable salts thereof. The compounds (1-13a) to (1-13aa) may be produced according to the processes described in literature (for example, WO01/74774, and R.C. Ralock, "Comprehensive Organic transformation", VCH publisher Inc., 1989). 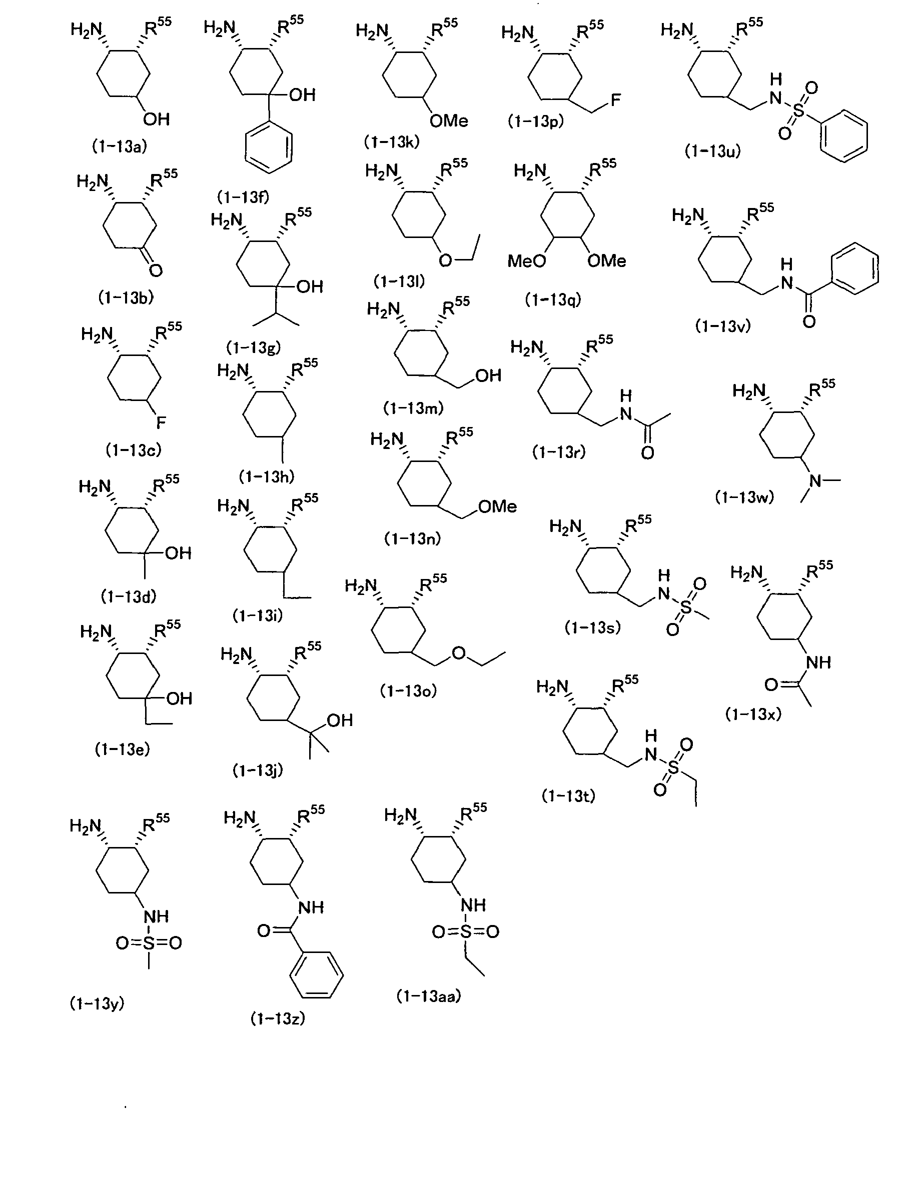 In the above formulas, R⁵⁵ is as defined in the production process 1.

Examples of synthesis of compounds (1-13bb) to (1-13tt) as specific examples of the compound (1-13) are given below. The compounds (1-13bb) to (1-13tt) include pharmaceutically acceptable salts thereof. The compounds (1-13bb) to (1-13tt) may be produced according to the processes described in literature (for example, WO01/74774, R.C. Ralock, "Comprehensive Organic transformation", VCH publisher Inc., 1989, and Protective Groups in Organic Synthesis 2nd Edition (John Wiley & Sons, Inc.)). 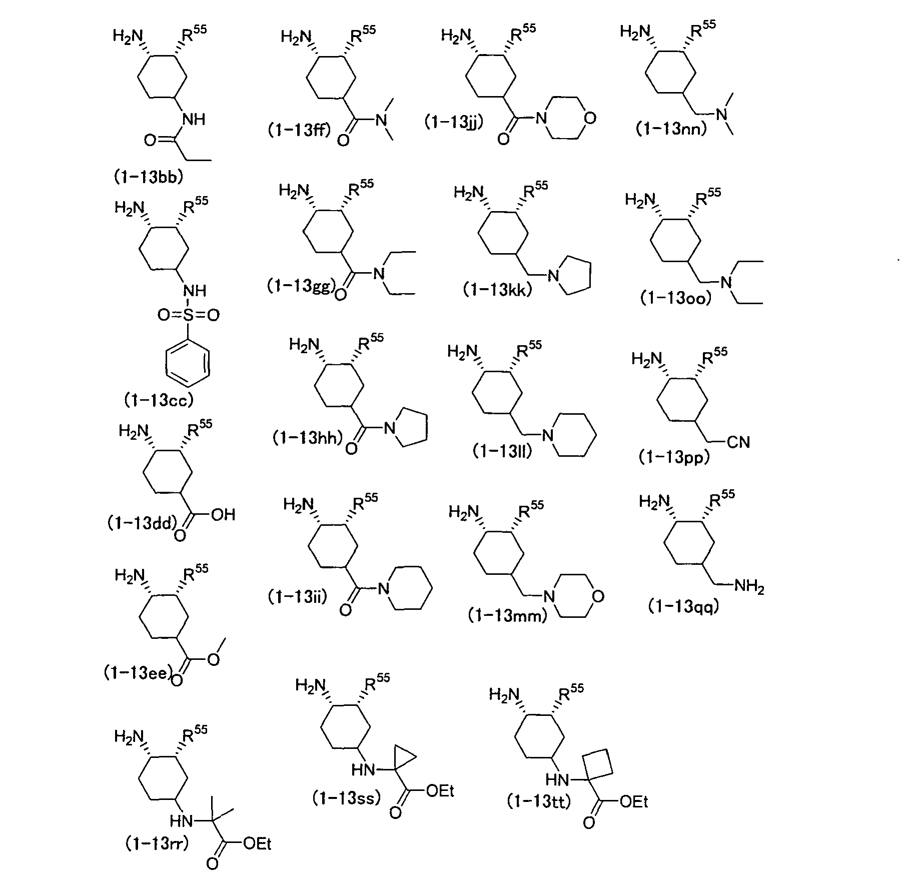 In the above formulas, R⁵⁵ is as defined in the production process 1.

### Production Process 25

Each of a compound (25-9) as a specific example of the compound (1-14) described in the production process 1 and a compound (25-8) as a specific example of the compound (13-10) described in the production process 13 may be produced according to, for example, the following process: 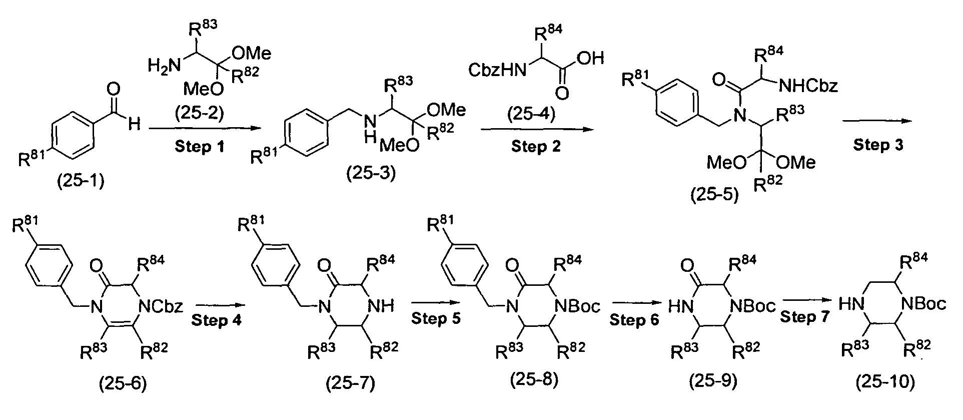 wherein R⁸², R⁸³ and R⁸⁴ are independently "a hydrogen atom", "an optionally substituted alkyl group", "an optionally substituted aryl group" or "an optionally substituted aralkyl group", and R⁸¹ is a hydrogen atom or methoxy.

### 1) Step 1

A compound (25-3) may be produced by carrying out reductive amination of a compound (25-1) with a compound (25-2) by the same method as described in literature (for example, R.C. Ralock, "Comprehensive Organic transformation", VCH publisher Inc., 1989).

### 2) Steps 2 to 4

A compound (25-7) may be produced from the compound (25-3) by the same production process as described in literature (WO01/07436 and the like).

### 3) Step 5

The compound (25-8) may be produced from the compound (25-7) by the same production process as described in literature (for example, Protective Groups in Organic Synthesis 2nd Edition (John Wiley & Sons, Inc.)).

### 4) Step 6

The compound (25-9) may be produced from the compound (25-8) by the same production process as described in literature (for example, J. Chem. Soc. Perkin Trans. I 3281 (2001), Heterocycles 38, 17 (1994), Tetrahedron Lett. 34, 6673 (1993), J. Org. Chem. 60, 4602 (1995) and J. Med. Chem. 38, 2866 (1995)).

### 5) Step 7

A compound (25-10) may be produced from the compound (25-9) by the same process as that described in literature (for example, R.C. Ralock, "Comprehensive Organic transformation", VCH publisher Inc., 1989) or the like.

Examples of synthesis of compounds (25-9a) to (25-91) as specific examples of the compound (25-9) are given below. The compounds (25-9a) to (25-91) include pharmaceutically acceptable salts thereof.

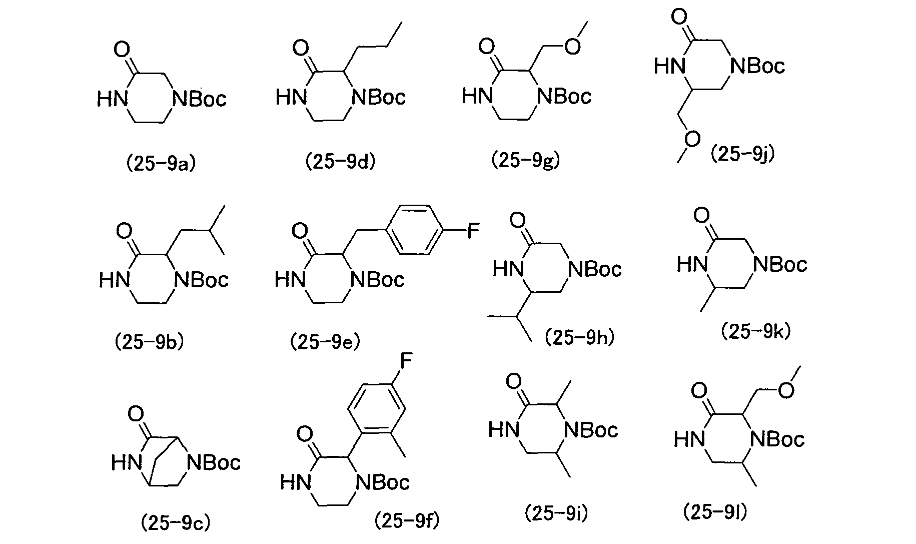

Examples of synthesis of compounds (25-10a) to (25-101) as specific examples of the compound (25-10) are given below. The compounds (25-10a) to (25-101) include pharmaceutically acceptable salts thereof.

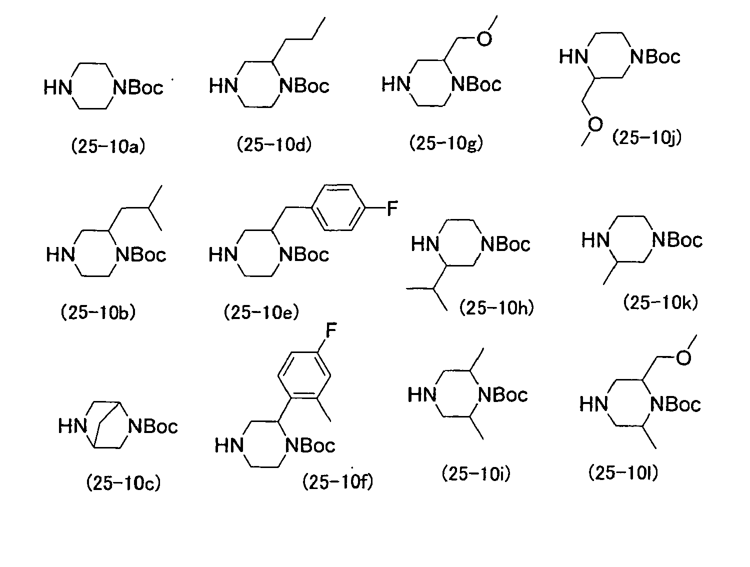

### Production Process 26

A compound of the formula (26-7) as the compound of the formula (I), or a salt thereof is produced, for example, by the following process: 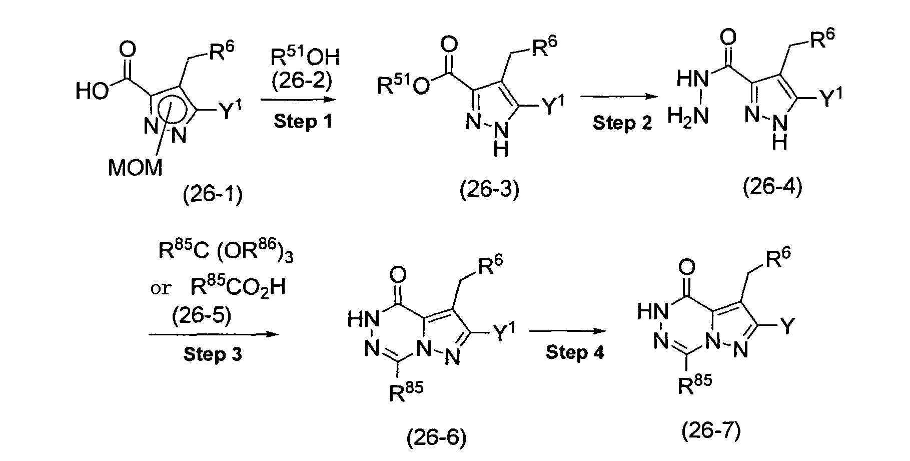 wherein R⁶ and Y are as defined in the item [1]; R⁵¹ and Y¹ are as defined in the production process 1; the compound of the formula (26-1) corresponds to the compound of the formula (6-11) described in the production process 6 when P¹ is a MOM group; R⁸⁵ is "a hydrogen atom", "an optionally substituted alkyl group", "an optionally substituted cycloalkyl group", "an optionally substituted alkoxy group", "an optionally substituted alkoxycarbonyl group", "an optionally substituted alkylamino group", "an optionally substituted aryl group", "an optionally substituted aralkyl group", "an optionally substituted heteroaryl group" or "an optionally substituted heteroarylalkyl group"; and R⁸⁶ is an alkyl group.

### 1) Step 1

A compound (26-3) may be produced by reacting a compound (26-1) with a compound (26-2) in an alcohol solvent in the presence of an acid. The acid includes hydrochloric acid, phosphoric acid, sulfuric acid and the like. A preferable example thereof is hydrochloric acid. The acid may be used as a solvent. The amount of the acid used is usually chosen in the range of a catalytic amount to a large excess over the alcohol solvent. The alcohol solvent includes, for example, methanol, ethanol and 2-propanol. The reaction temperature is chosen in the range of about 50°C to about 150°C, and the reaction is carried out with refluxing or in a sealed tube. In the reaction, a compound in which the protective group for primary amino group or secondary amino group in Y¹ has been removed is produced in some cases. The primary amino group or secondary amino group in Y may be protected again with a protective group (e.g. Boc or Cbz) by the same method as in the production process described in literature (for example, Protective Groups in Organic Synthesis 2nd Edition (John Wiley & Sons, Inc.)).

### 2) Step 2

A compound (26-4) is produced by reacting the compound (26-3) with hydrazine monohydrate in the presence or absence of an inert solvent. When the inert solvent is present, the amount of hydrazine monohydrate used is usually chosen in the range of 1 equivalent to large excess equivalents per equivalent of the compound (26-3). When the inert solvent is absent, hydrazine monohydrate may be used as a solvent. The inert solvent includes, for example, alcohol solvents (e.g. methanol, ethanol and 2-propanol) and dimethylformamide. The reaction temperature is chosen in the range of about 50°C to about 170°C, and the reaction is usually carried out with refluxing.

### 3) Step 3

When the compound (26-5) is R⁸⁵C(OR⁸⁶)₃, a compound (26-6) may be produced by reacting the compound (26-4) with the compound (26-5) in an inert solvent in the presence of an acid. The amount of the compound (26-5) used is usually chosen in the range of 1 equivalent to large excess equivalents per equivalent of the compound (26-4). The acid includes inorganic acids such as hydrochloric acid, sulfuric acid, etc.; and organic acids such as trifluoroacetic acid, acetic acid, etc. The inert solvent includes, for example, alcohol solvents (e.g. methanol, ethanol and 2-propanol) and dimethylformamide. The acid may be used as a solvent. The reaction temperature is chosen in the range of about 50°C to about 150°C. When the compound (26-5) is R⁸⁵CO₂H, a compound (26-6) may be produced by reacting the compound (26-4) with the compound (26-5) in the presence of an acid. The amount of the compound (26-5) used is usually chosen in the range of 1 equivalent to large excess equivalents per equivalent of the compound (26-4). The acid includes inorganic acids such as hydrochloric acid, sulfuric acid, polyphosphoric acid, etc. The acid may be used as a solvent. The reaction temperature is chosen in the range of about 50°C to about 200°C. In the reaction, a compound in which the protective group for primary amino group or secondary amino group in Y¹ has been removed is produced in some cases. The primary amino group or secondary amino group in Y may be protected again with a protective group (e.g. Boc or Cbz) by the same method as in the production process described in literature (for example, Protective Groups in Organic Synthesis 2nd Edition (John Wiley & Sons, Inc.)).

### 4) Step 4

The compound (26-7) may be produced from the compound (26-6) by the same production process as described in the step 6 in the production process 1.

### Production Process 27

A compound of the formula (27-4) as the compound of the formula (I), or a salt thereof is produced, for example, by the following process: 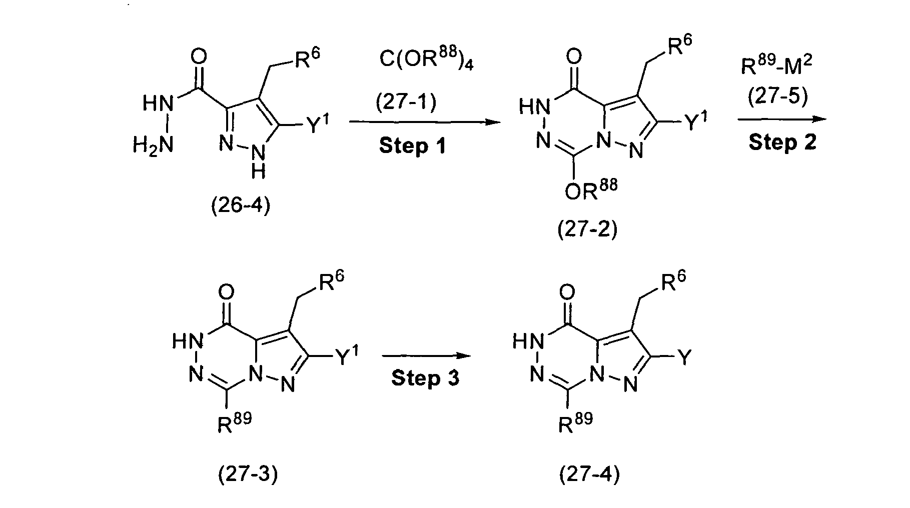 wherein R⁶ and Y are as defined in the item [1]; Y¹ is as defined in the production process 1; M² is as defined in the production process 10; R⁸⁸ is an alkyl group; and R⁸⁹ is "an optionally substituted alkoxy group", "an optionally substituted amino group", "an optionally substituted aryloxy group", "an optionally substituted alkylthio group" or "an optionally substituted arylthio group".

### 1) Step 1

A compound (27-2) may be produced from a compound (26-4) by the same production process as described in literature (for example, Synlett 11, 1670 (2000), Tetrahedron Lett. 34, 6127 (1998) and J. Org. Chem. 58, 3387 (1993)).

### 2) Step 2

A compound (27-3) may be produced from the compound (27-2) by the same production process as described in literature (for example, J. Heterocycl. Chem. 39, 97 (2002), Eur. J. Med. Chem. 27, 251 (1992) and J. Med. Chem. 38, 3558 (1995)).

### 3) Step 3

The compound (27-4) may be produced from the compound (27-3) by the same production process as described in the step 6 in the production process 1.

### Production Process 28

A compound of the formula (28-11) as the compound of the formula (I), or a salt thereof is produced, for example, by the following process: 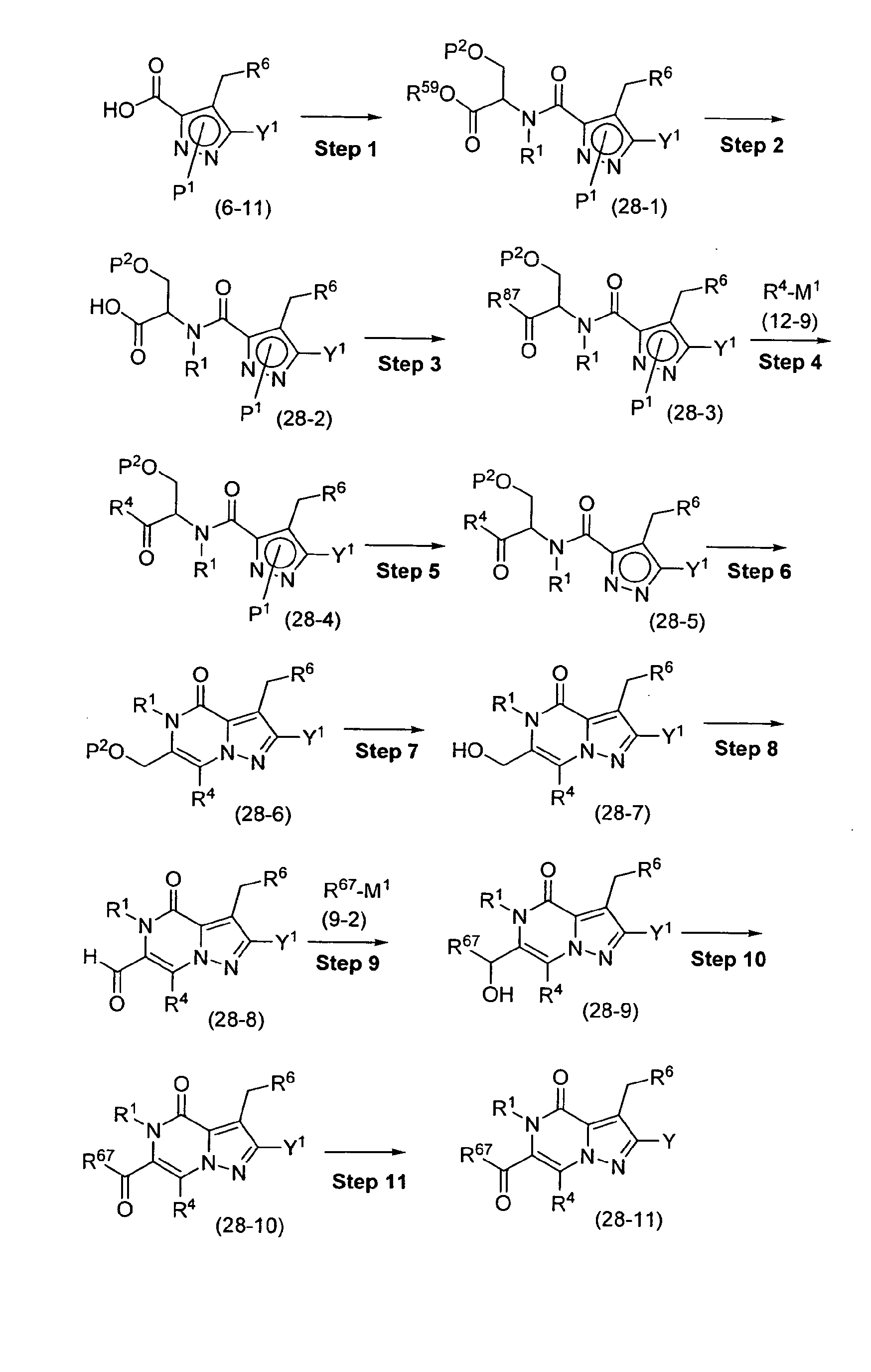 wherein R¹, R⁴, R⁶ and Y are as defined in the item [1]; Y¹ is as defined in the production process 1; R⁶⁷, R⁸⁷ and M¹ are as defined in the production process 9; R⁵⁹ and P² are as defined in the production process 5; and P¹ is as defined in the production process 6.

### 1) Step 1

A compound (28-1) may be produced from a compound (6-11) and a compound (5-17) represented by the formula: 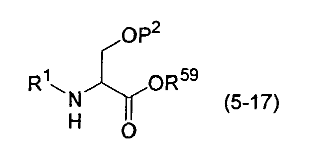 wherein R¹, R⁵⁹ and P² are as defined above, by the same production process as described in the step 3, (3) in the production process 1.

### 2) Step 2

A compound (28-2) may be produced from the compound (28-1) by the same production process as described in the step 3, (2) in the production process 1.

### 3) Step 3 to Step 4

A compound (28-4) may be produced from the compound (28-2) by the same production process as described in the step 1 to step 2 in the production process 9.

### 4) Step 5

A compound (28-5) may be produced from the compound (28-4) by the same process as that described in literature (for example, Protective Groups in Organic Synthesis 2nd Edition (John Wiley & Sons, Inc.)) or the like.

### 5) Step 6

A compound (28-6) may be produced from the compound (28-5) by the same production process as described in the step 3, (4) in the production process 1.

In the reaction, a compound in which the protective group for primary amino group or secondary amino group in Y¹ has been removed is produced in some cases. The primary amino group or secondary amino group in Y may be protected again with a protective group (e.g. Boc or Cbz) by the same method as in the production process described in literature (for example, Protective Groups in Organic Synthesis 2nd Edition (John Wiley & Sons, Inc.)).

### 6) Step 7

A compound (28-7) may be produced from the compound (28-6) by the same process as that described in literature (for example, Protective Groups in Organic Synthesis 2nd Edition (John Wiley & Sons, Inc.)) or the like.

### 7) Step 8

A compound (28-8) may be produced from the compound (28-7) by the same production process as described in the step 11 in the production process 5.

### 8) Step 9 to Step 10

A compound (28-10) may be produced from the compound (28-8) by the same process as that described in literature (for example, R.C. Ralock, "Comprehensive Organic transformation", VCH publisher Inc., (1989)) or the like.

### 9) Step 11

The compound (28-11) may be produced from the compound (28-10) by the same production process as described in the step 6 in the production process 1.

### Production Process 29

A compound of the formula (29-8) as the compound of the formula (I), or a salt thereof is produced, for example, by the following process: 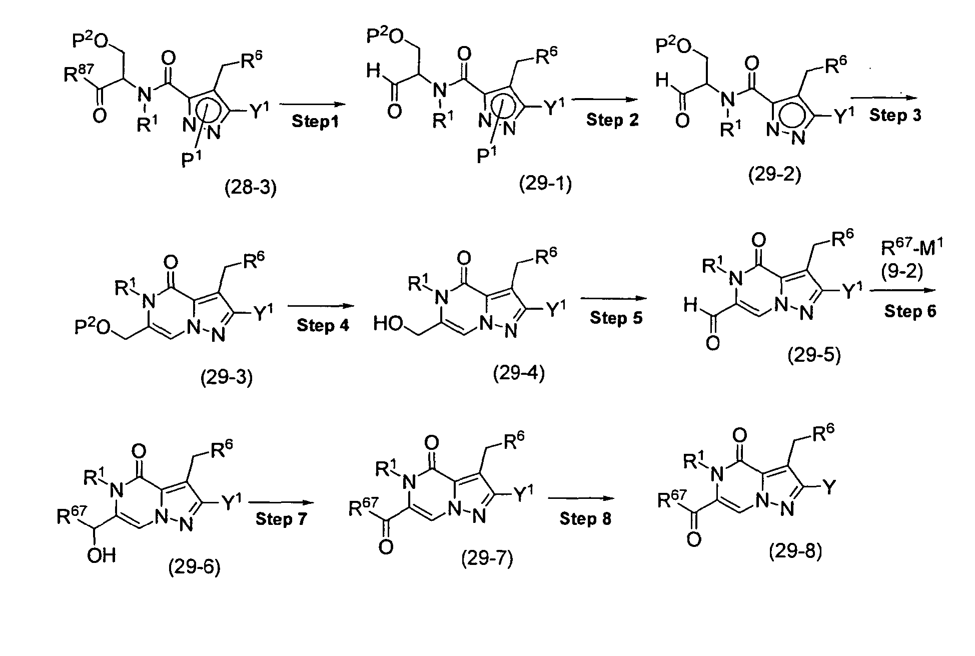 wherein R¹, R⁶ and Y are as defined in the item [1]; Y¹ is as defined in the production process 1; R⁶⁷, R⁸⁷ and M¹ are as defined in the production process 9; R⁵⁹ and P² are as defined in the production process 5; and P¹ is as defined in the production process 6.

### 1) Step 1

A compound (29-1) may be produced from a compound (28-3) by the same production process as described in the step 6 in the production process 5.

### 2) Step 2

A compound (29-2) may be produced from the compound (29-1) by the same process as that described in literature (for example, Protective Groups in Organic Synthesis 2nd Edition (John Wiley & Sons, Inc.)) or the like.

### 3) Step 3

A compound (29-3) may be produced from the compound (29-2) by the same production process as described in the step 3, (4) in the production process 1.

In the reaction, a compound in which the protective group for primary amino group or secondary amino group in Y¹ has been removed is produced in some cases. The primary amino group or secondary amino group in Y may be protected again with a protective group (e.g. Boc or Cbz) by the same method as in the production process described in literature (for example, Protective Groups in Organic Synthesis 2nd Edition (John Wiley & Sons, Inc.)).

### 4) Step 4

A compound (29-4) may be produced from the compound (29-3) by the same process as that described in literature (for example, Protective Groups in Organic Synthesis 2nd Edition (John Wiley & Sons, Inc.)) or the like.

### 5) Step 5

A compound (29-5) may be produced from the compound (29-4) by the same production process as described in the step 11 in the production process 5.

### 6) Step 6 to Step 7

A compound (29-7) may be produced from the compound (29-5) by the same process as that described in literature (for example, R.C. Ralock, "Comprehensive Organic transformation", VCH publisher Inc., (1989)) or the like.

### 7) Step 8

The compound (29-8) may be produced from the compound (29-7) by the same production process as described in the step 6 in the production process 1.

### Production Process 30

A compound of the formula (30-3) as the compound of the formula (I), or a salt thereof is produced, for example, by the following process: 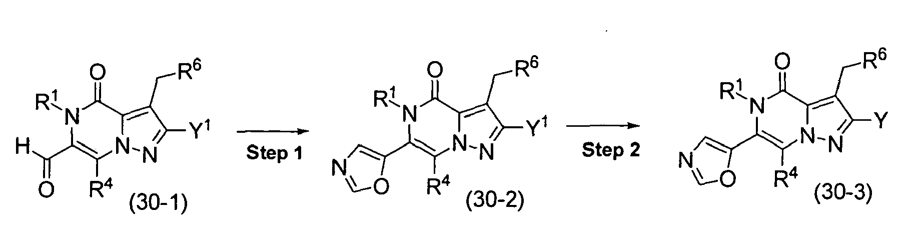 wherein R¹, R⁴, R⁶ and Y are as defined in the item [1]; Y¹ is as defined in the production process 1; and the compound of the formula (30-1) corresponds to the compound (28-8) described in the production process 28 or the compound (29-5) described in the production process 29.

### 1) Step 1

A compound (30-2) may be produced from a compound (30-1) by the same production process as described in literature (for example, Bioorg. Med. Chem. Lett. 12, 2879 (2002)).

### 2) Step 2

The compound (30-3) may be produced from the compound (30-2) by the same production process as described in the step 6 in the production process 1.

Unless otherwise specified, the starting materials, reagents and the like used above may be commercial compounds or may be produced from well-known compounds by well-known processes.

In each of the production processes described above, when the starting compound in each reaction has a reactive group such as hydroxyl group, amino group or carboxyl group, the reactive group in a site other than a site where the reaction is desired is previously protected with a suitable protective group if necessary, and the protective group is removed after carrying out each reaction or after carrying out several reactions, whereby a desired compound may be obtained. As the protective group for protecting hydroxyl group, amino group, carboxyl group or the like, conventional protective groups used in the field of organic synthetic chemistry may be used. The introduction and removal of such a protective group may be carried out according to a conventional method (for example, the method described in T.W. Greene, P.G.M. Wuts ,"Protective Groups in Organic Synthesis" 2nd Edition, John Wiley & Sons, Inc. (1991)).

For example, the protective group for the hydroxyl group includes tert-butyldimethylsilyl group, methoxymethyl group, tetrahydropyranyl group and the like. The protective group for the amino group includes tert-butoxycarbonyl group, benzyloxycarbonyl group and the like. The protective group for the hydroxyl group may be removed by reaction in a solvent such as aqueous methanol, aqueous ethanol or aqueous tetrahydrofuran in the presence of an acid such as hydrochloric acid, sulfuric acid or acetic acid. In the case of tert-butyldimethylsilyl group, it is also possible to carry out the removal in a solvent such as tetrahydrofuran in the presence of, for example, tetrabutylammonium fluoride. In the case of tert-butoxycarbonyl group, the protective group for the amino group may be removed, for example, by reaction in a solvent such as aqueous tetrahydrofuran, methylene chloride, chloroform or aqueous methanol in the presence of an acid such as hydrochloric acid or trifluoroacetic acid. In the case of benzyloxycarbonyl group, the removal may be carried out, for example, by reaction in a solvent such as acetic acid in the presence of an acid such as hydrobromic acid.

As a form in which the carboxyl group is protected, tert-butyl esters, orthoesters and acid amides are exemplified. Such a protective group is removed as follows. In the case of the tert-butyl ester, the removal is carried out, for example, by reaction in an aqueous solvent in the presence of hydrochloric acid. In the case of the orthoester, the removal is carried out, for example, by treatment with an acid and then an alkali such as sodium hydroxide in a solvent such as aqueous methanol, aqueous tetrahydrofuran or aqueous 1,2-dimethoxyethane. In the case of the acid amide, the removal is carried out by reaction in a solvent such as water, aqueous methanol or aqueous tetrahydrofuran in the presence of an acid such as hydrochloric acid or sulfuric acid.

The bicyclic pyrazole derivative of the formula (I) includes those having a center of optical activity. The compound having a center of optical activity may be obtained as a racemic modification, or it may be obtained as an optically active substance when an optically active starting material is used. If necessary, the racemic modification obtained may be physically or chemically resolved into optical antipodes by a well-known method. Preferably, diastereomers are formed from the racemic modification by a reaction using a reagent for optical resolution. The diastereomers different in form may be resolved by a well-known method such as fractional crystallization.

The bicyclic pyrazole derivative or prodrug thereof of the present invention may be converted to a salt, for example, by mixing with a pharmaceutically acceptable acid in a solvent such as water, methanol, ethanol or acetone. The pharmaceutically acceptable acid includes, for example, inorganic acids such as hydrochloric acid, hydrobromic acid, sulfuric acid, phosphoric acid, nitric acid, etc.; and organic acids such as acetic acid, propionic acid, oxalic acid, succinic acid, lactic acid, malic acid, tartaric acid, citric acid, maleic acid, fumaric acid, methanesulfonic acid, p-toluenesulfonic acid, ascorbic acid, etc.

The agents of the present invention are expected to be usable for the treatment of various diseases because of their inhibitory effect on DPP-IV. The compounds described in the present specification are useful for the suppression of postprandial hyperglycemia in a prediabetic, the treatment of non-insulin-dependent diabetes mellitus, the treatment of autoimmune diseases such as arthritis and articular rheumatism, the treatment of intestinal mucosa diseases, growth acceleration, the inhibition of rejection of a transplantate, the treatment of corpulence, the treatment of eating disorder, the treatment of HIV infection, the suppression of cancer metastasis, the treatment of prostatomegaly, the treatment of periodontitis, and the treatment of osteoporosis.

When used for the treatment, the bicyclic pyrazole derivative of the present invention, the prodrug thereof or the pharmaceutically acceptable salt of the derivative or prodrug may be administered as a pharmaceutical composition orally or parenterally (for example, by intravenous, subcutaneous or intramuscular injection, locally, intrarectally, percutaneously, or through nose). Compositions for the oral administration include, for example, tablets, capsules, pills, granules, powders, solutions and suspensions. Compositions for the parenteral administration include, for example, aqueous or oily preparations for injection, ointments, creams, lotions, aerosols, suppositories and patches. These pharmaceutical compositions are prepared by conventional techniques and may contain non-toxic and inactive carriers or excipients conventionally used in the field of formulation.

Although the dose is varied depending on the individual compounds, the disease, age, body weight and sex of a patient, symptom, administration route and the like, the bicyclic pyrazole derivative of the present invention, the prodrug thereof or the pharmaceutically acceptable salt of the derivative or prodrug is administered in a dose of 0.1 to 1000 mg/day, preferably 1 to 300 mg/day in one portion or two or three portions a day. It is also possible to administer the derivative, prodrug or salt at intervals of several days to several weeks.

The bicyclic pyrazole derivative of the present invention, the prodrug thereof or the pharmaceutically acceptable salt of the derivative or prodrug may be used in combination with other remedies for diabetes.

### EXAMPLES

The present invention is more concretely illustrated below with reference examples, working examples and test examples, which should not be construed as limiting the scope of the invention. The nomenclature of compounds shown in the reference examples and working examples mentioned below is not always based on IUPAC. Abbreviations are used in these examples for the simplification of description in some cases and they have the same meanings as defined above.

### Example 1

Methyl 2-[(3R)-3-aminopiperidin-1-yl]-3- (2-chlorobenzyl)-7-methyl-4-oxo-4,5-dihydropyrazolo[1,5-a]pyrazine-6-carboxylate hydrochloride

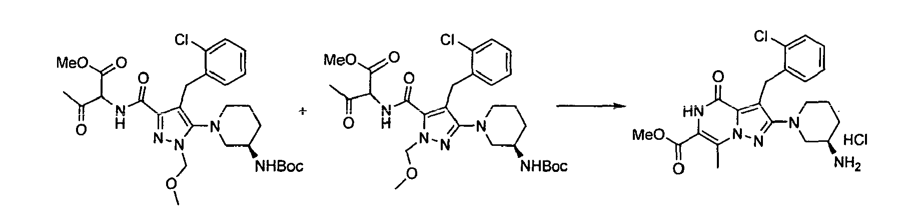

A methyl 2-({[5-{(3R)-3-[(tert-butoxycarbonyl)amino]piperidin-1-yl}-4-(2-chlorobenzyl)-1-(methoxymethyl)-1H-pyrazol-3-yl]carbonyl}amino)-3-oxobutanate mixture (55.0 mg) was dissolved in a 4N hydrochloric acid/1,4-dioxane solution (1.5 ml), followed by adding thereto water (0.5 ml), and the resulting mixture was stirred at 50°C for 2 hours. The reaction solution was concentrated under reduced pressure and the water remaining in the resulting residue was distilled off as an azeotrope with methanol to obtain the title compound (42.0 mg). ¹H NMR (300 MHz, DMSO-d₆) δ ppm 10.74 (s, 1H), 8.23 (bs, 3H), 7.50-7.35 (m, 1H), 7.30-7.05 (m, 2H), 7.05-6.80 (m, 1H), 4.35 (s, 2H), 3.83 (s, 3H), 3.80-3.60 (m, 1H), 3.20-2.90 (m, 2H), 2.75 (s, 3H), 2.65-2.40 (m, 2H), 2.00-1.80 (m, 1H), 1.70-1.20 (m, 3H). MS (ESI+) 430 (M⁺+1, 100%).

### Example 2

Ethyl 3-but-2-yn-1-yl-4-oxo-2-piperazin-1-yl-4,5-dihydropyrazolo[1,5-a]pyrazine-6-carboxylate hydrochloride

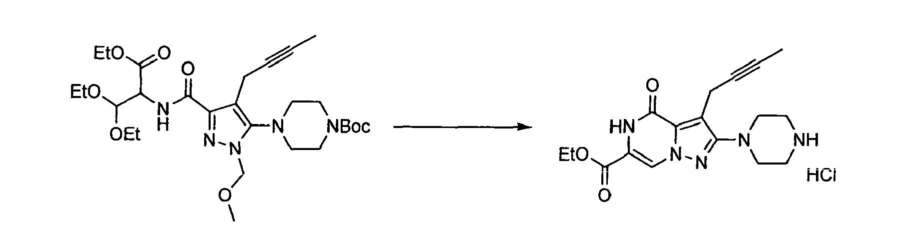

To a solution of tert-butyl 4-[4-but-2-yn-1-yl-3-({[1-diethoxymethyl]-2-ethoxy-2-oxoethyl}amino)carbonyl]-1-(methoxymethyl)-1H-pyrazol-5-yl]pyrazol-5-yl]piperazin-1-carboxylate (136 mg) in 1,4-dioxane (1 ml) were added 4N hydrochloric acid (2 ml) and water (1 ml), and the resulting mixture was stirred with heating at 50°C for two and a half hours. The reaction mixture was concentrated under reduced pressure to obtain the title compound (77 mg). ¹H NMR (400 MHz, DMSO-d₆) δ ppm 9.16 (bs, 2H), 8.11 (s, 1H), 4.29 (q, J = 7.2 Hz, 2H), 3.88-3.87 (bs, 2H), 3.57-3.50 (m, 4H), 3.28-3.24 (m, 4H), 1.76 (s, 3H), 1.30 (t, J = 7.2 Hz, 3H). MS (ESI+) 344 (M⁺+1, 100%).

### Example 3

2-[(3R)-3-Aminopiperidin-1-yl]-3-(2-chlorobenzyl)pyrazolo[1,5-a]pyrazin-4(5H)-one hydrochloride

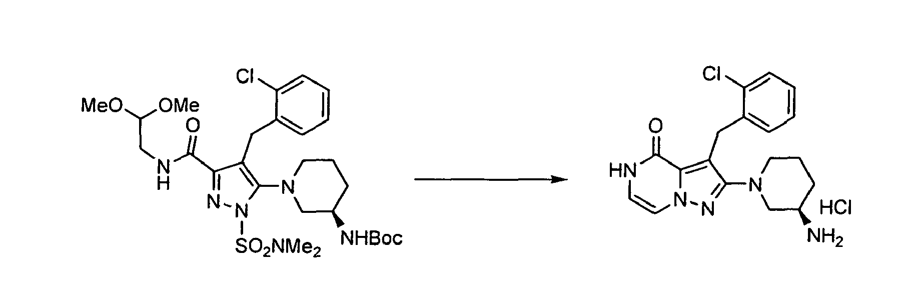

Water (20 ml) and a 4N hydrochloric acid/1,4-dioxane solution (40 ml) were added to a solution of tert-butyl ((3R)-1-{4-(2-chlorobenzyl)-3-{[(2,2-dimethoxyethyl)amino]carbonyl}-1-[(dimethylamino)sulfonyl]-1H-pyrazol-5-yl}piperidin-3-yl)carbamate (2.97 g) in 1,4-dioxane (20 ml), and the resulting mixture was stirred at 50°C for 2 hours. After cooling to 0°C, dioxane (30 ml), water (20 ml) and sodium hydrogencarbonate (30 mg) were added thereto. The resulting mixture was warmed to room temperature and di-tert-butyl dicarbonate (2.06 g) was added thereto and stirred overnight. The 1,4-dioxane was distilled off under reduced pressure and water (100 ml) was added to the residue, followed by two runs of extraction with ethyl acetate (100 ml). The combined organic layer was concentrated under reduced pressure. The resulting residue was purified by a silica gel column chromatography (chloroform/ethyl acetate = 1/1) to obtain a purified substance (1.30 g). MS (ESI+) 458 (M⁺+1, 36%).

Then, a 4N hydrochloric acid/1,4-dioxane solution (1.0 ml) was added to a suspension (0.1 ml) of the purified substance (53.0 mg) in 1,4-dioxane, and the resulting mixture was stirred at room temperature for 1 hour. The reaction mixture was concentrated under reduced pressure to obtain the title compound as a crude product (47.8 mg).
¹H NMR (400 MHz, CDCl₃) δ ppm 11.08 (d, J = 5.2Hz, 1H), 8.00-7.98 (m, 3H), 7.50-7.43 (m, 2H), 7.22-7.19 (m, 2H), 6.99-6.96 (m, 1H), 6.73-6.70 (m, 1H), 4.28 (s, 2H), 3.25-3.22 (m, 1H), 2.94-2.91 (m, 2H), 2.55-2.53 (m, 2H), 2.33-2.31 (m, 1H), 1.91-1.90 (m, 1H), 1.41-1.39 (m, 2H).
MS (ESI+) 358 (M⁺+1, 28%).

### Example 4

2-[(3R)-3-Aminopiperidin-1-yl]-3-(2-chlorobenzyl)-5,6-dimethylpyrazolo[1,5-a]pyrazin-4(5H)-one hydrochloride

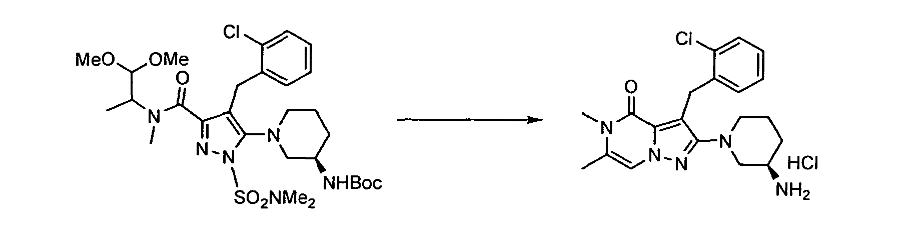

Water (1.0 ml) and a 4N hydrochloric acid/1,4-dioxane solution (2.0 ml) were added to a solution of tert-butyl ((3R)-1-{4-(2-chlorobenzyl)-3-{[(2,2-dimethoxy-1-methylethyl)(methyl)amino]carbonyl}-1-[(dimethylamino)sulfonyl]-1H-pyrazol-5-yl}piperidin-3-yl)carbamate (126 mg) in 1,4-dioxane (1.0 ml), and the resulting mixture was stirred at 50°C for 2 hours. After cooling to 0°C, 1,4-dioxane (1.0 ml), water (1.0 ml) and sodium hydrogencarbonate (1.5 g) were added thereto. The resulting mixture was warmed to room temperature and di-tert-butyl dicarbonate (83.8 mg) was added thereto and stirred overnight. The 1,4-dioxane was distilled off under reduced pressure and water (10 ml) was added to the residue, followed by two runs of extraction with ethyl acetate (10 ml). The combined organic layer was concentrated under reduced pressure. The resulting residue was purified by a silica gel column chromatography (hexane/ethyl acetate = 1/1) to obtain a purified substance (56.7 mg). A 4N hydrochloric acid/1,4-dioxane solution (1.0 ml) was added to a suspension (0.1 ml) of the purified substance (53.0 mg) in 1,4-dioxane, and the resulting mixture was stirred at room temperature for 1 hour. The reaction mixture was concentrated under reduced pressure to obtain the title compound as a crude product (51.9 mg).
¹H NMR (400 MHz, DMSO-d₆) δ ppm 8.09-8.06 (m, 3H), 7.52 (s, 1H), 7.45-7.42 (m, 1H), 7.23-7.15 (m, 2H), 6.92-6.89 (m, 1H), 4.26 (s, 2H), 3.55-3.51 (m, 1H), 3.34 (s, 3H), 3.28-3.25 (m, 1H), 2.99-2.90 (m, 2H), 2.67-2.63 (m, 1H), 2.26 (s, 3H), 1.98-1.95 (m, 1H), 1.75-1.70 (m, 1H), 1.46-1.41 (m, 2H).
MS (ESI+) 386 (M⁺+1, 35%).

### Example 5

2-[(3R)-3-Aminopiperidin-1-yl]-3-(2-chlorobenzyl)-6-phenylpyrazolo[1,5-a]pyrazin-4(5H)-one hydrochloride

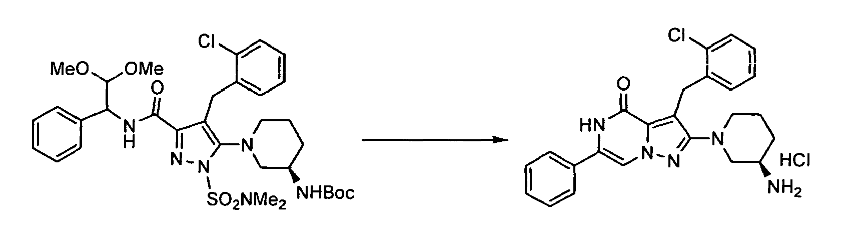

Water (2.0 ml) and a 4N hydrochloric acid/1,4-dioxane solution (4.0 ml) were added to a solution of tert-butyl ((3R)-1-{4-(2-chlorobenzyl)-3-{[(2,2-dimethoxy-1-phenylethyl)amino]carbonyl}-1-[(dimethylamino)sulfonyl]-lH-pyrazol-5-yl}piperidin-3-yl)carbamate (270 mg) in 1,4-dioxane (2.0 ml), and the resulting mixture was stirred at 50°C for 2 hours. After cooling to 0°C, dioxane (3.0 ml), water (1.0 ml) and sodium hydrogencarbonate (3.0 g) were added thereto. The resulting mixture was warmed to room temperature and di-tert-butyl dicarbonate (167 mg) was added thereto and stirred overnight. The 1,4-dioxane was distilled off under reduced pressure and water (10 ml) was added to the residue, followed by two runs of extraction with ethyl acetate (10 ml). The combined organic layer was concentrated under reduced pressure. The resulting residue was purified by a silica gel column chromatography (hexane/ethyl acetate = 2/1) to obtain a purified substance (94.3 mg). A 4N hydrochloric acid/1,4-dioxane solution (1.0 ml) was added to a suspension (0.1 ml) of the purified substance (92.0 mg) in 1,4-dioxane, and the resulting mixture was stirred at room temperature for 1 hour. The reaction mixture was concentrated under reduced pressure to obtain the title compound as a crude product (72.8 mg).
¹H NMR (400 MHz, DMSO-d₆) δ ppm 11.4 (s, 1H), 8.15-8.12 (m, 3H), 7.93 (s, 1H), 7.74-7.71 (m, 2H), 7.49-7.42 (m, 4H), 7.25-7.21 (m, 2H), 7.09-7.03 (m, 1H), 4.33 (s, 2H), 3.25-3.21 (m, 1H), 2.96-2.93 (m, 2H), 2.53-2.48 (m, 2H), 1.69-1.65 (m, 1H), 1.66-1.47 (m, 1H), 1.45-1.23 (m, 2H).
MS (ESI+) 434 (M⁺+1, 35%).

### Example 6

2-[(3R)-3-Aminopiperidin-1-yl]-3-(2-chlorobenzyl)-7-phenylpyrazolo[1,5-a]pyrazin-4(5H)-one hydrochloride

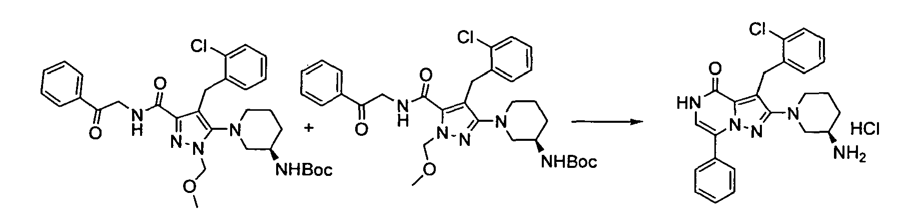

Water (2.0 ml) and a 4N hydrochloric acid/1,4-dioxane solution (4.0 ml) were added to a solution of a tert-butyl [(3R)-1-(4-(2-chlorobenzyl)-1-(methoxymethyl)-3-{[(2-oxo-2-phenylethyl)amino]-carbonyl}-1H-pyrazol-5-yl)piperidin-3-yl]carbamate mixture (150 mg) in 1,4-dioxane (2.0 ml), and the resulting mixture was stirred at 50°C for 2 hours. After cooling to 0°C, 1,4-dioxane (3.0 ml), water (1.0 ml) and sodium hydrogencarbonate (3.0 g) were added thereto. The resulting mixture was warmed to room temperature and di-tert-butyl dicarbonate (65.8 mg) was added thereto and stirred overnight. The 1,4-dioxane was distilled off under reduced pressure and water (20 ml) was added to the residue, followed by two runs of extraction with ethyl acetate (20 ml). The combined organic layer was concentrated under reduced pressure. The resulting residue was purified by a silica gel column chromatography (hexane/ethyl acetate = 2/1) to obtain a purified substance (54.8 mg). A 4N hydrochloric acid/1,4- dioxane solution (1.0 ml) was added to a suspension (0.1 ml) of the purified substance (54.8 mg) in 1,4-dioxane, and the resulting mixture was stirred at room temperature for 1 hour. The reaction mixture was concentrated under reduced pressure to obtain the title compound as a crude product (47.9 mg).
¹H NMR (400 MHz, DMSO-d₆) δ ppm 11.40-11.38 (d, J = 5.9Hz, 1H), 8.06-8.04 (m, 3H), 7.81-7.79 (m, 2H), 7.48-7.44 (m, 4H), 7.24-7.21 (m, 2H), 7.06-7.04 (m, 1H), 6.85-6.84 (m, 1H), 4.35 (s, 2H), 3.52-3.51 (m, 1H), 3.05-2.90 (m, 2H), 2.75-2.71 (m, 2H), 1.89-1.86 (m, 1H), 1.65-1.64 (m, 1H), 1.44-1.40 (m, 2H).
MS (ESI+) 434 (M⁺+1, 31%).

### Example 7

2-[(3R)-3-Aminopiperidin-1-yl]-3-(2-chloro-5-fluorobenzyl)-4-oxo-4,5-dihydropyrazolo[1,5-a]pyrazine-6- carboxylic acid methanesulfonate

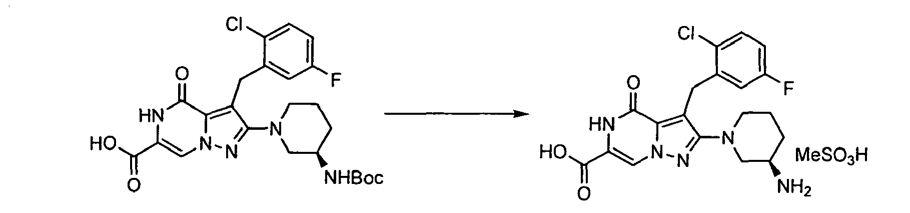

Methanesulfonic acid (214 mg) was added to a solution of 2-{(3R)-3-[(tert-butoxycarbonyl)amino]-piperidin-1-yl}-3-(2-chloro-5-fluorobenzyl)-4-oxo-4,5-dihydropyrazolo[1,5-a]pyrazine-6-carboxylic acid (580 mg) in chloroform (10 ml), and the resulting mixture was stirred at 50°C for 2 hours. After the mixture was allowed to cool, the chloroform was distilled off under reduced pressure and the resulting solid was recrystallized from 2-propanol. The solid thus obtained was collected by filtration and washed with 2-propanol to obtain the title compound (380 mg) as a white solid.
¹H NMR (400 MHz, CD₃OD) δ ppm 8.13 (s, 1H), 7.45-7.39 (m, 1H), 6.99-6.93 (m, 1H), 6.77-6.71 (m, 1H), 4.44 (d, J = 17Hz, 1H), 4.36 (d, J = 17 Hz, 1H), 3.72-3.62 (m, 1H), 3.39-3.30 (m, 1H), 3.14-3.02 (m, 2H), 3.86-3.73 (m, 1H), 2.68 (s, 3H), 2.07-1.95 (m, 1H), 1.78-1.67 (m, 1H), 1.62-1.47 (m, 2H).
MS (ESI+) 420 (M⁺+1, 33%).

### Example 8

2-[(3R)-3-Aminopiperidin-1-yl]-3-(2-chlorobenzyl)-5-(2-oxo-2-phenylethyl)pyrazolo[1,5-a]pyrazin-4(5H)-one hydrochloride

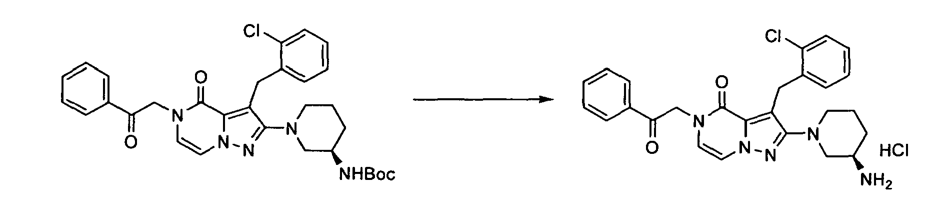

A 4N hydrochloric acid/1,4-dioxane solution (1.0 ml) was added to a suspension (0.1 ml) of tert-butyl {(3R)-1-[3-(2-chlorobenzyl)-4-oxo-5-(2-oxo-2-phenylethyl)-4,5-dihydropyrazolo[1,5-a]pyrazin-2-yl]piperidin-3-yl}carbamate (38.8 mg) in 1,4-dioxane, and the resulting mixture was stirred at room temperature for 1 hour. The reaction mixture was concentrated under reduced pressure to obtain the title compound as a crude product (38.9 mg).
¹H NMR (400 MHz, DMSO-d₆) δ ppm 8.06-8.04 (m, 5H), 7.73-7.69 (m, 2H), 7.62-7.58 (m, 2H), 7.45-7.42 (m, 1H), 7.22-7.20 (m, 2H), 7.00 (d, J = 6.0Hz, 1H), 6.95-6.93 (m, 1H), 5.47 (s, 2H), 4.28 (s, 2H), 3.55-3.51 (m, 1H), 3.25-3.20 (m, 1H), 3.10-2.91 (m, 2H), 2.85-2.75 (m, 1H), 2.01-1.95 (m, 1H), 1.75-1.69 (m, 1H), 1.51-1.45 (m, 2H).
MS (ESI+) 476 (M⁺+1, 46%).

The following compounds of Example 9 to Example 28 were synthesized by the same process as in Example 8.

| Example number | | Reference example number for starting material |
|---|---|---|
| Example 9 | 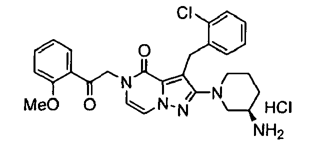 | Reference Example 12-1 |
| Example 10 | 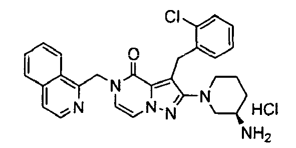 | Reference Example 12-3 |
| Example 11 | 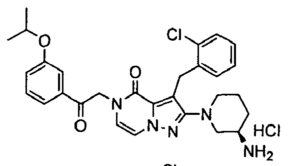 | Reference Example 12-2 |
| Example 12 | 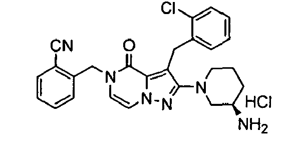 | Reference Example 12-4 |
| Example 13 | 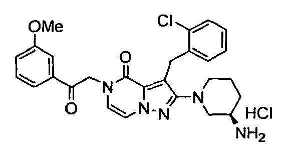 | Reference Example 12-5 |
| Example 14 | 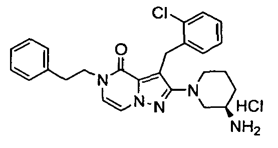 | Reference Example 12-6 |
| Example 15 | 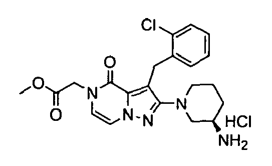 | Reference Example 12-7 |
| Example 16 | 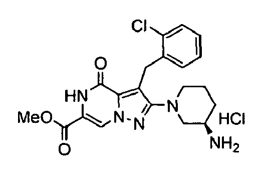 | Reference Example 10 |
| Example 17 | 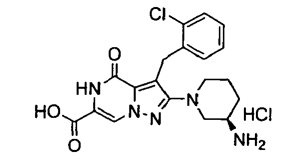 | Reference Example 11 |
| Example 18 | 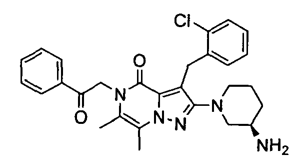 | Reference Example 25 |
| Example 19 | 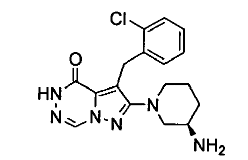 | Reference Example 29 |
| Example 20 | 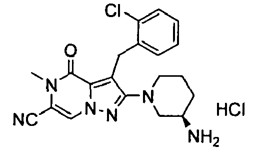 | Reference Example 15 |
| Example 21 | 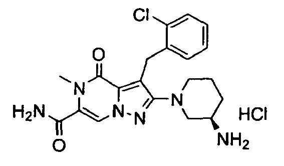 | Reference Example 16 |
| Example 22 | 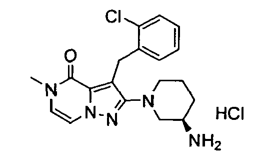 | Reference Example 13 |
| Example 23 | 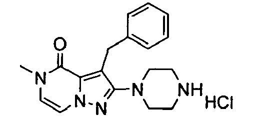 | Reference Example 19 |
| Example 24 | 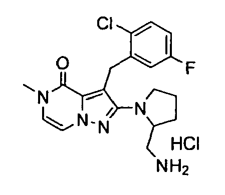 | Reference Example 36 |
| Example 25 | 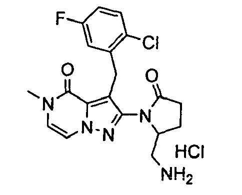 | Reference Example 37 |
| Example 26 | 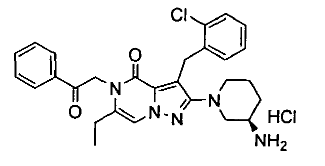 | Reference Example 33 |
| Example 27 | 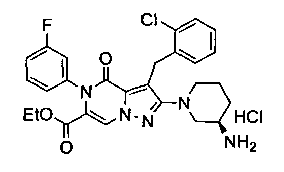 | Reference Example 9 |
| Example 28 | 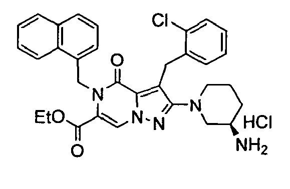 | Reference Example 91 |

### Example 9

¹H NMR (400 MHz, DMSO-d₆) δ ppm 8.31-8.21 (m, 3H), 7.77-7.75 (m, 1H), 7.67-7.65 (m, 2H), 7.44-7.42 (m, 1H), 7.28-7.19 (m, 3H), 7.11-7.09 (m, 1H), 7.03-7.01 (m, 1H), 7.00-6.94 (m, 1H), 5.24 (s, 2H), 4.28 (s, 2H), 3.97 (s, 3H), 3.72-3.68 (m, 1H), 3.20-3.11 (m, 1H), 3.05-2.95 (m, 2H), 2.70-2.68 (m, 1H), 1.95-1.85 (m, 1H), 1.71-1.65 (m, 1H), 1.55-1.35 (m, 2H).
MS (ESI+) 506 (M⁺+1, 73%).

### Example 10

¹H NMR (400 MHz, DMSO-d₆) δ ppm 8.44 (d, J = 8.4Hz, 1H), 8.40 (d, J= 5.9Hz, 1H), 8.21-8.11 (m, 4H), 7.96-7.94 (m, 2H), 7.84-7.80 (m, 1H), 7.71 (d, J = 5.9Hz, 1H), 7.43-7.41 (m, 1H), 7.21-7.18 (m, 2H), 7.13 (d, J = 5.9Hz, 1H), 7.02-6.99 (m, 1H), 5.84 (s, 2H), 4.22 (s, 2H), 3.19-3.16 (m, 1H), 3.02-2.91 (m, 2H), 2.61-2.53 (m, 2H), 1.92-1.91 (m, 1H), 1.68-1.65 (m, 1H), 1.46-1.40 (m, 2H).
MS (ESI+) 499 (M⁺+1, 100%).

### Example 11

¹H NMR (400 MHz, DMSO-d₆) δ ppm 8.06-8.03 (m, 3H), 7.67 (d, J = 5.9Hz, 1H), 7.59 (d, J = 8.0Hz, 1H), 7.50-7.42 (m, 3H), 7.28-7.27 (m, 1H), 7.22-7.19 (m, 2H), 6.98 (d, J = 5.9Hz, 1H), 6.96-6.92 (m, 1H), 5.44 (s, 2H), 4.75-4.69 (m, 1H), 4.27 (s, 2H), 3.30-3.28 (m, 1H), 3.15-3.11 (m, 1H), 3.05-2.98 (m, 1H), 2.52-2.45 (m, 2H), 2.10-2.07 (m, 1H), 1.91-1.90 (m, 1H), 1.67-1.42 (m, 2H), 1.28 (d, J = 6.0Hz, 6H).
MS (ESI+) 534 (M⁺+1, 80%).

### Example 12

¹H NMR (400 MHz, DMSO-d₆) δ ppm 8.13-8.10 (m, 3H), 7.88 (d, J = 7.7Hz, 1H), 7.72 (d, J = 6.0Hz, 1H), 7.69-7.65 (m, 1H), 7.52-7.48 (m, 1H), 7.43 (d, J = 7.7Hz, 1H), 7.26-7.17 (m, 3H), 7.10 (d, J = 6.0Hz, 1H), 7.00-6.97 (m, 1H), 5.25 (s, 2H), 4.27 (s, 2H), 3.19-3.16 (m, 1H), 3.01-2.88 (m, 2H), 2.60-2.51 (m, 2H), 1.93-1.90 (m, 1H), 1.67-1.60 (m, 1H), 1.47-1.41 (m, 2H).
MS (ESI+) 473 (M⁺+1, 66%).

### Example 13

¹H NMR (400 MHz, DMSO-d₆) δ ppm 8.15-8.11 (m, 3H), 7.68 (d, J = 5.9Hz, 1H), 7.65-7.63 (m, 1H), 7.53-7.48 (m, 2H), 7.44-7.41 (m, 1H), 7.32-7.29 (m, 1H), 7.21-7.18 (m, 2H), 7.00 (d, J = 5.9Hz, 1H), 6.95-6.93 (m, 1H), 5.45 (s, 2H), 4.27 (s, 2H), 3.83 (s, 3H), 3.61-3.57 (m, 1H), 3.25-3.20 (m, 1H), 3.04-2.91 (m, 2H), 2.66-2.63 (m, 1H), 1.92-1.88 (m, 1H), 1.68-1.65 (m, 1H), 1.48-1.40 (m, 2H).
MS (ESI+) 506 (M⁺+1, 89%).

### Example 14

¹H NMR (400 MHz, DMSO-d₆) δ ppm 8.09-8.03 (m, 3H), 7.57 (d, J = 5.9Hz, 1H), 7.45 (dd, J = 1.9Hz, 7.7Hz, 1H), 7.30-7.20 (m, 7H), 6.94 (d, J = 5.9Hz, 1H), 6.89 (dd, J = 1.6Hz, 7.1Hz, 1H), 4.29 (s, 2H), 4.04 (t, J = 6.8Hz, 2H), 3.55-3.50 (m, 1H), 3.20-3.14 (m, 1H), 2.95-2.89 (m, 4H), 2.57-2.55 (m, 1H), 1.91-1.89 (m, 1H), 1.66-1.64 (m, 1H), 1.45-1.41 (m, 2H).
MS (ESI+) 462 (M⁺+1, 37%).

### Example 15

¹H NMR (400 MHz, DMSO-d₆) δ ppm 8.18-8.16 (m, 3H), 7.67 (d, J = 5.9Hz, 1H), 7.46-7.44 (m, 1H), 7.22-7.19 (m, 2H), 7.03-6.97 (m, 2H), 4.68 (s, 2H), 4.28 (s, 2H), 3.68 (s, 3H), 3.52-3.44 (m, 1H), 3.18-3.16 (m, 1H), 3.03-2.89 (m, 2H), 2.62-2.60 (m, 1H), 1.92-1.90 (m, 1H), 1.67-1.64 (m, 1H), 1.45-1.41 (m, 2H).
MS (ESI+) 430 (M⁺+1, 44%).

### Example 16

¹H NMR (400 MHz, DMSO-d₆) δ ppm 8.30 (bs, 1H), 8.19 (s, 1H), 7.46 (dd, J = 1.7Hz, 7.7Hz, 1H), 7.27-7.18 (m, 2H), 7.00 (dd, J = 1.7Hz, 7.3Hz, 1H), 4.39-4.29 (m, 2H), 3.84 (s, 3H), 3.62-3.60 (m, 1H), 3.39-3.29 (m, 3H), 3.17-3.15 (m, 1H), 3.06-3.03 (m, 1H), 2.96-2.91 (m, 1H), 2.61-2.52 (m, 1H), 1.91-1.88 (m, 1H), 1.66-1.62 (m, 1H), 1.47-1.37 (m, 2H).
MS (ESI+) 416 (M⁺+1, 32%).

### Example 17

¹H NMR (400 MHz, DMSO-d₆) δ ppm 11.00 (s, 1H), 8.12-8.08 (m, 4H), 7.47-7.44 (m, 1H), 7.25-7.18 (m, 2H), 7.00-6.97 (m, 1H), 4.40-4.28 (m, 2H), 3.25-3.21 (m, 1H), 3.09-3.05 (m, 1H), 2.98-2.95 (m, 1H), 2.75-2.62 (m, 2H), 1.99-1.90 (m, 1H), 1.75-1.71 (m, 1H), 1.60-1.52 (m, 2H).
MS (ESI+) 402 (M⁺+1, 35%).

### Example 18

¹H NMR (400 MHz, DMSO-d₆) δ ppm 8.09-8.07 (m, 5H), 7.73-7.71 (m, 1H), 7.62-7.58 (m, 2H), 7.44-7.41 (m, 1H), 7.21-7.18 (m, 2H), 6.93-6.90 (m, 1H), 5.61 (s, 2H), 4.28 (s, 2H), 3.60-3.57 (m, 1H), 3.25-3.23 (m, 1H), 3.01-2.98 (m, 2H), 2.71-2.68 (m, 1H), 2.52 (s, 3H), 2.18 (s, 3H), 1.92-1.90 (m, 1H), 1.69-1.66 (m, 1H), 1.47-1.44 (m, 2H).
MS (ESI+) 504 (M⁺+1, 86%).

### Example 19

¹H NMR (300 MHz, CD₃OD) δ ppm 8.35 (s, 1H), 7.32-7.29 (m, 1H), 7.11-7.07 (m, 2H), 6.94-6.90 (m, 1H), 4.35 (d, J = 17.2Hz, 1H), 4.27 (d, J = 17.2Hz, 1H), 3.50-3.46 (m, 1H), 3.22-3.20 (m, 1H), 3.04-2.94 (m, 2H), 2.72-2.68 (m, 1H), 1.93-1.89 (m, 1H), 1.54-1.38 (m, 3H).
MS (ESI+) 359 (M⁺+1, 36%).

### Example 20

¹H NMR (400 MHz, DMSO-d₆) δ ppm 8.88 (s, 1H), 8.12 (bs, 3H), 7.47-7.45 (m, 1H), 7.25-7.17 (m, 2H), 6.97-6.95 (m, 1H), 4.33-4.32 (m, 2H), 3.65-3.62 (m, 1H), 3.43 (s, 3H), 3.15-3.14 (m, 1H), 3.07-3.04 (m, 1H), 2.96-2.91 (m, 1H), 2.63-2.61 (m, 1H), 1.92-1.88 (m, 1H), 1.64-1.63 (m, 1H), 1.47-1.33 (m, 2H).
MS (ESI+) 397 (M⁺+1, 28%).

### Example 21

¹H NMR (400 MHz, DMSO-d₆) δ ppm 8.20-8.17 (m, 4H), 7.89 (s, 1H), 7.80 (bs, 1H), 7.47-7.45 (m, 1H), 7.25-7.18 (m, 2H), 6.96-6.93 (m, 1H), 4.31(s, 2H), 3.67-3.66 (m, 1H), 3.39 (s, 3H), 3.25-3.23 (m, 1H), 2.99-2.94 (m, 2H), 2.62-2.60 (m, 1H), 1.91-1.89 (m, 1H), 1.65-1.64 (m, 1H), 1.46-1.40 (m, 2H).
MS (ESI+) 415 (M⁺+1, 35%).

### Example 22

¹H NMR (400 MHz, CD₃OD) δ ppm 7.49 (d, J = 5.9Hz, 1H), 7.42-7.40 (m, 1H), 7.20-7.16 (m, 2H), 6.99 (d, J = 7.2Hz, 1H), 6.87 (d, J = 5.9Hz, 1H), 4.13 (s, 2H), 3.76-3.74 (m, 1H), 3.69-3.61 (m, 2H), 3.61-3.58 (m, 2H), 3.46 (s, 3H), 3.10-3.04 (m, 2H), 2.80-2.77 (m, 1H), 2.02-2.00 (m, 1H), 1.75-1.73 (m, 1H), 1.61-1.54 (m, 2H).
MS (ESI+) 372 (M⁺+1, 38%).

### Example 23

¹H NMR (400 MHz, CD₃OD) δ ppm 7.45 (d, J = 5.9 Hz, 1H), 7.27-7.20 (m, 4H), 7.18-7.15 (m, 1H), 6.87 (d, J = 5.9 Hz, 1H), 4.35 (s, 2H), 3.48 (s, 3H), 3.28 (t, J = 5.1 Hz, 4H), 3.17 (t, J = 5.1 Hz, 4H).
MS (ESI+) 324 (M⁺+1, 100%).

### Example 24

¹H NMR (400 MHz, CD₃OD) δ ppm 7.48 (d, J = 6.0 Hz, 1H), 7.44-7.40 (m, 1H), 6.97-6.92 (m, 1H), 6.83 (d, J = 6.0 Hz, 1H), 6.66-6.63 (m, 1H), 4.50 (s, 2H), 4.29-4.25 (m, 1H), 3.55-3.48 (m, 1H), 3.45 (s, 3H), 3.17-3.05 (m, 3H), 2.13-2.06 (m, 1H), 1.91-1.75 (m, 3H).
MS (ESI+) 390 (M⁺+1, 100%).

### Example 25

¹H NMR (400 MHz, DMSO-d₆) δ ppm 8.14 (br, 3H), 7.73 (d, J = 6.0 Hz, 1H), 7.47-7.43 (m, 1H), 7.21 (d, J = 6.0 Hz, 1H), 7.12-7.07 (m, 1H), 6.93-6.89 (m, 1H), 4.30 (d, J = 16.3 Hz, 1H), 4.24 (d, J = 16.3 Hz, 1H), 4.06-4.00 (m, 1H), 3.41 (s, 3H), 2.99-2.94 (m, 1H), 2.73-2.67 (m, 1H), 2.54-2.45 (m, 1H), 2.32-2.24 (m, 1H), 2.10-1.97 (m, 2H).
MS (ESI+) 404 (M⁺+1, 100%).

### Example 26

¹H NMR (400 MHz, CD₃OD) δ ppm 8.11-8.08 (m, 2H), 7.68-7.66 (m, 1H), 7.58-7.54 (m, 2H), 7.38-7.34 (m, 4H), 7.16-7.13 (m, 3H), 7.00-6.98 (m, 1H), 5.55 (s, 2H), 4.36 (s, 2H), 3.64-3.58 (m, 1H), 3.31-3.30 (m, 1H), 3.05-3.04 (m, 2H), 2.85-2.81 (m, 1H), 2.55-2.53 (m, 2H), 2.05-2.01 (m, 1H), 1.80-1.75 (m, 1H), 1.58-1.52 (m, 2H), 1.24 (t, J = 7.3Hz, 3H).
MS (ESI+) 504 (M⁺+1, 71%).

### Example 27

¹H NMR (400 MHz, CD₃OD) δ ppm 8.24 (s, 1H), 7.46-7.37 (m, 2H), 7.18-7.04 (m, 6H), 4.46-4.40 (m, 2H), 4.07 (q, J = 7.1Hz, 2H), 3.73-3.65 (m, 4H), 3.34-3.33 (m, 1H), 3.12-3.08 (m, 2H), 2.81-2.80 (m, 1H), 2.00-1.99 (m, 1H), 1.70-1.69 (m, 1H), 1.59-1.50 (m, 2H), 1.10 (t, J = 7.1Hz, 3H).
MS (ESI+) 524 (M⁺+1, 99%).

### Example 28

¹H NMR (400 MHz, DMSO-d₆) δ ppm 8.38 (s, 1H), 8.11-8.10 (m, 1H), 8.10 (bs, 3H), 7.95-7.94 (m, 1H), 7.81 (d, J = 8.3Hz, 1H), 7.58-7.56 (m, 2H), 7.46-7.44 (m, 1H), 7.38-7.36 (m, 1H), 7.24-7.22 (m, 2H), 7.09-7.07 (m, 1H), 6.96 (d, J = 6.5Hz, 1H), 5.93 (s, 2H), 4.37-4.35 (m, 2H), 4.05 (q, J = 7.0Hz, 2H), 3.68-3.65 (m, 1H), 3.25-3.10 (m, 2H), 3.01-2.95 (m, 1H), 2.68-2.65 (m, 1H), 1.92-1.90 (m, 1H), 1.75-1.71 (m, 1H), 1.47-1.39 (m, 2H), 1.04 (t, J = 7.0Hz, 3H).
MS (ESI+) 570 (M⁺+1, 100%).

### Example 29

2-[(3R)-3-aminopiperidin-1-yl]-3-(2-chloro-5-fluorobenzyl)-5-methyl-6,7-dihydropyrazolo[1,5-a]pyrazin-4(5H)-one

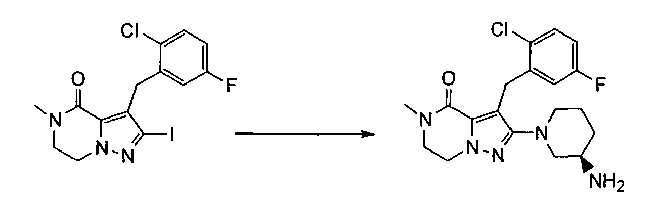

A suspension of 3-(2-chloro-5-fluorobenzyl)-2- iodo-5-methyl-6,7-dihydropyrazolo[1,5-a]pyrazin-4(5H)-one (420 mg), (R)-tert-3-butylpiperidin-3-yl carbamate (400 mg), cesium carbonate (652 mg) and copper iodide (9.5 mg) in n-butyronitrile (5 ml) was stirred in a sealed tube at 110°C for 20 hours. Then, copper iodide (20 mg) was added thereto, followed by stirring at the same temperature for 10 hours. After cooling to room temperature, a product (100 mg) was obtained by purification by a silica gel column chromatography (hexane/ethyl acetate = 1/1). Subsequently, to a solution of this product in methanol (4 ml) was added a 12N aqueous hydrochloric acid solution (2 ml) at room temperature, and after standing overnight, the reaction solution was concentrated under reduced pressure. Water and potassium carbonate were added to the residue to make the solution basic, followed by two runs of extraction with chloroform. The combined organic layer was dried over anhydrous sodium sulfate and filtered, and the filtrate was concentrated under reduced pressure. The residue was purified by a silica gel column chromatography (chloroform/methanol = 10/1 ~ chloroform/methanol/triethylamine = 10/1/0.1) to obtain the title compound (1 mg).
¹H NMR (400 MHz, CDCl₃) δ ppm 7.33-7.27 (m, 1H), 6.85-6.79 (m 1H), 6.76-6.70 (m, 1H), 4.32-4.18 (m, 4H), 3.79-3.72 (m, 2H), 3.20-3.12 (m, 1H), 3.10 (s, 3H), 3.02-2.93 (m, 1H), 2.90-2.76 (m, 1H), 2.74-2.66 (m, 1H), 2.57-2.48 (m, 1H), 2.10-1.10 (m, 4H).
MS (ESI+) 392 (M⁺+1, 100%).

### Example 30

2-[(3R)-3-Aminopiperidin-1-yl]-3-(2-chloro-5-fluorobenzyl)-5-methylpyrazolo[1,5-a]pyrazin-4(5H)-one

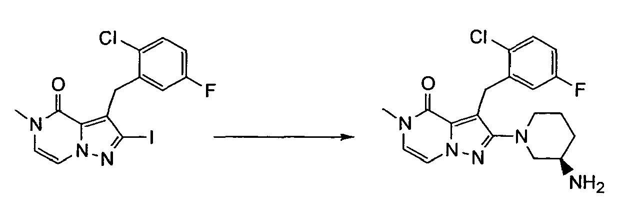

A suspension of 3-(2-chloro-5-fluorobenzyl)-2- iodo-5-methylpyrazolo[1,5-a]pyrazin-4(5H)-one (100 mg), (R)-tert-3-butylpiperidin-3-yl carbamate (96 mg), potassium phosphate (102 mg), ethylene glycol (30 mg) and copper iodide (2.3 mg) in 2-propanol (2 ml) was stirred in a sealed tube at 80°C for 8 hours and then at 110°C for 20 hours. After cooling to room temperature, water was added, followed by two runs of extraction with ethyl acetate. The combined organic layer was concentrated under reduced pressure. The resulting residue was purified by a silica gel column chromatography (hexane/ethyl acetate = 1/1) to obtain a product (25 mg). Subsequently, to a solution of this product in methanol (5 ml) was added a 12N aqueous hydrochloric acid solution (2 ml) at room temperature and the resulting mixture was allowed to stand for 2 hours. The reaction mixture was concentrated under reduced pressure and 1N hydrochloric acid was added to the residue, followed by extraction with ethyl acetate. The aqueous layer was made basic with potassium carbonate and extracted twice with chloroform. The combined organic layer was dried over anhydrous sodium sulfate and filtered, and the filtrate was concentrated under reduced pressure to obtain the title compound (2 mg) as a brown solid.
¹H NMR (400 MHz, CDCl₃) δ ppm 7.35-7.30 (m, 1H), 7.28 (d, J = 5.9Hz, 1H), 6.87-6.80 (m, 1H), 6.74-6.69 (m, 1H), 6.44 (d, J = 5.9Hz, 1H), 4.45 (d, J = 17.4Hz, 1H), 4.38 (d, J = 17.4Hz, 1H), 3.46 (s, 3H), 3.35-3.28 (m, 1H), 3.20-3.12 (m, 1H), 2.88-2.72 (m, 2H), 2.61-2.53 (m, 1H), 1.89-1.80 (m, 1H), 1.72-1.17 (m, 3H).
MS (ESI+) 390 (M⁺+1, 100%) .

### Example 31

2-[(3R)-3-Aminopiperidin-1-yl]-3-(2-chlorobenzyl)-5-(3-fluorophenyl)-4-oxo-4,5-dihydropyrazolo[1,5-a]pyrazine-6-carboxylic acid hydrochloride

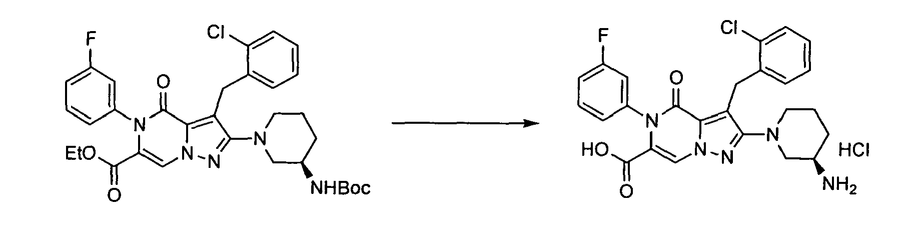

A 1N-aqueous sodium hydroxide solution (1.0 ml) was added to a solution of ethyl 2-{(3R)-3-[(tert-butoxycarbonyl)amino]piperidin-1-yl}-3-(2-chlorobenzyl)-5-(3-fluorophenyl)-4-oxo-4,5-dihydropyrazolo[1,5-a]pyrazine-6-carboxylate (88.0 mg) in 1,4-dioxane (2.0 ml), and the resulting mixture was stirred at 50°C for 2 hours. After cooling to room temperature, a 5% aqueous sodium hydrogensulfate solution (20 ml) was added, followed by two runs of extraction with ethyl acetate (20 ml). The combined organic layer was concentrated under reduced pressure. The resulting residue was purified by a silica gel column chromatography (silica gel, chloroform/methanol = 50/1) to obtain a purified substance (62.6 mg). A 4N hydrochloric acid/1,4-dioxane solution (1.0 ml) was added to a suspension (0.1 ml) of the purified substance (59.6 mg) in 1,4-dioxane, and the resulting mixture was stirred at room temperature for 1 hour. The reaction mixture was concentrated under reduced pressure to obtain the title compound as a crude product (60.0 mg).
¹H NMR (400 MHz, CD₃OD) δ ppm 8.27 (s, 1H), 8.22-8.20 (m, 3H), 7.46-7.44 (m, 2H), 7.25-7.21 (m, 4H), 7.14-7.12 (m, 1H), 7.04-7.03 (m, 1H), 4.31 (s, 2H), 3.67-3.66 (m, 1H), 3.30-3.28 (m, 1H), 3.15-3.13 (m, 1H), 3.02-2.99 (m, 1H), 2.68-2.65 (m, 1H), 1.93-1.91 (m, 1H), 1.68-1.66 (m, 1H), 1.50-1.38 (m, 2H).
MS (ESI+) 496 (M⁺+1, 100%).

### Example 32

Ethyl 2-[(3R)-3-aminopiperidin-1-yl]-3-(2-chloro-5-fluorobenzyl)-4-oxo-4,5-dihydropyrazolo[1,5-a]pyrazine-6-carboxylate methanesulfonate

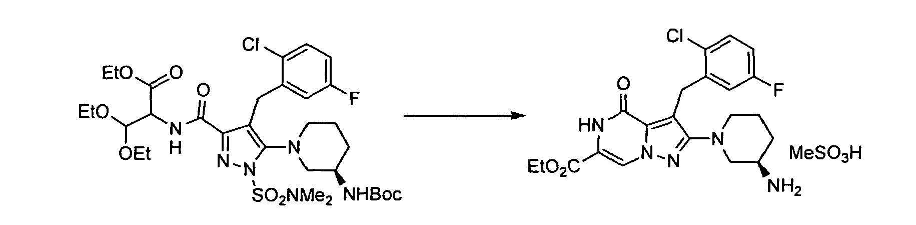

To a solution of ethyl N-({5-{(3R)-3-[(tert-butoxycarbonyl)amino]piperidin-1-yl}-4-(2-chloro-5-fluorobenzyl)-1-[(dimethylamino)sulfonyl]-1H-pyrazol-3-yl}carbonyl)-3-ethoxy-O-ethylserinate (300 mg) in 1,4-dioxane (4 ml) was added 4N hydrochloric acid/1,4-dioxane (8 ml), and the resulting mixture was stirred at 50°C for 1 hour. After the mixture was allowed to cool, a saturated aqueous sodium hydrogencarbonate solution was added, followed by extraction with chloroform. The organic layer was washed with a saturated aqueous sodium chloride solution, dried over sodium sulfate and then filtered, and the filtrate was concentrated under reduced pressure. The resulting solid was dissolved in 2-propanol (20 ml), followed by adding thereto methanesulfonic acid (77 mg). The resulting mixture was stirred at 60°C for 30 minutes and allowed to cool. The solid thus obtained was collected by filtration and washed with 2-propanol to obtain the title compound (115 mg) as a white solid.
¹H NMR (400 MHz, CD₃OD) δ ppm 8.18 (s, 1H), 7.47-7.43 (m, 1H), 7.01-6.96 (m, 1H), 6.80-6.75 (m, 1H), 4.50-4.38 (m, 4H), 3.74-3.65 (m, 1H), 3.20-3.05 (m, 3H), 2.90-2.80 (m, 1H), 2.71 (s, 3H), 2.08-1.98 (m, 1H), 1.81-1.72 (m, 1H), 1.63-1.50 (m, 2H), 1.41 (t, J = 7.1Hz, 3H).
MS (ESI+) 448 (M⁺+1, 53%).

### Example 33

2-[(3R)-3-Aminopiperidin-1-yl]-3-(2-chlorobenzyl)-5-methylpyrazolo[1,5-d][1,2,4]triazin-4(5H)-one hydrochloride

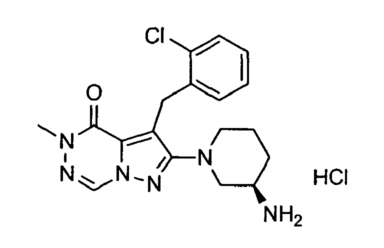

The title compound (82 mg) was synthesized by the same process as in Example 8.
¹H NMR(300MHz, DMSO-d₆) δ ppm 8.80 (s, 1H), 8.25 (bs, 3H), 7.47-7.43 (m, 1H), 7.26-7.17 (m, 2H), 7.02-6.99 (m, 1H), 4.34 (d, J=17.6Hz, 1H), 4.27 (d, J=17.6Hz, 1H), 3.49 (s, 3H), 3.47-3.45 (m, 1H), 3.13-3.09 (m, 2H), 2.96-2.89 (m, 1H), 2.62-2.58 (m, 1H), 1.92-1.88 (m, 1H), 1.63-1.60 (m, 1H), 1.46-1.32 (m, 2H).
MS (ESI+) 373 (M⁺+1, 43%) .

### Example 34

2-[(3R)-3-Aminopiperidin-1-yl]-3-(2-chlorobenzyl)-5-(2-oxo-2-phenylethyl)pyrazolo[1,5-d][1,2,4]triazin-4(5H)-one hydrochloride

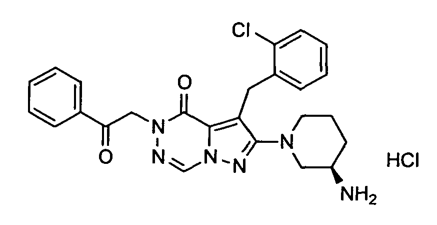

The title compound (125 mg) was synthesized by the same process as in Example 8.
¹H NMR(300MHz, DMSO-d₆) δ ppm 8.90 (s, 1H), 8.21 (bs, 3H), 8.03 (d, J=7.3Hz, 2H), 7.73-7.68 (m, 1H), 7.59-7.54 (m, 2H), 7.46-7.43 (m, 1H), 7.24-7.21 (m, 2H), 7.03-7.01 (m, 1H), 5.55 (s, 2H), 4.36 (d, J=17.7Hz, 1H), 4.29 (d, J=17.7Hz, 1H), 3.46-3.44 (m, 1H), 3.16-3.12 (m, 2H), 2.99-2.92 (m, 1H), 2.67-2.63 (m, 1H), 1.93-1.90 (m, 1H), 1.65-1.62 (m, 1H), 1.48-1.34 (m, 2H).
MS (ESI+) 477 (M⁺+1, 100%).

### Example 35

2-[(3R)-3-Aminopiperidin-1-yl]-3-(2-chlorobenzyl)-5-(isoquinolin-1-ylmethyl)pyrazolo[1,5-d][1,2,4]triazin-4(5H)-one hydrochloride

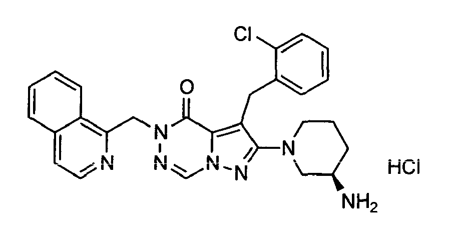

The title compound (141 mg) was synthesized by the same process as in Example 8.
¹H NMR(300MHz, DMSO-d₆) δ ppm 8.89 (s, 1H), 8.50-8.48 (m, 2H), 8.39 (bs, 3H), 8.24-8.21 (m, 2H), 8.08-8.02 (m, 1H), 7.92-7.89 (m, 1H), 7.45-7.42 (m, 1H), 7.24-7.21 (m, 2H), 7.14-7.11 (m, 1H), 6.02 (s, 2H), 4.38-4.26 (m, 2H), 3.73-3.71 (m, 1H), 3.15-3.13 (m, 2H), 3.00-2.95 (m, 1H), 2.64-2.60 (m, 1H), 1.94-1.90 (m, 1H), 1.65-1.61 (m, 1H), 1.53-1.25 (m, 2H).
MS (ESI+) 500 (M⁺+1, 100%).

### Example 36

2-[(3R)-3-Aminopiperidin-1-yl]-3-(2-chlorobenzyl)-7-(trifluoromethyl)pyrazolo[1,5-d][1,2,4]triazin-4(5H)-one hydrochloride

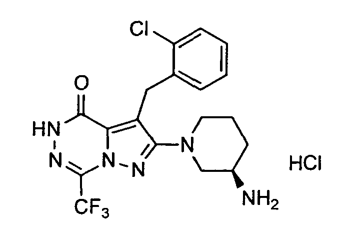

The title compound (6 mg) was synthesized by the same process as in Example 8.
¹H NMR(300MHz, CD₃OD) δ ppm 7.35-7.31 (m, 1H), 7.13-7.09 (m, 2H), 6.96-6.92 (m, 1H), 4.45-4.29 (m, 2H), 3.50-3.46 (m, 1H), 3.25-3.11 (m, 2H), 3.02-2.95 (m, 1H), 2.76-2.67 (m, 1H), 1.95-1.90 (m, 1H),1.64-1.30 (m, 3H).
MS (ESI+) 527 (M⁺+1, 51%).

### Example 37

2-[(3R)-3-Aminopiperidin-1-yl]-3-(2-chlorobenzyl)-5-(2-oxo-2-phenylethyl)-7-(trifluoromethyl)pyrazolo[1,5- d][1,2,4]triazin-4(5H)-one hydrochloride

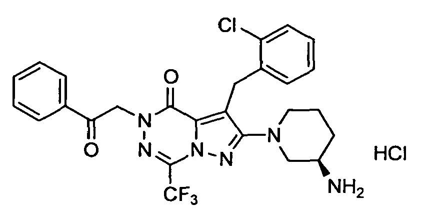

The title compound (18 mg) was synthesized by the same process as in Example 8.
¹H NMR(300MHz, CD₃OD) δ ppm 8.08-8.02 (m, 2H), 7.71-7.61 (m, 1H), 7.53-7.48 (m, 2H), 7.38-7.33 (m, 1H), 7.12-6.98 (m, 2H), 6.86-6.83 (m, 1H), 5.84-5.69 (m, 2H), 4.35-4.24 (m, 2H), 3.62-3.44 (m, 1H), 2.97-2.70 (m, 4H), 1.92-1.81 (m, 1H), 1.64-1.60 (m, 1H), 1.46-1.21 (m, 2H).
MS (ESI+) 545 (M⁺+1, 85%).

### Example 38

Methyl 2-[(3R)-3-aminopiperidin-1-yl]-3-(2-chlorobenzyl)-7-(2-methoxy-2-oxoethyl)-4-oxo-4,5-dihydropyrazolo[1,5-a]pyrazine-6-carboxylate hydrochloride

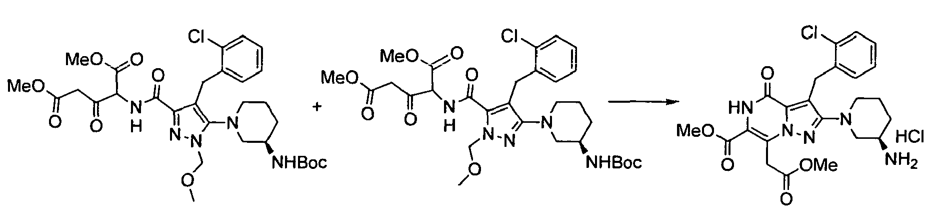

A dimethyl N-{[5-{(3R)-3-[(tert-butoxycarbonyl)amino]piperidin-1-yl}-4-(2-chlorobenzyl)-1-(methoxymethyl)-1H-pyrazol-3-yl]carbonyl}-3-oxoglutamate mixture (57.5 mg) was dissolved in a 4N hydrochloric acid/1,4-dioxane solution (1.5 ml), followed by adding thereto water (0.5 ml), and the resulting mixture was stirred at 50°C for 2 hours. The reaction solution was concentrated under reduced pressure and the water remaining in the resulting residue was distilled off as an azeotrope with methanol to obtain the title compound (40.4 mg).
¹H NMR (300 MHz, DMSO-d₆) δ ppm 11.05 (bs, 1H), 8.16 (bs, 3H), 7.50-7.35 (m, 1H), 7.30-7.10 (m, 2H), 7.00-6.90 (m, 1H), 4.40 (s, 2H), 4.35 (s, 2H), 3.81 (s, 3H), 3.80-3.60 (m, 1H), 3.68 (s, 3H), 3.20-2.80 (m, 4H), 1.95-1.75 (m, 1H), 1.70-1.55 (m, 1H), 1.55-1.20 (m, 2H).
MS (ESI+) 488 (M⁺+1, 100%).

### Example 39

2-[(3R)-3-Aminopiperidin-1-yl]-3-(2-chlorobenzyl)-9-methyl-7,8-dihydro-4H,6H-pyrazolo[1,5-a]pyrrolo[1,2-d]pyrazin-4-one hydrochloride

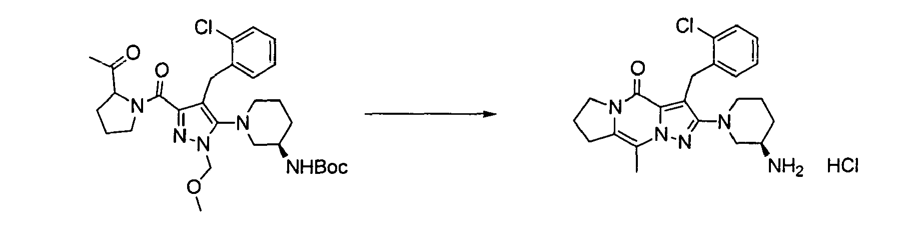

The title compound (15 mg) was synthesized by the same process as in Example 2.
¹H NMR (300 MHz, DMSO-d₆) δ ppm 11.05 (bs, 1H), 8.16 (bs, 3H), 7.50-7.35 (m, 1H), 7.30-7.10 (m, 2H), 7.00-6.90 (m, 1H), 4.40 (s, 2H), 4.35 (s, 2H), 3.81 (s, 3H), 3.80-3.60 (m, 1H), 3.68 (s, 3H), 3.20-2.80 (m, 4H), 1.95-1.75 (m, 1H), 1.70-1.55 (m, 1H), 1.55-1.20 (m, 2H).
MS (ESI+) 488 (M⁺+1, 100%).

### Example 40

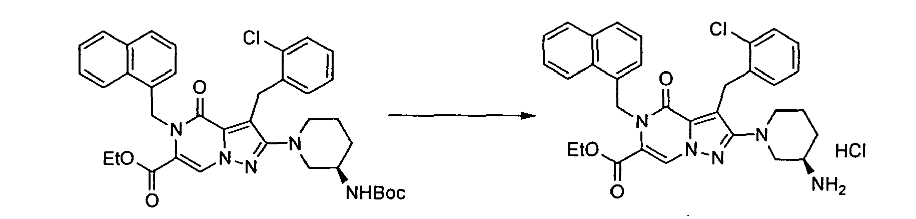

The title compound (127 mg) was synthesized by the same process as in Example 8.
¹H NMR (400 MHz, DMSO-d₆) δ ppm 8.30 (s, 1H), 8.18-8.14 (m, 4H), 7.97-7.94 (m, 1H), 7.80 (d, J = 8.2Hz, 1H), 7.58-7.55 (m, 2H), 7.45-7.42 (m, 1H), 7.37-7.36 (m, 1H), 7.23-7.21 (m, 2H), 7.05-7.03 (m, 1H), 6.92-6.90 (m, 1H), 6.03 (s, 2H), 4.34-4.31 (m, 2H), 3.68-3.64 (m, 1H), 3.19-3.17 (m, 1H), 3.08-3.06 (m, 1H), 3.00-2.95 (m, 1H), 2.67-2.65 (m, 1H), 1.93-1.91 (m, 1H), 1.67-1.65 (m, 1H), 1.50-1.40 (m, 2H).
MS (ESI+) 542 (M⁺+1, 76%).

### Example 41

Ethyl 2-[(3R)-3-aminopiperidin-1-yl]-3-(2-chlorobenzyl)-4-oxo-4,5-dihydropyrazolo[1,5-a]pyrazine-6-carboxylate hydrochloride

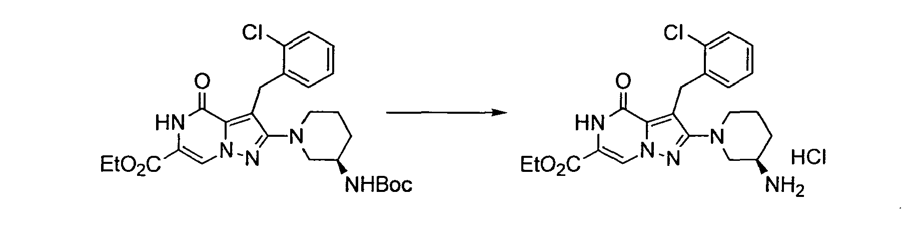

The title compound (23 mg) was synthesized by the same process as in Example 8.
¹H NMR (400 MHz, DMSO-d₆) 8.16 (s, 1H), 8.05-8.00 (m, 4H), 7.47-7.45 (m, 1H), 7.26-7.18 (m, 2H), 7.00-6.97 (m, 1H), 4.38-4.27 (m, 4H), 3.59-3.57 (m, 1H), 3.18-3.16 (m, 1H), 3.05-3.02 (m, 1H), 2.95-2.92 (m, 1H), 2.61-2.52 (m, 1H), 1.91-1.90 (m, 1H), 1.72-1.69 (m, 1H), 1.43-1.37 (m, 2H), 1.31 (t, J = 7.1Hz, 3H).
MS (ESI+) 430 (M⁺+1, 50%).

### Reference Example 1

3-(2-Chloro-5-fluorobenzyl)-2-iodo-5-methyl-6,7- dihydropyrazolo[1,5-a]pyrazin-4(5H)-one

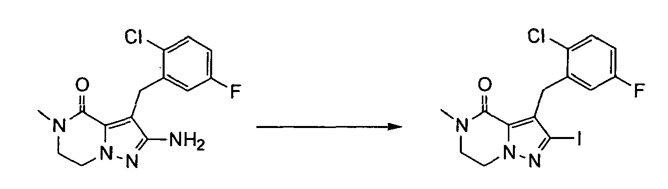

To a mixture of diiodomethane (5 ml) and isoamyl nitrite (2.2 ml) was added 2-amino-3-(2-chloro-5-fluorobenzyl)-5-methyl-6,7-dihydropyrazolo[1,5-a]pyrazin-4(5H)-one (1 g) at room temperature, and the resulting mixture was stirred at the same temperature for 1 hour. The reaction solution was purified by a silica gel column chromatography (hexane/ethyl acetate = 1/1) to obtain the title compound (0.98 g) as a yellow solid.
¹H NMR (400 MHz, CDCl₃) δ ppm 7.28-7.23 (m, 1H), 6.79-6.72 (m, 1H), 6.47-6.42 (m, 1H), 4.42-4.37 (m, 2H), 4.11 (s, 2H), 3.73-3.68 (m, 2H), 3.04 (s, 3H).
MS (ESI+) 420 (M⁺+1, 100%).

### Reference Example 2

3-(2-Chloro-5-fluorobenzyl)-2-iodo-5-methylpyrazolo[1,5-a]pyrazin-4(5H)-one

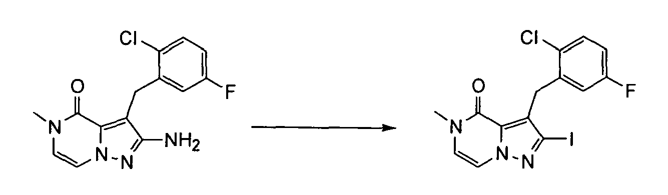

To a mixed solution of diiodomethane (5 ml) and isoamyl nitrite (2.2 ml) was added 2-amino-3-(2-chloro-5-fluorobenzyl)-5-methylpyrazolo[1,5-a]pyrazin-4(5H)-one (1 g) at room temperature, and the resulting mixture was stirred at the same temperature for 4 hours. The reaction solution was purified by a silica gel column chromatography (from chloroform to hexane/ethyl acetate = 2/1 ~ 1/1). The resulting purified substance was filtered and washed with ethyl acetate/hexane (about 1/3) and the precipitate on a filter was dried to obtain the title compound (0.77 g) as a yellow solid.
¹H NMR (400 MHz, CDCl₃) δ ppm 7.44 (d, J = 6.0Hz, 1H), 7.37-7.32 (m, 1H), 6.88-6.81 (m, 1H), 6.56 (d, J = 6.0Hz, 1H), 6.51-6.46 (m, 1H), 4.34 (s, 2H), 3.49 (s, 3H).
MS (ESI+) 418 (M⁺+1, 100%).

### Reference Example 3

2-Amino-3-(2-chloro-5-fluorobenzyl)-5-methyl-6,7-dihydropyrazolo[1,5-a]pyrazin-4(5H)-one

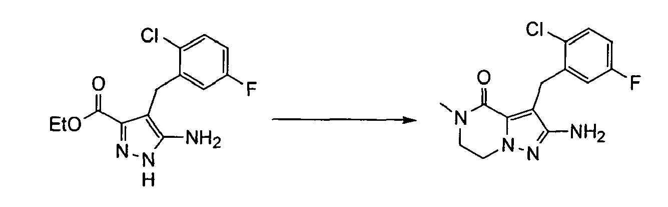

Di-tert-butyl dicarbonate (70.3 g) was added to a solution of ethyl 3-amino-4-(2-chloro-5-fluorobenzyl)-1H-pyrazole-5-carboxylate (24.0 g), 4-(dimethylamino)pyridine (9.8 g) and triethylamine (56.1 ml) in tetrahydrofuran (200 ml) under ice-cooling, and the resulting mixture was stirred at the same temperature for 2 hours and then allowed to stand overnight at room temperature. Water was added thereto, followed by extraction with ethyl acetate. The organic layer was washed three times with a 5% aqueous potassium hydrogensulfate solution and then with a saturated aqueous sodium hydrogencarbonate solution and a saturated aqueous sodium chloride solution, and dried over sodium sulfate. The dried organic layer was filtered and the filtrate was concentrated under reduced pressure to obtain a product (60.5 g). Then, to a solution of this product in methanol (200 ml) was added a solution of sodium hydroxide (12.9 g) in water (100 ml), and the resulting mixture was stirred at 50°C for 6 hours and then allowed to stand overnight at room temperature. The solvent was concentrated under reduced pressure and the residue was acidified with a 5% aqueous potassium hydrogensulfate solution (1000 ml) and extracted twice with ethyl acetate (400 ml). The combined organic layer was washed with a saturated aqueous sodium chloride solution, dried over magnesium sulfate and then filtered, and the filtrate was concentrated under reduced pressure to obtain a product (35.7 g). To a solution of this product (17.9 g) in N,N-dimethylformamide (100 ml) were added 1-hydroxybenzotriazole (6.16 g) and N-methylaminoethanol (3.03 g), followed by adding thereto 1-ethyl-3-(3-dimethylaminopropyl)carbodiimide hydrochloride (7.72 g) under ice-cooling, and the resulting mixture was stirred at the same temperature for 1 hour and allowed to stand overnight at room temperature. Ice water (500 ml) was added to the reaction mixture and the crystals precipitated were filtered. This solid was dissolved in methanol/chloroform (about 1/1) and the resulting solution was concentrated under reduced pressure. Ethanol/toluene (about 1/1) was added to the concentrate and the resulting mixture was concentrated under reduced pressure to obtain the residue. The aqueous layer of the filtrate was extracted with ethyl acetate (500 ml) and the extract solution was washed with a saturated aqueous sodium chloride solution (300 ml), dried over sodium sulfate and then filtered. The filtrate was concentrated under reduced pressure to obtain the residue. These residues were combined to obtain a product (24.8 g). To a solution of this product in N,N-dimethylformamide (100 ml) was added triphenylphosphine (15.8 g), followed by adding thereto carbon tetrabromide (20 g) under ice-cooling, and the resulting mixture was stirred at the same temperature for 15 minutes and then at room temperature for 1 hour. Potassium carbonate (16.7 g) was added to the reaction solution, followed by stirring at 70°C for 2 hours. The resulting mixture was cooled to room temperature, poured into ice water (500 ml) and then extracted twice with ethyl acetate (300 ml). The combined organic layer was washed twice with water (300 ml) and then once with a saturated aqueous sodium chloride solution, and concentrated under reduced pressure to obtain a product (48 g). To a solution of this product in methanol (200 ml) was added a 12N aqueous hydrochloric acid solution (100 ml) and the resulting mixture was allowed to stand overnight at room temperature. After the solvent was concentrated under reduced pressure, a 3N aqueous hydrochloric acid solution (200 ml) and water (300 ml) were added, and the resulting mixture was washed with ethyl acetate (500 ml). The aqueous layer was made basic with potassium carbonate and extracted twice with chloroform (300 ml). The combined organic layer was washed with a saturated aqueous sodium chloride solution, dried over sodium sulfate and then filtered, and the filtrate was concentrated under reduced pressure. The resulting residue was purified by a silica gel column chromatography (from chloroform/ethyl acetate = 2/3 to ethyl acetate, ethyl acetate/methanol = 10/1). The resulting purified substance was filtered and washed with toluene/hexane (about 1/2) and dried to obtain the title compound (3.9 g) as a white solid.
¹H NMR (400 MHz, CDCl₃) δ ppm 7.32-7.28 (m, 1H), 6.98-6.93 (m, 1H), 6.86-6.81 (m, 1H), 4.20 (s, 2H), 4.22-4.18 (m, 2H), 3.75-3.71 (m, 2H),3.62 (s, 2H), 3.11 (s, 3H).

### Reference Example 4

2-Amino-3-(2-chloro-5-fluorobenzyl)-5-methylpyrazolo[1,5-a]pyrazin-4(5H)-one

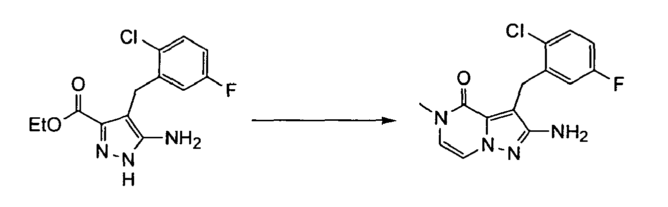

Di-tert-butyl dicarbonate (70.3 g) was added to a solution of ethyl 3-amino-4-(2-chloro-5-fluorobenzyl)-1H-pyrazole-5-carboxylate (24.0 g), dimethylaminopyridine (9.8 g) and triethylamine (56.1 ml) in tetrahydrofuran (200 ml) under ice-cooling, and the resulting mixture was stirred at the same temperature for 2 hours and then allowed to stand overnight at room temperature. Water was added thereto, followed by extraction with ethyl acetate, and the organic layer was washed three times with a 5% aqueous potassium hydrogensulfate solution and then with a saturated aqueous sodium hydrogencarbonate solution and a saturated aqueous sodium chloride solution, dried over sodium sulfate and then filtered. The filtrate was concentrated under reduced pressure to obtain a product (60.5 g). Subsequently, to a solution of this product in methanol (200 ml) was added a solution of sodium hydroxide (12.9 g) in water (100 ml), and the resulting mixture was stirred at 50°C for 6 hours and then allowed to stand overnight at room temperature. The solution thus obtained was concentrated under reduced pressure, acidified with a 5% aqueous potassium hydrogensulfate solution (1000 ml), and then extracted twice with ethyl acetate (400 ml). The combined organic layer was washed with a saturated aqueous sodium chloride solution, dried over magnesium sulfate and then filtered, and the filtrate was concentrated under reduced pressure to obtain a product (35.7 g). To a solution of this product (17.9 g) in N,N-dimethylformamide (100 ml) were added 1-hydroxybenzotriazole (6.16 g) and N-methylacetaldehyde dimethylacetal (4.8 g), followed by adding thereto 1-ethyl-3-(3-dimethylaminopropyl)carbodiimide hydrochloride (7.72 g) under ice-cooling, and the resulting mixture was stirred at the same temperature for 1 hour and allowed to stand overnight at room temperature. Ice water was added to the reaction mixture, followed by two runs of extraction with ethyl acetate. The combined organic layer was washed twice with a 5% aqueous potassium hydrogensulfate solution and then successively with a saturated aqueous sodium hydrogencarbonate solution and a saturated aqueous sodium chloride solution, dried over sodium sulfate and then filtered. The filtrate was concentrated under reduced pressure. To a solution of the resulting residue in 1,4-dioxane (50 ml) was added 4N hydrochloric acid/1,4-dioxane (100 ml) at room temperature and the resulting mixture was allowed to stand at the same temperature for 5 hours. Toluene/hexane was added to the reaction solution and the solid precipitated was filtered. The solid thus obtained was dissolved in water and the resulting solution was made basic with a saturated aqueous sodium hydrogencarbonate solution and extracted twice with chloroform. The combined organic layer was concentrated under reduced pressure. The residue was washed with chloroform and dried to obtain the title compound (4.1 g) as a white solid.
¹H NMR (400 MHz, CDCl₃) δ ppm 7.34-7.30 (m, 1H), 7.19 (d, J = 5.9Hz, 1H), 7.00-6.95 (m, 1H), 6.88-6.82 (m, 1H), 6.41 (d, J = 5.9Hz, 1H), 4.35 (s, 2H), 3.86 (s, 2H), 3.49 (s, 3H).
MS (ESI+) 307 (M⁺+1, 100%).

### Reference Example 5

Ethyl 3-amino-4-(2-chloro-5-fluorobenzyl)-1H-pyrazole-5-carboxylate

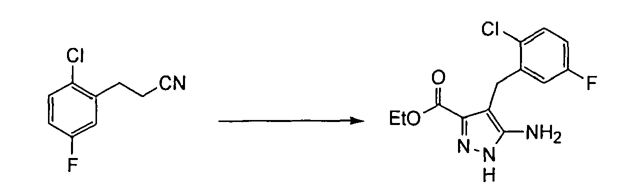

Sodium (3.7 g) was added to ethanol (100 ml) in small portions under ice-cooling and stirred until it was completely dissolved. A solution of 3-(2-chloro-5-fluorophenyl)propanenitrile (24.0 g) and diethyl oxalate (16.8 ml) in ethanol (50 ml) was added dropwise thereto, and the resulting mixture was stirred at 80°C for 6 hours and allowed to stand overnight. Ice water was added thereto and the pH was adjusted to 1 with a 1N aqueous hydrochloric acid solution, followed by two runs of extraction with ethyl acetate. The combined organic layer was washed with a saturated aqueous sodium chloride solution, dried over magnesium sulfate and then filtered, and the filtrate was concentrated under reduced pressure to obtain a product (36.1 g). Subsequently, to a suspension of this product in ethanol (150 ml) were added hydrazine hydrate (9 ml) and acetic acid (20 ml), and the resulting mixture was stirred at 80°C for 3 hours. The reaction solution was cooled to room temperature and ice water was added thereto. Then, the resulting mixture was made basic with a 5% aqueous potassium carbonate solution and the insoluble material precipitated was filtered. The insoluble material was dissolved in methanol, followed by adding thereto toluene, and the resulting mixture was concentrated under reduced pressure. The solid precipitated was washed with hexane/toluene and dried to obtain the title compound (24.0 g) as a white solid.
¹H NMR (400 MHz, CDCl₃) δ ppm 7.35-7.31 (m, 1H), 6.88-6.83 (m, 1H), 6.77-6.73(m, 1H), 4.30 (q, J = 7.1Hz, 2H), 4.08 (s, 2H), 1.23 (t, J = 7.1Hz, 3H).
MS (ESI+) 298 (M⁺+1, 100%).

### Reference Example 6

3-(2-Chloro-5-fluorophenyl)propanenitrile

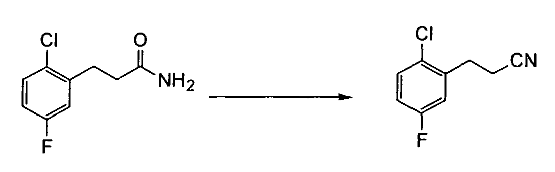

Phosphorus oxychloride (23.66 g) was added to a suspension of 3-(2-chloro-5-fluorophenyl)propanamide (24.9 g) in toluene (200 ml) at room temperature, and the resulting mixture was stirred at 80°C for 6 hours. The reaction solution was cooled to room temperature and ice water was added thereto, followed by extraction with ethyl acetate. The organic layer was washed successively with water, a saturated aqueous sodium hydrogencarbonate solution and a saturated aqueous sodium chloride solution, dried over magnesium sulfate and then filtered, and the filtrate was concentrated under reduced pressure to obtain the title compound (24.0 g) as a yellow oil.
¹H NMR (400 MHz, CDCl₃) δ ppm 7.37-7.33 (m, 1H), 7.07-7.02 (m, 1H), 7.00-6.94 (m, 1H), 3.06 (t, J = 7.3Hz, 2H), 2.69 (t, J = 7.3Hz, 2H).
MS (ESI+) 184 (M⁺+1, 100%).

### Reference Example 7

3-(2-Chloro-5-fluorophenyl)propanamide

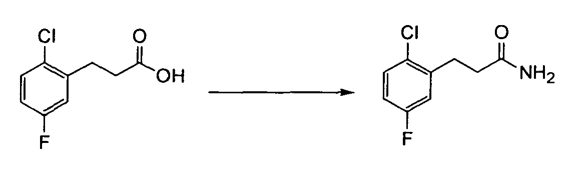

Thionyl chloride (15 ml) was added dropwise to a solution of 3-(2-chloro-5-fluorophenyl)propionic acid (38.8 g) and N,N-dimethylformamide (0.1 ml) in toluene (400 ml) at room temperature, and the resulting mixture was stirred at 60°C for 3 hours. The solvent was concentrated under reduced pressure and toluene (200 ml) was added, followed by concentration under reduced pressure. Toluene (50 ml) was added to the residue to obtain a solution. This solution was added dropwise to 28% aqueous ammonia (233 ml) under ice-cooling and the resulting mixture was stirred at the same temperature for 30 minutes and then at room temperature for 6 hours, and allowed to stand overnight at the same temperature. Water was added to the reaction mixture, followed by extraction with ethyl acetate. The combined organic layer was washed with a 1N aqueous hydrochloric acid solution, a saturated aqueous sodium hydrogencarbonate solution and a saturated aqueous sodium chloride solution, dried over magnesium sulfate and then filtered, and the filtrate was concentrated under reduced pressure. The solid precipitated was washed with hexane/ethyl acetate and dried to obtain the title compound (23.6 g) as a white solid.
¹H NMR (400 MHz, CDCl₃) δ ppm 7.33-7.28 (m, 1H), 7.04-6.99 (m, 1H), 6.92-6.85(m, 1H), 5.52 (d, J = 6.4Hz, 2H), 3.06 (t, J = 7.7Hz, 2H), 2.54 (t, J = 7.7Hz, 2H).
MS (ESI+) 202 (M⁺+1, 100%).

### Reference Example 8

3-(2-Chloro-5-fluorophenyl)propionic acid

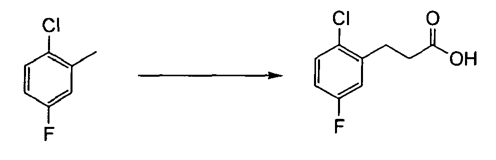

To a solution of 2-chloro-5-fluorotoluene (50.0 g) in carbon tetrachloride (750 ml) were added N-bromosuccinimide (67.7 g) and azoisobutyronitrile (0.7 g), and the resulting mixture was stirred with heating under reflux for 3 hours and allowed to stand overnight at room temperature. The insoluble material precipitated was filtered off and the filtrate was concentrated under reduced pressure. The resulting residue was purified by a silica gel column chromatography (hexane/ethyl acetate = 1/1) to obtain a purified substance (82.2 g). A solution (150 ml) of this product in dimethyl sulfoxide was added dropwise to a solution of meldramic acid (150 g) and triethylamine (100 ml) in dimethyl sulfoxide (500 ml) under ice-cooling over a period of 30 minutes, and the resulting mixture was allowed to stand at room temperature for 3 days. The reaction solution was poured into ice water (2000 ml) and extracted twice with ethyl acetate (1000 ml), and the combined organic layer was concentrated under reduced pressure to obtain a product. Then, a suspension of this product in methanol (600 ml) was stirred with heating under reflux for 10 hours and allowed to stand overnight at room temperature. The reaction mixture was concentrated under reduced pressure and water (500 ml) was added thereto, followed by two runs of extraction with ethyl acetate (300 ml). The combined organic layer was concentrated under reduced pressure to obtain a product (110 g). Subsequently, to this product were added 1,4-dioxane (500 ml) and a 12N aqueous hydrochloric acid solution (200 ml), and the resulting mixture was stirred with heating under reflux for 20 hours. The mixture was cooled to room temperature and then concentrated under reduced pressure and water (500 ml) was added thereto, followed by two runs of extraction with ethyl acetate (500 ml). The combined organic layer was concentrated under reduced pressure, followed by adding thereto a solution of potassium carbonate (70 g) in water (500 ml) and a solution of sodium hydroxide (40 g) in water (300 ml), and the resulting mixture was washed with hexane (500 ml). The aqueous layer was acidified with 12N hydrochloric acid and extracted three times with chloroform (1000 ml). After the combined organic layer was concentrated under reduced pressure, tetrahydrofuran was added to the residue and the insoluble material was filtered off. The filtrate was concentrated under reduced pressure to obtain the title compound (38.8 g).
¹H NMR (400 MHz, CDCl₃) δ ppm 7.33-7.29 (m, 1H), 7.02-6.98 (m, 1H), 6.93-6.87(m, 1H), 3.04 (t, J = 7.7Hz, 2H), 2.71 (t, J = 7.7Hz, 2H).
MS (ESI+) 203 (M⁺+1, 100%).

### Reference Example 9

Ethyl 2-{(3R)-3-[(tert-butoxycarbonyl)amino]-piperidin-1-yl}-3-(2-chlorobenzyl)-5-(3-fluorophenyl)-4-oxo-4,5-dihydropyrazolo[1,5-a]pyrazine-6-carboxylate

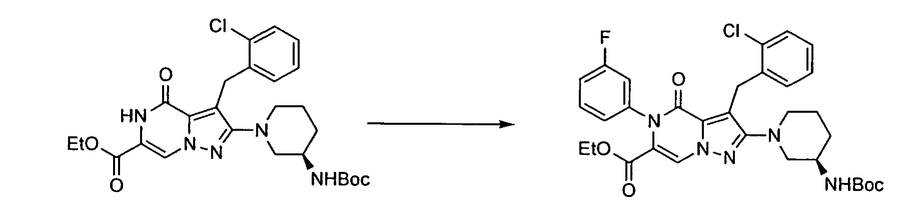

To a solution of ethyl 2-{(3R)-3-[(tert-butoxycarbonyl)amino]piperidin-1-yl}-3-(2-chlorobenzyl)-4-oxo-4,5-dihydropyrazolo[1,5-a]pyrazine-6-carboxylate (100 mg) in dichloromethane (1.5 ml) were added 3-fluorophenylboric acid (79.3 mg), copper (II) acetate (68.5 mg), molecular sieves (4Å, 80 mg) and pyridine (0.0611 ml), and the resulting mixture was stirred overnight at room temperature. The reaction mixture was filtered through Celite and the filtrate was concentrated under reduced pressure. The resulting residue was purified by a silica gel column chromatography (hexane/ethyl acetate = 3/1) to obtain the title compound (90.0 mg).
MS (ESI+) 624 (M⁺+1, 92%).

### Reference Example 10

Methyl 2-{(3R)-3-[(tert-butoxycarbonyl)amino]-piperidin-1-yl}-3-(2-chlorobenzyl)-4-oxo-4,5-dihydropyrazolo[1,5-a]pyrazine-6-carboxylate

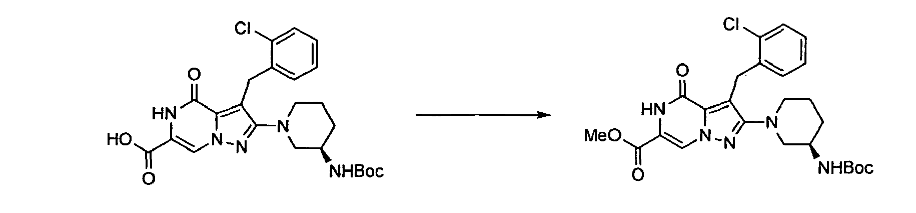

Methanol (0.072 ml), 1-ethyl-3-(3-dimethylaminopropyl)carbodiimide hydrochloride (343 mg), 1-hydroxybenzotriazole monohydrate (242 mg) and triethylamine (0.248 ml) were added to a solution of 2-{(3R)-3-[(tert-butoxycarbonyl)amino]piperidin-1-yl}-3-(2-chlorobenzyl)-4-oxo-4,5-dihydropyrazolo[1,5-a]pyrazine-6-carboxylic acid (448 mg) in N,N-dimethylformamide (20 ml), and the resulting mixture was stirred overnight at room temperature. A saturated aqueous sodium hydrogencarbonate solution (200 ml) was added thereto, followed by two runs of extraction with ethyl acetate (100 ml), and the combined organic layer was concentrated under reduced pressure. The resulting residue was purified by a silica gel column chromatography (hexane/ethyl acetate = 2/1) to obtain the title compound (255 mg).
MS (ESI+) 516 (M⁺+1, 35%).

### Reference Example 11

2-{(3R)-3-[(tert-Butoxycarbonyl)aminolpiperidin-1-yl}-3-(2-chlorobenzyl)-4-oxo-4,5-dihydropyrazolo[1,5-a]pyrazine-6-carboxylic acid

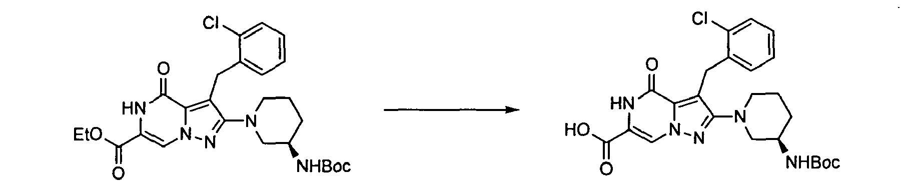

A 1N-aqueous sodium hydroxide solution (10 ml) was added to a solution of ethyl 2-{(3R)-3-[(tert-butoxycarbonyl)amino]piperidin-1-yl}-3-(2-chlorobenzyl)-4-oxo-4,5-dihydropyrazolo[1,5-a]pyrazine-6-carboxylate (1.00 g) in 1,4-dioxane (10 ml), and the resulting mixture was stirred at 50°C for 2 hours. The mixture was cooled to room temperature and a 5% aqueous sodium hydrogensulfate solution (20 ml) was added thereto, followed by two runs of extraction with ethyl acetate (20 ml). The combined organic layer was concentrated under reduced pressure. The resulting residue was purified by a silica gel column chromatography (chloroform/methanol = 10/1) to obtain the title compound (872 mg).
MS (ESI+) 502 (M⁺+1, 66%).

### Reference Example 12

tert-Butyl {(3R)-1-[3-(2-chlorobenzyl)-4-oxo-5-(2-oxo-2-phenylethyl)-4,5-dihydropyrazolo[1,5-a]pyrazin-2-yl]piperidin-3-yl}carbamate

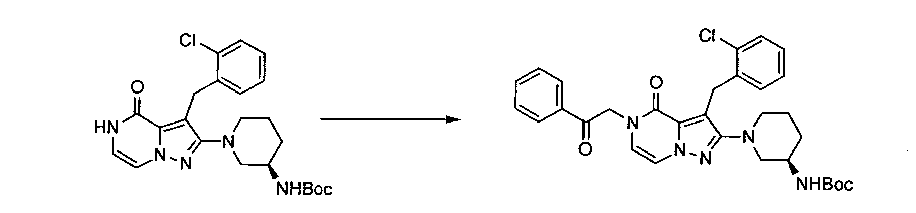

Phenacyl bromide (59.7 mg) and potassium carbonate (82.8 mg) were added to a solution of tert-butyl {(3R)-1-[3-(2-chlorobenzyl)-4-oxo-4,5-dihydropyrazolo[1,5-a]pyrazin-2-yl]piperidin-3-yl}carbamate (91.6 mg) in N,N-dimethylformamide (1.0 ml), and the resulting mixture was stirred overnight at room temperature. Water (20 ml) was added to the reaction mixture, followed by two runs of extraction with ethyl acetate (20 ml), and the combined organic layer was concentrated under reduced pressure. The resulting residue was purified by a silica gel column chromatography (hexane/ethyl acetate = 2/1) to obtain the title compound (38.8 mg).
MS (ESI+) 576 (M⁺+1, 100%).

The following compounds of Reference Examples 12-1 to 12-8 were synthesized by the same process as above.

| 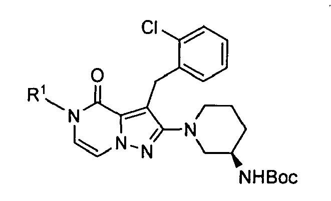 | | | |
|---|---|---|---|
| Reference example number | R¹ | Reference example number | R¹ |
| Reference Example 12-1 | 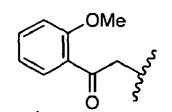 | Reference Example 12-5 | 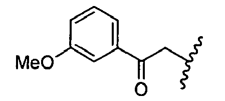 |
| Reference Example 12-2 | 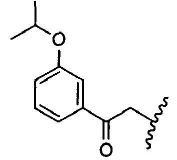 | Reference Example 12-6 | 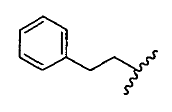 |
| Reference Example 12-13 | 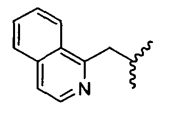 | Reference Example 12-7 | 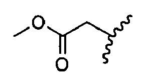 |
| Reference Example 12-4 | 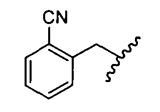 | | |

### Reference Example 12-1

MS (ESI+) 606 (M⁺+1, 100%).

### Reference Example 12-2

MS (ESI+) 634 (M⁺+1, 100%).

### Reference Example 12-3

MS (ESI+) 599 (M⁺+1, 100%).

### Reference Example 12-4

MS (ESI+) 573 (M⁺+1, 100%).

### Reference Example 12-5

MS (ESI+) 606 (M⁺+1, 100%).

### Reference Example 12-6

MS (ESI+) 562 (M⁺+1, 100%).

### Reference Example 12-7

MS (ESI+) 530 (M⁺+1, 59%).

### Reference Example 12-8

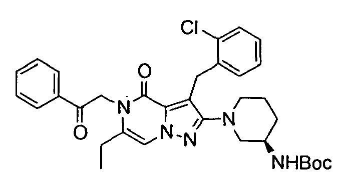

MS (ESI+) 604 (M⁺+1, 100%).

### Reference Example 13

tert-Butyl {(3R)-1-[3-(2-chlorobenzyl)-5-methyl-4-oxo-4,5-dihydropyrazolo[1,5-a]pyrazin-2-yl]piperidin-3-yl}carbamate

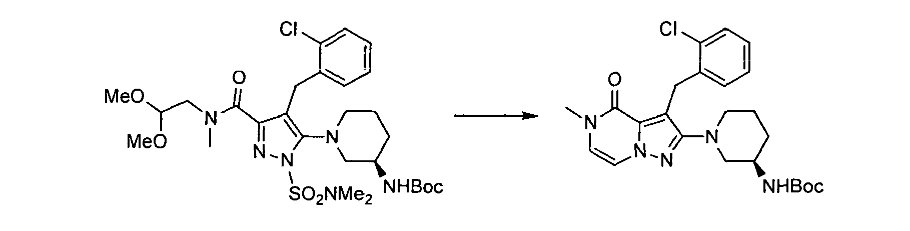

Water (4.0 ml) and a 4N hydrochloric acid/1,4-dioxane solution (8.0 ml) were added to a solution of tert-butyl ((3R)-1-{4-(2-chlorobenzyl)-3-{[(2,2-dimethoxyethyl)(methyl)-amino]carbonyl}-1-[(dimethylamino)sulfonyl]-1H-pyrazol-5-yl}piperidin-3-yl)carbamate (386 mg) in 1,4-dioxane (4.0 ml), and the resulting mixture was stirred at 50°C for 2 hours. A 5% aqueous potassium carbonate solution (100 ml) was added to the reaction mixture, followed by two runs of extraction with chloroform (100 ml), and the combined organic layer was concentrated under reduced pressure. To a solution of the resulting residue in 1,4-dioxane (1.0 ml) were added sodium hydrogencarbonate (168.0 mg) and di-tert-butyl dicarbonate (328 mg), and stirred overnight. Water (100 ml) was added to the reaction mixture, followed by two runs of extraction with ethyl acetate (100 ml), and the combined organic layer was concentrated under reduced pressure. The resulting residue was purified by a silica gel column chromatography (hexane/ethyl acetate = 1/1) to obtain the title compound (16.5 mg).
MS (ESI+) 472 (M⁺+1, 49%).

### Reference Example 14

tert-Butyl {(3R)-1-[3-(2-chlorobenzyl)-6-cyano-5-methyl-4-oxo-4,5-dihydropyrazolo[1,5-a]pyrazin-2-yl]piperidin-3-yl}carbamate

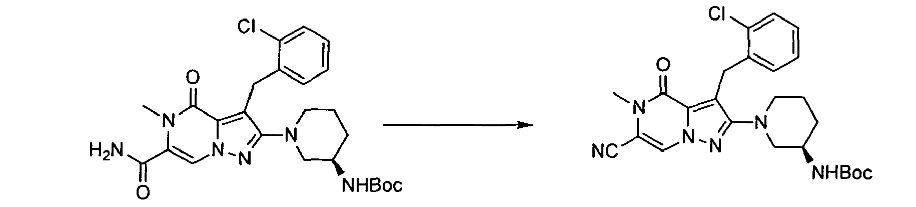

Trifluoroacetic anhydride (0.18 ml) was added to a solution of tert-butyl {(3R)-1-[6-(aminocarbonyl)-3-(2-chlorobenzyl)-5-methyl-4-oxo-4,5-dihydropyrazolo[1,5-a]pyrazin-2-yl]piperidin-3-yl}carbamate (120 mg) in tetrahydrofuran (3.0 ml), and the resulting mixture was stirred overnight at room temperature. The reaction mixture was concentrated under reduced pressure and the residue was dissolved in methanol (2.5 ml). Water (0.05 ml) and potassium carbonate (48.3 mg) were added thereto and the resulting mixture was stirred at room temperature for 1 hour. Water (50 ml) was added to the reaction mixture, followed by two runs of extraction with ethyl acetate (50 ml), and the combined organic layer was concentrated under reduced pressure. The resulting residue was purified by a silica gel column chromatography (hexane/ethyl acetate = 2/1) to obtain the title compound (68.5 mg).
MS (ESI+) 497 (M⁺+1, 67%).

### Reference Example 15

tert-Butyl {(3R)-1-[6-(aminocarbonyl)-3-(2-chlorobenzyl)-5-methyl-4-oxo-4,5-dihydropyrazolo[1,5-a]pyrazin-2-yl]piperidin-3-yl}carbamate

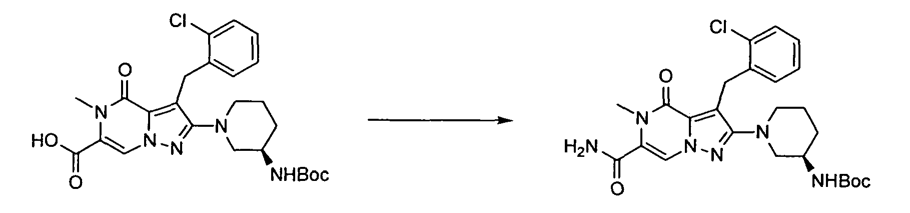

Ammonium chloride (106 mg), 1-ethyl-3-(3-dimethylaminopropyl)carbodiimide hydrochloride (380 mg), 1-hydroxybenzotriazole monohydrate (268 mg) and triethylamine (0.551 m) were added to a solution of 2-{(3R)-3-[(tert-butoxycarbonyl)amino]piperidin-1-yl}-3-(2-chlorobenzyl)-5-methyl-4-oxo-4,5-dihydropyrazolo[1,5-a]pyrazin-6-carboxylic acid (340 mg) in dimethylformamide (6.0 ml), and the resulting mixture was stirred overnight at room temperature. A saturated aqueous sodium hydrogencarbonate solution (200 ml) was added thereto, followed by two runs of extraction with ethyl acetate (100 ml), and the combined organic layer was concentrated under reduced pressure. The resulting residue was purified by a silica gel column chromatography (hexane/ethyl acetate = 1/2) to obtain the title compound (236 mg).
MS (ESI+) 515 (M⁺+1, 72%).

### Reference Example 16

2-{(3R)-3-[(tert-Butoxycarbonyl)amino]piperidin- 1-yl}-3-(2-chlorobenzyl)-5-methyl-4-oxo-4,5-dihydropyrazolo[1,5-a]pyrazin-6-carboxylic acid

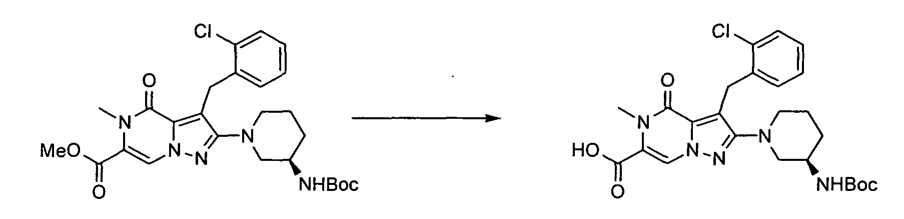

A 1N-aqueous sodium hydroxide solution (4.0 ml) was added to a solution of methyl 2-{(3R)-3-[(tert-butoxycarbonyl)amino]piperidin-1-yl}-3-(2-chlorobenzyl)-5-methyl-4-oxo-4,5-dihydropyrazolo[1,5-a]pyrazin-6-carboxylate (340 mg) in 1,4-dioxane (4.0 ml), and the resulting mixture was stirred at 50°C for 2 hours. After cooling to room temperature, a 5% aqueous sodium hydrogensulfate solution (50 ml) was added, followed by two runs of extraction with ethyl acetate (50 ml), and the combined organic layer was concentrated under reduced pressure to obtain the title compound as a crude product (340 mg).
¹H NMR (400 MHz, CDCl₃) δ ppm 8.27 (s, 1H), 7.40-7.37 (m, 1H), 7.16-7.12 (m, 2H), 6.96-6.93 (m, 1H), 4.70-4.68 (m, 1H), 4.64-4.41 (m, 2H), 3.76-3.72 (m, 1H), 3.71 (s, 3H), 3.33-3.29 (m, 1H), 3.03-3.00 (m, 2H), 2.91-2.89 (m, 1H), 1.67-1.65 (m, 2H), 1.50-1.45 (m, 2H), 1.45 (s, 9H).
MS (ESI+) 516 (M⁺+1, 73%).

### Reference Example 17

Methyl 2-{(3R)-3-[(tert-butoxycarbonyl)amino]piperidin-1-yl}-3-(2-chlorobenzyl)-5-methyl-4-oxo-4,5-dihydropyrazolo[1,5-a]pyrazin-6-carboxylate

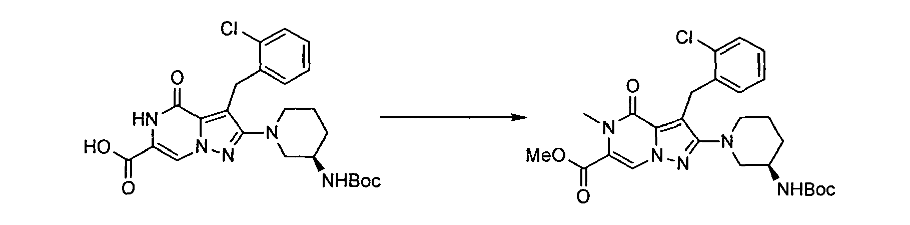

Methyl iodide (0.097 ml) and potassium carbonate (495 mg) were added to a solution of 2-{(3R)-3-[(tert-butoxycarbonyl)amino]piperidin-1-yl}-3-(2-chlorobenzyl)-4-oxo-4,5-dihydropyrazolo[1,5-a]pyrazin-6-carboxylic acid (490 mg) in N,N-dimethylformamide (10 ml), and the resulting mixture was stirred at room temperature for 6 hours. Water (100 ml) was added to the reaction mixture, followed by two runs of extraction with ethyl acetate (100 ml), and the combined organic layer was concentrated under reduced pressure to obtain the title compound as a crude product (349 mg).
¹H NMR (400 MHz, CDCl₃) δ ppm 8.13 (s, 1H), 7.40-7.37 (m, 1H), 7.17-7.12 (m, 2H), 6.96-6.94 (m, 1H), 4.69-4.67 (m, 1H), 4.67-4.41 (m, 2H), 3.90 (s, 3H), 3.76-3.74 (m, 1H), 3.73 (s, 3H), 3.27-3.24 (m, 1H), 3.05-3.01 (m, 2H), 2.89-2.84 (m, 1H), 1.53-1.51 (m, 4H), 1.44 (s, 9H).
MS (ESI+) 530 (M⁺+1, 100%).

### Reference Example 18

tert-Butyl 4-(3-benzyl-5-methyl-4-oxo-4,5-dihydropyrazolo[1,5-a]pyrazin-2-yl)piperazin-1-carboxylate

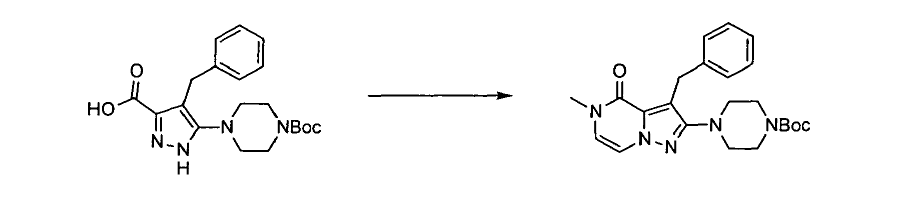

(Methylamino)acetaldehyde dimethyl acetal (69.5 mg) was added to a solution of 4-benzyl-3-[4-(tert-butoxycarbonyl)piperazin-1-yl]-1H-pyrazole-5-carboxylic acid (150 mg), 1-hydroxy-1H-benzotriazole (52.5 mg) and N-ethyl-N'-(3-dimethylaminopropyl)carbodiimide hydrochloride (74.5 mg) in N,N-dimethylformamide (1.5 ml), and the resulting mixture was stirred at room temperature for 24 hours. The reaction solution was poured into water (50 ml) and extracted with toluene/ethyl acetate (1 : 1, 100 ml). The extracted solution was dried over anhydrous magnesium sulfate and filtered, and the filtrate was concentrated under reduced pressure. The oil thus obtained was dissolved in 1,4-dioxane (15 ml), followed by adding thereto 1N hydrochloric acid (3 ml), and the resulting mixture was stirred at 50°C for 2 hours. The reaction solution was cooled to room temperature and made basic with a saturated aqueous sodium hydrogencarbonate solution. Di-tert-butyl dicarbonate (170 mg) was added to the thus treated reaction solution and the resulting mixture was stirred at room temperature for 2 hours. The reaction solution was extracted twice with ethyl acetate (100 ml). The combined organic layer was dried over anhydrous magnesium sulfate and filtered. The filtrate was concentrated under reduced pressure and the resulting residue was purified by a silica gel column chromatography (hexane/ethyl acetate = 1/1) to obtain the title compound (102 mg) as a white solid.
¹H NMR (400 MHz, CDCl₃) δ ppm 7.25-7.21 (m, 5H), 7.17-7.14 (m, 1H), 6.42 (d, J = 5.9 Hz, 1H), 4.34 (s, 2H), 3.46 (s, 3H), 3.42 (t, J = 5.1 Hz, 4H), 3.04 (t, J = 5.1 Hz, 4H), 1.45 (s, 9H).
MS (ESI+) 424 (M⁺+1, 100%).

### Reference Example 19

tert-Butyl ((3R)-1-{4-(2-chlorobenzyl)-3-{[(2,2-dimethoxyethyl)amino]carbonyl}-1-[(dimethylamino)sulfonyl]-1H-pyrazol-5-yl}piperidin-3-yl)carbamate

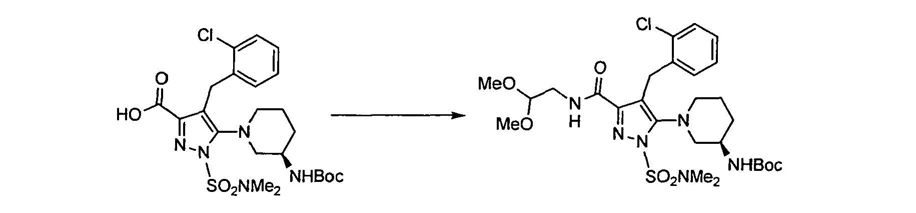

Aminoacetaldehyde dimethyl acetal (1.20 ml), 1-ethyl-3-(3-dimethylaminopropyl)carbodiimide hydrochloride (2.12 g), 1-hydroxybenzotriazole monohydrate (1.50 g) and triethylamine (1.54 ml) were added to a solution of 5-{(3R)-3-[(tert-butoxylcarbonyl)amino]piperidin-1-yl}-4-(2-chlorobenzyl)-1-[(dimethylamino)sulfonyl]-1H-pyrazole-3-carboxylic acid (3.00 g) in N,N-dimethylformamide (6.0 ml), and the resulting mixture was stirred overnight at room temperature. A saturated aqueous sodium hydrogencarbonate solution (200 ml) was added thereto, followed by two runs of extraction with ethyl acetate (100 ml), and the combined organic layer was concentrated under reduced pressure to obtain the title compound as a crude product (2.97). MS (ESI+) 629 (M⁺+1, 75%).

Compounds of Reference Examples 19-1 to 19-8 were synthesized by the same process as above.

| Reference example number | | Reference example number | |
|---|---|---|---|
| Reference Example 19-1 | 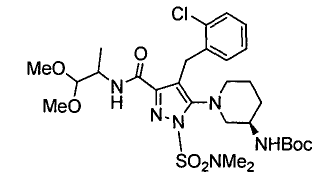 | Reference Example 19-4 | 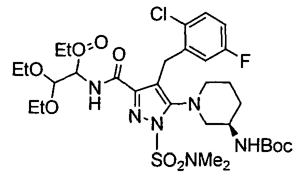 |
| Reference Example 19-2 | 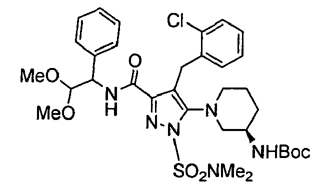 | Reference Example 19-5 | 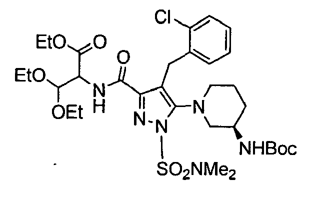 |
| Reference Example 19-3 | 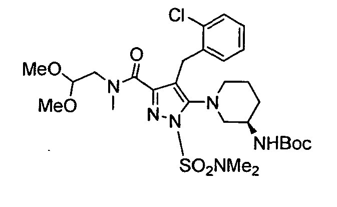 | | |

| Reference example number | | | |
|---|---|---|---|
| Reference Example 19-6 | 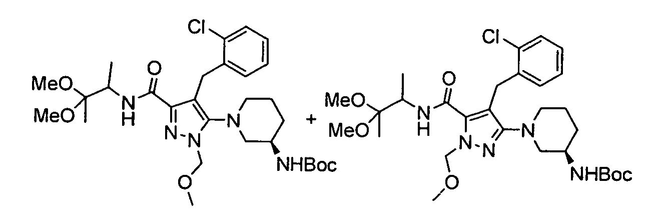 | | |
| Reference Example 19-7 | 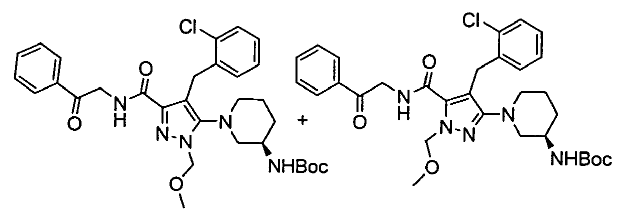 | | |
| Reference Example 19-8 | 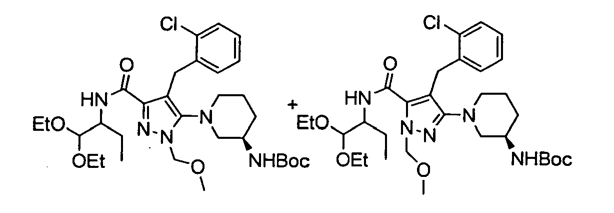 | | |

### Reference Example 20

Methyl 2-({[5-{(3R)-3-[(tert-butoxycarbonyl)-amino]piperidin-1-yl}-4-(2-chlorobenzyl)-1-(methoxymethyl)-1H-pyrazol-3-yl]carbonyl}amino)-3-oxobutanoate mixture

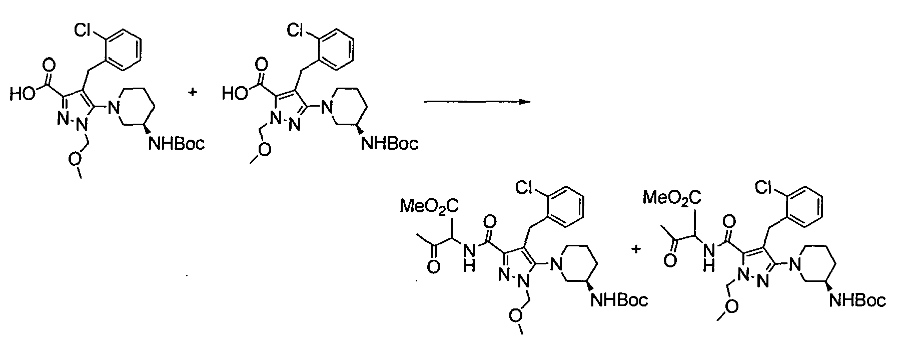

N-methylmorpholine (46.5 mg) and isobutyl chlorocarbonate (62.7 mg) were added to a solution of a 5-{(3R)-3-[(tert-butoxycarbonyl)amino]piperidin-1-yl}-4-(2-chlorobenzyl)-1-(methoxymethyl)-1H-pyrazole-3-carboxylic acid mixture (200.0 mg) in tetrahydrofuran (5 ml), and the resulting mixture was stirred at -20°C for 30 minutes. Then, methyl 2-amino-3-oxobutanoate hydrochloride (200.0 mg) was added thereto, followed by adding dropwise thereto N-methylmorpholine (120.7 mg) slowly, and the resulting mixture was stirred overnight while being slowly warmed from -20°C to room temperature. A 10% aqueous potassium hydrogensulfate solution (50 ml) was added to the reaction solution and organic substances were extracted twice therefrom with ethyl acetate (30 ml). The combined organic layer was washed with a saturated aqueous sodium chloride solution (50 ml), dried over anhydrous magnesium sulfate and then filtered, and the filtrate was concentrated under reduced pressure. The resulting residue was purified by a silica gel thin-layer chromatography (hexane/ethyl acetate = 2/1) to obtain the title compound (91.5 mg).
MS (ESI+) 593 (M⁺+1, 100%).

### Reference Example 21

tert-Butyl {(3R)-1-[3-(2-chlorobenzyl)-6,7-dimethyl-4-oxo-5-(2-oxo-2-phenylethyl)-4,5-dihydropyrazolo[1,5-a]pyrazin-2-yl]piperidin-3-yl}carbamate

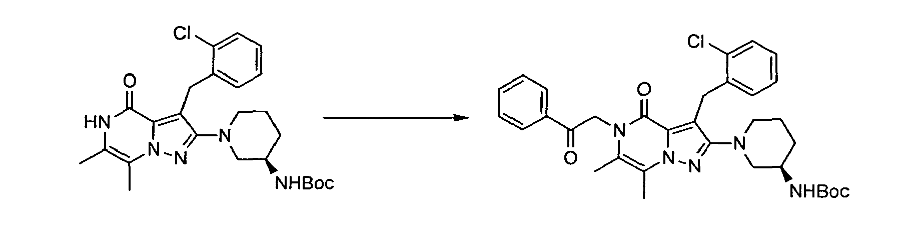

Phenacyl bromide (57.7 mg) and potassium carbonate (79.9 mg) were added to a solution of tert-butyl {(3R)-1-[3-(2-chlorobenzyl)-6,7-dimethyl-4-oxo-4,5-dihydropyrazolo[1,5-a]pyrazin-2-yl]piperidin-3-yl}carbamate (94.0 mg) in N,N-dimethylformamide (2.0 ml), and the resulting mixture was stirred overnight at room temperature. Water (20 ml) was added to the reaction mixture, followed by two runs of extraction with ethyl acetate (20 ml), and the combined organic layer was concentrated under reduced pressure. The resulting residue was purified by a preparative thin-layer silica gel chromatography (chloroform/ethyl acetate = 20/1) to obtain the title compound (40.0 mg).
MS (ESI+) 604 (M⁺+1, 86%).

### Reference Example 22

tert-Butyl {(3R)-1-[3-(2-chlorobenzyl)-6,7-dimethyl-4-oxo-4,5-dihydropyrazolo[1,5-a]pyrazin-2-yl]piperidin-3-yl}carbamate

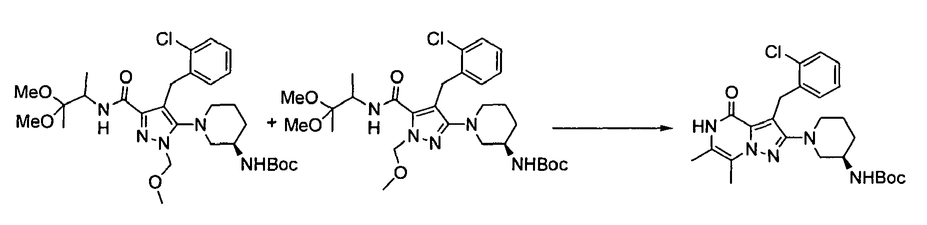

Water (2.0 ml) and a 4N hydrochloric acid/1,4-dioxane solution (4.0 ml) were added to a solution of a tert-butyl {(3R)-1-[4-(2-chlorobenzyl)-3-{[(2,2-dimethoxy-1-methylpropyl)amino]carbonyl}-1-(methoxymethyl)-1H-pyrazol-5-yl]piperidin-3-yl}carbamate mixture (430 mg) in 1,4-dioxane (2.0 ml), and the resulting mixture was stirred at 50°C for 2 hours. The reaction mixture was cooled to 0°C and 1,4-dioxane (3.0 ml), water (1.0 ml) and sodium hydrogencarbonate (3.0 g) were added thereto. The resulting mixture was warmed to room temperature and di-tert-butyl dicarbonate (182 mg) was added thereto and stirred overnight. The 1,4-dioxane was distilled off under reduced pressure and water (50 ml) was added to the residue, followed by two runs of extraction with ethyl acetate (50 ml). The combined organic layer was concentrated under reduced pressure. The resulting residue was purified by a silica gel column chromatography (chloroform/ethyl acetate = 1/1) to obtain the title compound (96.4 mg).
¹H NMR (400 MHz, CDCl₃) δ ppm 9.40 (s, 1H), 7.38-7.34 (m, 1H), 7.14-7.02 (m, 2H), 7.01-6.99 (m, 1H), 5.11-5.09 (m, 1H), 4.57-4.36 (m, 2H), 3.78-3.76 (m, 1H), 3.18-3.02 (m, 3H), 2.80-2.78 (m, 1H), 2.39 (s, 3H), 2.14 (s, 3H), 1.54-1.48 (m, 4H), 1.44 (s, 9H).
MS (ESI+) 486 (M⁺+1, 56%).

### Reference Example 23

tert-Butyl {(3R)-1-[3-(2-chlorobenzyl)-4-oxo-4,5-dihydropyrazolo[1,5-d][1,2,4]triazin-2-yl]piperidin-3-yl}carbamate

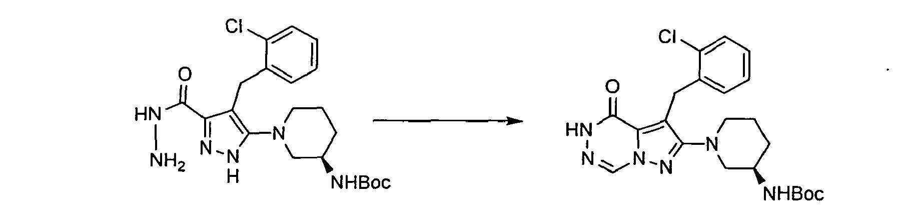

Trimethyl orthoformate (1.09 ml) was added to a solution of tert-butyl {(3R)-1-[4-(2-chlorobenzyl)-3-(hydrazinecarbonyl)-1H-pyrazol-5-yl]piperidin-3-yl}carbamate (478 mg) in a mixture of N,N-dimethylformamide (8 ml) and acetic acid (2 ml), and the resulting mixture was stirred with heating at 80°C for 3 hours. The reaction solution was cooled to room temperature and toluene (20 ml) was added thereto and then distilled off under reduced pressure. This procedure was repeated four times. A 10% aqueous potassium carbonate solution (50 ml) was added to the residue, followed by two runs of extraction with chloroform (50 ml). The organic layer was dried over anhydrous sodium sulfate and filtered, and the filtrate was concentrated under reduced pressure. The resulting residue was purified by a silica gel column chromatography (hexane/ethyl acetate = 4/1 to 2/1) to obtain the title compound (274 mg) as a white solid. ¹H NMR (300 MHz, CDCl₃) δ ppm 9.93 (s, 1H), 8.19 (s, 1H), 7.41-7.36 (m, 1H), 7.19-7.14 (m, 2H), 6.99-6.96 (m, 1H), 4.64-4.62 (m, 1H), 4.54-4.39 (m, 2H), 3.70 (bs, 1H), 3.32-3.29 (m, 1H), 3.06-2.92 (m, 3H), 1.76-1.65 (m, 2H), 1.51-1.45 (m, 2H), 1.44 (s, 9H).
MS (ESI+) 459 (M⁺+1, 52%).

### Reference Example 24

tert-Butyl {(3R)-1-[4-(2-chlorobenzyl)-3-(hydrazinecarbonyl)-1H-pyrazol-5-yl]piperidin-3-yl}carbamate

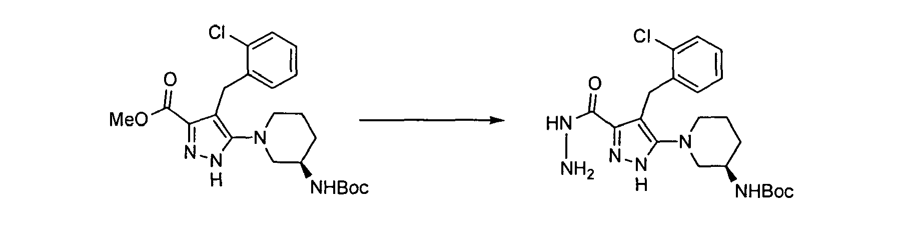

Hydrazine monohydrate (2.0 ml) was added to methyl 5-{(3R)-3-[(tert-butoxycarbonyl)amino]piperidin-1-yl}-4-(2-chlorobenzyl)-1H-pyrazole-3-carboxylate (449 mg), and the resulting mixture was stirred with heating at 110°C for 15 hours. The reaction solution was cooled to room temperature and a 10% aqueous potassium carbonate solution (40 ml) was added thereto, followed by extraction with ethyl acetate (100 ml). The organic layer was dried over anhydrous sodium sulfate and filtered, and the filtrate was concentrated under reduced pressure to obtain the title compound (274 mg).
MS (ESI+) 449 (M⁺+1, 100%).

### Reference Example 25

Methyl 5-{(3R)-3-[(tert-butoxycarbonyl)amino]-piperidin-1-yl}-4-(2-chlorobenzyl)-1H-pyrazole-3-carboxylate

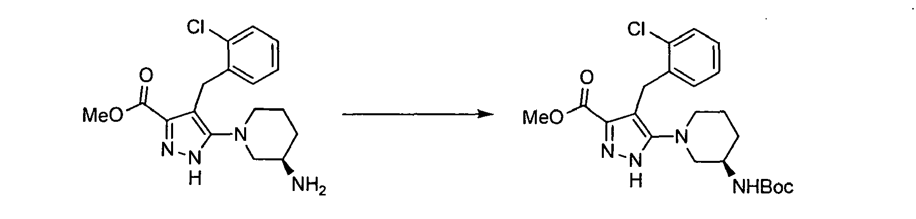

A saturated aqueous sodium hydrogencarbonate solution (10 ml) and di-tert-butyl dicarbonate (218 mg) were added to a solution of methyl 5-[(3R)-3-aminopiperidin-1-yl]-4-(2-chlorobenzyl)-1H-pyrazole-3-carboxylate (180 mg) in tetrahydrofuran (10 ml), and the resulting mixture was vigorously stirred overnight at room temperature. Tetrahydrofuran was distilled off under reduced pressure and the residue was extracted twice with chloroform (50 ml). The organic layer was dried over anhydrous sodium sulfate and filtered, and the filtrate was concentrated under reduced pressure. The resulting residue was purified by a silica gel column chromatography (hexane/ethyl acetate = 3/1 to 1/1) to obtain the title compound (191 mg) as a white solid.
¹H NMR (300 MHz, CDCl₃) δ ppm 7.39-7.36 (m, 1H), 7.16-7.11 (m, 2H), 6.94-6.91 (m, 1H), 4.92-4.90 (m, 1H), 4.29-4.11 (m, 2H), 3.86-3.77 (m, 1H), 3.82 (s, 3H), 3.18-3.14 (m, 1H), 2.90-2.80 (m, 3H), 1.64-1.46 (m, 4H), 1.44 (s, 9H).
MS (ESI+) 449 (M⁺+1, 85%).

### Reference Example 26

Methyl 5-[(3R)-3-aminopiperidin-1-yl]-4-(2-chlorobenzyl)-1H-pyrazole-3-carboxylate

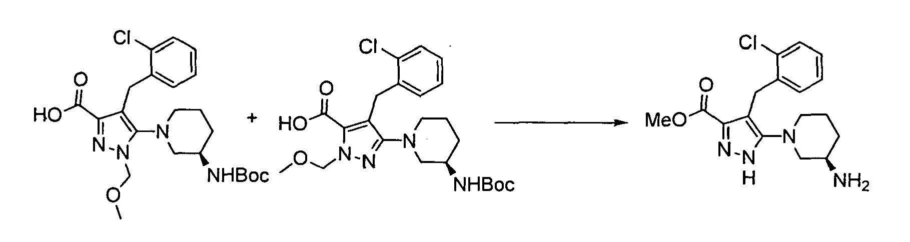

A solution of a 5-{(3R)-3-[(tert-butoxycarbonyl)amino]piperidin-1-yl}-4-(2-chlorobenzyl)-1-(methoxymethyl)-1H-pyrazole-3-carboxylic acid mixture (240 mg) in 10% hydrochloric acid/methanol (10 ml) was stirred in a sealed tube with heating at 80°C for 8 hours. The reaction solution was cooled to room temperature and then distilled under reduced pressure to remove the solvent, whereby the title compound was obtained as a crude product (180 mg).
MS (ESI+) 349 (M⁺+1, 60%).

### Reference Example 27

tert-Butyl {(3R)-1-[3-(2-chlorobenzyl)-6-ethyl-4-oxo-5-(2-oxo-2-phenylethyl)-4,5-dihydropyrazolo[1,5-a]pyrazin-2-yl]piperidin-3-yl}carbamate

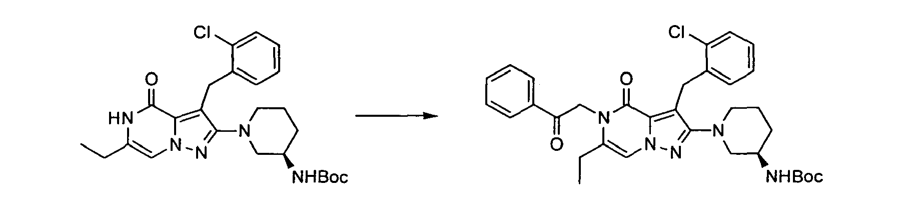

Phenacyl bromide (36.9 mg) and potassium carbonate (50.9 mg) were added to a solution of tert-butyl {(3R)-1-[3-(2-chlorobenzyl)-6-ethyl-4-oxo-4,5-dihydropyrazolo[1,5-a]pyrazin-2-yl]piperidin-3-yl}carbamate (60.0 mg) in N,N-dimethylformamide (1.5 ml), and the resulting mixture was stirred overnight at room temperature. Water (20 ml) was added to the reaction mixture, followed by two runs of extraction with ethyl acetate (20 ml), and the combined organic layer was concentrated under reduced pressure. The resulting residue was purified by a silica gel column chromatography (hexane/ethyl acetate = 2/1) to obtain the title compound (23.2 mg).
MS (ESI+) 604 (M⁺+1, 100%).

### Reference Example 28

tert-Butyl {(3R)-1-[3-(2-chlorobenzyl)-6-ethyl-4-oxo-4,5-dihydropyrazolo[1,5-a]pyrazin-2-yl]piperidin-3-yl}carbamate

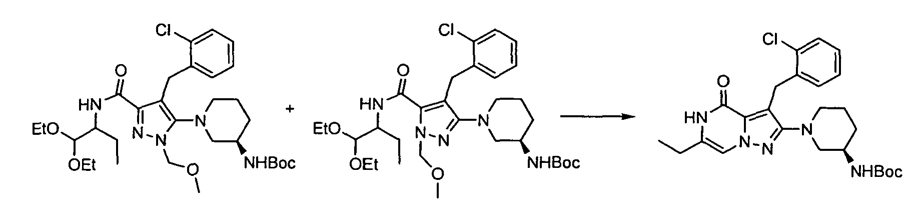

Water (2.5 ml) and a 4N hydrochloric acid/1,4-dioxane solution (5.0 ml) were added to a solution of a tert-butyl {(3R)-1-[4-(2-chlorobenzyl)-3-({[1-(dimethoxymethyl)propyl]amino}carbonyl)-1-(methoxymethyl)-1H-pyrazol-5-yl]piperidin-3-yl}carbamate mixture (324 mg) in 1,4-dioxane (2.5 ml), and the resulting mixture was stirred at 50°C for 2 hours. The reaction mixture was cooled to 0°C and 1,4-dioxane (4.0 ml), water (2.0 ml) and sodium hydrogencarbonate (3.8 g) were added thereto. The resulting mixture was warmed to room temperature and di-tert-butyl dicarbonate (228 mg) was added thereto and stirred overnight. 1,4-dioxane was distilled off under reduced pressure and water (50 ml) was added to the residue, followed by two runs of extraction with ethyl acetate (50 ml). The combined organic layer was concentrated under reduced pressure. The resulting residue was purified by a silica gel column chromatography (hexane/ethyl acetate = 2/1) to obtain the title compound (80.9 mg).
¹H NMR (400 MHz, CDCl₃) δ ppm 9.47 (s, 1H), 7.39-7.36 (m, 1H), 7.14-7.11 (m, 2H), 7.06-7.05 (m, 1H), 7.02-7.00 (m, 1H), 4.83-4.80 (m, 1H), 4.54-4.34 (m, 2H), 3.85-3.82 (m, 1H), 3.16-3.15 (m, 1H), 3.01-2.98 (m, 2H), 2.85-2.81 (m, 1H), 2.45-2.39 (m, 2H), 1.57-1.49 (m, 4H), 1.44 (s, 9H), 1.21 (t, J = 7.5Hz, 3H).
MS (ESI+) 486 (M⁺+1, 67%).

### Reference Example 29

tert-Butyl ({1-[3-(2-chloro-5-fluorobenzyl)-5-methyl-4-oxo-4,5-dihydropyrazolo[1,5-a]pyrazin-2-yl]pyrrolidin-2-yl}methyl)carbamate

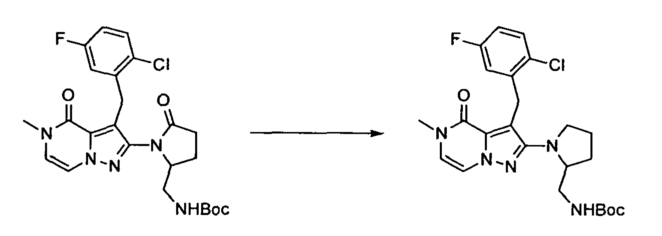

A reactor containing a solution of tert-butyl ({1-[3-(2-chloro-5-fluorobenzyl)-5-methyl-4-oxo-4,5-dihydropyrazolo[1,5-a]pyrazin-2-yl]-5-oxopyrrolidin-2-yl}methyl)carbamate (406.1 mg) in tetrahydrofuran (50 ml) was cooled with a water-ice bath. To this solution was added a borane-tetrahydrofuran complex (a 1.1M tetrahydrofuran solution, 7.33 ml), and the resulting mixture was stirred at room temperature for 2 hours. The reactor was cooled again with a water-ice bath and methanol (10 ml) was added. Then, the resulting mixture was stirred at room temperature for 30 minutes and the reaction solution was concentrated under reduced pressure. The resulting oil was dissolved in methanol (30 ml) and the resulting solution was concentrated under reduced pressure. The resulting residue was purified by a silica gel column chromatography (hexane/ethyl acetate = 5/1 to 1/1) to obtain the title compound (69.1 mg) as a white solid. ¹H NMR (400 MHz, CDCl₃) δ ppm 7.34-7.30 (m, 1H), 7.23 (d, J = 6.0 Hz, 1H), 6.85-6.80 (m, 1H), 6.63-6.60 (m, 1H), 6.42 (d, J = 6.0 Hz, 1H), 4.79 (br, 1H), 4.60 (d, J = 17.9 Hz, 1H), 4.39 (d, J = 17.9 Hz, 1H), 4.08-4.02 (m, 1H), 3.45 (s, 3H), 3.46-3.40 (m, 1H), 3.32-3.26 (m, 2H), 3.08-3.02 (m, 1H), 1.94-1.71 (m, 4H), 1.42 (s, 9H).
MS (ESI+) 490 (M⁺+1, 100%).

### Reference Example 30

Ethyl 4-benzyl-1-(4-methoxybenzyl)-3-piperazin-1-yl-1H-pyrazole-5-carboxylate

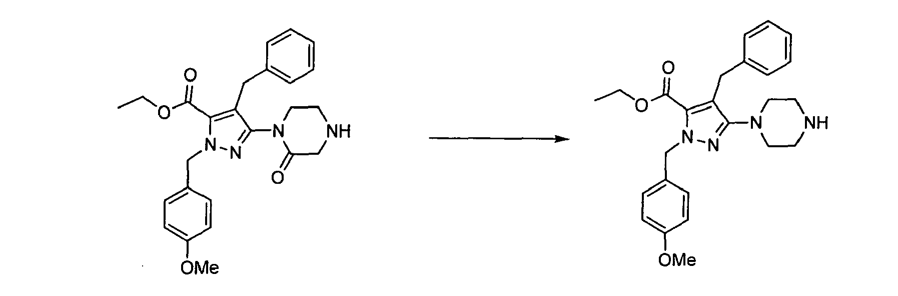

The title compound (1.22 g) was synthesized by the same process as in Reference Example 29.
¹H NMR (400 MHz, CDCl₃) δ ppm 7.25-7.06 (m, 7H), 6.85-6.80 (m, 2H), 5.59 (s, 2H), 4.11 (q, J = 7.1 Hz, 2H), 4.03 (s, 2H), 3.77 (s, 3H), 3.08-2.94 (m, 4H), 2.75-2.71 (m, 2H), 2.56-2.50 (m, 2H), 1.02 (t, J = 7.1 Hz, 3H).
MS (ESI+) 435 (M⁺+1, 100%).

### Reference Example 31

tert-Butyl ({1-[3-(2-chloro-5-fluorobenzyl)-5-methyl-4-oxo-4,5-dihydropyrazolo[1,5-a]pyrazin-2-yl]-5-oxopyrrolidin-2-yl}methyl)carbamate

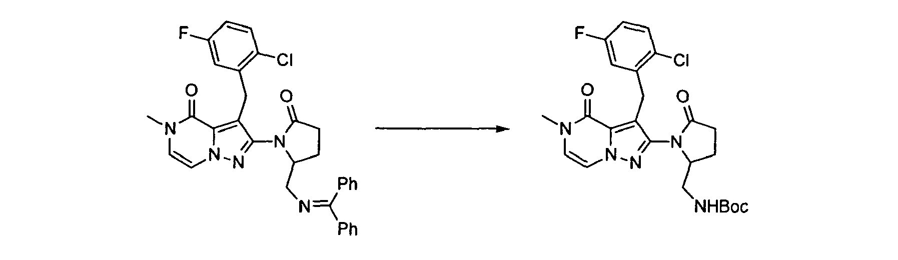

To a solution of 3-(2-chloro-5-fluorobenzyl)-2-(2-{[(diphenylmethylene)amino]methyl}-5-oxopyrrolidin-1-yl)-5-methylpyrazolo[1,5-a]pyrazin-4(5H)-one (663.6 mg) in tetrahydrofuran (20 ml) was added 1N hydrochloric acid (10 ml), and the resulting mixture was stirred at room temperature for 1 hour. Tetrahydrofuran was removed under reduced pressure and the residue was extracted twice with diethyl ether (20 ml). After the aqueous layer was concentrated under reduced pressure, toluene was added thereto and the resulting mixture was similarly concentrated under reduced pressure. The residue was dissolved in tetrahydrofuran (50 ml), followed by adding thereto a saturated aqueous sodium hydrogencarbonate solution (10 ml) and then di-tert-butyl dicarbonate (511 mg), and the resulting mixture was stirred at room temperature for 2 hours. The reaction solution was extracted twice with ethyl acetate (20 ml). The combined organic layer was dried over anhydrous magnesium sulfate and filtered, and the filtrate was concentrated under reduced pressure. The resulting residue was purified by a silica gel column chromatography (hexane/ethyl acetate = 1/1) to obtain the title compound (408.9 mg) as a white solid.
¹H NMR (400 MHz, CDCl₃) δ ppm 7.33 (d, J = 6.0 Hz, 1H), 7.30-7.28 (m, 1H), 6.98-6.94 (m, 1H), 6.89-6.84 (m, 1H), 6.62 (d, J = 6.0 Hz, 1H), 4.89 (br, 1H), 4.59 (d, J = 16.1 Hz, 1H), 4.37 (d, J = 16.1 Hz, 1H), 3.62-3.58 (m, 1H), 3.53 (s, 3H), 3.37-3.31 (m, 1H), 3.00-2.94 (m, 1H), 2.52-2.32 (m, 2H), 2.09-1.90 (m, 2H), 1.42 (s, 9H).
MS (ESI+) 504 (M⁺+1, 100%).

### Reference Example 32

3-(2-Chloro-5-fluorobenzyl)-2-(2-{[(diphenylmethylene)amino]methyl}-5-oxopyrrolidin-1-yl)-5-methylpyrazolo[1,5-a]pyrazin-4(5H)-one

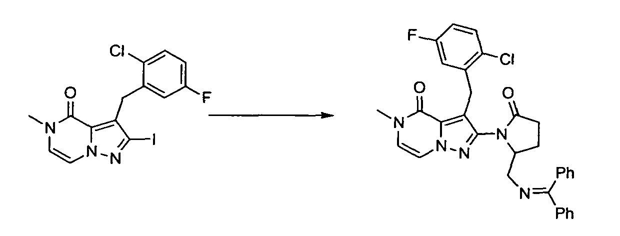

A mixture of 3-(2-chloro-5-fluorobenzyl)-2-iodo-5-methylpyrazolo[1,5-a]pyrazin-4(5H)-one (908 mg), 5-{[(diphenylmethylene)amino]methyl}pyrrolidin-2-one (1.21 g), potassium phosphate (924 mg), copper(I) iodide (87 mg), 1,4-dioxane (10 ml) and N,N'-dimethylethylenediamine (767 mg) was stirred at 100°C for 50 hours. The reaction mixture was cooled to room temperature and then filtered, and the filtrate was concentrated under reduced pressure. The resulting residue was purified by a silica gel column chromatography (chloroform/methanol = 95.5/0.5) to obtain the title compound (663.6 mg) as a yellow solid. ¹H NMR (400 MHz, CDCl₃) δ ppm 7.52-7.25 (m, 8H), 7.23 (d, J = 6.0 Hz, 1H), 7.08-7.05 (m, 1H), 7.04-7.00 (m, 2H), 6.84-6.81 (m, 1H), 6.71-6.66 (m, 1H), 6.54 (d, J = 6.0 Hz, 1H), 4.46 (d, J = 16.4 Hz, 1H), 4.38 (d, J = 16.4 Hz, 1H), 4.28-4.23 (m, 1H), 3.48 (s, 3H), 3.38 (dd, J = 14.4 and 3.8 Hz, 1H), 3.21 (dd, J = 14.4 and 6.2 Hz, 1H), 2.64-2.56 (m, 1H),2.45-2.37 (m, 1H), 2.27-2.17 (m, 1H), 1.94-1.85 (m, 1H).
MS (ESI+) 568 (M⁺+1, 100%).

### Reference Example 33

Ethyl 4-benzyl-1-(4-methoxybenzyl)-3-(2-oxopiperazin-1-yl)-1H-pyrazole-5-carboxylate

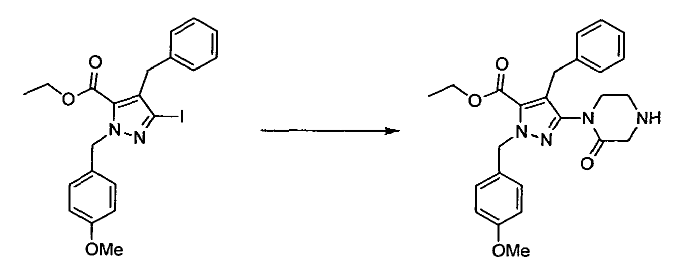

The title compound (2.21 g) was synthesized by the same process as in Reference Example 32.
¹H NMR (400 MHz, CDCl₃) δ ppm 7.29-7.18 (m, 5H), 7.14-7.12 (m, 2H), 6.84 (d, J = 8.7 Hz, 2H), 5.63 (s, 2H), 4.31 (q, J = 7.1 Hz, 2H), 4.09 (s, 2H), 3.78 (s, 3H), 3.41 (s, 2H), 3.21 (t, J = 5.5 Hz, 2H), 2.74 (t, J = 5.5 Hz, 2H), 1.28 (t, J = 7.1 Hz, 3H).
MS (ESI+) 449 (M⁺+1, 100%).

### Reference Example 34

5-{[(Diphenylmethylene)amino]methyl}-pyrrolidin-2-one

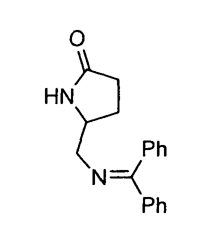

In methanol (500 ml) was suspended 4,5-diaminovaleric acid dihydrochloride (9.40 g), and the resulting suspension was stirred at room temperature for 3 hours. To the resulting transparent solution was added sodium carbonate (11.56 g), and the resulting mixture was stirred at room temperature for 2 days. The reaction mixture was filtered and benzophenoneimine (16.62 g) was added to the filtrate, followed by stirring at room temperature for 24 hours. The reaction solution was concentrated and the residue was suspended in ethyl acetate. The resulting suspension was filtered and the filtrate was concentrated. The resulting residue was purified by a silica gel column chromatography (ethyl acetate) to obtain the title compound (1.21 g) as an oil.
¹H NMR (400 MHz, CD₃OD) δ ppm 7.63-7.33 (m, 8H), 7.20-7.17 (m, 2H), 4.02-3.98 (m, 1H), 3.44 (d, J = 5.5 Hz, 2H), 2.39-2.27 (m, 3H), 1.94-1.87 (m, 1H).
MS (ESI+) 279 (M⁺+1, 100%).

### Reference Example 35

4-Benzyl-3-[4-(tert-butoxycarbonyl)piperazin-1-yl]-1H-pyrazole-5-carboxylic acid

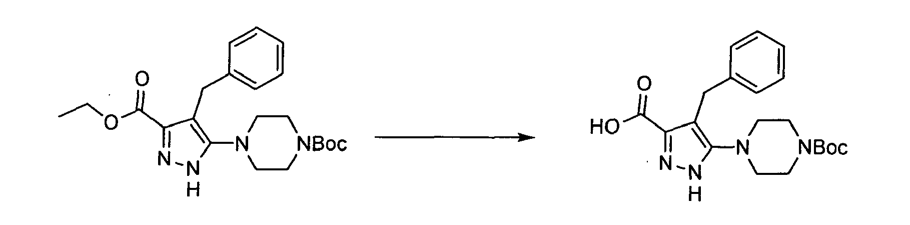

In a mixture of methanol (10 ml) and tetrahydrofuran (10 ml) was dissolved tert-butyl 4-[4-benzyl-3-(ethoxycarbonyl)-1H-pyrazol-5-yl]piperazin-1-carboxylate (732.4 mg), followed by adding thereto a 2N aqueous sodium hydroxide solution (4.4 ml), and the resulting mixture was allowed to stand at room temperature for 24 hours and then stirred at 50°C for 3 hours. The reaction solution was cooled and the tetrahydrofuran was removed under reduced pressure. To the resulting residue was added water (50 ml), and a reactor containing thus obtained mixture was cooled in a water-ice bath. This mixture was acidified with a 5% aqueous potassium hydrogensulfate solution. The reaction solution was extracted twice with ethyl acetate (100 ml). The combined organic layer was dried over anhydrous magnesium sulfate and filtered, and the filtrate was concentrated under reduced pressure to obtain the title compound (635 mg) as a white solid.
¹H NMR (400 MHz, CD₃OD) δ ppm 7.24-7.11 (m, 5H), 4.12 (s, 2H), 3.39 (t, J = 5.1 Hz, 4H), 2.85 (t, J = 5.1 Hz, 4H), 1.45 (s, 9H) .
MS (ESI+) 387 (M⁺+1, 100%).

### Reference Example 36

tert-Butyl 4-[4-benzyl-3-(ethoxycarbonyl)-1H-pyrazol-5-yl]piperazin-1-carboxylate

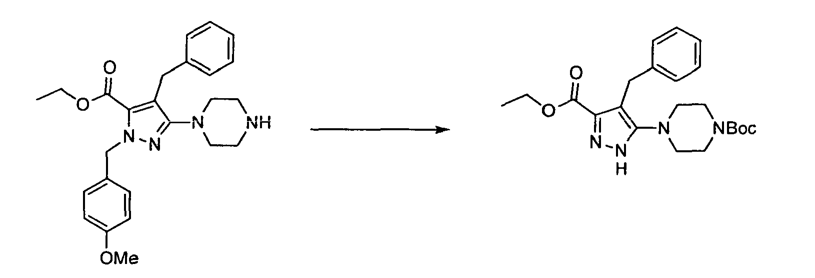

Ethyl 4-benzyl-1-(4-methoxybenzyl)-3-piperazin-1-yl-1H-pyrazole-5-carboxylate (1.22 g) was dissolved in a mixture of anisole (1.0 ml) and trifluoroacetic acid (16 ml), followed by adding thereto 96% sulfuric acid (0.5 ml), and the resulting mixture was allowed to stand at room temperature for 5 days. The reaction solution was concentrated and tetrahydrofuran (40 ml) was added thereto. A reactor containing the reaction solution was cooled in a water-ice bath and the reaction solution was made basic with a saturated aqueous sodium hydrogencarbonate solution, followed by adding thereto di-tert-butyl dicarbonate (1.23 g). The reaction solution was stirred at room temperature for 2 hours and extracted twice with ethyl acetate (50 ml). The combined organic layer was dried over anhydrous magnesium sulfate and filtered, and the filtrate was concentrated under reduced pressure. The resulting residue was purified by a silica gel column chromatography (hexane/ethyl acetate = 4/1 to 1/1) to obtain the title compound (732.4 mg) as a white solid.
¹H NMR (400 MHz, CDCl₃) δ ppm 7.25-7.23 (m, 2H), 7.18-7.15 (m, 3H), 4.30 (q, J = 7.1 Hz, 2H), 4.10 (s, 2H), 3.43 (t, J = 5.1 Hz, 4H), 2.94 (t, J = 5.1 Hz, 4H), 1.45 (s, 9H), 1.24 (t, J = 7.1 Hz, 3H).
MS (ESI+) 415 (M⁺+1, 100%).

### Reference Example 37

Ethyl 4-benzyl-3-iodo-1-(4-methoxybenzyl)-1H-pyrazole-5-carboxylate

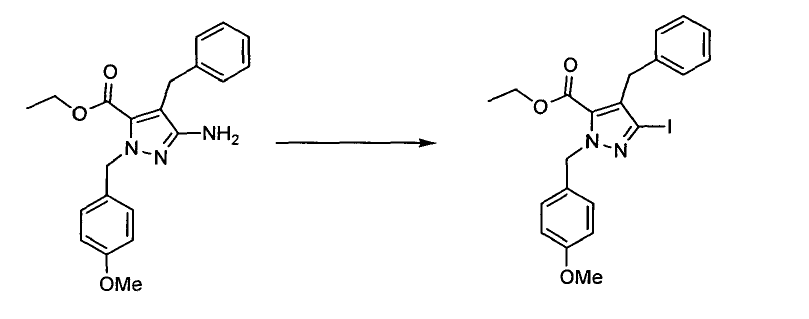

Ethyl 3-amino-4-benzyl-1-(4-methoxybenzyl)-1H-pyrazole-5-carboxylate (14.05 g) was dissolved in a mixture of diiodomethane (32 ml) and isoamyl nitrite (22.6 g), and the resulting solution was stirred at room temperature for 2 hours. The reaction solution was concentrated and the resulting residue was purified by a silica gel column chromatography (hexane/ethyl acetate = 12.5/1) to obtain the title compound (9.33 g) as an oil.
¹H NMR (400 MHz, CDCl₃) δ ppm 7.26-7.20 (m, 4H), 7.18-7.10 (m, 3H), 6.83 (d, J = 8.7 Hz, 2H), 5.67 (s, 2H), 4.22 (q, J = 7.1 Hz, 2H), 4.02 (s, 2H), 3.77 (s, 3H), 1.18 (t, J = 7.1 Hz, 3H).
MS (ESI+) 477 (M⁺+1, 100%).

### Reference Example 38

Ethyl 3-amino-4-benzyl-1-(4-methoxybenzyl)-1H-pyrazole-5-carboxylate

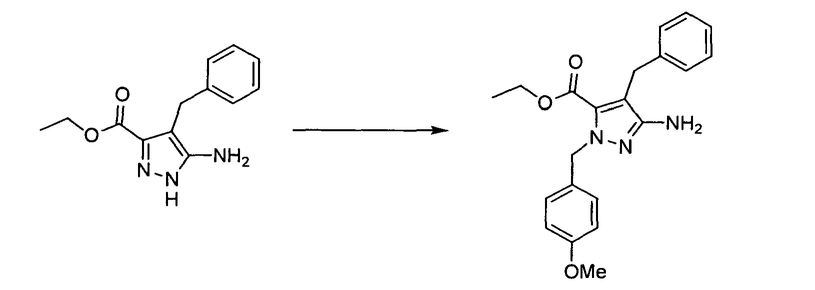

Cesium carbonate (25.0 g) and 4-methoxybenzyl chloride (12.02 g) were added to a solution of ethyl 5-amino-4-benzyl-1H-pyrazole-3-carboxylate (17.09 g) in N,N-dimethylformamide (500 ml), and the resulting mixture was stirred at 70°C for 15 hours. The reaction mixture was cooled to room temperature and filtered, and the filtrate was concentrated under reduced pressure. The resulting residue was purified by a silica gel column chromatography (hexane/ethyl acetate = 5/1) to obtain the title compound (14.05 g) as an oil.
¹H NMR (400 MHz, CDCl₃) δ ppm 7.29-7.25 (m, 2H), 7.20-7.16 (m, 5H), 6.83 (d, J = 8.7 Hz, 2H), 5.51 (s, 2H), 4.27 (q, J = 7.1 Hz, 2H), 4.01 (s, 2H), 3.77 (s, 3H), 3.43 (br, 2H), 1.24 (t, J = 7.1 Hz, 3H).
MS (ESI+) 366 (M⁺+1, 100%).

### Reference Example 39

Ethyl 5-amino-4-benzyl-1H-pyrazole-3-carboxylate

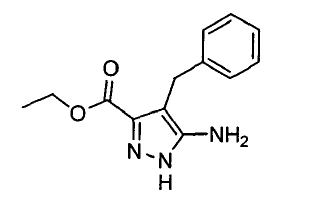

Sodium ethoxide (a 21% ethanol solution, 150 ml) was added to a solution of 3-phenylpropionitrile (43.56 g) and diethyl oxalate (48.53 g) in ethanol (500 ml), and the resulting mixture was stirred with heating under reflux for 10 hours. The reaction solution was cooled to room temperature, followed by adding thereto 35% hydrochloric acid (45 g) and then acetic acid (200 ml) and hydrazine monohydrate (21.6 g). The reaction solution was stirred with heating under reflux for 10 hours, cooled to room temperature, and then concentrated under reduced pressure. The resulting residue was suspended in ethyl acetate (300 ml) and the resulting suspension was made basic with a saturated aqueous sodium hydrogencarbonate solution and extracted four times with ethyl acetate (500 ml). The combined organic layer was dried over anhydrous magnesium sulfate and filtered, and the filtrate was concentrated under reduced pressure. The resulting residue was washed 5 times with hexane (500 ml) and dried to obtain the title compound (31.76 g) as a brown solid.
¹H NMR (400 MHz, CDCl₃) δ ppm 7.30-7.26 (m, 2H), 7.22-7.18 (m, 3H), 4.36 (q, J = 7.1 Hz, 2H), 4.06 (s, 2H), 3.55 (br, 2H), 1.34 (t, J = 7.1 Hz, 3H).
MS (ESI+) 246 (M⁺+1, 100%).

### Reference Example 40

5-{(3R)-3-[(tert-Butoxylcarbonyl)amino]-piperidin-1-yl}-4-(2-chlorobenzyl)-1-[(dimethylamino)sulfonyl]-1H-pyrazole-3-carboxylic acid

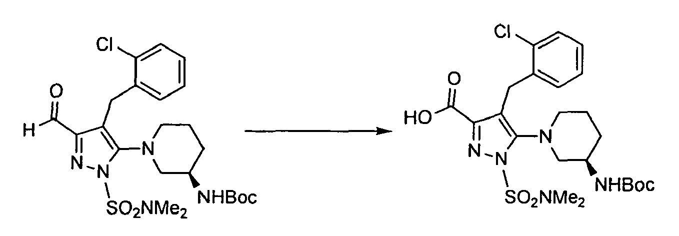

An aqueous solution (80 ml) of sodium dihydrogenphosphate dihydrate (7.69 g) was added to a solution of tert-butyl ((3R)-1-{4-(2-chlorobenzyl)-1-[(dimethylamino)sulfonyl]-3-formyl-1H-pyrazol-5-yl}piperidin-3-yl)carbamate (8.64 g) in tert-butyl alcohol (400 ml), and the reaction mixture was ice-cooled. After 2-methyl-2-butene (8.7 ml) and sodium chlorite (1.78 g) were added thereto, the resulting mixture was stirred at 0°C for 3 hours. An aqueous solution (50 ml) of sodium sulfite (0.41 g) and a 5% potassium hydrogensulfate solution (1000 ml) were added to the reaction mixture, followed by two runs of extraction with ethyl acetate (500 ml), and the combined organic layer was concentrated under reduced pressure to obtain the title compound as a crude product (8.88 g).
¹H NMR (400 MHz, CDCl₃) δ ppm 7.38-7.36 (m, 1H), 7.12-7.11 (m, 2H), 6.99-6.97 (m, 1H), 4.71-4.68 (m, 1H), 4.09-4.03 (m, 2H), 3.74-3.72 (m, 1H), 3.20-3.19 (m, 1H), 3.06 (s, 6H), 2.99-2.94 (m, 1H), 2.88-2.82 (m, 2H), 1.52-1.47 (m, 4H), 1.44 (s, 9H).
MS (ESI+) 542 (M⁺+1, 60%).

### Reference Example 41

5-{(3R)-3-[(tert-Butoxylcarbonyl)amino]-piperidin-1-yl}-4-(2-chloro-5-fluorobenzyl)-1-[(dimethylamino)sulfonyl]-1H-pyrazole-3-carboxylic acid

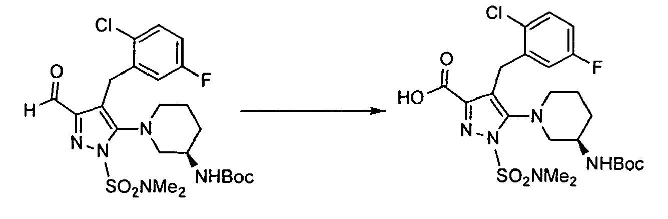

The title compound was synthesized as a crude product (4.2 g) by the same process as in Reference Example 40.
MS (ESI+) 560 (M⁺+1, 47%).

### Reference Example 42

5-{(3R)-3-[(tert-Butoxycarbonyl)amino]-piperidin-1-yl}-4-(2-chlorobenzyl)-1-(methoxymethyl)-1H-pyrazole-3-carboxylic acid mixture

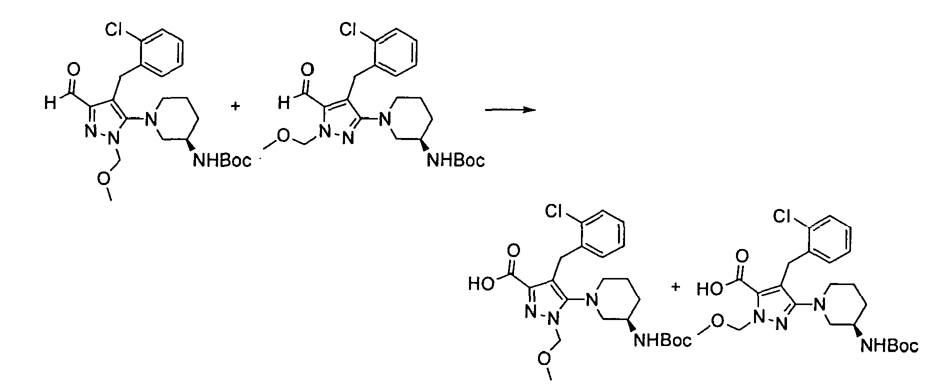

An aqueous solution (12.46 g / 30 ml water) of sodium dihydrogenphosphate dihydrate was added to a solution of a tert-butyl {(3R)-1-[4-(2-chlorobenzyl)-3-formyl-1-(methoxymethyl)-1H-pyrazol-5-yl]piperidin-3-yl}carbamate mixture (12.33 g) in tert-butanol (140 ml), and the resulting mixture was cooled to 0°C. To the reaction solution were added dropwise 2-methyl-2-butene (14.11 ml) and an aqueous sodium chlorite solution (3.66 g / 20 ml water), and the resulting mixture was vigorously stirred at 0°C for 7 hours. After a 1M aqueous sodium sulfite solution (55 ml) was added thereto, the resulting mixture was adjusted to pH2 with a 10% aqueous potassium hydrogensulfate solution (200 ml) and extracted twice with ethyl acetate (300 ml). The organic layer was dried over anhydrous sodium sulfate and filtered, and the filtrate was concentrated under reduced pressure to obtain the title compound (12.73 g) as a light-yellow solid.
¹H NMR (300 MHz, CDCl₃) δ ppm 7.37-7.34 (m, 1H), 7.12-7.08 (m, 2H), 6.88-6.81 (m, 1H), 5.73-5.69 (m, 2H), 4.90-4.88 (m, 1H), 4.25-4.10 (m, 2H), 3.74-3.72 (m, 1H), 3.39-3.32 (m, 3H), 3.11-3.08 (m, 1H), 2.86-2.71 (m, 3H), 1.54-1.46 (m, 4H), 1.43-1.41 (m, 9H).
MS (ESI+) 479 (M⁺+1, 37%).

### Reference Example 43

tert-Butyl ((3R)-1-{4-(2-chlorobenzyl)-1-[(dimethylamino)sulfonyl]-3-formyl-1H-pyrazol-5-yl}piperidin-3-yl)carbamate

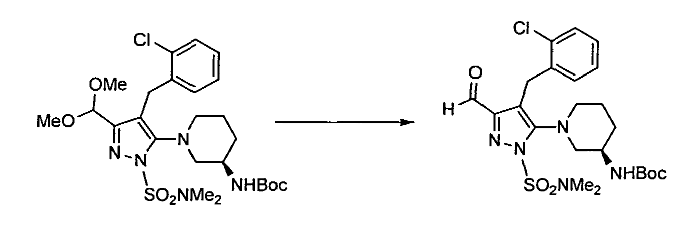

Acetic acid (100 ml) and water (50 ml) were added to a solution of tert-butyl ((3R)-1-{4-(2-chlorobenzyl)-3-(dimethoxymethyl)-1-[(dimethylamino)sulfonyl]-1H-pyrazol-5-yl}piperidin-3-yl)carbamate (10.2 g) in 1,4-dioxane (50 ml), and the resulting mixture was stirred at 50°C for 8 hours. The reaction mixture was concentrated under reduced pressure and water (500 ml) was added to the residue, followed by two runs of extraction with ethyl acetate (300 ml). The combined organic layer was concentrated under reduced pressure to obtain the title compound as a crude product (9.49 g).
¹H NMR (400 MHz, CDCl₃) δ ppm 10.41(s, 1H), 7.40-7.37 (m, 1H), 7.19-7.13 (m, 2H), 6.88-6.77 (m, 1H), 4.68-4.66 (m, 1H), 4.31-4.14 (m, 2H), 3.75-3.73 (m, 1H), 3.07 (s, 6H), 2.99-2.94 (m, 2H), 2.92-2.76 (m, 2H), 1.65-1.49 (m, 4H), 1.43 (s, 9H).
MS (ESI+) 526 (M⁺+1, 50%).

### Reference Example 44

tert-Butyl ((3R)-1-{4-(2-chloro-5-fluorobenzyl)-1-[(dimethylamino)sulfonyl]-3-formyl-1H-pyrazol-5-yl}piperidin-3-yl)carbamate

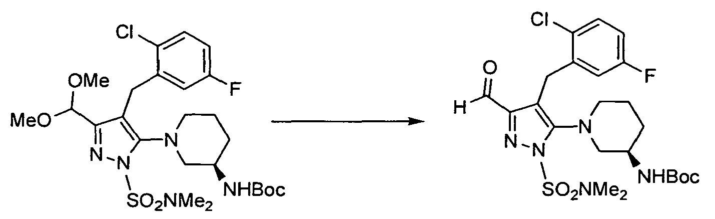

The title compound (4.3 g) was synthesized by the same process as in Reference Example 43.
MS (ESI+) 544 (M⁺+1, 44%).

### Reference Example 45

tert-Butyl ((3R)-1-{4-(2-chlorobenzyl)-3-(dimethoxymethyl)-1-[(dimethylamino)sulfonyl]-1H-pyrazol-5-yl}piperidin-3-yl)carbamate

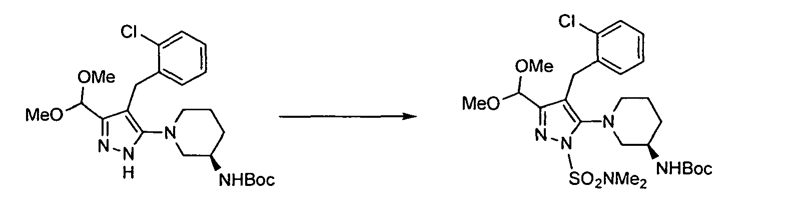

Triethylamine (5.5 ml) and potassium tert-butoxide (13.4 g) were added to a solution of tert-butyl {(3R)-1-[4-(2-chlorobenzyl)-3-(dimethoxymethyl)-1H-pyrazol-5-yl]piperidin-3-yl}carbamate (18.6 g) in N,N-dimethylformamide (160 ml) under ice-cooling, and the resulting mixture was warmed to room temperature and then stirred for 1 hour. The reaction mixture was ice-cooled again and N,N-dimethylsulfamoyl chloride (6.73 ml) was added thereto and stirred for 2 hours. Water (500 ml) was added to the reaction mixture, followed by two runs of extraction with ethyl acetate (300 ml), and the combined organic layer was concentrated under reduced pressure. The resulting residue was purified by a silica gel column chromatography (hexane/ethyl acetate = 3/1) to obtain the title compound (10.2 g).
¹H NMR (400 MHz, CDCl₃) δ ppm 7.38-7.36 (m, 1H), 7.18-7.12 (m, 2H), 6.98-6.96 (m, 1H), 5.89 (s, 1H), 4.67-4.65 (m, 1H), 4.27-4.07 (m, 2H), 3.71-3.70 (m, 1H), 3.37 (s, 6H), 3.04 (s, 6H), 3.05-3.01 (m, 3H), 2.69-2.64 (m, 1H), 1.53-1.52 (m, 2H), 1.44 (s, 9H), 1.39-1.37 (m, 2H).
MS (ESI+) 572 (M⁺+1, 31%).

### Reference Example 46

tert-Butyl ((3R)-1-{4-(2-chloro-5-fluorobenzyl)-3-(dimethoxymethyl)-1-[(dimethylamino)sulfonyl]-1H-pyrazol-5-yl}piperidin-3-yl)carbamate

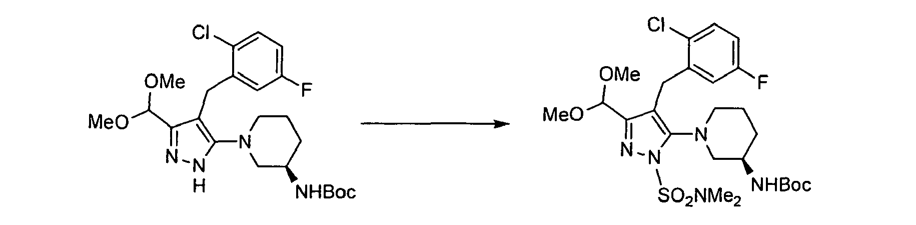

The title compound (4.6 g) was synthesized by the same process as in Reference Example 45.
¹H NMR (400 MHz, CDCl₃) δ ppm 7.37-7.30 (m, 1H), 6.90-6.82 (m, 1H), 6.78-6.69 (m, 1H), 5.89 (s, 1H), 4.14 (d, J = 18Hz, 1H), 4.06 (d, J = 18Hz, 1H), 3.77-3.68 (m, 1H), 3.37 (s, 3H), 3.36 (s, 3H), 3.05 (s, 3H), 2.99-2.86 (m, 2H), 2.82 (s, 3H), 2.80-2.70 (m, 1H), 1.64-1.36 (m, 5H), 1.43 (s, 9H).
MS (ESI+) 590 (M⁺+1, 67%).

### Reference Example 47

tert-Butyl {(3R)-1-[4-(2-chlorobenzyl)-3-formyl-1-(methoxymethyl)-1H-pyrazol-5-yl]piperidin-3-yl}carbamate mixture

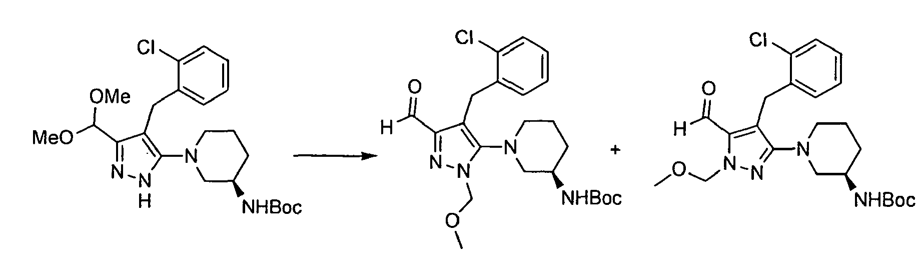

A solution (60 ml) of tert-butyl {(3R)-1-[4-(2-chlorobenzyl)-3-(dimethoxymethyl)-1H-pyrazol-5-yl]piperidin-3-yl}carbamate (15.00 g) in N,N-dimethylformamide was cooled to 0°C and 55% sodium hydride (1.69 g) was added thereto. The resulting mixture was warmed to room temperature 30 minutes after the addition and then stirred for 30 minutes. The reaction solution was cooled to 0°C again and chloromethyl methyl ether (3.27 g) was added thereto. The resulting mixture was warmed to room temperature 4 hours after the addition and then stirred for 1 day. Water (500 ml) was added to the reaction solution, followed by extraction with ethyl acetate (500 ml). The organic layer was dried over anhydrous sodium sulfate and filtered, and the filtrate was concentrated under reduced pressure. The crude product thus obtained was made into a mixed solution with 1,4-dioxane (50 ml), water (50 ml) and acetic acid (150 ml), and the mixed solution was stirred with heating at 50°C for 6 hours. The mixed solution was cooled to 0°C, adjusted to pH 9 with an aqueous sodium carbonate solution, and then extracted twice with ethyl acetate (300 ml). The organic layer was dried over anhydrous sodium sulfate and filtered, and the filtrate was concentrated under reduced pressure. The resulting residue was purified by a silica gel column chromatography (hexane/ethyl acetate = 4/1 to 1/1) to obtain the title compound (8.62 g) as a light-yellow liquid.
¹H NMR (300 MHz, CDCl₃) δ ppm 9.83 (s, 1H), 7.41-7.35 (m, 1H), 7.20-7.17 (m, 2H), 7.03-7.00 (m, 1H), 5.65 (s, 2H), 4.82-4.80 (m, 1H), 4.26-4.18 (m, 2H), 3.77-3.75 (m, 1H), 3.36 (s, 3H), 3.20-3.17 (m, 1H), 2.93-2.84 (m, 3H), 1.64-1.47 (m, 4H), 1.43 (s, 9H).
MS (ESI+) 463 (M⁺+1, 70%).

### Reference Example 48

tert-Butyl {(3R)-1-[4-(2-chlorobenzyl)-3-(dimethoxymethyl)-1H-pyrazol-5-yl]piperidin-3-yl}carbamate

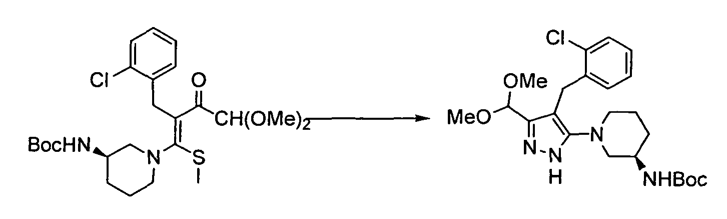

Hydrazine monohydrate (6.91 ml) was added to a solution of tert-butyl {(3R)-1-[2-(2-chlorobenzyl)-4,4-dimethoxy-1-(methylthio)-3-oxobut-1-en-1-yl]piperidin-3-yl}carbamate (8.87 g) in N,N-dimethylformamide (90 ml), and the resulting mixture was stirred at 100°C for 2 hours. The reaction mixture was allowed to cool and water (500 ml) was added thereto, followed by two runs of extraction with ethyl acetate (300 ml). The combined organic layer was concentrated under reduced pressure. The resulting residue was purified by a silica gel column chromatography (hexane/ethyl acetate = 1/1) to obtain the title compound (7.40 g).
¹H NMR (400 MHz, CDCl₃) δ ppm 9.90 (bs, 1H), 7.38-7.36 (m, 1H), 7.18-7.13 (m, 2H), 7.05-7.03 (m, 1H), 5.33 (s, 1H), 4.92-4.90 (m, 1H), 3.92 (s, 2H), 3.77-3.75 (m, 1H), 3.28 (s, 3H), 3.26 (s, 3H), 3.12-3.10 (m, 1H), 3.00-2.97 (m, 2H), 2.78-2.76 (m, 1H), 1.58-1.49 (m, 4H), 1.43 (s, 9H).
MS (ESI+) 465 (M⁺+1, 56%).

### Reference Example 49

tert-Butyl {(3R)-1-[4-(2-chloro-5-fluorobenzyl)-3-(dimethoxymethyl)-1H-pyrazol-5-yl]piperidin-3-yl}carbamate

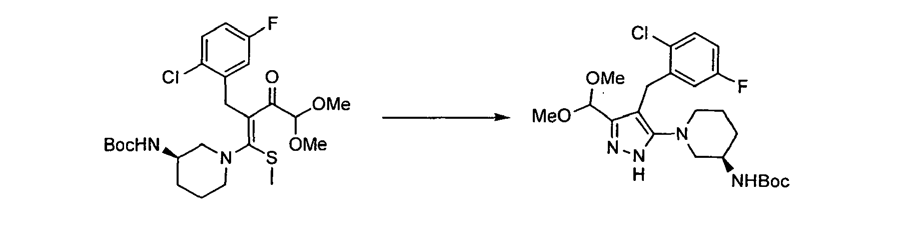

The title compound (22 g) was synthesized by the same process as in Reference Example 48.
¹H NMR (400 MHz, CDCl₃) δ ppm 7.35-7.29 (m, 1H), 6.88-6.78 (m, 1H), 6.76-6.73(m, 1H), 5.34 (s, 1H), 3.88 (s, 2H), 3.80-3.74 (m, 1H), 3.26 (s, 3H), 3.24 (s, 3H), 2.98-2.80 (m, 3H), 1.70-1.46 (m, 5H), 1.42 (s, 9H).
MS (ESI+) 483 (M⁺+1, 47%).

### Reference Example 50

tert-Butyl {(3R)-1-[2-(2-chlorobenzyl)-4,4-dimethoxy-1-(methylthio)-3-oxobut-1-en-1-yl]piperidin-3-yl}carbamate

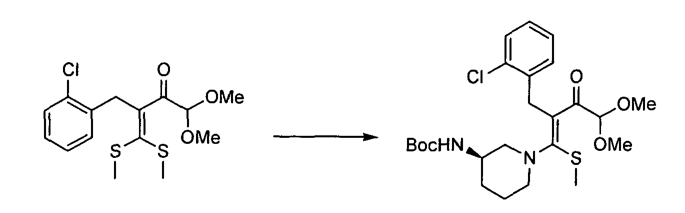

To a solution of 3-(2-chlorobenzyl)-1,1-dimethoxy-4,4-bis(methylthio)but-3-en-2-one (30.4 g) in xylene (220 ml) was added (R)-tert-3-butylpiperidin-3-yl carbamate (21.0 g), and the resulting mixture was heated under reflux for 4 hours. The reaction mixture was allowed to cool, and purified by a silica gel column chromatography (hexane/ethyl acetate = 1/2) to obtain the title compound (8.87 g).
¹H NMR (400 MHz, CDCl₃) δ ppm 7.36-7.34 (m, 1H), 7.17-7.06 (m, 2H), 4.62-4.59 (m, 1H), 4.58 (s, 1H), 4.10-4.08 (m, 2H), 3.70-3.63 (m, 2H), 3.33-3.30 (m, 1H), 3.29 (s, 3H), 3.26 (s, 3H), 3.09-3.07 (m, 1H), 2.84-2.79 (m, 2H), 2.30 (s, 3H), 2.04-1.99 (m, 1H), 1.85-1.81 (m, 1H), 1.64-1.61 (m, 2H), 1.48 (s, 9H).
MS (ESI+) 499 (M⁺+1, 100%).

### Reference Example 51

tert-Butyl {(3R)-1-[(1Z)-2-(2-chloro-5-fluorobenzyl)-4,4-dimethoxy-1-(methylthio)-3-oxobut-1-en-1-yl]piperidin-3-yl}carbamate

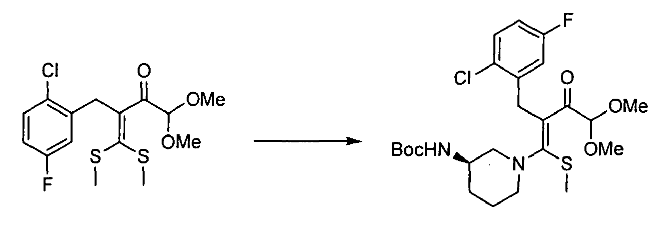

The title compound (18.2 g) was synthesized by the same process as in Reference Example 50.
MS (ESI+) 517 (M⁺+1, 100%).

### Reference Example 52

3-(2-Chlorobenzyl)-1,1-dimethoxy-4,4-bis(methylthio)but-3-en-2-one

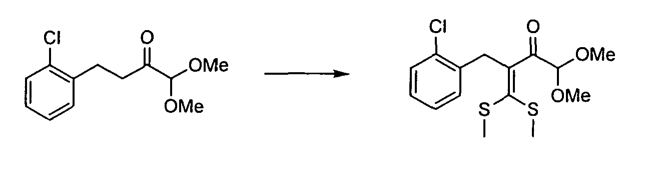

Tetrahydrofuran (210 ml) and hexamethylphosphoramide (39.1 g) were added to a potassium bis(trimethylsilyl)amide solution (toluene, 15%, 350 ml), and the resulting mixture was stirred at -78°C for 40 minutes. A solution of 4-(2-chlorophenyl)-1,1-dimethoxybutan-2-one (50.5 g) in tetrahydrofuran (50 ml) was added dropwise thereto over a period of 50 minutes. The resulting mixture was stirred at -78°C for 20 minutes and carbon disulfide (16.6 g) was added thereto. The reaction mixture was warmed to 0°C over a period of 80 minutes and then stirred under ice-cooling for 30 minutes. The reaction mixture was cooled to -78°C again and a potassium bis(trimethylsilyl)amide solution (toluene, 15%, 350 ml) was added dropwise thereto over a period of 30 minutes. The resulting mixture was stirred at -78°C for 30 minutes and methyl iodide (28.5 ml) was added thereto. The reaction mixture was warmed to room temperature over a period of 100 minutes and stirred at room temperature for 30 minutes. A saturated aqueous ammonium chloride solution (200 ml) was added thereto and the tetrahydrofuran was distilled off under reduced pressure. Water (500 ml) was added to the residue, followed by two runs of extraction with ethyl acetate (500 ml), and the combined organic layer was concentrated under reduced pressure. The resulting residue was purified by a silica gel column chromatography (hexane/ethyl acetate = 8/1) to obtain the title compound (36.6 g).
¹H NMR (400 MHz, CDCl₃) δ ppm 7.34-7.32 (m, 1H), 7.19-7.14 (m, 3H), 5.00 (s, 1H), 4.11 (s, 2H), 3.24 (s, 6H), 2.35 (s, 3H), 2.34 (s, 3H).
MS (ESI+) 347 (M⁺+1, 5%).

### Reference Example 53

3-(2-Chloro-5-fluorobenzyl)-1,1-dimethoxy-4,4- bis(methylthio)but-3-en-2-one

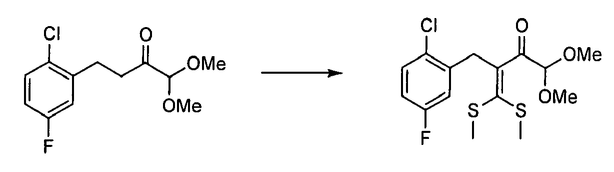

The title compound (34.3 g) was synthesized by the same process as in Reference Example 52.
¹H NMR (400 MHz, CDCl₃) δ ppm 7.30-7.26 (m, 1H), 7.01-6.95 (m, 1H), 6.89-6.83(m, 1H), 5.10 (s, 1H), 4.05 (s, 2H), 3.29 (s, 6H), 2.37 (s, 3H), 2.35 (s, 3H).
MS (ESI+) 365 (M⁺+1, 11%).

### Reference Example 54

4-(2-Chlorophenyl)-1,1-dimethoxybutan-2-one

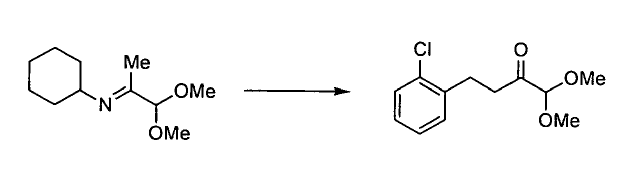

Tetrahydrofuran (200 ml) was added to a lithium diisopropylamide solution (heptane, 2.0 M, 200 ml) at -78°C and a solution of N-(2,2-dimethoxy-1-methylethylidene)cyclohexaneamine (72.4 g) in tetrahydrofuran (360 ml) was added dropwise thereto over a period of 30 minutes. The resulting mixture was stirred at -78°C for 1 hour and 2-chlorobenzyl bromide (82.2 g) was added dropwise thereto over a period of 20 minutes. The reaction mixture was warmed to room temperature over a period of 3 hours. The reaction mixture was cooled to 0°C and 3N-hydrochloric acid (290 ml) was added thereto and vigorously stirred. The reaction mixture was adjusted to pH 8 with a 5% aqueous potassium carbonate solution and the tetrahydrofuran was distilled off under reduced pressure. The residue was extracted twice with ethyl acetate (500 ml) and the combined organic layer was concentrated under reduced pressure. The resulting residue was purified by a silica gel column chromatography (hexane/ethyl acetate = 10/1) to obtain the title compound (62.4 g).
¹H NMR (400 MHz, CDCl₃) δ ppm 7.34-7.32 (m, 1H), 7.26-7.24 (m, 1H), 7.17-7.14 (m, 2H), 4.47 (s, 1H), 3.39 (s, 6H), 3.03-2.98 (m, 2H), 2.93-2.89 (m, 2H).

### Reference Example 55

4-(2-Chloro-5-fluorophenyl)-1,1-dimethoxybutan-2-one

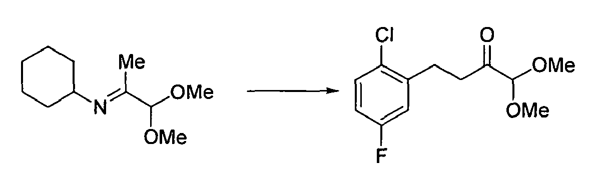

The title compound (38 g) was synthesized by the same process as in Reference Example 54.
¹H NMR (400 MHz, CDCl₃) δ ppm 7.32-7.25 (m, 1H), 7.01-6.97 (m, 1H), 6.89-6.80(m, 1H), 4.47 (s, 1H), 3.58 (s, 6H), 3.01-2.96 (m, 2H), 2.93-2.87 (m, 2H).
MS (ESI+) 261 (M⁺+1, 19%).

### Reference Example 56

N-(2,2-Dimethoxy-1-methylethylidene)cyclohexaneamine

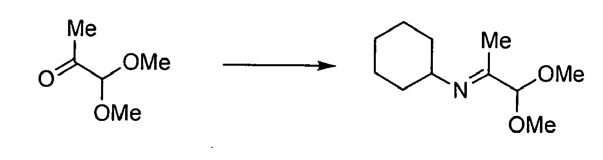

Molecular sieves (4Å, 28 g) and cyclohexylamine (35.2 ml) were added to a solution of pyruvic aldehyde dimethyl acetal (28.0 g) in dichloromethane (237 ml), and the resulting mixture was stirred overnight at room temperature. The reaction mixture was filtered through Celite and the filtrate was concentrated under reduced pressure to obtain the title compound as a crude product (48.4 g).
¹H NMR (400 MHz, CDCl₃) δ ppm 4.43 (s, 1H), 3.39 (s, 6H), 3.38-3.32 (m, 1H), 1.83 (s, 3H), 1.81-1.77 (m, 2H), 1.7.-1.60 (m, 4H), 1.47-1.45 (m, 2H) 1.42-1.28 (m, 2H).

### Reference Example 57

tert-Butyl 4-[4-but-2-yn-1-yl-3-({[1-diethoxymethyl]-2-ethoxy-2-oxoethyl}amino)carbonyl]-1-(methoxymethyl)-1H-pyrazol-5-yl]pyrazol-5-yl]piperazine-1-carboxylate

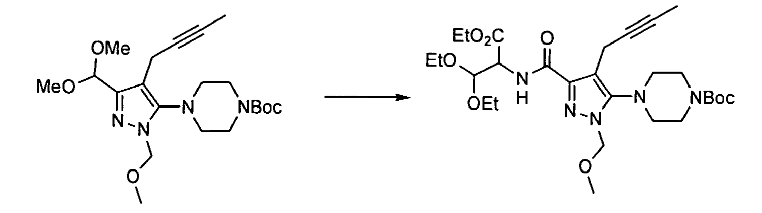

To an ice-cooled solution of tert-butyl 4-[3-(dimethoxy)-4-iodo-1-(methoxymethyl)-1H-pyrazol- 5-yl]piperazine-1-carboxylate (400 mg) in dimethylformamide (5 ml) were added 1-ethyl-3-(3-dimethylaminopropyl)carbodiimide hydrochloride (586 mg), 1-hydroxybenzotriazole monohydrate (413 mg) and ethyl 3-ethoxy-O-ethylserinate (627 mg), and the resulting mixture was stirred overnight at room temperature. Water was added to the reaction solution, followed by extraction with ethyl acetate, and the combined organic layer was concentrated under reduced pressure to obtain a crude product. The crude product was purified by a silica gel column chromatography (from chloroform to hexane/ethyl acetate = 2/1 to 1/1) to obtain the title compound (360 mg).
¹H NMR (400 MHz, CDCl₃) δ ppm 5.58-5.48 (m, 2H), 5.06-5.03 (m, 1H), 4.87-4.86 (m, 1H), 4.25-4.23 (m, 2H), 3.82-3.74 (m, 2H), 3.68-3.60 (m, 2H), 3.56-3.53 (m, 4H), 3.45-3.33 (m, 2H), 3.38 (s, 3H), 3.15-3.12 (m, 4H), 1.78 (s, 3H), 1.48 (s, 9H), 1.27-1.16 (m, 9H).
MS (ESI+) 580 (M⁺+1, 100%).

### Reference Example 58

tert-Butyl 4-[4-but-2-yn-1-yl-3-(diethoxymethyl)-1-(methoxymethyl)-1H-pyrazol-5-yl]piperazine-1-carboxylate

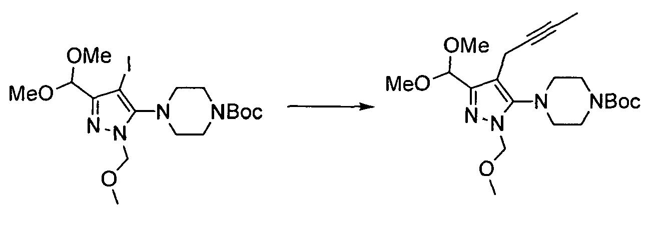

A solution of tert-butyl 4-[3-(dimethoxymethyl)-4-iodo-1-(methoxymethyl)-1H-pyrazol-5-yl]piperazine-1-carboxylate (2.50 g) in tetrahydrofuran (50 ml) was cooled to -40°C, followed by adding dropwise thereto 2N isopropylmagnesium chloride (a tetrahydrofuran solution) (25 ml), and the resulting mixture was stirred at -40°C for 1 hour. A 1M solution of copper(I) cyanide•lithium chloride in tetrahydrofuran (66 ml) was added to the reaction solution and the resulting mixture was stirred at -20°C or lower for 30 minutes. After 2-butyn-1-ol (13.3 g) was added dropwise thereto, the resulting mixture was stirred at -20°C for one and a half hours. The reaction mixture was quenched with methanol and then concentrated under reduced pressure, and water was added to the residue, followed by extraction with ethyl acetate. The combined organic layer was concentrated under reduced pressure and the residue was purified by a silica gel column chromatography (hexane/ethyl acetate = 2/1) to obtain the title compound (1.18 g).
¹H NMR (400 MHz, CDCl₃) δ ppm 5.55 (s, 1H), 5.34 (s, 2H), 3.55-3.52 (m, 4H), 3.38 (s, 6H), 3.37-3.36 (m, 2H), 3.34 (s, 3H), 3.33-3.10 (m, 4H), 1.76-1.74 (m, 3H), 1.47 (s. 9H).
MS (ESI+) 423 (M⁺+1, 100%).

### Reference Example 59

tert-Butyl {(3R)-1-[3-(dimethoxymethyl)-4-iodo-1-(methoxymethyl)-1H-pyrazol-5-yl]piperidin-3-yl}carbamate

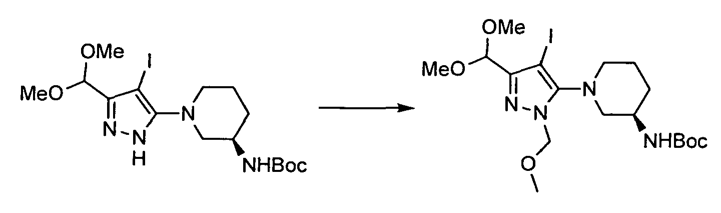

To a solution of tert-butyl {(3R)-1-[3-(dimethoxymethyl)-4-iodo-1H-pyrazol-5-yl]piperidin-3-yl}carbamate (4.66 g) in N,N-dimethylformamide (20 ml) was added 55% sodium hydride (500.0 mg) at 0°C, and stirred for 20 minutes, followed by adding thereto chloromethyl methyl ether (885.6 mg), and the resulting mixture was stirred at 0°C for 2 hours and then at room temperature for 2 hours. A 10% aqueous potassium hydrogensulfate solution (200 ml) was added to the reaction solution and organic substances were extracted twice therefrom with ethyl acetate (200 ml). The combined organic layer was washed with water (200 ml) and then a saturated aqueous sodium chloride solution (200 ml), dried over anhydrous magnesium sulfate, and then filtered, and the filtrate was concentrated under reduced pressure. The resulting residue was purified by a silica gel column chromatography (hexane/ethyl acetate = 10/1 to 1/1) to obtain the title compound (3.39 g).
¹H NMR (300 MHz, CDCl₃) δ ppm 5.41 (s, 2H), 5.37 (s, 1H), 5.28 (bs, 1H), 3.90-3.84 (m, 1H), 3.42 (s, 6H), 3.38 (s, 3H), 3.25-2.95 (m, 4H), 1.93-1.58 (m, 4H), 1.45 (s, 9H).
MS (ESI+) 511 (M⁺+1, 100%).

### Reference Example 60

tert-Butyl 4-[3-(dimethoxymethyl)-4-iodo-1-(methoxymethyl)-1H-pyrazol-5-yl]piperazine-1-carboxylate

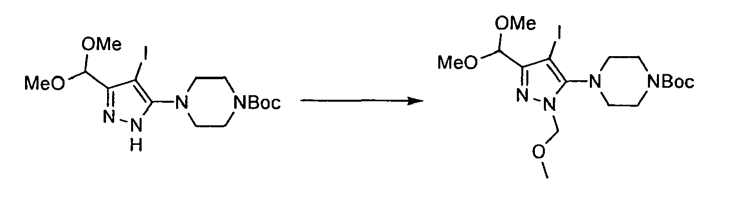

The title compound (3.44 g) was synthesized by the same process as in Reference Example 59.
¹H NMR (300 MHz, CDCl₃) δ ppm 5.41 (s, 2H), 5.37 (s, 1H), 3.56 (t, J = 5.1Hz, 4H), 3.43 (s, 6H), 3.38 (s, 3H), 3.16 (t, J = 5.1Hz, 4H), 1.47 (s, 9H).
MS (ESI+) 497 (M⁺+1, 100%).

### Reference Example 61

tert-Butyl {(3R)-1-[3-(dimethoxymethyl)-4-iodo-1H-pyrazol-5-yl]piperidin-3-yl}carbamate

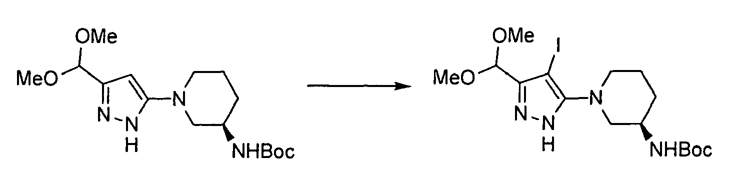

Anhydrous potassium carbonate (2.00 g) and iodine (3.40 g) were added to a solution of tert-butyl {(3R)-1-[3-(dimethoxymethyl)-1H-pyrazol-5-yl]piperidin-3-yl}carbamate (4.40 g) in chloroform (50 ml), and the resulting mixture was stirred at room temperature for 3 hours. A 1M aqueous potassium hydrogensulfite solution (200 ml) was added to the reaction solution and organic substances were extracted twice therefrom with chloroform (200 ml). The combined organic layer was dried over anhydrous sodium sulfate and filtered, and the filtrate was concentrated under reduced pressure to obtain the title compound (6.44 g).
¹H NMR (300 MHz, CDCl₃) δ ppm 5.39 (s, 1H), 5.20 (bs, 1H), 3.90-3.84 (m, 1H), 3.31 (s, 6H), 3.25-2.95 (m, 4H), 1.85-1.55 (m, 4H), 1.43 (s, 9H).
MS (ESI+) 467 (M⁺+1, 100%).

### Reference Example 62

tert-Butyl 4-[3-(dimethoxymethyl)-4-iodo-1H-pyrazol-5-yl]piperazine-1-carboxylate

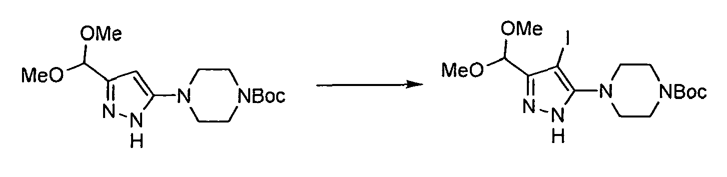

The title compound (10.14 g) was synthesized by the same process as in Reference Example 61. ¹H NMR (300 MHz, CDCl₃) δ ppm 5.42 (s, 1H), 3.57 (t, J = 5.1Hz, 4H), 3.34 (s, 6H), 3.17 (t, J = 5.1Hz, 4H), 1.48 (s, 9H).
MS (ESI+) 453 (M⁺+1, 100%).

### Reference Example 63

tert-Butyl {(3R)-1-[3-(dimethoxymethyl)-1H-pyrazol-5-yl]piperidin-3-yl}carbamate

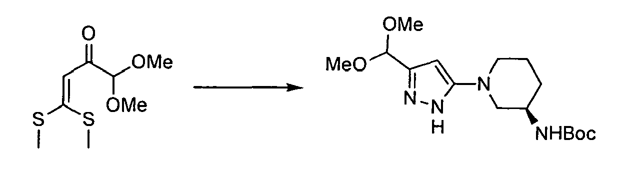

To a solution of 1,1-dimethoxy-4,4-bis(methylthio)-3-buten-2-one (25.0 g) in ethanol (300 ml) was added (R)-tert-3-butylpiperidin-3-yl carbamate (24.0 g), and the resulting mixture was stirred with heating under reflux for 10 hours. After the reaction solution was cooled to room temperature, a solution of hydrazine monohydrate (12.0 g) in ethanol (50 ml) was added thereto, and the resulting mixture was stirred with heating under reflux for 4 hours. The reaction solution was concentrated under reduced pressure and the resulting residue was diluted with and dissolved in chloroform (500 ml). The resulting solution was washed with water (500 ml), dried over anhydrous sodium sulfate and then filtered, and the filtrate was concentrated under reduced pressure. To the resulting residue was added chloroform (500 ml) and the residue was dissolved with heating under reflux. After hexane (250 ml) was added thereto, the resulting mixture was stirred for 1 hour while being cooled to 0°C, and the solid precipitated was collected by filtration to obtain the title compound (9.52 g).
¹H NMR (300 MHz, CDCl₃) δ ppm 5.71 (s, 1H), 5.47 (s, 1H), 4.85 (bs, 1H), 3.85-3.70 (m, 1H), 3.31 (s, 6H), 3.25-2.95 (m, 4H), 1.85-1.50 (m, 4H), 1.42 (s, 9H).
MS (ESI+) 341 (M⁺+1, 100%).

### Reference Example 64

tert-Butyl 4-[3-(dimethoxymethyl)-1H-pyrazol-5-yl]piperazine-1-carboxylate

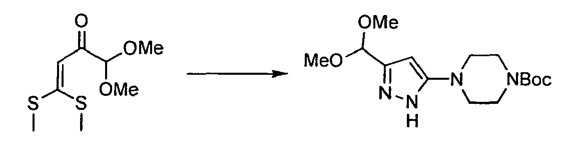

The title compound (16.10 g) was synthesized by the same process as in Reference Example 63.
¹H NMR (300 MHz, CDCl₃) δ ppm 5.74 (s, 1H), 5.50 (s, 1H), 3.55 (t, J = 5.1Hz, 4H), 3.33 (s, 6H), 3.16 (t, J = 5.1Hz, 4H), 1.48 (s, 9H).
MS (ESI+) 327 (M⁺+1, 100%).

### Reference Example 65

1,1-Dimethoxy-4,4-bis(methylthio)-3-buten-2-one

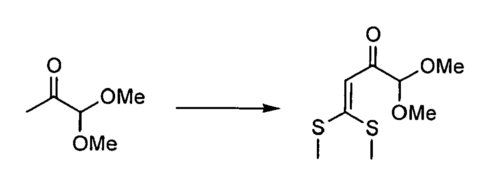

Carbon disulfide (7.61 g) was added to a suspension of 55% sodium hydride (9.60 g) in dimethylformamide (250 ml) at 0°C over a period of 10 minutes and stirred for 30 minutes, and then a solution of 1,1-dimethoxyacetone (11.8 g) in N,N-dimethylformamide (50 ml) was added thereto over a period of 30 minutes. An ice bath was removed, followed by stirring with warming for 2 hours. After tetrahydrofuran (200 ml) was added to the reaction solution, the resulting mixture was cooled to 0°C and then a solution of iodomethane (35.5 g) in tetrahydrofuran (50 ml) was added thereto over a period of 30 minutes. An ice bath was removed and the reaction solution was stirred for 1 hour while being warmed to room temperature. A 10% aqueous potassium hydrogensulfate solution (500 ml) was added to the reaction solution and organic substances were extracted twice therefrom with ethyl acetate (500 ml). The combined organic layer was washed with water (500 ml) and then a saturated aqueous sodium chloride solution (500 ml), dried over anhydrous magnesium sulfate and filtered, and the filtrate was concentrated under reduced pressure to obtain the title compound (25.0 g).
¹H NMR (300 MHz, CDCl₃) δ ppm 6.34 (s, 1H), 4.53 (s, 1H), 3.38 (s, 6H), 2.46 (s, 6H).

### Reference Example 66

Methyl 2-amino-3-oxobutanoate hydrochloride

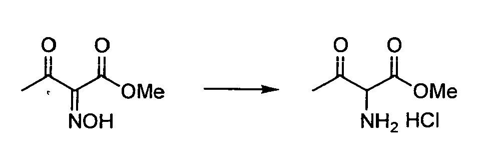

A 10% palladium-active carbon carrier (containing 50% water, 0.60 g) and a 10% solution of hydrochloric acid in methanol (15 ml) were added to a solution of methyl 2-(hydroxyimino)-3-oxobutanoate (3.00 g) in methanol (15 ml), and the resulting mixture was stirred for 2 hours at room temperature under a hydrogen atmosphere at atmospheric pressure and then treated with nitrogen to replace the air. The palladium-active carbon carrier was filtered off and the filtrate was concentrated under reduced pressure to obtain the title compound (3.46 g).
¹H NMR (300 MHz, DMSO-d₆) δ ppm 8.90 (bs, 3H), 5.31 (s, 1H), 3.81 (s, 3H), 2.38 (s, 3H).

### Reference Example 67

Methyl 2-(hydroxyimino)-3-oxobutanoate

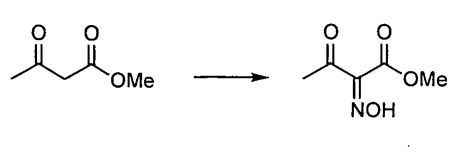

An aqueous solution (30 ml) of sodium nitrite (16.3 g) was added to a solution of methyl acetoacetate (25.0 g) in acetic acid (100 ml) over a period of 1 hour while maintaining the internal temperature at 12°C or lower, and the resulting mixture was stirred overnight while being slowly warmed to room temperature. The reaction solution was diluted with water (1000 ml) and sodium hydrogencarbonate was added thereto with stirring until no more foaming was observed. Organic substances were extracted twice therefrom with ethyl acetate (500 ml) and the combined organic layer was dried over anhydrous magnesium sulfate and filtered. The filtrate was concentrated under reduced pressure to obtain the title compound (20.3 g).
¹H NMR (300 MHz, CDCl₃) δ ppm 10.00 (bs, 1H), 3.91 (s, 3H), 2.42 (s, 3H).

### Reference Example 68

Ethyl 2-{(3R)-3-[(tert-butoxycarbonyl)amino]-piperidin-1-yl}-3-(2-chloro-5-fluorobenzyl)-4-oxo-4,5-dihydropyrazolo[1,5-a]pyrazin-6-carboxylate

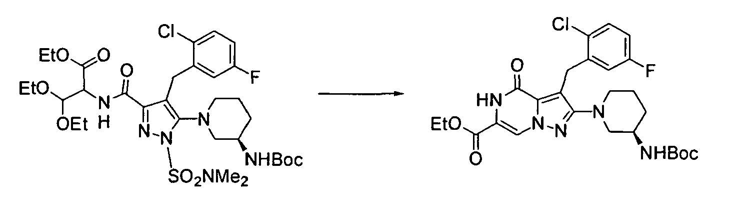

Water (20 ml) and a 4N hydrochloric acid/1,4-dioxane solution (40 ml) were added to a solution of ethyl N-({5-{(3R)-3-[(tert-butoxycarbonyl)aminopiperidin-1-yl]-4-(2-chloro-5-fluorobenzyl)-1-[(dimethylamino)sulfonyl]-1H- pyrazol-3-yl}carbonyl}-3-ethoxy-O-ethylserinate (1.50 g) in 1,4-dioxane (20 ml), and the resulting mixture was stirred at 50°C for 2 hours. The reaction solution was cooled to 0°C and 1,4-dioxane (30 ml), water (20 ml) and sodium hydrogencarbonate (30 g) were added thereto. The resulting mixture was warmed to room temperature and di-tert-butyl dicarbonate (1.61 g) was added thereto and stirred overnight. 1,4-Dioxane was distilled off under reduced pressure and water (100 ml) was added to the residue, followed by two runs of extraction with ethyl acetate (100 ml). The combined organic layer was concentrated under reduced pressure. The resulting residue was purified by a silica gel column chromatography (hexane/ethyl acetate = 2/1) to obtain the title compound (630 mg).
MS (ESI+) 448 (M⁺+1, 30%).

### Reference Example 69

2-{(3R)-3-[(tert-butoxycarbonyl)amino]piperidin-1-yl}-3-(2-chloro-5-fluorobenzyl)-4-oxo-4,5-dihydropyrazolo[1,5-a]pyrazin-6-carboxylic acid

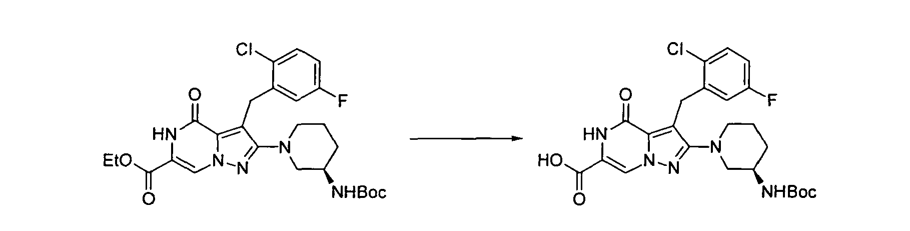

A solution consisting of ethyl 2-{(3R)-3-[(tert-butoxycarbonyl)amino]piperidin-1-yl}-3-(2-chloro-5-fluorobenzyl)-4-oxo-4,5-dihydropyrazolo[1,5-a]pyrazin-6-carboxylate (630 mg), ethanol (10 ml) and a 1N aqueous sodium hydroxide solution (10 ml) was stirred at 80°C for 1 hour. The reaction solution was allowed to cool, adjusted to pH 6 with a saturated aqueous ammonium chloride solution, and then extracted with ethyl acetate. The organic layer was washed with a saturated aqueous sodium chloride solution, dried over sodium sulfate and then filtered, and the filtrate was concentrated under reduced pressure to obtain the title compound (580 mg).
MS (ESI+) 520 (M⁺+1, 33%).

### Reference Example 70

Ethyl 2-{(3R)-3-[(tert-butoxycarbonyl)amino]-piperidin-1-yl}-3-(2-chlorobenzyl)-4-oxo-4,5-dihydropyrazolo[1,5-a]pyrazin-6-carboxylate

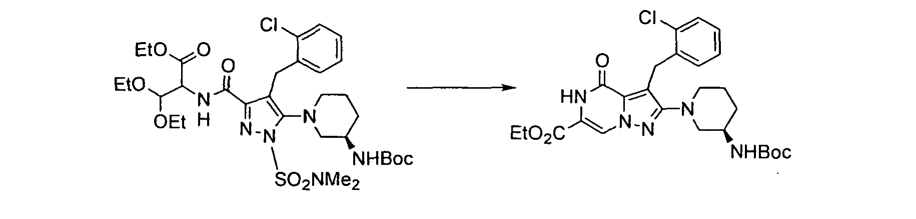

Water (20 ml) and a 4N hydrochloric acid/1,4-dioxane solution (40 ml) were added to a solution of ethyl N-({5-{(3R)-3-[(tert-butoxycarbonyl)aminopiperidin-1-yl]-4-(2-chlorobenzyl)-1-[(dimethylamino)sulfonyl]-1H-pyrazol-3-yl}carbonyl}-3-ethoxy-O-ethylserinate (2.69 g) in 1,4-dioxane (20 ml), and the resulting mixture was stirred at 50°C for 2 hours. The reaction solution was cooled to 0°C and 1,4-dioxane (30 ml), water (20 ml) and sodium hydrogencarbonate (30 g) were added thereto. The resulting mixture was warmed to room temperature and di-tert-butyl dicarbonate (1.61 g) was added thereto and stirred overnight. The 1,4-dioxane was distilled off under reduced pressure and water (100 ml) was added to the residue, followed by two runs of extraction with ethyl acetate (100 ml). The combined organic layer was concentrated under reduced pressure. The resulting residue was purified by a silica gel column chromatography (hexane/ethyl acetate = 2/1) to obtain the title compound (1.03 g).
¹H NMR (400 MHz, CDCl₃) δ ppm 8.47 (bs, 1H), 8.06 (s, 1H), 7.40-7.3 (m, 1H), 7.18-7.13 (m, 2H), 6.98-6.96 (m, 1H), 4.65-4.37 (m, 5H), 3.73-3.71 (m, 1H), 3.27-3.24 (m, 1H), 3.06-3.02 (m, 2H), 2.88-2.85 (m, 1H), 1.53-1.48 (m, 4H), 1.44 (s, 9H), 1.40 (t, J = 7.2Hz, 3H).
MS (ESI+) 530 (M⁺+1, 52%).

### Reference Example 71

Dimethyl N-{[5-{(3R)-3-[(tert-butoxycarbonyl)amino]piperidin-1-yl}-4-(2-chlorobenzyl)-1-(methoxymethyl)-1H-pyrazol-3-yl]carbonyl}-3-oxoglutamate mixture

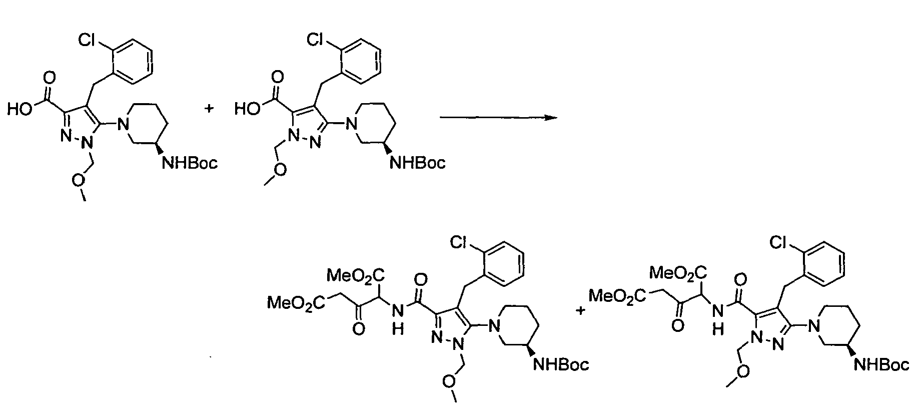

N-methylmorpholine (105.6 mg) and isobutyl chlorocarbonate (142.6 mg) were added to a solution of a 5-{(3R)-3-[(tert-butoxycarbonyl)amino]piperidin-1-yl}-4-(2-chlorobenzyl)-1-(methoxymethyl)-1H-pyrazole-3-carboxylic acid mixture (500.0 mg) in tetrahydrofuran (10 ml), and the resulting mixture was stirred at -20°C for 30 minutes. Then, dimethyl 3-oxoglutamate hydrochloride (300.0 mg) was added thereto, followed by adding dropwise thereto N-methylmorpholine (134.5 mg) slowly, and the resulting mixture was stirred overnight while being slowly warmed from -20°C to room temperature. A 10% aqueous potassium hydrogensulfate solution (50 ml) was added to the reaction solution and organic substances were extracted twice therefrom with ethyl acetate (30 ml). The combined organic layer was washed with a saturated aqueous sodium chloride solution (50 ml), dried over anhydrous magnesium sulfate and then filtered, and the filtrate was concentrated under reduced pressure. The resulting residue was purified by a silica gel thin-layer chromatography (hexane/ethyl acetate = 1/1) to obtain the title compound (57.5 mg).
MS (ESI+) 650 (M⁺+1, 100%).

### Reference Example 72

Dimethyl 3-oxoglutamate hydrochloride

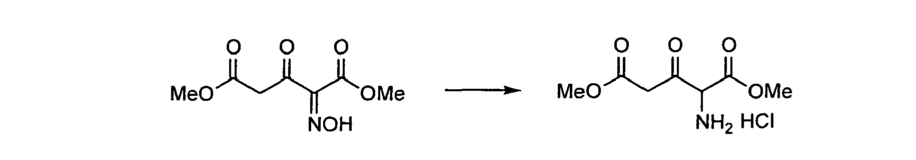

A 10% palladium-active carbon carrier (containing 50% water, 0.30 g) and a 10% solution of hydrochloric acid in methanol (5 ml) were added to a solution of dimethyl 2-(hydroxyimino)-3-oxopentanedioate (1.26 g) in methanol (5 ml), and the resulting mixture was stirred for 2 hours at room temperature under a hydrogen atmosphere at atmospheric pressure and then treated with nitrogen to replace the air. The palladium-active carbon carrier was filtered off and the filtrate was concentrated under reduced pressure to obtain the title compound (1.40 g).
¹H NMR (300 MHz, DMSO-d₆) δ ppm 9.03 (bs, 3H), 5.46 (bs, 1H), 4.03 (d, J = 16.8Hz, 1H), 3.92 (d, J = 16.8Hz, 1H), 3.77 (s, 3H), 3.64 (s, 3H).

### Reference Example 73

Dimethyl 2-(hydroxyimino)-3-oxopentanedioate

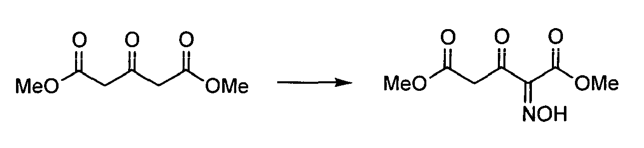

Chlorotrimethylsilane (1.20 g) and then isopentyl nitrite (1.40 g) were added to a solution of dimethyl 3-oxopentanedioate (1.74 g) in dichloromethane (5 ml) while maintaining the internal temperature at -20°C or lower, and the resulting mixture was stirred for 3 hours while being slowly warmed to room temperature. An aqueous sodium hydrogencarbonate solution (50 ml) was added to the reaction solution and organic substances were extracted twice therefrom with ethyl acetate (30 ml). The combined organic layer was dried over anhydrous sodium sulfate and then concentrated under reduced pressure. The resulting residue was purified by a silica gel column chromatography (hexane/ethyl acetate = 4/1) to obtain the title compound (1.26 g).
¹H NMR (300 MHz, CDCl₃) δ ppm 10.37 (bs, 1H), 3.92 (s, 3H), 3.84 (s, 2H), 3.76 (s, 3H).

### Reference Example 74

tert-Butyl {(3R)-1-[3-(2-chlorobenzyl)-5-methyl-4-oxo-4,5-dihydropyrazolo[1,5-d][1,2,4]triazin-2-yl]piperidin-3-yl}carbamate

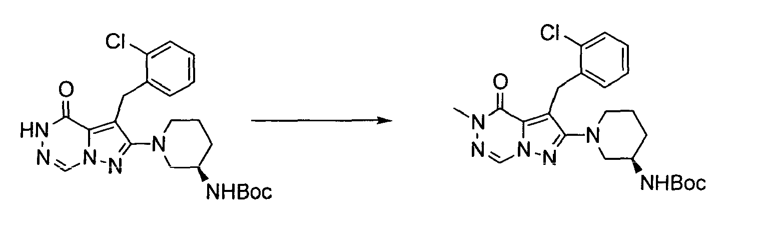

The title compound (87 mg) was synthesized by the same process as in Reference Example 17.
MS (ESI+) 473 (M⁺+1, 100%).

### Reference Example 75

tert-Butyl {(3R)-1-[3-(2-chlorobenzyl)-4-oxo-5-(2-oxo-2-phenylethyl)-4,5-dihydropyrazolo[1,5-d][1,2,4]triazin-2-yl]piperidin-3-yl}carbamate

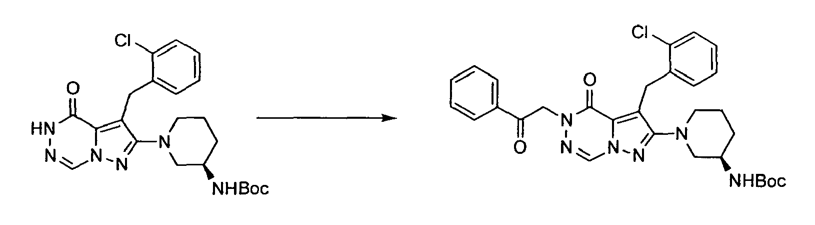

The title compound (131 mg) was synthesized by the same process as in Reference Example 12.
MS (ESI+) 577 (M⁺+1, 100%).

### Reference Example 76

tert-Butyl {(3R)-1-[3-(2-chlorobenzyl)-5-(isoquinolin-1-ylmethyl)-4-oxo-4,5-dihydropyrazolo[1,5-d][1,2,4]triazin-2-yl]piperidin-3-yl}carbamate

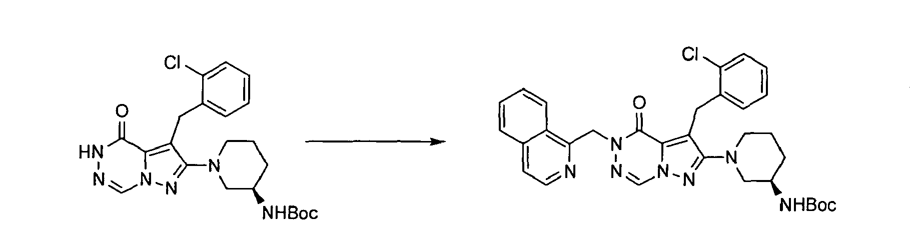

The title compound (122 mg) was synthesized by the same process as in Reference Example 12.
MS (ESI+) 600 (M⁺+1, 100%).

### Reference Example 77

tert-Butyl {(3R)-1-[3-(2-chlorobenzyl)-4-oxo-7-(trifluoromethyl)-4,5-dihydropyrazolo[1,5-d][1,2,4]triazin- 2-yl]piperidin-3-yl}carbamate

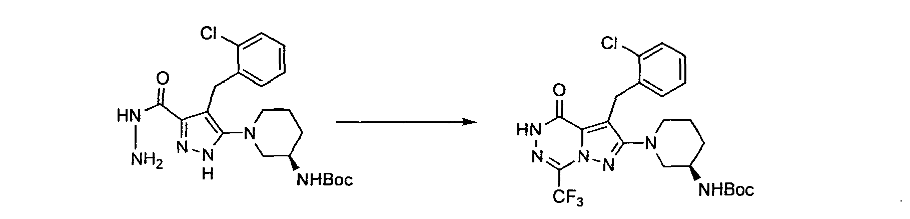

A solution of tert-butyl {(3R)-1-[4-(2-chlorobenzyl)-3-(hydrazinecarbonyl)-1H-pyrazol-5-yl]piperidin-3-yl}carbamate (80 mg) in a mixture of trifluoroacetic acid (0.5 ml) and polyphosphoric acid (2 ml) was stirred with heating at 140°C for 16 hours. The reaction solution was cooled to room temperature, adjusted to pH 10 with a 10% aqueous potassium carbonate solution and then extracted twice with chloroform (50 ml). The organic layer was dried over anhydrous sodium sulfate and filtered, and the filtrate was concentrated under reduced pressure. To a solution of the resulting residue (70 mg) in tetrahydrofuran (5 ml) were added a saturated aqueous sodium hydrogencarbonate solution (5 ml) and di-tert-butyl dicarbonate (81 mg), and the resulting mixture was stirred overnight at room temperature. After the tetrahydrofuran was distilled off under reduced pressure, a 10% aqueous potassium carbonate solution was added to the residue, followed by extraction with ethyl acetate (100 ml). The organic layer was dried over anhydrous sodium sulfate and filtered, and the filtrate was concentrated under reduced pressure. The residue was purified by a thin-layer chromatography (silica gel, hexane/ethyl acetate = 3/1) to obtain the title compound (6 mg).
¹H NMR(300MHz, CDCl₃) δ ppm 9.58 (bs, 1H), 7.43-7.39 (m, 1H), 7.20-7.17 (m, 2H), 6.96-6.93 (m, 1H), 4.62-4.41 (m, 3H), 3.68-3.66 (m, 1H), 3.31-3.29 (m, 1H), 3.15-2.98 (m, 3H), 1.65-1.48 (m, 4H), 1.44 (s, 9H).
MS (ESI+) 527 (M⁺+1, 55%).

### Reference Example 78

tert-Butyl {(3R)-1-[3-(2-chlorobenzyl)-4-oxo-5-(2-oxo-2-phenylethyl)-7-(trifluoromethyl)-4,5-dihydropyrazolo[1,5-d][1,2,4]triazin-2-yl]piperidin-3-yl}carbamate

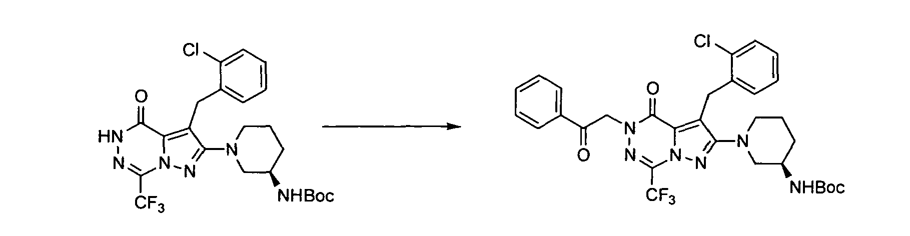

The title compound (21 mg) was synthesized by the same process as in Reference Example 12.
MS (ESI+) 645 (M⁺+1, 13%).

### Reference Example 79

Methyl 1-{[5-{(3R)-3-[(tert-butoxycarbonyl)-amino]piperidin-1-yl}-4-(2-chlorobenzyl)-1-(methoxymethyl)-1H-pyrazol-3-yl]carbonyl}pyrrolinate

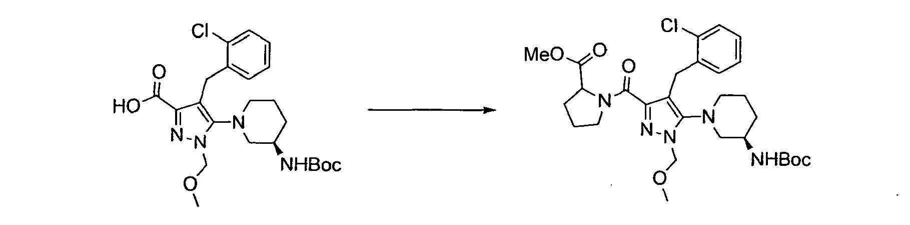

The title compound (810 mg) was synthesized by the same process as in Reference Example 57.
¹H NMR (400 MHz, CDCl₃) δ ppm 5.58-5.48 (m, 2H), 5.06-5.03 (m, 1H), 4.87-4.86 (m, 1H), 4.25-4.23 (m, 2H), 3.82-3.74 (m, 2H), 3.68-3.60 (m, 2H), 3.56-3.53 (m, 4H), 3.45-3.33 (m, 2H), 3.38 (s, 3H), 3.15-3.12 (m, 4H), 1.78 (s, 3H), 1.48 (s, 9H), 1.27-1.16 (m, 9H).
MS (ESI+) 580 (M⁺+1, 100%).

### Reference Example 80

1-{[5-{(3R)-3-[(tert-butoxycarbonyl)amino]-piperidin-1-yl}-4-(2-chlorobenzyl)-1-(methoxymethyl)-1H- pyrazolo-3-yl]carbonyl}proline

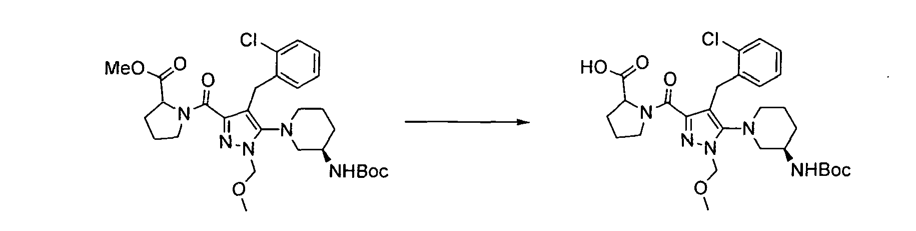

A 1N-aqueous sodium hydroxide solution (1.0 ml) was added to a solution of methyl 1-{[5-{(3R)-3-[(tert-butoxycarbonyl)amino]piperidin-1-yl}-4-(2-chlorobenzyl)-1-(methoxymethyl)-1H-pyrazolo-3-yl]carbonyl}prolinate (430 mg) in tetrahydrofuran (3.0 ml), and the resulting mixture was stirred at 50°C for 2 hours. The mixture was cooled to room temperature and a 5% aqueous sodium hydrogensulfate solution (50 ml) was added thereto, followed by extraction with ethyl acetate. The organic layer was concentrated under reduced pressure to obtain the title compound as a crude product (718 mg).
MS (ESI+) 576 (M⁺+1, 73%).

### Reference Example 81

tert-Butyl {(3R)-1-[3-[(3-acetylpyrrolidin-1-yl)carbonyl]-4-(2-chlorobenzyl)-1-methoxymethyl]-1H-pyrazolo-5-yl}piperidin-3-yl}carbamate

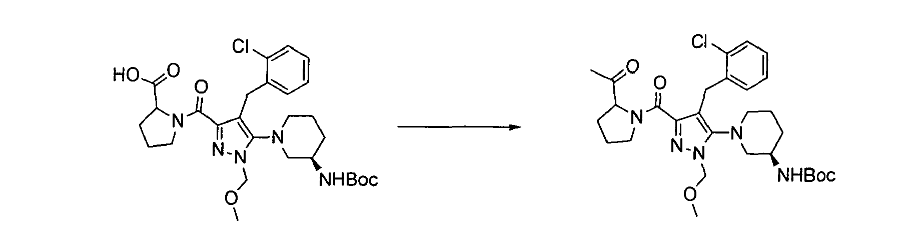

To a solution of 1-{[5-{(3R)-3-[(tert-butoxycarbonyl)amino]piperidin-1-yl}-4-(2-chlorobenzyl)-1-(methoxymethyl)-1H-pyrazolo-3-yl]carbonyl}proline (718 mg) in N,N-dimethylformamide (5.0 ml) were added 1-ethyl-3-(3-dimethylaminopropyl)carbodiimide hydrochloride (665 mg), 1-hydroxybenzotriazole monohydrate (512 mg) and N,O-dimethylhydroxylamine hydrochloride (243 mg), and the resulting mixture was stirred overnight at room temperature. Water was added to the reaction solution, followed by extraction with ethyl acetate, and the combined organic layer was concentrated under reduced pressure to obtain a crude product. The crude product was purified by a silica gel column chromatography (hexane/ethyl acetate = 2/1 to 1/2) to obtain a product (585 mg). MS (ESI+) 619 (M⁺+1, 100%).

Heated and dried cerium chloride (464 mg) and methylmagnesium bromide (4.4 mol) were added to a solution of the product in tetrahydrofuran (5.0 ml) at 0°C, and the resulting mixture was stirred with heating at 50°C for one and a half hours. After the reaction solution was cooled to room temperature, a saturated aqueous ammonium chloride solution was added thereto, followed by extraction with ethyl acetate. The combined organic layer was concentrated under reduced pressure to obtain a crude product. The crude product was purified by a silica gel column chromatography (chloroform/methanol = 10/1) to obtain the desired title compound (118 mg).
¹H NMR (300 MHz, DMSO-d₆) δ ppm 10.74 (s, 1H), 8.23 (bs, 3H), 7.50-7.35 (m, 1H), 7.30-7.05 (m, 2H), 7.05-6.80 (m, 1H), 4.35 (s, 2H), 3.83 (s, 3H), 3.80-3.60 (m, 1H), 3.20-2.90 (m, 2H), 2.75 (s, 3H), 2.65-2.40 (m, 2H), 2.00-1.80 (m, 1H), 1.70-1.20 (m; 3H).
MS (ESI+) 574 (M⁺+1, 100%).

### Reference Example 82

1-tert-Butyl 2-methyl-(2S, 4S)-4-hydroxypyrrolidine-1,2-dicarboxylate

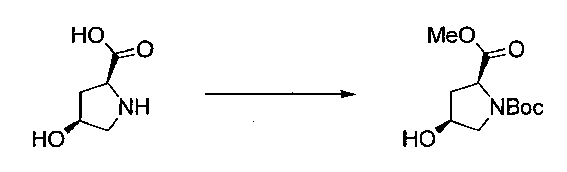

Thionyl chloride (612 ml) was added dropwise to a solution of L-hydroxyproline (1.0 g) in methanol (10 ml) at room temperature, and the resulting mixture was stirred overnight at room temperature. The reaction solution was concentrated under reduced pressure, followed by adding thereto chloroform (10 ml) and a saturated aqueous sodium hydrogencarbonate solution and then di-tert-butyl dicarbonate (2.5 g), and the resulting mixture was stirred at room temperature for 2 hours. The organic layer was concentrated under reduced pressure to obtain the title compound as a crude product (3.38 g).
¹H NMR (400 MHz, CDCl₃) δ ppm 5.58-5.48 (m, 2H), 5.06-5.03 (m, 1H), 4.87-4.86 (m, 1H), 4.25-4.23 (m, 2H), 3.82-3.74 (m, 2H), 3.68-3.60 (m, 2H), 3.56-3.53 (m, 4H), 3.45-3.33 (m, 2H), 3.38 (s, 3H), 3.15-3.12 (m, 4H), 1.78 (s, 3H), 1.48 (s, 9H), 1.27-1.16 (m, 9H).
MS (ESI+) 580 (M⁺+1, 100%).

### Reference Example 83

Methyl (4S)-4-fluoro-L-prolinate hydrochloride

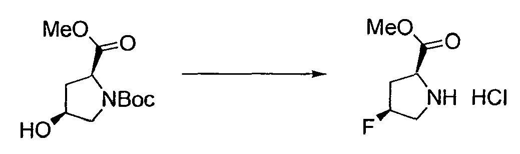

Diethylamine sulfur=trifluoride (1.4 ml) was added to a solution (5.0 ml) of 1-tert-butyl 2-methyl-(2S, 4S)-4-hydroxypyrrolidine-1,2-dicarboxylate (2.0 g) in dichloromethane at -78°C, and the resulting mixture was stirred at room temperature for 4 hours. A saturated aqueous ammonium chloride solution was added to the reaction solution, followed by extraction with ethyl acetate, and the combined organic layer was concentrated under reduced pressure to obtain a crude product. The crude product was purified by a silica gel column chromatography (chloroform/methanol = 10/1) to obtain a product (1.87 g). MS (ESI+) 248 (M⁺+1, 100%).

To a solution of this product in chloroform was added a 4N hydrochloric acid/1,4-dioxane solution, and the resulting mixture was stirred at room temperature for 3 hours. The reaction solution was concentrated under reduced pressure to obtain the title compound (552 mg).
MS (ESI+) 148 (M⁺+1, 100%).

### Reference Example 84

Methyl (4S)-1-{[5-{(3R)-3-[(tert-butoxycarbonyl)amino]piperidin-1-yl}-4-(2-chlorobenzyl)-1-(methoxymethyl)-1H-pyrazol-3-yl]carbonyl}-4-fluoro-L-prolinate

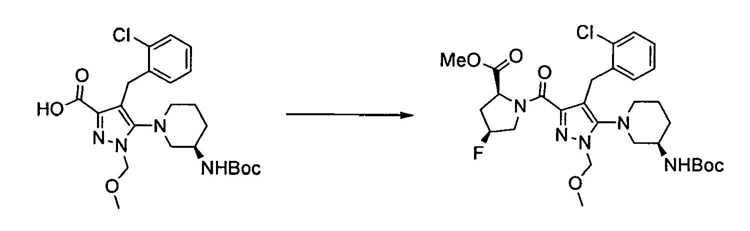

The title compound (810 mg) was synthesized by the same process as in Reference Example 57.
¹H NMR (400 MHz, CDCl₃) δ ppm 5.58-5.48 (m, 2H), 5.06-5.03 (m, 1H), 4.87-4.86 (m, 1H), 4.25-4.23 (m, 2H), 3.82-3.74 (m, 2H), 3.68-3.60 (m, 2H), 3.56-3.53 (m, 4H), 3.45-3.33 (m, 2H), 3.38 (s, 3H), 3.15-3.12 (m, 4H), 1.78 (s, 3H), 1.48 (s, 9H), 1.27-1.16 (m, 9H).
MS (ESI+) 580 (M⁺+1, 100%).

### Reference Example 85

(4S)-1-{[5-{(3R)-3-[(tert-butoxycarbonyl)amino]piperidin-1-yl}-4-(2-chlorobenzyl)-1-(methoxymethyl)-1H-pyrazol-3-yl]carbonyl}-4-fluoro-L-proline

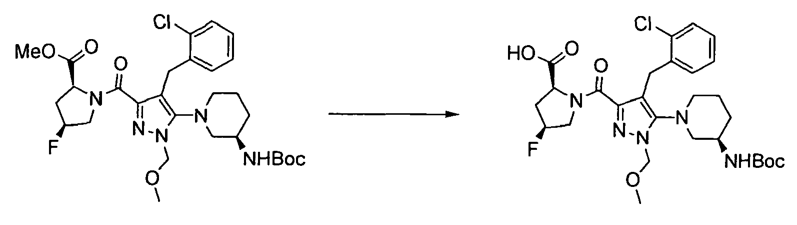

The title compound (810 mg) was synthesized by the same process as in Reference Example 80.
MS (ESI+) 580 (M⁺+1, 100%).

### Reference Example 86

tert-Butyl {(3R)-1-[4-(2-chlorobenzyl)-3-[((2S,4S)-4-fluoro-2-{[methoxy(methyl)amino]-carbonyl}pyrrolidin-1-yl)carbonyl]-1-(methoxymethyl)-1H-pyrazol-5-yl]piperidin-3-yl}carbamate

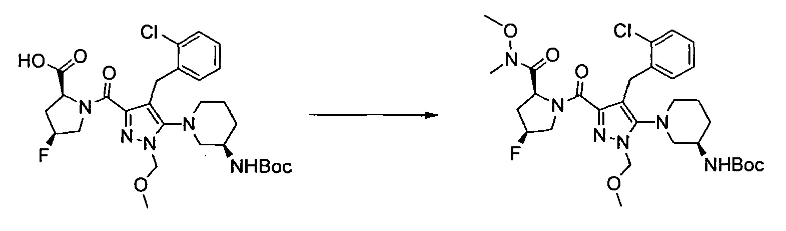

The title compound (440 mg) was obtained by the same process as in Reference Example 81.
MS (ESI+) 637 (M⁺+1, 30%).

### Reference Example 87

tert-Butyl {(3R)-1-[3-(dimethoxymethyl)-1-(methoxymethyl)-4-(2-methylbenzyl)-1H-pyrazolo-5-yl]piperidin-3-yl}carbamate

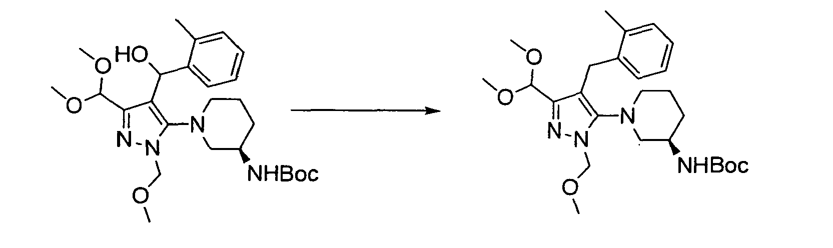

Trifluoroacetic acid (0.1 ml) and triethylsilane (0.32 ml) were added to a solution (1.0 ml) of tert-butyl {(3R)-1-[3-(dimethoxymethyl)-4-[hydroxy(2-methylphenyl)methyl]-1-(methoxymethyl)-1H-pyrazolo-5-yl]piperidin-3-yl}carbamate (50 mg) in dichloromethane, and the resulting mixture was stirred overnight at room temperature. A saturated aqueous sodium hydrogencarbonate solution was added to the reaction solution, followed by extraction with chloroform, and the combined organic layer was concentrated under reduced pressure to obtain a crude product. The crude product was purified by a silica gel column chromatography (hexane/ethyl acetate = 2/1) to obtain the title compound (31.5 mg).
MS (ESI+) 487 (M⁺+1, 100%).

### Reference Example 88

tert-Butyl {(3R)-1-[3-(dimethoxymethyl)-4-[hydroxy(2-methylphenyl)methyl]-1-(methoxymethyl)-1H-pyrazolo-5-yl]piperidin-3-yl}carbamate

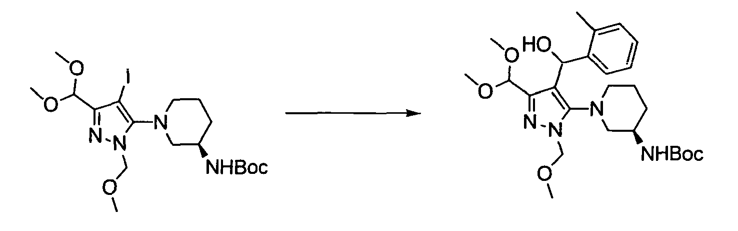

To a solution (40 ml) of tert-butyl {(3R)-1-[3-(dimethoxymethyl)-4-iodo-1-(methoxymethyl)-1H-pyrazol-5-yl]piperidin-3-yl}carbamate (2.7 g) in tetrahydrofuran was added 2M isopropylmagnesium chloride (27 ml) at -20°C, and stirred for 30 minutes. To the reaction solution was added dropwise o-tolualdehyde (7.8 g), and the resulting mixture was warmed to room temperature and then stirred for 3 hours. A saturated aqueous ammonium chloride solution was added to the reaction solution, followed by extraction with ethyl acetate, and the combined organic layer was concentrated under reduced pressure to obtain a crude product. The crude product was purified by a silica gel column chromatography (hexane/ethyl acetate = 2/1) to obtain the title compound (1.37 g).
MS (ESI+) 505 (M⁺+1, 100%).

### Reference Example 89

Ethyl N-{[5-{(3R)-3-[(tert-butoxycarbonyl)amino]piperidin-1-yl}-4-(2-chlorobenzyl)-1-(methoxymethyl)-1H- pyrazol-3-yl]carbonyl}-L-phenylalaninate

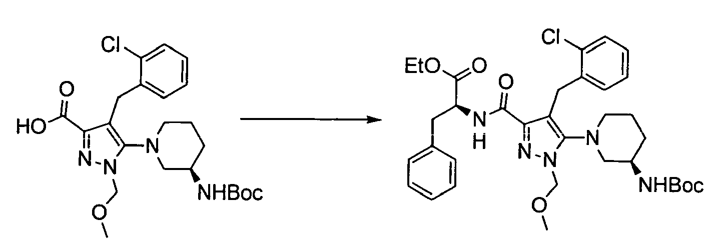

The title compound (630 mg) was synthesized by the same process as in Reference Example 57.
¹H NMR (400 MHz, CDCl₃) δ ppm 7.38-7.28 (m, 2H), 7.25-7.18 (m, 2H), 7.17-7.11 (m, 1H), 7.08-7.05 (m, 1H), 6.98-6.95 (m, 1H), 5.45 (d, J = 10.6 Hz, 1H), 5.24 (d, J = 10.6 Hz, 1H), 4.97-4.92 (m, 1H), 4.87-4.82 (m, 1H), 4.18 (q, J = 7.1 Hz, 2H), 4.07 (d, J = 13.8 Hz, 1H), 4.05 (d, J = 13.8 Hz, 1H), 3.73-3.68 (m, 1H), 3.27 (s, 3H), 3.22-3.15 (m ,1H), 3.08-3.01 (m, 2H), 2.93-2.84 (m, 2H), 2.73-2.65 (m, 1H), 1.60-1.48 (m, 4H), 1.43 (s, 9H), 1.24 (t J = 7.1 Hz, 3H).
MS (ESI+) 654 (M⁺+1, 100%).

### Reference Example 90

N-{[5-{(3R)-3-[(tert-butoxycarbonyl)amino]-piperidin-1-yl}-4-(2-chlorobenzyl)-1-(methoxymethyl)-1H-pyrazol-3-yl]carbonyl}-N-methoxy-N-methyl-L-phenylalaninamide

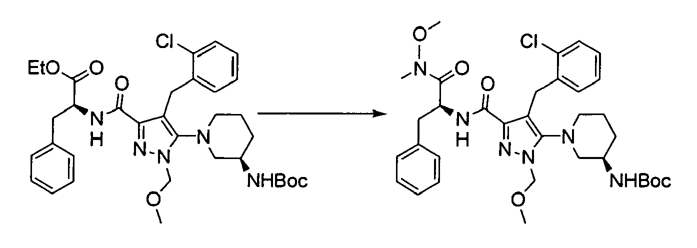

A 1N-aqueous sodium hydroxide solution (1.1 ml) was added to a solution of ethyl N-{[5-{(3R)-3-[(tert-butoxycarbonyl)amino]piperidin-1-yl}-4-(2-chlorobenzyl)-1-(methoxymethyl)-1H-pyrazol-3-yl]carbonyl}-L-phenylalaninate (430 mg) in tetrahydrofuran (20 ml), and the resulting mixture was stirred at 50°C for 3 hours. The reaction solution was cooled to room temperature, neutralized with 1N-hydrochloric acid, poured into water (120 ml) and then extracted with ethyl acetate (50 ml x 2). The organic layer was dried over anhydrous magnesium sulfate and filtered, and the filtrate was concentrated under reduced pressure. To a solution of N-{[5-{(3R)-3-[(tert-butoxycarbonyl)amino]piperidin-1-yl}-4-(2-chlorobenzyl)-1-(methoxymethyl)-1H-pyrazol-3-yl]carbonyl}-L-phenylalanine (718 mg) as a crude product in N,N-dimethylformamide (30 ml) were added 1-ethyl-3-(3-dimethylaminopropyl)carbodiimide hydrochloride (387 mg), 1-hydroxybenzotriazole monohydrate (272 mg) and N,O-dimethylhydroxylamine hydrochloride (197 mg), and the resulting mixture was stirred at room temperature for 18 hours. The reaction solution was poured into a saturated aqueous sodium chloride solution (200 ml) and extracted with ethyl acetate (60 ml x 2). The combined organic layer was dried over anhydrous magnesium sulfate and filtered, and the filtrate was concentrated under reduced pressure. The resulting residue was purified by a silica gel column chromatography (hexane/ethyl acetate = 2/1) to obtain the title compound (200 mg). MS (ESI+) 669 (M⁺+1, 38%).

### Reference Example 91

Ethyl 2-{(3R)-3-[(tert-butoxycarbonyl)amino]-piperidin-1-yl}-3-(2-chlorobenzyl)-5-(1-naphthylmethyl)-4-oxo-4,5-dihydropyrazolo[1,5-a]pyrazin-6-carboxylate

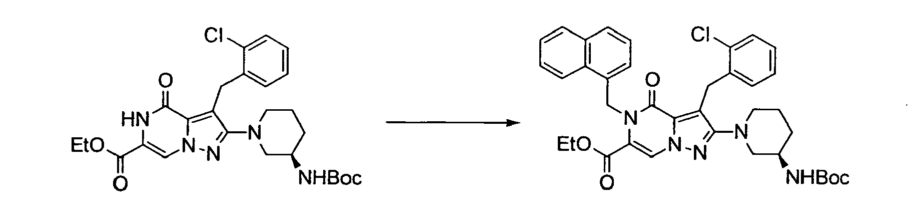

Sodium iodide (11.3 mg), potassium carbonate (156 mg) and 1-(chloromethyl)naphthalene (0.0847 ml) were added to a solution of ethyl 2-{(3R)-3-[(tert-butoxycarbonyl)amino]piperidin-1-yl}-3-(2-chlorobenzyl)-4-oxo-4,5-dihydropyrazolo[1,5-a]pyrazin-6-carboxylate (200 mg) in N,N-dimethylformamide (5.0 ml), and the resulting mixture was stirred overnight at room temperature. Water (50 ml) was added to the reaction mixture, followed by two runs of extraction with ethyl acetate (50 ml). The combined organic layer was dried over anhydrous magnesium sulfate and filtered, and the filtrate was concentrated under reduced pressure. The resulting residue was purified by a silica gel column chromatography (hexane/ethyl acetate = 5/1) to obtain the title compound (222 mg).
MS (ESI+) 670 (M⁺+1, 100%).

### In vitro DPP-IV inhibitory effect measurement test

Human serum containing DPP-IV enzyme was used in an experiment after being diluted with assay buffer (25mM Tris-HCl, 140mM NaCl, 10mM KCl, pH 7.9) (finally, diluted 10-fold). Each of solutions of each test compound having various concentrations was added to the diluted serum and the resulting mixture was incubated at room temperature. Then, a substrate (Glycyl-L-Proline 4-Methyl-Coumaryl-7-Amide, Peptide Laboratories Co., Ltd.) was added thereto to a final concentration of 100 µM and the reaction was carried out at room temperature. Acetic acid was added to the reaction mixture to a final concentration of 12.5% to terminate the reaction, and the intensity of fluorescence at an excitation wavelength of 360 nm and a measuring wavelength of 460 nm was measured by the use of a fluorescent plate reader. A compound concentration for 50% inhibition was calculated as an IC₅₀ value from enzyme inhibitory activity values obtained by adding each test compound to a plurality of concentrations.

**Table 1**

| Test Compound | Inhibitory activity against human DPP IV IC₅₀ (nM) |
|---|---|
| Example 1 | 20 |
| Example 3 | 30 |
| Example 4 | 37 |
| Example 5 | 82 |
| Example 7 | 0.7 |
| Example 8 | 7.0 |
| Example 9 | 71 |
| Example 10 | 2.8 |
| Example 11 | 25 |
| Example 12 | 24 |
| Example 13 | 6.1 |
| Example 14 | 61 |
| Example 16 | 2.5 |
| Example 17 | 1.6 |
| Example 18 | 45 |
| Example 19 | 15 |
| Example 20 | 5.0 |
| Example 21 | 13 |
| Example 26 | 28 |
| Example 32 | 0.9 |
| Example 33 | 12 |
| Example 34 | 2.8 |
| Example 35 | 2.3 |
| Example 38 | 9.6 |
| Example 41 | 2.1 |

### INDUSTRIAL APPLICABILITY

The present invention makes it possible to provide compounds having DPP-IV inhibitory activity and improved in safety, nontoxity and the like.

The present inventive compounds are useful for the suppression of postprandial hyperglycemia in a prediabetic, the treatment of non-insulin-dependent diabetes mellitus, the treatment of autoimmune diseases such as arthritis and articular rheumatism, the treatment of intestinal mucosa diseases, growth acceleration, the inhibition of rejection of a transplantate, the treatment of corpulence, the treatment of eating disorder, the treatment of HIV infection, the suppression of cancer metastasis, the treatment of prostatomegaly, the treatment of periodontitis, and the treatment of osteoporosis.

## Claims

1. A compound represented by the formula (I): 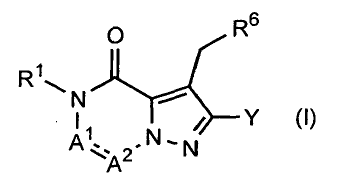 wherein R¹ is a hydrogen atom, an optionally substituted alkyl group, an optionally substituted cycloalkyl group, an optionally substituted aryl group, or an optionally substituted heteroaryl group;
the solid line and dotted line between A¹ and A² indicate a double bond (A¹=A²) or a single bond (A¹⁻A²);
A¹ is a nitrogen atom or a group represented by the formula C(R²) and A² is a group represented by the formula C(R⁴), in the case of the solid line and dotted line between A¹ and A² being a double bond (A¹=A²);
A¹ is a group represented by the formula C(R²)(R³) and A² is a group represented by the formula C(R⁴)(R⁵), in the case of the solid line and dotted line between A¹ and A² being a single bond (A¹-A²);
R¹ and R² may be taken together with the adjacent nitrogen atom and carbon atom to form an optionally substituted 4- to 7-membered ring in the case of the solid line and dotted line between A¹ and A² being a double bond (A¹=A²) and A¹ being a group represented by the formula C(R²);
R² is a hydrogen atom, a halogen atom, a cyano group, a formyl group, an optionally substituted alkyl group, an optionally substituted cycloalkyl group, an optionally substituted cycloalkyloxy group, an optionally substituted alkenyl group, an optionally substituted alkynyl group, an optionally substituted amino group, an optionally substituted carbamoyl group, a carboxyl group, an optionally substituted alkoxy group, an optionally substituted alkoxycarbonyl group, an optionally substituted cycloalkyloxycarbonyl group, a tetrahydrofuranyloxycarbonyl group, an optionally substituted aryl group, an optionally substituted aryloxy group, an optionally substituted aryloxycarbonyl group, an optionally substituted aralkyl group, an optionally substituted aralkyloxy group, an optionally substituted aroyl group, an optionally substituted arylthio group, an optionally substituted arylsulfinyl group, an optionally substituted arylsulfonyl group, an optionally substituted alkylthio group, an optionally substituted alkylsulfinyl group, an optionally substituted alkylsulfonyl group, an optionally substituted heteroaryl group, an optionally substituted heteroarylalkyl group, an optionally substituted heteroarylcarbonyl group, an optionally substituted heteroaryloxy group, an optionally substituted alkylcarbonyl group, an optionally substituted cycloalkylcarbonyl group, or an optionally substituted nitrogen-containing saturated heterocyclic group;
R³ is a hydrogen atom, a halogen atom, a cyano group, an optionally substituted alkyl group, an optionally substituted cycloalkyl group, an optionally substituted carbamoyl group, a carboxyl group, an optionally substituted alkoxy group, an optionally substituted alkoxycarbonyl group, an optionally substituted aryl group, an optionally substituted aryloxycarbonyl group, an optionally substituted aralkyl group, an optionally substituted aroyl group, an optionally substituted aryloxy group, an optionally substituted arylthio group, an optionally substituted arylsulfonyl group, an optionally substituted heteroaryl group, an optionally substituted heteroarylalkyl group, an optionally substituted heteroarylcarbonyl group, an optionally substituted heteroaryloxy group, or an optionally substituted alkylcarbonyl group;
R⁴ and R⁵ are independently a hydrogen atom, a halogen atom, a cyano group, an optionally substituted alkyl group, an optionally substituted cycloalkyl group, an optionally substituted carbamoyl group, a carboxyl group, an optionally substituted alkoxy group, an optionally substituted cycloalkyloxy group, an optionally substituted alkoxycarbonyl group, an optionally substituted amino group, an optionally substituted aryl group, an optionally substituted aryloxycarbonyl group, an optionally substituted aralkyl group, an optionally substituted aroyl group, an optionally substituted aryloxy group, an optionally substituted arylthio group, an optionally substituted arylsulfonyl group, an optionally substituted heteroaryl group, an optionally substituted heteroarylalkyl group, an optionally substituted heteroarylcarbonyl group, or an optionally substituted alkylcarbonyl group;
R⁶ is a hydrogen atom, an optionally substituted alkyl group, an optionally substituted cycloalkyl group, an optionally substituted aryl group, an optionally substituted vinyl group, an optionally substituted ethynyl group, an optionally substituted nitrogen-containing saturated heterocyclic group, or an optionally substituted heteroaryl group; and
-Y is any of groups represented by the formula (A), formula (B), formula (C) and formula (D) shown below: 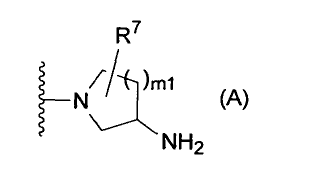 wherein m1 is 0, 1, 2 or 3, and R⁷ is absent or one or two R⁷s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁷s, when taken together, represent methylene or ethylene and may bind to one or more carbon atoms constituting the ring, to form a new ring; 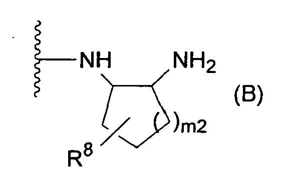 wherein m2 is 0, 1, 2 or 3, and R⁸ is absent or one or two R⁸s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁸s, when taken together, represent methylene or ethylene and may bind to one or more carbon atoms constituting the ring, to form a new ring; 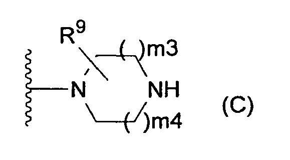 wherein m3 and m4 are independently 0 or 1, and R⁹ is absent or one or two R⁹s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁹s, when taken together, represent methylene or ethylene and may bind to one or more carbon atoms constituting the ring, to form a new ring; and 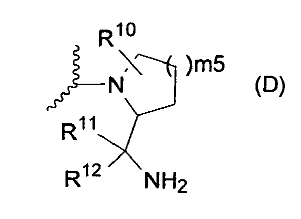 wherein m5 is 1, 2 or 3, R¹⁰ is absent or one or two R¹⁰S are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R¹⁰s, when taken together, represent methylene or ethylene and may bind to one or more carbon atoms constituting the ring, to form a new ring, and R¹¹ and R¹² are independently a hydrogen atom, methyl, ethyl, propyl or isopropyl, or R¹¹ and R¹², when taken together with the adjacent carbon atom, represent cyclopropyl, cyclobutyl or cyclopentyl, a prodrug of said compound, or a pharmaceutically acceptable salt of said compound or prodrug.

2. A compound according to claim 1, which is represented by the formula (II): 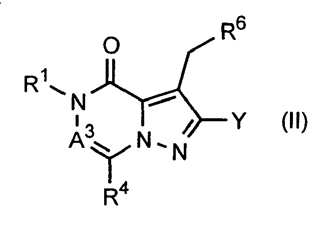 wherein A³ is a nitrogen atom or a group represented by the formula C(R²), and R¹, R², R⁴, R⁶ and Y are as defined in claim 1, a prodrug thereof or a pharmaceutically acceptable salt of the compound or prodrug.

3. A compound, a prodrug thereof or a pharmaceutically acceptable salt of the compound or prodrug according to either of claims 1 and 2, wherein R⁶ is the following formula (E), formula (F), formula (G) or formula (H): 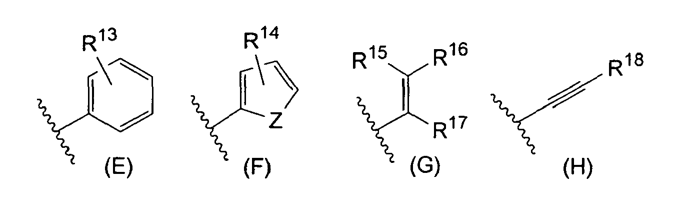 wherein Z is an oxygen atom, the formula S(O)p or N(R¹⁹);
R¹³ is absent or one or two R¹³s are present and are independently a halogen atom, a hydroxyl group, a formyl group, a carboxyl group, a cyano group, an alkylthio group, an alkylsulfinyl group, an alkylsulfonyl group, an alkyl group, a haloalkyl group, a cycloalkyl group, an alkoxy group, a haloalkoxy group, an optionally substituted amino group, an optionally substituted carbamoyl group, an alkoxycarbonyl group, an optionally substituted alkylcarbonyl group, a cycloalkylcarbonyl group, an optionally substituted phenyl group, an optionally substituted heteroaryl group or a nitrogen-containing saturated heterocyclic group, or two R¹³s, when taken together, represent a C₁₋₃ alkylenedioxy group;
R¹⁴ is absent or one or two R¹⁴s are present and are independently a halogen atom, a cyano group, an alkyl group, a haloalkyl group, a cycloalkyl group, an alkoxy group or a haloalkoxy group;
R¹⁵ is methyl, ethyl, a chlorine atom or a bromine atom;
R¹⁶ is a hydrogen atom, methyl, ethyl, a chlorine atom or a bromine atom;
R¹⁷ is a hydrogen atom, methyl or ethyl;
R¹⁸ is a hydrogen atom, methyl, ethyl, cyclopropyl or cyclobutyl;
p is 0, 1 or 2; and
R¹⁹ is a hydrogen atom or an alkyl group.

4. A compound, a prodrug thereof or a pharmaceutically acceptable salt of the compound or prodrug according to claim 3, wherein R⁶ is the formula (E) or the formula (H).

5. A compound, a prodrug thereof or a pharmaceutically acceptable salt of the compound or prodrug according to claim 4, wherein R⁶ is the formula (E) and one or two R¹³ are present and are independently a halogen atom, a cyano group, an alkylthio group, an alkylsulfonyl group, a C₁₋₃ alkylenedioxy group, an alkyl group, a haloalkyl group, a cycloalkyl group, an alkoxy group or a haloalkoxy group.

6. A compound, a prodrug thereof or a pharmaceutically acceptable salt of the compound or prodrug according to either of claims 1 and 2, wherein R⁶ is the following formula (J): 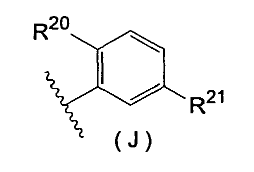 wherein R²⁰ is a halogen atom, a cyano group, an alkylthio group, an alkylsulfonyl group, an alkyl group, a haloalkyl group, a cycloalkyl group, an alkoxy group or a haloalkoxy group, and R²¹ is a hydrogen atom or a fluorine atom.

7. A compound, a prodrug thereof or a pharmaceutically acceptable salt of the compound or prodrug according to any one of claims 1 to 6, wherein Y is the formula (A) or (B).

8. A compound, a prodrug thereof or a pharmaceutically acceptable salt of the compound or prodrug according to any one of claims 1 to 6, wherein Y is the formula (A).

9. A compound, a prodrug thereof or a pharmaceutically acceptable salt of the compound or prodrug according to any one of claims 1 to 8, wherein R² is a hydrogen atom, a cyano group, an optionally substituted alkyl group, an optionally substituted cycloalkyl group, an optionally substituted alkenyl group, an optionally substituted carbamoyl group, a carboxyl group, an optionally substituted alkoxycarbonyl group, an optionally substituted cycloalkyloxycarbonyl group, a tetrahydrofuranyloxycarbonyl group, an optionally substituted aryl group, an optionally substituted aryloxycarbonyl group, an optionally substituted aralkyl group, an optionally substituted aroyl group, an optionally substituted heteroaryl group, an optionally substituted heteroarylalkyl group, an optionally substituted heteroarylcarbonyl group or an optionally substituted alkylcarbonyl group.

10. A compound, a prodrug thereof or a pharmaceutically acceptable salt of the compound or prodrug according to any one of claims 1 to 8, wherein R² is a hydrogen atom, a cyano group, an optionally substituted alkyl group, an optionally substituted carbamoyl group, a carboxyl group, an optionally substituted alkoxycarbonyl group, an optionally substituted cycloalkyloxycarbonyl group, a tetrahydrofuranyloxycarbonyl group, an optionally substituted aryl group, an optionally substituted aryloxycarbonyl group, an optionally substituted aralkyl group, an optionally substituted aroyl group, an optionally substituted heteroaryl group or an optionally substituted alkylcarbonyl group.

11. A compound, a prodrug thereof or a pharmaceutically acceptable salt of the compound or prodrug according to any one of claims 1 to 10, wherein R⁴ is a hydrogen atom, an optionally substituted alkyl group, a cyano group, an optionally substituted carbamoyl group, a carboxyl group, an optionally substituted alkoxy group, an optionally substituted cycloalkyloxy group, an optionally substituted alkoxycarbonyl group, an optionally substituted aryl group, an optionally substituted aryloxycarbonyl group, an optionally substituted aralkyl group, an optionally substituted aroyl group, an optionally substituted heteroaryl group, an optionally substituted heteroarylalkyl group, an optionally substituted heteroarylcarbonyl group or an optionally substituted alkylcarbonyl group.

12. A compound, a prodrug thereof or a pharmaceutically acceptable salt of the compound or prodrug according to any one of claims 1 to 10, wherein R⁴ is a hydrogen atom or an optionally substituted alkyl group.

13. A compound, a prodrug thereof or a pharmaceutically acceptable salt of the compound or prodrug according to any one of claims 1 to 10, wherein R⁴ is the formula: -C(R²²)(R²³)-A⁴-R²⁴ in which R²² and R²³ are independently a hydrogen atom, methyl, ethyl, propyl, isopropyl, methoxy or ethoxy, or R²² and R²³, when taken together with the adjacent carbon atom, represent cyclopropyl, cyclobutyl or cyclopentyl; A⁴ is a single bond, methylene or ethylene; and R²⁴ is a hydrogen atom, a cyano group, an alkyl group, a haloalkyl group, a cycloalkyl group, a tetrahydrofuranyl group, an optionally substituted carbamoyl group, a carboxyl group, an optionally substituted alkoxy group, an optionally substituted aryloxy group, an optionally substituted alkoxycarbonyl group, an optionally substituted aryl group, an optionally substituted aryloxycarbonyl group, an optionally substituted aroyl group, an optionally substituted heteroarylcarbonyl group or an optionally substituted alkylcarbonyl group.

14. A compound, a prodrug thereof or a pharmaceutically acceptable salt of the compound or prodrug according to any one of claims 1 to 13, wherein R¹ is a hydrogen atom, an optionally substituted alkyl group of 1 to 3 carbon atoms, or an optionally substituted aryl group, and the substituent(s) of the optionally substituted alkyl group is selected from fluorine atom, optionally substituted aroyl groups, carboxyl group, optionally substituted alkoxycarbonyl groups, optionally substituted aryl groups and optionally substituted aryloxy groups.

15. A compound, a prodrug thereof or a pharmaceutically acceptable salt of the compound or prodrug according to any one of claims 1 to 13, wherein R¹ is a hydrogen atom, methyl or a group represented by the formula: -Ra-Rb-Rc in which
Ra is an alkylene chain;
Rb is a single bond or a carbonyl group; and
Rc is an optionally substituted alkyl group, an optionally substituted alkoxy group, an optionally substituted aryl group, an optionally substituted heteroaryl group, an optionally substituted heteroaryloxy group or an optionally substituted aryloxy group.

16. A pharmaceutical composition comprising a compound, a prodrug thereof or a pharmaceutically acceptable salt of the compound or prodrug according to any one of claims 1 to 15 as an active ingredient.

17. A dipeptidyl peptidase IV inhibitor comprising a compound, a prodrug thereof or a pharmaceutically acceptable salt of the compound or prodrug according to any one of claims 1 to 15 as an active ingredient.

18. A pharmaceutical composition for the treatment of diabetes comprising a compound, a prodrug thereof or a pharmaceutically acceptable salt of the compound or prodrug according to any one of claims 1 to 15 as an active ingredient.

19. Use of a compound, a prodrug thereof or a pharmaceutically acceptable salt of the compound or prodrug according to any one of claims 1 to 15 in the manufacture of a dipeptidyl peptidase IV inhibitor.

20. Use of a compound, a prodrug thereof or a pharmaceutically acceptable salt of the compound or prodrug according to any one of claims 1 to 15 in the manufacture of a pharmaceutical composition for the treatment of diabetes.

21. A method for treating diabetes comprising administering an effective amount of a compound, a prodrug thereof or a pharmaceutically acceptable salt of the compound or prodrug according to any one of claims 1 to 15 to a patient who needs treatment.
